# DEMANDE DE BREVET EUROPEEN

(11) **EP 1 724 352 A2**
(43) Date de publication de la demande: **22.11.2006**
(21) Numéro de dépôt: 06004814.7
(22) Date de dépôt: 28.10.1999
(51) Int. Cl.: C12N 15/31, C12N 1/21, C07K 14/22, C07K 16/12, A61K 38/16

(54) **Acides nucléiques et polypeptides spécifiques des souches pathogènes du genre neisseria**

(30) Priorité: 30.10.1998 FR 9813693
(62) Demande divisionnaire de: 04016071.5
(71) Demandeur: Sanofi Pasteur, 69007 Lyon Cedex (FR); INSTITUT NATIONAL DE LA SANTE ET DE LA RECHERCHE MEDICALE (INSERM), 75013 Paris (FR)
(72) Inventeur: Aujame, Luc, 69210 Fleurieux sur l'Arbresle (FR); Bouchardon, Annabelle, 69500 Bron (FR); Renauld-Mongenie, Geneviève, 69630 Chaponost (FR); Rokbi, Bachra, 69003 Lyon (FR); Nassif, Xavier, 75015 Paris (FR); Tinsley, Colin, 75015 Paris (FR); Perrin, Agnès, 75015 Paris (FR)
(74) Mandataire: Colombet, Alain André

(57) **Abrégé**

Cette invention concerne des acides nucléiques codant pour les polypeptides spécifiques des souches pathogènes du genre *Neisseria,* les polypeptides correspondants, et leurs applications diagnostiques et thérapeutiques.

## Description

La présente invention a trait à des acides nucléiques codant pour des polypeptides spécifiques des souches pathogènes du genre *Neisseria,* notamment utiles pour prévenir ou traiter une infection par *Neisseria meningitidis.*

D'une manière générale, les méningites sont soit d'origine virale, soit d'origine bactérienne. Les bactéries principalement responsables sont : *Haemophilus influenzae* de type b, *Neisseria meningitidis* et *Streptococcus pneumoniae.* L'espèce *Neisseria meningitidis* est subdivisée en sérogroupes selon la nature des polysaccharides capsulaires. Bien qu'il existe une douzaine de sérogroupes, 90% des cas de méningites sont attribuables à trois sérogroupes : A, B et C.

Il existe des vaccins efficaces à base de polysaccharides capsulaires pour prévenir les méningites à *Neisseria meningitidis* sérogroupes A et C. Ces polysaccharides tels quels ne sont que peu ou pas immunogènes chez les enfants de moins de deux ans et n'induisent pas de mémoire immunitaire. Toutefois, ces inconvénients peuvent être surmontés en conjuguant ces polysaccharides à une protéine porteuse.

En revanche, le polysaccharide de *Neisseria meningitidis* sérogroupe B n'est pas ou peu immunogène chez l'homme, qu'il soit sous forme conjuguée ou non. Ainsi il apparaît hautement souhaitable de rechercher un vaccin à l'encontre des méningites causées par *Neisseria meningitidis,* notamment du sérogroupe B, autre qu'un vaccin à base de polysaccharide.

A cette fin, différentes protéines de la membrane externe de *N. meningitidis* ont déjà été proposées, telles que le récepteur membranaire de la transferrine humaine (WO 90/12591 et WO93/06861).

*Neisseria meningitidis* est génétiquement très proche de *Neisseria gonorrhoeae et Neisseria lactamica. N. gonorrhoeae* est surtout responsable d'infections localisées dans le tractus urogénital. Elle colonise la muqueuse génitale, puis traverse l'épithélium, envahit ensuite le sous-épithélium où elle se multiplie et est responsable d'une forte réaction inflammatoire. Par contre, *N*. *lactamica* est considérée comme une espèce non pathogène.

Des séquences présentes chez *N. gonorrhoeae* et *N. meningitidis,* absentes de *N. lactamica* ont été divulguées dans la demande de brevet WO 98/02 547, mais cette demandé de brevet antérieure ne localise pas et n'identifie pas les séquences codantes.

Les auteurs de la présente invention sont maintenant parvenus à identifier certains de ces gènes en recherchant dans le génome du méningoccoque, les cadres de lecture ouverts spécifiques des souches pathogènes du genre *Neisseria,* en mettant en oeuvre la stratégie suivante :

Certaines des séquences divulguées dans la demande de brevet WO98/02547 (désignées dans ladite demande antérieure SEQ ID n° 66, 67, 69, 70, 72 à 96, 98, et 99), ont été positionnées sur la séquence du génome de la souche *N. meningitidis* de sérogroupe B (ATCC 13090), disponible à partir de la banque Pathoseq® d'incyte Pharmaceuticals ainsi que sur la séquence du génome de la souche *Neisseria meningitidis* Z2491 (Sanger Center). Ceci a permis d'identifier, dans le génome de *N. meningitidis* qui compte 2,3 Méga bases, 19 contigs représentant 220 000 paires de bases.

Les auteurs de la présente invention ont ensuite analysé, pour chacun des 19 contigs, la présence de phases de lecture ouvertes (" open reading frames-ORF "), contenant au moins 100 acides aminés (et par définition, bornées par un codon d'initiation et un codon stop), à l'aide du logiciel Gene Jockey Il sequence processor® (Biosoft). Cette analyse a permis de sélectionner environ 400 ORFs candidates.

Les séquences de chacune de ces ORFs ont ensuite été analysées à l'aide du logiciel Codon Use® (Conrad Halling), qui tient compte de la fréquence d'utilisation des codons chez *N. meningitidis.* N'ont été retenues que les ORFs dont les séquences présentent une fréquence d'utilisation maximale de ces codons. A l'issue de cette analyse, 197 ORFs candidates ont été retenues.

Les ORFs sélectionnées par cette double analyse ont été soumises à une recherche d'homologies sur l'ensemble des banques disponibles à l'aide du programme BLASTX® depuis l'accès à la banque Pathoseq® d'Incyte Pharmaceuticals. Cette recherche d'homologies a permis d'exclure les ORFs codant *a priori* pour des protéines cytoplasmiques ou périplasmiques, en particulier des protéines du métabolisme. Les ORFs ont également été soumises à une analyse des motifs protéiques éventuels, à l'aide du programme Protean® de DNA Star (Lasergene software).

Les auteurs de la présente invention ont ensuite recherché si les ORFs retenues à l'issue de l'étape précédente (au nombre de 118) étaient effectivement absentes de *N. lactamica,* comme le prévoyait la demande de l'art antérieur WO 98/02547.

A cette fin, une amplification par PCR a été mise en oeuvre. Cette amplification s'est révélée inopérante pour 78 des 118 ORFs testées. Seules les ORFs pour lesquelles l'amplification chez *N. lactamica* était négative (séquences appelées "lactamica⁻") ont été retenues. Afin de vérifier que ces résultats négatifs n'étaient pas des " faux-négatifs ", les séquences lactamica⁻ retenues ont été soumises à un contrôle par dot blot. A l'issue de cette étape, seules 23 ORFs ont été confirmées *N. meningitidis*^{*+*}/*N. lactamica*^{*-*}*.*

Enfin, ces 23 ORFs ont été repositionnées dans leur ensemble sur le génome de *N. meningitidis* ATCC13090. Ceci a permis de mettre en évidence que trois ORFs préalablement éliminées sur la base de leur fonction protéique putative, apparaissaient localisées à proximité ou même encadrées par certaines des 23 ORFs *N. meningitidis*^{*+*}*l N. lactamica*^{*-*}*.* Ces trois ORFs (SEQ ID n° 29, 35 et 37) ont été réintroduites dans l'étude, et il s'est avéré qu'elles étaient elles aussi *N. meningitidis*^{*+*}*l N. lactamica*^{*-*}*.*

Les auteurs de la présente invention ont ensuite cherché à savoir si les ORFs identifiées à partir du génome de la souche *N. meningitidis* de sérogroupe B ATCC 13090 étaient aussi présentes dans les génomes de *N*. *meningitidis* Z2491 (Sanger Center) de sérogroupe A et de *N. gonorrhoeae* FA1090 (Advanced Center of Genome Technology, Oklahoma University).Puis ils ont comparé les séquences dérivées de ces différents génomes, par alignement multiple (Clustal, Infobiogen). Ceci a permis de redéfinir pour certaines des ORFs la position la plus probable des codons d'initiation et de fin de traduction. Les séquences des cadres de lecture ouverts issues de la souche ATCC13090 sont données dans les SEQ ID N° 1 - 51 (numéros impairs) et les séquences d'acides aminés qui en sont déduites, dans les SEQ ID N° 2 - 52 (numéros pairs).

La présente invention a donc pour objet un acide nucléique sous forme isolée codant pour un polypeptide, ou un fragment antigénique de celui-ci à l'exclusion des acides nucléiques divulgués dans les SEQ ID n° 70, 73, 74, 77, 80, 81, 87, 88, 89, 94, 95 et 98 de la demande WO 98/02547 (séquences annexées à la présente description et numérotées SEQ ID n° 70A, 73A, 74A, 77A, 80A, 81A, 87A, 88A, 89A, 94A, 95A, 98A pour les distinguer des séquences de l'invention) ; ledit polypeptide ayant une séquence d'acides aminés qui est identique ou homologue à une séquence choisie parmi celles du groupe II ; le groupe II étant constitué par les séquences montrées dans les SEQ ID n° 2 - 52 (numéros pairs) et la séquence SEQ ID n°53.

De manière préférentielle, ledit acide nucléique peut avoir une séquence nucléotidique choisie parmi celles du groupe 1, le groupe I étant constitué par les séquences montrées dans les SEQ ID n°1-51 (numéros impairs).

Le terme « acide nucléique » inclut et signifie également ORF, gène, polynucléotide, ADN et ARN. Le terme « acide nucléique sous forme isolée » signifie un acide nucléique séparé de l'environnement biologique dans lequel il se trouve dans des conditions naturelles. Par exemple, une molécule d'ADN existe dans des conditions naturelles lorsqu'elle se trouve intégrée dans un génome ou bien lorsqu'elle fait partie d'une banque de gènes. Dans ce cas là, elle ne peut être sous forme isolée. Par contre, la même molécule séparée du génome par clonage (par exemple suite à une amplification par PCR) doit être considérée comme étant sous forme isolée. De manière typique, une molécule d'ADN sous forme isolée, ne contient pas les régions codantes qui lui sont contiguës en 5' et 3' dans le génome dont elle est issue. Les acides nucléiques sous forme isolée peuvent être intégrés dans des vecteurs (par exemple plasmides or vecteurs viraux ou bactériens) sans pour cela se départir de leur caractéristique d'être séparés de leur environnement naturel.

Les auteurs de la présente invention ont plus particulièrement trouvé que les ORFs qui, lorsqu'elles étaient issues de la souche ATCC 13090, étaient caractérisées par les séquences telles montrées dans les SEQ ID n° 19, 27, 39, 45, 47 et 49, étaient spécifiques de *Neisseria meningitidis* dans la mesure où il n'a pas été possible de mettre en évidence des séquences identiques ou homologues dans le génome de *N. gonorrhoeae.* Ils ont aussi trouvé que l'ORF caractérisée par la séquence de souche telle que montrée dans le SEQ ID n° 39 était spécifique de *Neisseria meningitidis* de sérogroupe B.

L'invention a également pour objet un polypeptide sous forme isolée ou un fragment de celui-ci ; ledit polypeptide ayant une séquence d'acides aminés identique ou homologue à une séquence choisie parmi celles du groupe II.

Les acides aminés encadrés dans la séquence SEQ ID n° 8 correspondent à la séquence signal, l'acide aminé en gras représente le premier acide aminé de la forme mature. La séquence d'acides aminés de la forme protéique mature est représentée dans le SEQ ID n° 53.

Dans le cadre de la présente invention, les termes de "polypeptide " et " protéine " sont équivalents et interchangeables entre eux. Ils désignent n'importe quelle chaîne d'acides aminés, quelle qu'en soit sa longueur et ses modifications post-traductionnelles (par exemple, phosphorylation ou glycosylation).

Par " fragments antigéniques des polypeptides spécifiques des souches pathogènes du genre *Neisseria* ", on entend les polypeptides dérivés des polypeptides de l'invention tels que définis précédemment, par des délétions de parties desdits polypeptides sans destruction de l'antigénicité (par exemple, sans perte notable de l'activité antigénique) desdits polypeptides. L'antigénicité spécifique peut être déterminée en utilisant diverses méthodes connues de l'homme du métier, comme expliqué plus loin.

Ces fragments ont de préférence au moins 12 acides aminés de long, de préférence encore au moins 20 acides aminés de long, préférentiellement 50 acides aminés de long, de préférence encore 75 acides aminés de long, préférentiellement 100 acides aminés de long.

Ces fragments peuvent être utilisés pour révéler des épitopes qui peuvent être masqués chez les polypeptides parents. Ils sont également avantageux pour induire une réponse immune protectrice dépendante des lymphocytes T. Les délétions peuvent en effet permettre d'éliminer des régions immunodominantes de haute variabilité entre différentes souches.

De tels fragments peuvent être obtenus en utilisant des techniques standard connues de l'homme du métier (par exemple, Ausubel et al, Current Protocols in Molecular Biology, John Wiley & sons Inc, 1994), par exemple par PCR, RT-PCR, traitement par enzymes de restriction des molécules d'ADN clonées, ou par la méthode de Kunkel et al (Proc. Natl. Acad. Sci. USA (1985) 82:448).

Par " séquence d'acides aminés homologue ", on entend une séquence qui diffère d'une des séquences du groupe Il par substitution, délétion, et/ou insertion d'un ou plusieurs acides aminés, à des positions telles que ces modifications ne détruisent pas l'antigénicité spécifique du polypeptide en question.

Lesdites substitutions sont de préférence des substitutions conservatives, c'est-à-dire des substitutions d'acides aminés de même classe, tels que des substitutions d'acides aminés aux chaînes latérales non chargées (tels que l'asparagine, la glutamine, la serine, la thréonine, et la tyrosine), d'acides aminés aux chaînes latérales basiques (tels que la lysine, l'arginine, et l'histidine), d'acides aminés aux chaînes latérales acides (tels que l'acide aspartique et l'acide glutamique) ; d'acides aminés aux chaînes latérales apolaires (tels que la glycine, l'alanine, la valine, la leucine, l'isoleucine, la proline, la phénylalanine, la méthionine, le tryptophane, et la cystéine).

De manière avantageuse, une séquence d'acides aminés homologue possède un degré d'homologie (i.e., d'identité) d'au moins 75 % avec une des séquences du groupe Il ; de préférence ce degré d'homologie est d'au moins 80 %, de manière tout à fait préférée, d'au moins 90 %. Les séquences d'acides aminés homologues incluent en particulier les séquences qui sont substantiellement identiques à une des séquences du groupe II. Par séquence substantiellement identique " on signifie une séquence dont le degré d'homologie *(i.e.,* d'identité) avec une des séquences du groupe II est d'au moins 90%, avantageusement d'au moins 95%, de préférence d'au moins 97%, de manière tout à fait préférée d'au moins 99%. De plus, elle peut ne différer de la séquence de référence que par une majorité de substitutions conservatives.

Le degré d'homologie (aussi appelé degré d'identité) est généralement déterminé en utilisant un logiciel d'analyse de séquence (par exemple, Sequence Analysis Software Package of the Genetics Computer Group, University of Wisconsin Biotechnology Center, 1710 University Avenue, Madison, Wl 53705). Des séquences d'acides aminés similaires sont alignées pour obtenir le maximum de degré d'homologie *(i.e.* identité). A cette fin, il peut être nécessaire d'introduire de manière artificielle des espaces (" gaps ") dans la séquence. Une fois l'alignement optimal réalisé, le degré d'homologie (i.e. identité) est établi par enregistrement de toutes les positions pour lesquelles les acides aminés des deux séquences comparées sont identiques, par rapport au nombre total de positions.

Par " séquences nucléotidiques homologues ", on entend des séquences qui diffèrent des séquences du groupe I par substitution d'un nucléotide ou plusieurs nucléotides, ou par délétion et/ou insertion d'un ou plusieurs codons, à des positions telles que ces séquences codent (i) toujours pour des polypeptides ayant les séquences du groupe II, par effet de la dégénérescence du code génétique ; ou bien (ii) pour des polypeptides possédant des séquences homologues tels que définies précédemment.

De manière avantageuse, une séquence nucléotidique homologue possède un degré d'homologie d'au moins 60 % avec une des séquences du groupe 1 ; de préférence ce degré d'homologie est d'au moins 80 %, de manière tout à fait préférée d'au moins 90 %.

De manière typique, une séquence nucléotidique homologue s'hybride spécifiquement aux séquences complémentaires des séquences du groupe 1 dans des conditions stringentes. La température à laquelle le test d'hybridation est mis en oeuvre constitue un facteur important influençant la stringence. De manière conventionnelle, cette température, dite température d'hybridation (Th), est choisie de 5 à 40°C, de préférence de 20 à 25°C au dessous de la température à laquelle 50% des brins appariés se séparent (Tm). De manière générale, on considère que des conditions de forte stringence sont remplies lorsque Th est inférieure à Tm de 5 à 25°C approximativement, par exemple de 5 à 10°C ou le plus souvent, de 20 à 25°C approximativement. Une stringence modérée s'établit lorsque Th est inférieure à Tm de 30 à 40°C.

Pour les séquences comprenant plus de 30 bases, la température Tm est définie par la relation : Tm = 81,5 + 0,41 (%G+C) + 16,6Log(concentration en cations) - 0,63 (%formamide) - (600/nombre de bases). Ainsi, la force ionique a un impact majeur sur la valeur de Tm. La température Tm augmente de 16,6°C chaque fois que la concentration en cation monovalent augmente d'un facteur 10. L'addition de formamide dans le tampon d'hybridation fait par contre baisser la valeur de Tm. (Pour une référence complète voir Sambrook et al, Molecular Cloning, A laboratory manual, Cold Spring Harbor laboratory Press, 1989, pages 9.54-9.62).

De manière conventionnelle, les expériences d'hybridation sont conduites à une température de 60 à 68°C, par exemple à 65°C. A cette température, des conditions d'hybridation stringentes peuvent être par exemple mises en oeuvre en 6xSSC, avantageusement en 2xSSC ou 1×SSC, de préférence, en 0,5×SSC, 0,3×SSC ou 0,1×SSC (en absence de formamide). Une solution de 1×SSC contient 0,15 M de NaCl et 0,015 de citrate de sodium.

C'est pourquoi, en d'autres termes, l'invention a pour objet un polynucléotide sous forme isolée, qui est capable de s'hybrider dans des conditions stringentes avec une molécule d'ADN ayant une des séquences nucléotidiques telles que montrées dans les SEQ ID N° 1 - 51 (numéros impairs) ou leurs complémentaires.

Une classe particulière de séquences homologues est constituée par celles que l'on rencontre dans la nature en vertu du phénomène extrêmement fréquent de la variation allélique. Une espèce bactérienne par exemple, *N*. *meningitidis* ou *N. gonorrhoeae,* est constituée par une grande variété de souches qui diffèrent entre elles par des variations mineures, dites variations alléliques. Ainsi, un polypeptide qui est présent dans différentes souches et qui bien évidemment remplit dans chacune d'entre elles, la même fonction biologique, peut posséder une séquence d'acides aminés qui n'est pas identique d'une souche à l'autre. En d'autres termes, les séquences issues de la variation allélique sont purement des séquences équivalentes ou alternatives à celles du groupe II. La classe de séquences variantes alléliques de l'une des séquences du groupe II est constituée par les séquences du polypeptide tel qu'il se trouve dans une espèce pathogène du genre *Neisseria* (par exemple *N*. *meningitidis* ou de *N. gonorrhoeae)* autre que la souche de *N*. *meningitidis* ATCC 13090. La fonction biologique qui est associée aux séquences variantes allèliques est la même que celle qui est associée à la séquence de référence. Les différences (substitution, délétion ou addition d'un ou plusieurs acides aminés) qu'elles présentent entre elles (y compris la séquence de référence) n'altèrent pas la fonction biologique du polypeptide. Par " fonction biologique ", on entend la fonction exercée par le polypeptide dans les cellules qui le produisent de manière naturelle.

La variation allélique s'exprime également au niveau des séquences codantes. Un polynucléotide codant pour un polypeptide possédant une séquence qui est une variante allélique de l'une des séquences du groupe I peut être facilement cloné par amplification de l'ADN génomique des souches d'espèces pathogènes du genre *Neisseria ,* par exemple par PCR (réaction en chaîne par la polymérase), en utilisant des amorces oligonucléotidiques synthétiques capables de s'hybrider au niveau des extrémités 5' et 3' de la région codante. Les séquences de telles amorces peuvent facilement être établies par un homme du métier à partir des séquences nucléotidiques données dans les SEQ ID N° 1 - 51 (numéros impairs). Les amorces ont en général de 10 à 40 nucléotides, de préférence de 15 à 25 nucléotides.

C'est pourquoi, en d'autres termes, l'invention a pour objet une molécule d'ADN sous forme isolée qui peut être amplifiée et/ou clonée par PCR à partir du génome d'une souche de *Neisseria* pathogène en utilisant un couple d'amorces PCR 5' et 3' ; les séquences de ces amorces étant établies à partir d'une des séquences nucléotidiques telles que montrées dans les SEQ ID N° 1 - 51 (numéros impairs). Un exemple est donné pour chaque couple d'amorces dans l'exemple 1.1 ci-après.

La présente invention a plus particulièrement pour objet les variants alléliques ayant les séquences nucléotidiques SEQ ID n° 54 à 76 (numéros pairs) et les produits codés par ces séquences nucléotidiques, ayant les séquences d'acides aminés SEQ ID n° 55 à 77 (numéros impairs).

Les polypeptides de l'invention peuvent être fusionnés à d'autres polypeptides, par exemple par traduction d'un gène hybride. Des vecteurs pour l'expression de polypeptides de fusion sont disponibles dans le commerce, comme les vecteurs pMal-c2 ou pMal-p2 de New England Biolabs, dans lesquels la protéine à laquelle peuvent être fusionnés les polypeptides de l'invention est une protéine de liaison au maltose, ou le système glutathion-S-transférase de Pharmacia, ou le système His-Tag de Novagen. De tels systèmes sont en particulier utiles pour une purification des polypeptides de l'invention. Les polypeptides de l'invention peuvent être fusionnés à des polypeptides présentant une activité d'adjuvant, comme par exemple la sous-unité B de la toxine cholérique ou de la toxine thermosensible de *E. coli.*

Les acides nucléiques de la présente invention peuvent être utilisés (i) dans un procédé de production des polypeptides codés par lesdits acides nucléiques dans un système hôte recombinant, (ii) pour la construction de vecteurs vaccinaux tels que des poxvirus, destinés à être utilisés dans des méthodes et des compositions de prévention et/ou de traitement d'une infection par les souches pathogènes de *Neisseria,* en particulier par *Neisseria meningitidis,* (iii) comme agent vaccinal sous une forme nue ou en association avec un véhicule favorisant le transfert aux cellules cible et, (iv) dans la construction de souches de *Neisseria* atténuées qui peuvent surexprimer un acide nucléique de l'invention ou l'exprimer sous une forme non toxique, mutée.

La présente invention fournit également (i) une cassette d'expression contenant un polynucléotide de l'invention placée sous le contrôle d'éléments permettant son expression, en particulier sous le contrôle d'un promoteur approprié ; (ii) un vecteur d'expression contenant ladite cassette d'expression ; (iii) une cellule hôte (procaryote ou eucaryote) transformée avec une cassette d'expression et/ou un vecteur d'expression tels que définis ci-dessus, ainsi que (iv) une méthode pour obtenir un polypeptide codé par ledit polynucléotide de l'invention, comprenant la culture de ladite cellule transformée, dans des conditions permettant l'expression du polynuctéotide de l'invention, et la récupération du polypeptide de la culture cellulaire.

Parmi les hôtes eucaryotes pouvant être utilisés, on peut notamment citer les cellules de levure (par exemple, *Saccharomyces cerevisiae* ou *Pichia Pastoris),* les cellules de mammifères (par exemple, COS1, NIH3T3, ou JEG3), des cellules d'arthropodes (par exemple, *Spodoptera frugiperda* (SF9)), et des cellules végétales. Parmi les hôtes procaryotes pouvant être utilisés, on peut notamment citer *E. coli.*

Le choix de la cassette d'expression dépend du système hôte choisi ainsi que des caractéristiques désirées pour le polypeptide exprimé. De manière générale, les cassettes d'expression incluent un promoteur qui est fonctionnel dans le système hôte sélectionné et qui peut être constitutif ou inductible ; un site de liaison au ribosome ; un codon d'initiation (ATG) ; si nécessaire une région codant pour un peptide signal ; une séquence nucléotidique de l'invention ; un codon stop ; éventuellement une région 3' terminale (terminateur de traduction et/ou de transcription). Le cadre de lecture ouverte ("open reading frame ORF") constitué par la séquence nucléotidique de l'invention, seule ou associée à la région codant pour le peptide signal, est placé sous le contrôle du promoteur de telle sorte que la traduction et la transcription aient lieu dans le système hôte. Les promoteurs et les régions codant pour les peptides signal sont connus de l'homme du métier. Parmi ceux-ci, on peut notamment citer le promoteur de *Salmonella typhimurium* inductible par l'arabinose (promoteur araB) et qui est fonctionnel dans les bactéries Gram⁻ tel que *E*. *coli* (US 5,028,530 et Cagnon et al., Protein Engineering (1991) 4(7) : 843), le promoteur du gène du bactériophage T7 codant pour l'ARN polymérase, (US 4,952,496) ; le peptide signal OspA et RlpB (Takase et al, J. Bact. (1987) 169:5692).

Le polypeptide exprimé peut être recueilli sous une forme pratiquement purifiée à partir de l'extrait cellulaire ou du surnageant après centrifugation de la culture de cellules recombinantes. Le polypeptide recombinant peut être notamment purifié par des méthodes de purification par affinité à l'aide d'anticorps ou par toute autre méthode connue de l'homme du métier, telle que par fusion génétique avec un petit domaine de liaison.

Les acides nucléiques de l'invention peuvent également être utiles dans le domaine de la vaccination, soit en utilisant un hôte viral ou bactérien comme véhicule de libération de l'ADN, soit en administrant l'acide nucléique d'intérêt sous une forme libre.

La présente invention a également pour objet (i) un vecteur vaccinal contenant un acide nucléique de l'invention, placé sous le contrôle d'éléments permettant son expression ; (ii) une composition pharmaceutique contenant une quantité thérapeutiquement ou prophylactiquement efficace dudit vecteur vaccinal ; (iii) une méthode pour induire une réponse immune contre *Neisseria* chez un vertébré, en particulier un mammifère, de préférence un humain, ladite méthode comprenant l'administration audit vertébré d'une quantité immunologiquement efficace dudit vecteur vaccinal pour provoquer une réponse immune, en particulier une réponse protectrice ou thérapeutique à *Neisseria meningitidis ;* (iv) une méthode pour prévenir et/ou traiter une infection par les souches pathogènes de *Neisseria,* en particulier par *Neisseria meningitidis,* qui comprend l'administration d'une quantité prophylactique ou thérapeutique dudit vecteur vaccinai de l'invention à un individu nécessitant un tel traitement.

En association avec les polypeptides de l'invention, le vecteur vaccinal tel que défini ci-dessus peut également comprendre des séquences nucléotidiques dont l'expression permet la stimulation de la réponse immune, telles que les séquences codant pour des cytokines.

Ledit vecteur vaccinal de l'invention peut être administré par n'importe quelle voie conventionnelle dans le domaine de la vaccination, en particulier par la voie parentérale (par exemple, sous-cutanée, intra-dermique, intra-musculaire, intraveineuse ou intra-péritonéale). Le dosage dépend de nombreux paramètres connus de l'homme du métier, tels que le vecteur lui-même, la voie d'administration, le poids, l'âge ou le sexe de l'animal ou de l'homme à vacciner.

La présente invention a également pour objet (i) une composition pharmaceutique contenant une quantité thérapeutiquement ou prophylactiquement efficace d'un polynucléotide de l'invention ; (ii) une méthode pour induire une réponse immune contre les souches pathogènes de *Neisseria,* en particulier par *Neisseria meningitidis* chez un vertébré par administration audit vertébré d'une quantité immunologiquement efficace dudit polynucléotide pour provoquer une réponse immune, en particulier une réponse immune protectrice envers les souches pathogènes de *Neisseria,* en particulier par *Neisseria meningitidis ;* et (iii) une méthode de prévention et de traitement à une infection par les souches pathogènes de *Neisseria,* en particulier par *Neisseria meningitidis,* par administration d'une quantité thérapeutique ou prophylactique dudit polynucléotide à un individu nécessitant un tel traitement.

Les polynucléotides de l'invention (ADN ou ARN) peuvent être administrés en tant que tels à un vertébré. Lorsqu'une molécule d'ADN de l'invention est utilisée, elle peut être sous la forme d'un plasmide incapable de se répliquer dans une cellule de vertébré et incapable d'intégrer le génome dudit vertébré. Ladite molécule d'ADN est typiquement placée sous le contrôle d'un promoteur adapté pour l'expression dans une cellule de vertébré. Ledit polynucléotide utilisé comme vaccin peut être formulé selon diverses méthodes connues de l'homme du métier. Ledit polynucléotide peut en particulier être utilisé sous une forme nue, exempt de tout véhicule favorisant le transfert à la cellule cible, tels que des liposomes anioniques, des lipides cationiques, des microparticules, par exemple des microparticules d'or, des agents de précipitation, par exemple du phosphate de calcium, ou tout autre agent facilitant la transfection. Dans ce cas, le polynucléotide peut être simplement dilué dans une solution physiologiquement acceptable, telle qu'une solution stérile ou une solution stérile tampon, en présence ou en l'absence d'un véhicule. Lorsqu'il est présent, ce véhicule peut être de préférence isotonique, hypotonique, ou faiblement hypertonique, et a une force ionique relativement faible. Il peut par exemple s'agir d'une solution de saccharose (par exemple une solution contenant 20 % de saccharose).

De manière alternative, un polynucléotide de l'invention peut être associé à des agents qui facilitent la transfection. Il peut être, entre autres , (i) associé à un agent chimique qui modifie la perméabilité cellulaire tel que la bupivacaïne (WO 94/16737) ; (ii) encapsulé dans des liposomes, éventuellement en présence de substances supplémentaires facilitant la transfection (WO 93/18759, WO 93/19768, WO 94/25608 et WO 95/2397, WO 93/18759 et WO 93/19768) ; ou (iii) associé à des lipides cationiques ou des microparticules de silice, d'or ou de tungstène.

Lorsque les polynucléotides de l'invention recouvrent des microparticules, celles-ci peuvent être injectées par voie intradermique ou intraépidermique par la technique du canon à gènes, "gene gun" (US 4,945,050, U.S. No. 5,015,580 et WO 94/24263).

La quantité d'ADN à utiliser comme vaccin dépend notamment de la force du promoteur utilisé dans la construction de l'ADN, de l'immunogénicité du produit exprimé, de l'individu auquel cet ADN est administré, du mode d'administration et du type de formulation. De manière générale, une quantité thérapeutiquement ou prophylactiquement efficace variant d'environ 1 µg à environ 1 mg, de préférence d'environ 10 µg à environ 800 µg et, de manière préférentielle d'environ 25 µg à environ 250 µg, peut être administrée à des adultes humains.

Le polynucléotide de l'invention peut être administré par toute voie d'administration conventionnelle telle que notamment par voie parentérale. Le choix de la voie d'administration dépend en particulier de la formulation choisie. Un polynucléotide formulé en association avec la bupivacaïne est avantageusement administré au muscle. Lorsque des liposomes neutres ou anioniques ou un lipide cationique tel que DOTMA (chlorure de N-[1-(2,3-dioléyloxy)propyl]-N,N,N-triméthylammonium) ou DC-Chol (3 beta-(N-(N',N'-dimethyl aminomethane)-carbamoyl) cholesterol), sont utilisés, la formulation peut être avantageusement injectée par voie intraveineuse, intramusculaire, intradermique ou sous-cutanée. Un polynucléotide sous une forme nue peut de manière avantageuse être administré par voie intramusculaire, intradermique ou sous-cutanée.

Les séquences nucléotidiques de l'invention permettent la construction de sondes et d'amorces nucléotidiques spécifiques utiles en diagnostic. Lesdites sondes ou amorces sont des acides nucléiques ayant des séquences identiques ou homologues à des portions des séquences du groupe I ou à leurs séquences complémentaires.

De manière préférentielle, lesdites sondes contiennent d'environ 5 à environ 100, de préférence d'environ 10 à environ 80 nucléotides. Elles peuvent contenir des bases modifiées, les résidus sucre et phosphate pouvant être également modifiés ou substitués. Les sondes de l'invention peuvent être utilisées dans des tests de diagnostic, pour capturer ou détecter des polynucléotides spécifiques des souches pathogènes de *Neisseria.* De telles sondes de capture peuvent être de manière conventionnelle immobilisées sur un support solide directement ou indirectement, par liaison covalente ou par adsorption passive. Une sonde de détection peut être marquée notamment par un isotope radioactif, une enzyme telle que la peroxidase ou la phosphatase alcaline, ou des enzymes capables d'hydrolyser un substrat chromogène, fluorogène ou luminescent, ou encore par des composés eux-mêmes homogènes, fluorogènes ou luminescents, des analogues nucléotidiques ; ou la biotine.

Une amorce contient généralement d'environ 10 à environ 40 nucléotides, et peut être utilisée pour initier une polymérisation enzymatique de l'ADN dans un processus d'amplification (par exemple la PCR), dans un processus d'élongation ou dans une méthode de transcription inverse. Une amorce de l'invention peut être notamment une amorce telle que décrite dans l'exemple II.1 ci-après.

La présente invention a également pour objet :
(i) un réactif contenant une sonde de l'invention pour la détection et/ou l'identification de la présence des souches pathogènes de *Neisseria* dans un échantillon biologique ;
(ii) un procédé de détection et/ou d'identification de la présence des souches pathogènes de *Neisseria* dans un échantillon biologique ; ladite méthode comprenant les étapes consistant à a) extraire l'ADN ou l'ARN d'un échantillon biologique et le dénaturer ; b) exposer ledit ADN ou ledit ARN à une sonde de l'invention, dans des conditions d'hybridation stringentes, de manière à détecter l'hybridation ; et
(iii) une méthode de détection et/ou d'identification des souches pathogènes de *Neisseria* dans un échantillon biologique dans lequel l'ADN est extrait d'un échantillon biologique et est mis en présence d'au moins une et de préférence de deux amorces de l'invention et est amplifié par exemple par PCR.

Comme mentionné précédemment, les polypeptides produits par l'expression des séquences ORF identifiées sont utiles comme agents vaccinaux. L'antigénicité spécifique des polypeptides homologues des polypeptides de séquences du groupe Il peut être évaluée en testant la réactivité croisée avec un antisérum dirigé contre les polypeptides de séquences du groupe II. Un antisérum hyper-immun monospécifique peut être produit contre un polypeptide de séquence du groupe II purifié ou un polypeptide de fusion, par exemple un produit d'expression des systèmes MBP, GST, ou His-tag.

L'antigénicité spécifique peut être déterminée en utilisant diverses méthodes connues de l'homme du métier, en particulier les techniques de Western-Blot, Dot-Blot, et ELISA, décrites ci-dessous.

Dans la technique de Western-Blot, la préparation protéique à tester est soumise à une électrophorèse sur gel SDS-PAGE. Après transfert sur une membrane de nitrocellulose, le matériel est incubé avec un antisérum hyper-immmun monospécifique obtenu après avoir immunisé un animal avec le matériel référent ; c'est-à-dire dans le cas présent, avec un polypeptide ayant une séquence d'acides aminés du groupe Il. Cet antisérum est au préalable dilué dans un domaine de dilution allant d'environ 1:50 à 1:5000, de préférence d'environ 1:100 à 1:500. L'antigénicité spécifique est révélée lorsqu'une bande correspondant au produit présente une réactivité à l'une des dilutions ci-dessus.

Dans le test ELISA, on utilise de préférence une préparation protéique purifiée, bien qu'un extrait cellulaire entier puisse être également utilisé. Environ 100 µl d'une préparation à environ 10 µg/ml sont distribués dans les puits d'une plaqué. La plaque est incubée pendant deux heures à 37°C puis une nuit à 4°C. La plaque est ensuite lavée avec une solution saline de tampon phosphate (PBS) comprenant 0,05 % de Tween 20. Les puits sont saturés avec 250 µl de PBS contenant 1 % d'albumine de sérum bovin (BSA) pour empêcher la liaison non spécifique d'anticorps. Après une heure d'incubation à 37°C, la plaque est lavée avec le tampon PBS/Tween. L'antisérum est dilué en série dans du tampon PBS/Tween contenant 0,5 % BSA. 100 µl de cette dilution sont ajoutés par puits. La plaque est incubée pendant 90 minutes à 37°C, lavée et évaluée selon les procédures standard. Par exemple, lorsque des anticorps spécifiques sont produits chez des lapins, un conjugué peroxydase de chèvre anti-lapin est ajouté au puits. L'incubation est réalisée pendant 90 minutes à 37°C et la plaque est ensuite lavée. La réaction est mesurée par colorimétrie (une réaction est positive lorsque la valeur de densité optique est de 1 si la dilution est d'au moins 1:50, de préférence d'au moins 1:500).

Dans le test Dot-Blot, on utilise de préférence une protéine purifiée, étant entendu qu'il est également possible d'utiliser un extrait cellulaire entier. Une solution protéique à environ 100 µg/ml est diluée deux fois en série dans un tampon Tris-HCl 50mM, pH : 7,5. 100 µl de chaque dilution sont appliqués à une membrane de nitrocellulose (appareil BioRad). Le tampon est enlevé en appliquant du vide au système. Les puits sont lavés par addition de 50 mM de tampon Tris-HCl (pH : 7,5) et la membrane est séchée à l'air. La membrane est ensuite saturée dans un tampon bloquant (50 mM Tris-HCl (pH : 7,5) 0,15M NaCl, 10 g/l de lait écrémé) et incubée avec une dilution d'antisérum allant d'environ 1:50 à 1:5000, de préférence à environ 1:500. La réaction est révélée conformément aux procédures standard. Par exemple, lorsque des anticorps spécifiques sont produits chez des lapins, un conjugué peroxydase de chèvre anti-lapin est ajouté aux puits. L'incubation est réalisée pendant 90 minutes à 37°C. La réaction est développée avec le substrat approprié et mesurée, par exemple par colorimétrie, par l'apparition d'une tache colorée (une réaction est positive lorsqu'une tache colorée apparaît en association avec une dilution d'au moins 1:50, de préférence d'au moins 1:500).

La présente invention a également pour objet (i) une composition pharmaceutique contenant -une quantité thérapeutiquement ou prophylactiquement efficace d'un polypeptide de l'invention ; (ii) une méthode pour induire une réponse immune contre les souches pathogènes de *Neisseria* chez un vertébré, par administration audit vertébré d'une quantité immunogéniquement efficace d'un polypeptide de l'invention pour provoquer une réponse immune, en particulier une réponse immune protectrice contre les souches pathogènes de *Neisseria ;* et (iii) une méthode de prévention et/ou de traitement d'une infection par les souches pathogènes de *Neisseria,* par administration d'une quantité thérapeutique ou prophylactique d'un polypeptide de l'invention à un individu nécessitant un tel traitement.

Les compositions immunogènes de l'invention peuvent être administrées par n'importe quelle voie conventionnelle dans le domaine de la vaccination, en particulier par la voie parentérale (par exemple, sous-cutanée, intra-dermique, intra-musculaire, intraveineuse ou intra-péritonéale). Le choix de la voie d'administration dépend d'un certain nombre de paramètres tel que l'adjuvant associé au polypeptide.

Une composition de l'invention contient au moins un polypeptide tel que défini précédemment. Il peut également contenir au moins un antigène supplémentaire de *Neisseria meningitidis* et/ou *Neisseria gonorrhoeae.*

Les polypeptides de l'invention peuvent être formulés avec des liposomes, de préférence des liposomes neutres ou anioniques, des microsphères, des ISCOMS, des particules "virus-like" pour faciliter le transfert du polypeptide et/ou augmenter la réponse immune.

L'administration peut être réalisée par une dose unique ou par des doses répétées si nécessaire à des intervalles qui peuvent être déterminés par l'homme du métier.

Par exemple, une dose initiale peut être suivie de trois doses de rappel à des intervalles d'une ou plusieurs semaines ou d'un ou plusieurs mois. La dose appropriée dépend de nombreux paramètres incluant l'individu traité (adulte ou enfant), l'antigène vaccinal particulier, la voie d'administration et la fréquence d'administration, la présence ou l'absence ou encore le type d'adjuvant, et l'effet désiré (par exemple, protection et/ou traitement) et peut être déterminée par l'homme du métier. Si la voie d'administration est parentérale, la dose est préférentiellement inférieure à 1 mg, de préférence d'environ 100 µg. Les polypeptides et polynucléotides de l'invention utilisés en tant qu'agents vaccinaux, peuvent être utilisés de manière séquentielle, dans un procédé d'immunisation en plusieurs étapes. Par exemple, un vertébré peut être initialement sensibilisé avec un vecteur vaccinai de l'invention, tel qu'un poxvirus, par exemple par la voie parentérale, puis être stimulé deux fois avec le polypeptide codé par le vecteur vaccinal.

Un polypeptide de l'invention peut également être utile comme agent de diagnostic pour détecter la présence d'anticorps anti*-Neisseria meningitidis* et/ou anti-Neisseria *gonorrhoeae* dans un échantillon biologique tel qu'un échantillon de sang.

La présente invention a également pour objet des anticorps monospécifiques dirigés contre les polypeptides de l'invention.

Par "anticorps monospécifique" on entend un anticorps capable de réagir de manière spécifique avec un polypeptide de *Neisseria* de l'invention. De tels anticorps peuvent être polyclonaux ou monoclonaux, et peuvent être des anticorps recombinants par exemple chimériques (par exemple, constitués par une région variable d'origine murine associée à une région constante d'origine humaine), humanisés et/ou à chaîne unique. Lesdits anticorps peuvent être également sous la forme de fragments d'immunoglobuline par exemple de fragments F(ab)'2 ou Fab. Les anticorps de l'invention peuvent être de n'importe quel isotype par exemple IgA ou IgG, les anticorps polyclonaux pouvant être d'un isotype unique ou pouvant contenir un mélange de plusieurs isotypes.

Les anticorps de l'invention dirigés contre les polypeptides de l'invention, peuvent être produits et identifiés en utilisant des méthodes immunologiques standard, par exemple l'analyse de Western-Blot, un essai Dot-Blot, un test ELISA (Coligan et al, Current Protocols in lmmunology (1994) John Wiley & Sons, Inc., New York, NY). Lesdits anticorps peuvent être utilisés dans des procédés de diagnostic pour détecter la présence d'un antigène de *Neisseria meningitidis* dans un échantillon tel que notamment un échantillon biologique (par exemple du sang).

Les anticorps de l'invention peuvent également être utilisés dans des procédés de chromatographie par affinité pour purifier un polypeptide de l'invention. Enfin, de tels anticorps peuvent également être utilisés dans des méthodes d'immunisation passive prophylactique ou thérapeutique.

La présente invention a également pour objet une méthode de diagnostic pour la détection de la présence de souches pathogènes de *Neisseria* dans un échantillon biologique comprenant la mise en contact dudit échantillon biologique avec un anticorps, ou un polypeptide de l'invention, de telle sorte qu'un complexe immun soit formé, et la détection dudit complexe indicatrice de souches pathogènes de *Neisseria* dans l'organisme dont provient l'échantillon. L'homme du métier comprend que le complexe immun est formé entre un composant de l'échantillon et l'anticorps ou le polypeptide de l'invention, toute substance non liée pouvant être éliminée préalablement à la détection du complexe.

Ainsi, un réactif de type polypeptide peut être utilisé pour la détection de la présence d'anticorps anti*-Neisseria meningitidis* et/ou *Neisseria gonorrhoeae* dans un échantillon, tandis qu'un anticorps de l'invention peut être utilisé comme réactif pour tester la présence de polypeptide de *Neisseria meningitidis* et/ou *Neisseria gonorrhoeae* dans un échantillon.

Pour une utilisation dans des applications de diagnostic, le réactif (par exemple l'anticorps ou le polypeptide de l'invention) peut être à l'état libre ou immobilisé sur un support solide, par des moyens directs ou indirects.

Les moyens directs incluent l'adsorption passive ou la liaison covalente entre le support et le réactif.

Par moyen indirect, on entend qu'une substance qui interagit avec ledit réactif est fixée au support solide. Par exemple, si un réactif de type polypeptide est utilisé, un anticorps qui se lie à celui-ci peut servir de substance anti-réactif, étant entendu que celle-ci se lie à un anticorps qui n'est pas impliqué dans la reconnaissance des anticorps dans les échantillons biologiques.

Parmi les moyens indirects pouvant être utilisés, on peut également citer le système ligand récepteur, une molécule telle qu'une vitamine pouvant être greffée sur le réactif de type polypeptide et le récepteur correspondant pouvant être immobilisé sur la phase solide. Ceci est illustré par le système biotine-streptavidine. On peut également ajouter au réactif une queue peptidique par génie chimique ou génie génétique, et immobiliser le produit greffé ou fusionné par adsorption passive ou liaison covalente avec la queue peptidique.

La présente invention a également pour objet un procédé de purification, à partir d'un échantillon biologique, d'un polypeptide de *Neisseria* de l'invention, par chromatographie d'affinité avec un anticorps monospécifique de l'invention. Ledit anticorps est de préférence d'isotype IgG.

Selon un exemple de réalisation, un échantillon biologique, de préférence dans une solution tampon, est appliqué à un matériel chromatographique, de préférence équilibré avec le tampon utilisé pour diluer l'échantillon biologique, de telle sorte que le polypeptide de l'invention (c'est-à-dire l'antigène) puisse adsorber sur le matériel. Les composants non liés sont lavés et l'antigène est ensuite élué avec un tampon d'élution approprié, tel qu'un tampon Glycine ou un tampon contenant un agent chaotropique, par exemple la guanidine HCl, ou une forte concentration en sel (par exemple, 3 M MgCl₂). Les fractions éluées sont recueillies et la présence d'antigène est détectée par exemple par mesure de l'absorbance à 280 nm.

La présente invention a également pour objet (i) une composition pharmaceutique contenant une quantité thérapeutiquement ou prophylactiquement efficace d'un anticorps monospécifique de l'invention ; et (ii) une méthode de prévention et/ou de traitement d'une infection par les souches pathogènes de *Neisseria,* par administration d'une quantité thérapeutique ou prophylactique d'un anticorps monospécifique de l'invention à un individu nécessitant un tel traitement.

A cette fin, l'anticorps monospécifique de l'invention est de préférence d'isotype IgG, et de préférence fixe le complément. Ledit anticorps monospécifique selon l'invention peut être administré seul ou en mélange avec au moins un autre anticorps monospécifique, spécifique d'un polypeptide de *Neisseria meningitidis* et/ou *Neisseria gonorrhoeae* différent, selon l'invention. La quantité d'anticorps peut être déterminée facilement par l'homme du métier. Par exemple, une administration quotidienne d'environ 100 à 1000 mg d'anticorps sur une semaine ou trois doses quotidiennes d'environ 100 à 1000 mg d'anticorps sur deux ou trois jours, peut être une posologie efficace.

L'efficacité thérapeutique ou prophylactique peut être évaluée en utilisant des méthodes standard connues de l'homme du métier, par exemple en mesurant l'induction d'une réponse immune ou l'induction d'une immunité protectrice et/ou thérapeutique (chez des souris ou des rats nouveau-nés), par évaluation de la charge bactérienne dans le liquide céphalo-rachidien). La protection peut être déterminée en comparant le degré d'infection de *Neisseria* à un groupe contrôle. La protection est mise en évidence lorsque l'infection est réduite par comparaison au groupe contrôle. Une telle évaluation peut être faite avec les polynucléotides, les vecteurs vaccinaux, les polypeptides ainsi que les anticorps selon l'invention. L'efficacité thérapeutique ou prophylactique d'un produit selon l'invention (polynucléotide ou polypeptide) peut être aussi évaluée en test de bactéricidie tel que décrit par Danve et al., Vaccine (1993) 11 (12):1214, à l'encontre de la souche méningoccoque d'origine du polynucléotide ou polypeptide utilisé. Dans le domaine des vaccins méningoccoques, le test de bactéricidie est en effet reconnu comme étant le test de référence à partir duquel on peut valablement prédire l'intérêt vaccinal d'un produit. En bref, on administre un produit selon l'invention à des animaux tels que le lapin afin d'obtenir un antisérum à l'encontre de ce produit. Puis cet antisérum est testé pour sa capacité de lyse. Le titre de bactéricidie d'un antisérum représente l'inverse de la dilution de cet antisérum pour lequel 50 % de la charge en méningoccoques est lysée. On considère que l'antisérum est bactéricide lorsque le titre est supérieur à 4 vis à vis de la souche méningoccoque d'origine du polynucléotide ou polypeptide utilisé. Dans ce cas-là, il est démontré que le produit à l'encontre duquel l'antisérum a été généré, est potentiellement intéressant d'un point de vue pharmaceutique.

Les exemples suivant illustrent l'invention sans en limiter la portée.

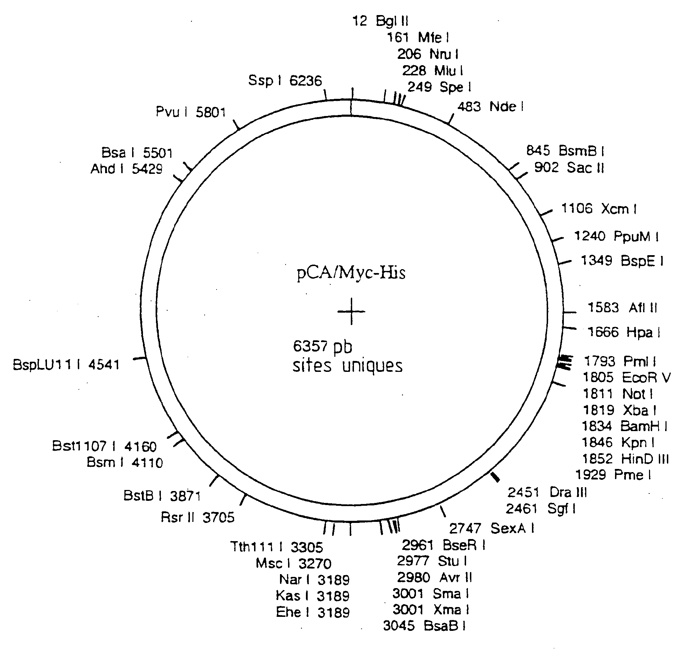

### Légende de la figure

La figure en annexe représente le vecteur pCAMyc-His utilisé comme vecteur de clonage.

### Détails de la stratégie d'identification des ORF :

Afin de sélectionner les séquences ORF spécifiques des souches pathogènes du genre *Neisseria,* une amplification par PCR est menée sur les séquences des 118 ORFs retenues après analyse par le logiciel Gene Jocke ® , Codon Use® , et la recherche d'homologies. Seules les séquences pour lesquelles l'amplification chez *N. lactamica* est négative (séquences appelées "lactamica⁻ ") sont retenues. Afin de vérifier que ces résultats négatifs ne sont pas des " faux-négatifs ", les séquences lactamica⁻ retenues sont soumises à un dot blot.

### A - Amplification par PCR :

### A.1. Extraction des ADN génomiques :

Les ADN génomiques de l'ensemble des souches de Neisseria utilisées dans cette étude ont été préparés selon un protocole identique. Les souches de *N. meningitidis, N. lactamica, N. flava, N. subflava* et *N. mucosa* ont été cultivées sur boite de milieu MHA (Muller Hinton Agar, Difco), les *N*. *gonorrhoeae* ont été cultivées sur boite de milieu MHA supplémenté par 10% de sang cuit de cheval (Biomérieux) et 1% d'Isovitalex (Biomérieux). La culture se fait à 37°C pendant 18 h sous une atmosphère contenant 10% CO2 une nuit à 37°C. Puis les cellules sont récoltées, lavées dans du tampon phosphate PBS (pH 7.2) et l'ADN est extrait selon le protocole D du Kit " Rapid Prep genomic DNA isolation kit for cells and tissue " (Pharmacia Biotech).

Les ADN génomiques ont alors été contrôlés sur gel d'agarose pour leur intégrité et par réaction PCR pour leur pureté.

### A.2. Réaction de PCR pour cribler les ORFs absentes dans N. lactamica 2314 :

Une amplification PCR a été réalisée sur les ADN génomiques de la souche *N. meningitidis* ATCC 13090 et *N. lactamica* 2314 (ATCC 23970) selon le protocole suivant :

La réaction de PCR a été réalisée sur un volume de 50 µl avec 10 ng d'ADN génomique, 250 µM de chacun des dNTPs, 300 nM de chacune des amorces, Tampon Taq DNA polymerase 1X, et 2 u de Taq DNA Polymerase (Appligène).

Les cycles d'amplification sont :

| | | |
|---|---|---|
| 97°C | 45 secondes | 25 cycles |
| 56°C | 1 minute | 25 cycles |
| 72°C | 2.30 minutes | 25 cycles |

Pour chacune des ORFs analysées, des contrôles positifs et négatifs de la réaction PCR ont été réalisés. A ce stade, seules les ORFs *N*. *meningitidis* + et *N. lactamica -* sont retenues.

### B - Sélection des ORFs N. meningitidis⁺ N. lactamica⁻ par dot blot sur ADN génomique :

L'absence d'un produit d'amplification par PCR d'une ORF avec de l'ADN génomique de *N. lactamica* 2314 comme matrice, ne certifie pas l'absence de cette ORF dans le génome de *N. lactamica* 2314. En effet, une certaine variabilité dans la région où devraient s'hybrider les oligonucléotides peut être responsable de l'absence de produit amplifié pour une ORF donnée.

Dans ce contexte, une vérification supplémentaire est mise en oeuvre par dot blot sur ADN génomique en utilisant comme sonde les produits d'amplification génomique sur la souche de *N. meningitidis* correspondant à chacune des phases de lecture identifiées. Les filtres du dot blot contiennent de l'ADN génomique des souches suivantes : 2 souches de *N. lactamica* 8064, 2314, une souche de *N*. *flava* ATCC 30008, une souche de *N*. *mucosa* ATCC 9297, 3 souches de *N. meningitidis* de sérogroupe B ATCC13090, M982, B16B6, une souche de *N. meningitidis* de sérogroupe A Z2491, une souche de *N*. *meningitidis* de sérogroupe C (souche Z4182), 2 souches de *N. gonorrhoeae* MS11 et FA1090. Cette analyse en dot blot permet de valider l'absence de l'ORF dans *N. lactamica* 2314 et 8064 et elle est aussi indicatrice du degré de variabilité d'une ORF au sein des souches de *Neisseria.*

La technique de dot blot utilisée est la suivante. Environ 50ng d'ADN génomique dénaturé 5 min à 100°C des différentes souches de *Neisseria* sont déposés sous vide sur une membrane de nitrocellulose Hybond N+ (Amersham) placée entre les machoires d'un appareil à dot blot (Bio-Rad). Puis l'ADN est fixé sur les membranes 5 min à un rayonnement UV 315nm.

Les membranes sont incubées dans un tampon de pré-hybridation (contenant de l'ADN de sperme de saumon dénaturé). Elles sont ensuite hybridées avec une sonde correspondant au produit d'amplification de l'ORF d'intérêt, marquée selon un protocole de marquage froid, comme le système 'DIG DNA labelling and détection kit' (Boehringer Mannheim).

L'ORF qui ne s'hybride pas sur l'ADN génomique de *N. lactamica* 2314 et 8064 est retenue définitivement comme candidat vaccinal potentiel.

### Exemple I : Clonage

### 1. Amplification par PCR

Chacune des ORFs a été amplifiée par PCR à partir de l'ADN génomique de *N. meningitidis* sérogroupe B (souche ATCC 13090), selon un protocole standard.

Deux oligonucléotides, amorces du côté N-terminal et du côté C-Terminal ont été définis pour chacune des séquences ORF de l'invention.

L'amorce du côté N-Terminal comprend un site de restriction enzymatique pour le clonage, une séquence Kozak CCACC pour l'initiation de la traduction (M. Kozak,J. Mol. Biol. **196 :** 947-950), l'ATG de l'ORF potentielle et environ 17 bases spécifiques de la partie 5' de l'ORF.

L'amorce du côté C-Terminal a été définie de manière à ce que l'ORF clonée soit en fusion dans sa partie 3' avec une répétition de 8 histidines et un codon stop présents dans le vecteur derrière le site multiple de clonage, d'où l'insertion d'une base "A " pour garder la bonne phase de lecture après le clonage et la disparition du codon stop de l'ORF. L'amorce du côté C-Terminal comprend ainsi un site de restriction enzymatique pour le clonage, une base "A", puis environ 20 bases spécifiques de la partie 3' du gène à partir du codon précédent le codon stop.

Après recherche des sites de restriction absents chez chacune des ORFs à l'aide du sous-programme MapDraw de DNASTAR (Lasergene Software), les sites de restriction Xbal en 5' et BgIII en 3' sont utilisés pour l'ORF SEQ ID n°19. Pour l'ORF SEQ ID n°41, les sites Spel en 5' et BgIII en 3' sont utilisés. Les sites de restriction Xbal en 5' et BamHI en 3' sont utilisés pour cloner les ORFs restantes.

Le mélange de PCR comprend pour un volume final de 100 µl, 10-50 ng ADN génomique, les amorces N-Terminale et C-Terminale à 200 nM chacun, les dNTPs à 250 µM chacun, le tampon PCR 1X (Composition du tampon PCR 10X : 200 mM Tris-HCl (pH8.8), 20 mM MgSO4, 100 mM KCI, 100 mM (NH4)2SO4, 1% TritonX-100, 1 mg/ml de sérumalbumine de boeuf exempte de nucléase) et 2,5 U de Polymerase.

L'amplification se fait comme suit :

| Etape | Température (°C) | Temps (min.) | Nombre de cycles |
|---|---|---|---|
| Dénaturation | 97 | 0.45 | 25 |
| Hybridation | cf tableau | 1 | 25 |
| Elongation | 72 | 1/kb ADN | 25 |

Les amorces utilisées et les conditions de PCR présentées dans le tableau ci-dessous, dans lequel "variant allélique N. g" signifie qu'un variant allélique est présent chez *Neisseria gonorrhoeae* et "variant allélique N. m A" signifie qu'un variant allélique est présent chez *Neisseria meningitidis* de sérogroupe A.

| **N° ORF (réf. interne)** | **SEQ ID n°** | **Amorce 5'** | **Amorce 3'** | **Polymérase** | **T° Hybridation** |
|---|---|---|---|---|---|
| 22 | 1-2 variant allélique N.g : 54, 55 | GCT CTA GAC CAC CAT GTC TGA AGA AAA ATT GAA AAT GAG (SEQ ID n° 78) | CGG GAT CCA GAA ATG GCT GGA TTC GCT ATC AG (SEQID n° 79) | Tfu (Appligene) | 56°C |
| 41 | 3-4 | GCT CTA GAC CAC CAT GAA ACA CTT ACT CAT CG (SEQ ID n° 80) | CGG GAT CCA ATA CGT AGG ACT TGG GTC (SEQ ID n° 81) | Tfu (Appligene) | 43°C |
| 42-43 | 5-6 variant allélique N.g. : 56, 57 | GCT CTA GAC CAC CAT GAA AAA ATC CCT TTT CGT TC (SEQ ID n° 82) | CGG GAT CCA TTG CGG ATA AAC ATA TTC CGC C (SEQ ID n° 83) | Tfu (Appligene) | 56°C |
| 47 | 7-8 variant allélique N.g. : 58, 59 | GCT CTA GAC CAC CAT GCG AAC GAC CCC AAC CTT C (SEQ ID n° 84) | CGG GAT CCA GAA CCG GTA GCC TAC GCC GAC (SEQ ID n° 85) | Tfit (Appligene) | 56°C |
| 55 | 9-10 variant allélique N.g. : 60, 61 | GCT CTA GAC CAC CAT GAA CAC ACG CAT CAT CGT TTC (SEQ ID n° 86) | CGG GAT CCA GCA ACG GCC TGC CGC TTT AAG (SEQ ID n° 87) | Pfu Turbo (Stratagene) | 56°C |
| 68 | 11-12 | GCT CTA GAC CAC CAT GCT GAC GTT TAT CGG ACT G (SEQ ID n° 88) | CGG GAT CCA CGG CAG AGG CAC GAT TCC (SEQ ID n° 89) | Tfu (Appligene) | 56°C |
| 71 | 13-14 | GCT CTA GAC CAC CAT GGG CAT CCA TCT CGA CTT C (SEQ ID n° 90) | CGG GAT CCA CAA AAG TTC CAG AAA ATC TAA CTC (SEQID n° 91) | Tfu (Appligene) | 56°C |
| 72 | 15-16 variant allélique N.mA : 62,63 | GCT CTA GAC CAC CAT GAA TAG ACC CAA GCA ACC (SEQ ID n° 92) | CGG GAT CCA TGC CGC TTG GGG GAG GC (SEQ ID n° 93) | Pfu Turbo (Stratagene) | 56°C |
| 73 | 17-18 variant allélique N.g : 64, 65 | GCT CTA GAC CAC CAT GAT GAA TGT CGA GGC AGA G (SEQ ID n° 94) | CGG GAT CCA CAG TTT GCC CGA CAT AC (SEQ ID n° 95) | Pfu Turbo (Stratagene) | 56°C |
| 74 | 19-20 | GCT CTA GAC CAC CAT GAA ATT TTT TCC TGC TCC (SEQ ID n° 96) | GAA GAT CTA GAA ACT GTA ATT CAA GTT GAA G (SEQ ID n° 97) | Pfu Turbo (Stratagene) | 56°C |
| 98 | 21-22 | GCT CTA GAC CAC CAT GAT TGA ATT TGT CCG AGC (SEQ ID n° 98) | CGG GAT CCA ACC CTG CGA CGA GTT GCG (SEQ ID n° 99) | Pfu Turbo (Stratagene) | 56°C |
| 116 | 23-24 variant allélique N.g : 66, 67 | GCT CTA GAC CAC CAT GCA ATA CAG CAC ACT GGC (SEQ ID n° 100) | CGG GAT CCA GTC CTT TTT CGC ACC TTG AAG (SEQ ID n° 101) | Pfu Turbo (Stratagene) | 56°C |
| 122 | 25-26 | GCT CTA GAC CAC CAT GGA GCA GTC GGG CAA ATT C (SEQ ID n° 102) | CGG GAT CCA AGC TGT TTG GCG ATT TCG GTG (SEQ ID n° 103) | Pfu Turbo (Stratagene) | 56°C |
| 125 | 27-28 | GCT CTA GAC CAC CAT GCA AAA CGG CGG GGG AAA G C (SEQ ID n° 104) | CGG GAT CCA GTG CCT GCG CAG CTT GGA ATC (SEQ ID n° 105) | Pfu turbo (Stratagene) | 56° C |
| 128 | 29-30 variant allélique N.m A : 68, 69 | GCT CTA GAC CAC CAT GAC ATT GCT CAA TCT AAT GAT AAT G (SEQ ID n° 106) | CGG GAT CCA TTC CGC AAA TAC CTG TTT CCA ACC (SEQ ID n° 107) | Tfu (Appligene) | 56° C |
| 152 | 31-32 variant allélique N.g:70,71 | GCT CTA GAC CAC CAT GAA ACA ATC CGC CCG (SEQ ID n° 108) | CGG GAT CCA TAC TTG GGC GCA ACA TGA C (SEQ ID n° 109) | Pfu turbo (Stratagene) | 56° C |
| 153 | 33-34 | GCT CTA GAC CAC CAT GAA TGT TTA CGG TTT CCC (SEQ ID n° 110) | CGG GAT CCA TTT TTT AGA CGT ATT TTT AGT CG (SEQ ID n° 111) | Tfu (Appligene) | 56° C |
| 155 | 35-36 | GCT CTA GAC CAC CAT GAT GAG TCA ACA CTC TGC C (SEQ ID n° 112) | CGG GAT CCA TCC AGT TTT TGC TCG AAG GC (SEQ ID n° 113) | Tfu (Appligene) | 56° C |
| 156 | 37-38 | GCT CTA GAC CAC CAT GCC TTC GAG CAA AAA CTG G (SEQ ID n° 114) | CGG GAT CCA TCG TTC TTC AAT CTC CAC AAA CG (SEQ ID n° 115) | Tfu (Appligene) | 56° C |
| 157 | 39-40 | GCT CTA GAC CAC CAT GCA CCT ATG TGG AAA G (SEQ ID n° 116) | CGG GAT CCA TTC AAT TCG CTT CAA CAA TG (SEQ ID n° 117) | Tfu (Appligene) | 56° C |
| 158 | 41-42 variant allélique N.mA : 72, 73 | GGA CTA GTC CAC CAT GCC TGC CAA CCA ACG TTA CCG (SEQ ID n° 118) | GAA GAT CTA AGC CGC GTT CCC TTC CAA AAA ATC (SEQ ID n° 119) | Tfu (Appligene) | 56° C |
| 159 | 43-44 variant allélique N.mA :74, 75 | GCT CTA GAC CAC CAT GCC GCA AAT TAA AAT TCC C (SEQ ID n° 120) | CGG GAT CCA AAA ACA ATC TTC CGG CAC CC (SEQ ID n° 121) | Tfu (Appligene) | 56° C |
| 161 | 45-46 | GCT CTA GAC CAC CAT GCG CAC GCC GTT TTG TTG (SEQ ID n° 122) | CGG GAT CCA TTG GGC AAC GAC GAA GGC AC (SEQ ID n° 123) | Tfu (Appligene) | 56° C. |
| 163-1 | 47-48 | GCT CTA GAC CAC CAT GAG AAT AGA GAT CAC ACC (SEQ ID n° 124) | CGG GAT CCA TGG CTC AAT CCT TTC TGC (SEQ ID n° 125) | Pfu Turbo (Stratagene) | 56° C |
| 163-2 | 49-50 | GCT CTA GAC CAC CAT GAT TCA CGT TTC GGC AGT G (SEQ ID n° 126) | CGG GAT CCA ACC TGC TTC ATG GGT GAT TC (SEQ ID n° 127) | Tfu (Appligene) | 56° C |
| 167-168 | 51-52 variant allélique N. g : 76, 77 | GCT CTA GAC CAC CAT GAA TTC GAC CGC AAG TAA AAC (SEQ ID n° 128) | CGG GAT CCA AAT CCC TCT GCC GTA TTT G (SEQ ID n° 129) | Tfu (Appligene) | 56° C |

### 2 - Clonage, transformation et sélection des recombinants

Le vecteur de clonage utilisé est le vecteur pCA/Myc-His ou pM1070 de 6.357 kb (cf figure), issu du plasmide pCDNA 3.1 (Invitrogen). Le pCA/Myc-His comprend notamment le promoteur ie1 de CMV (bases 249-902), l'intron A du gène ie1 de CMV (Chapman et al., 1991 Nucleic Acids Research, 19, 3979-3986), un site multiple de clonage (bases 1792-1852) avec les sites Pmll, EcoRV, Notl, Xbal, BamHl, Kpnl et HindIII, une séquence codant pour une polyhistidine et un codon stop (bases 1908-1928), une séquence de terminaison 3'bgh (bases 1853-2197) et le gène de résistance à l'ampicilline pour la sélection des clones recombinants dans *E*. *coli.*

Après purification (Kit GeneClean Bio101), les produits d'amplification PCR sont digérés 2 heures à 37°C par les enzymes adéquates (XbaI-BamHI, Xbal-Bglll, ou Spel-Bglll) dans un volume réactionnel final de 20 µl. Les produits de digestion sont alors ligués avec le vecteur pCA/Myc-His préalablement digéré par Xbal et BamHl selon le protocole de "Rapid DNA Ligation Kit" (Boehringer Mannheim). 15 µl de la ligature est utilisé pour transformer 100 µl de cellules compétentes *E*. *coli* XLI-blue (Novagen). Les cellules sont incubées 30 minutes dans la glace, 30 secondes à 42°C et 2 minutes dans la glace. Puis on ajoute 500 µl de milieu LB sans antibiotique et on incube 1 heure à 37°C. Ensuite 50 et 550 µl de la culture sont étalés sur des boites de milieu LB plus ampicilline (50 µg/ml concentration finale) et incubés une nuit à 37°C.

Le lendemain 36 colonies sont mises en culture dans 2 ml de LB plus ampicilline (50 µg/ml) et incubées une nuit à 37°C.

Le lendemain l'ADN plasmidique est extrait selon le protocole Qiagen mini-prep (Qiagen) et les recombinants sont identifiés par restriction enzymatique suivi d'une électrophorèse sur gel d'agarose. Les jonctions de clonage sont ensuite vérifiées par séquençage.

### Exemple II : Evaluation de l'activité protectrice des ORF de l'invention

### A. Préparation de l'ADN destiné aux expériences d'immunisations :

Une colonie isolée d'un clone recombinant est utilisée pour inoculer une préculture en milieu LB+ ampicilline et 5 ml de cette préculture représente l'inoculat d'une culture de 2,5 litres en milieu LB+ ampicilline. Le protocole de purification pour préparer l'ADN plasmidique est celui décrit dans le Kit EndoFree Giga (Qiagen). L'ADN purifié est élué de la colonne de purification avec un tampon Tris-HCl 10 mM EDTA 1 mM pH 8 et conservé à - 20°C. Avant l'injection, le plasmide recombinant purifié est dilué à raison de 100 µg/ml avec de l'eau (de qualité pour préparation injectable) et la concentration en NaCl est amenée à 150 mM.

### B. Production d'un sérum polyclonal spécifique :

### B. 1. Hyperimunisation dans un modèle animal :

Le modèle animal utilisé est la souris ou le lapin. La voie d'administration de l'ADN injecté est la voie intramusculaire ou la voie intradermique. Les plasmides recombinants à injecter sont éventuellement appliqués sur des billes s'ils sont injectés chez l'animal avec un appareil gene gun (BioRad). Le protocole d'immunisation suit un schéma comportant deux injections à 3 semaines d'intervalles.

### B.2. Analyse de l'activité bactéricide des anticorps induits :

Dix jours après la dernière injection, les animaux sont saignés et les sera sont analysés avec le test de bactéricidie selon le protocole de Danve et al., Vaccine (1993) 11 (12) :1214. Brièvement, les sera sont incubés à différentes dilutions (raison 2) en présence de complément de lapin et de méningococoques cultivés en présence ou en absence d'un agent chélatant du fer. Le titre de bactéricidie d'un sérum représente l'inverse de la dilution de cet antisérum pour lequel 50% des bactéries sont lysées.

On considère que l'antisérum n'est pas bactéricide lorsque son titre est inférieur à 4 contre la souche homologue.

Quand le titre de bactéricidie correspond à une séroconversion d'un facteur 4 contre la souche homologue, l'activité bactéricide de l'antisérum est analysée vis-à-vis des autres souches de Neisseria pour mesurer l'étendue de la réactivité croisée de l'antisérum d'intérêt.

### Exemple III : Production de protéines recombinantes purifiées

### 1. Production recombinante de protéines

### a. Préparation des transformants :

Le produit de PCR obtenu est ensuite digéré à 37°C pendant deux heures avec des enzymes de restriction dans 20 µl de volume réactionnel. Le produit de digestion est ligaturé dans un plasmide pET28a (Novagen), clivé de manière similaire, qui est déphosphorylé avant la ligature par traitement avec la phosphatase alcaline intestinale de veau. Le gène de fusion construit de cette manière permet la purification par affinité en une étape de la protéine de fusion résultante en raison de la présence de résidus histidine à l'extrémité N-terminale de la protéine de fusion qui sont codés par ce vecteur.

La réaction de ligature (20 µl) est effectuée à 14°C une nuit, avant transformation de 100 µl de cellules compétentes fraîches *E. coli* XL1-blue (Novagen). Les cellules sont incubées sur de la glace pendant deux heures puis soumises à un choc thermique à 42°C pendant 30 secondes avant d'être remises dans la glace pendant 90 secondes. Les échantillons sont ensuite ajoutés à 1 ml de bouillon LB en l'absence de sélection et cultivés à 37°C pendant deux heures. Les cellules sont ensuite étalées sur du milieu gélosé LB additionné de kanamycine (50 µg/ml de concentration finale) à une dilution 10x, et sont incubées une nuit à 37°C. Le jour suivant, 50 colonies sont repiquées sur des boîtes secondaires et sont incubées à 37°C une nuit.

### b. Production de la protéine :

Les transformants stockés (10 µl) sont étalés sur des boîtes de sélection et cultivés une nuit à 37°C. Quelques cellules sont recueillies à partir de la boîte utilisée comme inoculum pour une culture starter une nuit (3 ml) à 37°C. Le jour suivant, un échantillon (temps T=0) est recueilli et centrifugé à 14 000 tpm pendant 3 minutes. La culture starter est ensuite utilisée pour inoculer un milieu LB contenant de la kanamycine (100 µg/ml) à une dilution de 1:50 (densité optique de départ DO₆₀₀ = 0,05-0,1). Les cellules sont cultivées à une DO₆₀₀ de 1,0, un échantillon est recueilli pour une SDS-PAGE (échantillon de pré-induction) et la culture restante est induite avec 1 mM d'IPTG. Les cultures sont cultivées pendant quatre heures et des échantillons sont prélevés toutes les heures. La culture est centrifugée à 600 x g pendant 20 minutes à 4°C. Le surnageant est écarté et les culots sont resuspendus dans 50 mM de Tris-HCl (pH : 8,0), 2 mM EDTA, et recentrifugé. Le surnageant est écarté et les cellules sont stockées à -70°C.

### 2. Purification des protéines

Les culots obtenus à partir d'un litre de culture préparé selon l'exemple 1.4 précédent sont séchés et resuspendus dans 20 ml de 20 mM Tris-HCl (pH 8.0), 0.5 M NaCl, 5 mM Imidazole, refroidis dans la glace. Du lysozyme est ajoutée à une concentration de 0,1 mg/ml et la suspension est homogénéisée en utilisant un homogénéiseur à haute vitesse (Turrax) puis traitée avec un sonicateur (Branson, Sonofier 450). De la Benzonase (Merck) est utilisée à une concentration finale de 1 U/ml pour éliminer l'ADN. La suspension est centrifugée à 40 000 x g pendant 20 minutes et le surnageant est filtré à travers une membrane de 0,45 µm. Le surnageant est chargé sur une colonne IMAC (12 ml de résine) qui a été préparée en immobilisant des cations Ni⁺⁺ selon les recommandations du fabricant (Pharmacia). La colonne est lavée avec 10 volumes de colonnes de 20 mM Tris-HCl (pH 8.0), 0.5 M NaCl, 60 mM Imidazole. La protéine recombinante est éluée avec six volumes de 20-mM Tris-HCl (pH : 7.9), 0.5 M NaCl, 500 mM Imidazole, 0.1 % Zwittergent 3-14.

Le profil d'élution est contrôlé en mesurant l'absorbance des fractions à une densité optique de 280 nm. Une fraction aliquote est analysée sur un gel SDS-PAGE et colorée avec du bleu de Coomassie (Phast System - Pharmacia), et les fractions correspondant au pic de protéine sont ensuite regroupées et concentrées. Pour éliminer le tampon d'élution, la fraction est passée sur une colonne G24 Sephadex (Pharmacia), équilibrée dans du tampon PBS (pH : 7,4).La solution protéique est stérilisée par filtration à travers une membrane de 0,45 µm, et la concentration protéique est déterminée par la microméthode BCA (Pierce). La solution protéique est stockée à -70°C.

### Exemple IV : Production d'anticorps polyclonaux monospécifiques

### 1. Antisérum hyperimmun de lapin

On injecte à des lapins de Nouvelle Zélande à la fois par voie cutanée et par voie intraveineuse 100 µg (au total) du polypeptide purifié à l'exemple III, en présence d'adjuvant complet de Freund dans un volume total d'approximativement 2 ml. 21 et 42 jours après l'injection initiale, les doses de rappel qui sont identiques aux doses initiales, sont administrées de la même manière, à l'exception du fait que de l'adjuvant incomplet de Freund est utilisé. 15 jours après la dernière injection, le sérum de l'animal est recueilli, décomplémenté, et filtré à travers une membrane de 0,45 µm.

### 2. Liquide d'ascites hyperimmun de souris

On injecte à 10 souris par voie sous-cutanée 10-50 µg du polypeptide de fusion purifié obtenu à l'exemple II, en présence d'adjuvant complet de Freund, dans un volume d'approximativement 200 µl. 7 et 14 jours après l'injection initiale, des doses de rappel, qui sont identiques aux doses initiales, sont administrées de la même manière, à l'exception du fait que de l'adjuvant incomplet de Freund est utilisé. 21 et 28 jours après l'injection initiale, les souris reçoivent 50 µg de l'antigène seul par voie intrapéritonéale. Au 21^{ème} jour, on injecte également aux souris par voie intrapéritonéale, les cellules 180/TG CM26684 de sarcome (Lennette & Schmidt, Diagnostic procedures for viral, rickettsial, and chlamydial infections, (1979) 5th Ed. Washington DC, American Public Health Association). Les liquides d'ascites sont recueillis 10 à 13 jours après la première injection.

### Exemple V : Purification des polypeptides de l'invention par immunoaffinité

### 1. Purification d'IgG spécifique

Un sérum immun tel que préparé à l'exemple IV est appliqué à une colonne de protéine A Sépharose 4 Fast Flow (Pharmacia) équilibrée avec 100 mM Tris-HCl (pH: 8,0). La résine est lavée en appliquant 10 volumes de colonnes de 100 mM Tris-HCl et 10 volumes de 10 mM Tris-HCl (pH : 8,0) à la colonne. Les IgG sont éluées avec un tampon glycine de 0,1 M (pH : 3,0) et sont recueillies par fraction de 5 ml auxquelles on ajoute 0,25 ml de Tris-HCl 1 M (pH : 8,0). La densité optique de l'éluat est mesurée à 280 nm et les fractions contenant les IgG sont regroupées et si nécessaire stockées à -70°C.

### 2. Préparation de la colonne

Une quantité appropriée de gel Sépharose 4B activé par CNBr (1 g de gel séché fournissant approximativement 3,5 ml de gel hydraté, et la capacité du gel allant de 5 à 10 mg d'IgG couplée par ml de gel) fabriqué par Pharmacia (17-0430-01) et suspendue dans un tampon HCl de 1 mM et lavée avec un buchner par addition de petites quantités de tampon HCl 1 mM. Le volume total du tampon est de 200 ml par gramme de gel.

Les IgG purifiées sont dialysées pendant quatre heures à 20±5°C contre 5 volumes de tampon PBS de 500 mM (pH : 7,5). Puis elles sont diluées dans 500 mM de PBS (pH : 7,5) pour une concentration finale de 3 mg/ml.

Les IgG sont incubées avec le gel une nuit à 5±3°C, sous agitation. Le gel est tassé dans une colonne de chromatographie et lavé avec 2 volumes de colonne de tampon phosphate, 500 mM (pH : 7,5) puis un volume de tampon sodium 50 mM NaCl (pH : 7,5). Le gel est ensuite transféré dans un tube puis incubé avec 100 mM d'éthanolamine, (pH : 7,5) pendant 4 heures à température ambiante sous agitation, puis lavé deux fois avec deux volumes de colonnes de PBS. Le gel est ensuite stocké dans du PBS merthiolate à 1/10 000. La quantité d'IgG couplé au gel est déterminée en mesurant la densité optique à 280 nm de la solution d'IgG et de l'éluat direct.

### 3. Adsorption et élution de l'antigène.

Une solution d'antigène dans 50 mM Tris-HCl (pH : 8,0), 2 mM EDTA, par exemple, le surnageant obtenu à l'exemple III.5 après traitement par la Benzonase, centrifugation et filtration à travers une membrane de 0,45 µm, est appliquée à une colonne équilibrée avec 50 mM Tris-HCl (pH :8,0), 2 mM EDTA à une vitesse de flux d'environ 10 ml/heure. Puis, la colonne est lavée avec 20 volumes de 50 mM Tris-HCl (pH : 8,0), 2 mM EDTA. De manière alternative, l'adsorption peut être réalisée en "batch " qui est laissé une nuit à 5 ±3°C, sous agitation.

Le gel est lavé avec 2 à 6 volumes de tampon PBS 10 mM (pH : 6,8). L'antigène est élué avec un tampon glycine 100 mM (pH : 2,5). L'éluat est recueilli dans des fractions de 3 ml auquel on ajoute 150 µl de tampon PBS 1 mM (pH : 8,0). La densité optique est mesurée à 280 nm pour chaque fraction ; celles contenant l'antigène sont recueillies et stockées à -20°C.

Fragments du génome de *N. meningitidis* Z2491 décrits dans la demande de brevet WO98/02 547

(2) INFORMATIONS POUR LA SEQ ID NO: 70A:

(i) CARACTERISTIQUES DE LA SEQUENCE:
(A) LONGUEUR: 243 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 70A:

GATCAGACCC ATTTTCAGCG CACCGTAAGC GCGGATTTTC TCGAATTTTT CCAAAGCTGC 60

GGCATCGTTG TTGATGTCGT CTTGCAACTC TTTGCCCGTG TAGCCCAAGT CGGCGGCATT 120

CAGGAAAACG GTCGGAATGC CCGCGTTGAT GAGCGTGGCT TTCAAACGGC CTATATTCGG 180

CACATCAATT TCATCGACCA AATTGCCGGT TGGGAACATA CTGCCTTCGC CGTCGGCTGG 240

ATC 243

(2) INFORMATIONS POUR LA SEQ ID NO: 73A:

(i) CARACTERISTIQUES DE LA SEQUENCE:
(A) LONGUEUR: 120 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 73A:

CGGTCAGAAA CAGGCAAGGT AATGAAAATG CCTGAGGCAC GGACTGTGCT GCGAACGAAA 60

ACTCCTTACC GAAGTCTTCT ATACCCAGGC TCAATAGCCG CTCAAGGAGA GAGCTATCAT 120

(2) INFORMATIONS POUR LA SEQ ID NO: 74A:

(i) CARACTERISTIQUES DE LA SEQUENCE:
(A) LONGUEUR: 120 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 74A:

CGGTCAGAAA CAGGCAAGGT AATGAAAATG CCTGAGGCAC GGACTGTGCT GCGAACGAAA 60

ACTCCTTACC GAAGTCTTCT ATACCCAGGC TCAATAGCCG CTCAAGGAGA GAGCTATCAT 120

(2) INFORMATIONS POUR LA SEQ ID NO: 77A

(i) CARACTERISTIQUES DE LA SEQUENCE:
(A) LONGUEUR: 269 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 77A:

CGGAGCATAA AATCGTTATT AAAGATAATG GTATAGGAAC GAGCTTCGAT GAAATCAATG 60

ATTTTTATTT GAGAATCGGT CGGAACAGAA GGGAAGAAAA ACAAGCCTCC CCGTGCGGAA 120

GAATTCCAAC GGGTAAAAAA GGCCTTGGTA AATTGGCATT ATTCGGGCTT GGCAACAAAA 180

TTGAAATTTC TACTATCCAG GGAAACGAAA GGGTTACTTT TACTTTGGAT TATGCAGAGA 240

TTCGAAGAAG CAAGGGTATT TATCAACCG 269

(2) INFORMATIONS POUR LA SEQ ID NO: 80A:

(i) CARACTERISTIQUES DE LA SEQUENCE:
(A) LONGUEUR: 207 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 80A:

CGGGTCGCTT TATTTTGTGC AGGCATTATT TTTCATTTTT GGCTTGACAG TTTGGAAATA 60

TTGTGTATCG GGGGGGGGTA TTTGCTGACG TAAAAAACTA TAAACGCCGC GCAAAATATG 120

GCTGACTATA TTATTGACTT TGATTTTGTC CTGCGCGGTG ATGGATAAAA TCGCCAGCGA 180

TAAAGAATTT GCGAGAACCT GATGCCG 207

(2) INFORMATIONS POUR LA SEQ ID NO: 81A :

(i) CARACTERISTIQUES DE LA SEQUENCE:
(A) LONGUEUR: 224 paires de bases
(B) TYPE: nucléotide

(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 81A:

CGGCAACGAT TTGAGCTATC GCGGTTACGA CATTCTGGAT TTGGCACAAA AATGCGAGTT 60

TGAAGAAGTC GCCCACCTGC TGATTCACGG CCATCTGCCC AACAAATTCG AGCTGGCCGC 120

TTATAAAACC AAGCTCAAAT CCATGCGCGG CCTGCCTATC CGTGTGATTA AAGTTTTGGA 180

AAGCCTGCCT GCACATACCC ATCCGATGGA CGTAATGCGT ACCG 224

(2) INFORMATIONS POUR LA SEQ ID NO: 87A:

(i) CARACTERISTIQUES DE LA SEQUENCE:
(A) LONGUEUR: 273 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 87A:

AATTTCCACC TATGCCCTAC GCAGCGATTA TCCGTGGTTT ACCCAAAGGG TGATTATGGC 60

AAAAGCGCGG GGTTGAGCGA CCGCCTTTTG TTGCCGGCGT TCAAACGGGT TTTGATAGGA 120

AATGCAGGCA CGAAGCCTCG GCTGATTGTG ATGCACCTGA TGGGTTCGCA CAGTGATTTT 180

TGCACACGTT TGGATAAGGA TGCGCGGCGG TTTCAGTATC AAACTGAAAA AATATCCTGC 240

TATGTTTCCA TCAATCGCGC AAACCGATAA ATT 273

(2) INFORMATIONS POUR LA SEQ ID NO: 88A:

(i) CARACTERISTIQUES DE LA SEQUENCE:
(A) LONGUEUR: 270 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 88A:

AATTCTTCCG CACGGGGAGG CTTGTTTTTC TTCCCTTCTG TTCCGACCGA TTCTCAAATA 60

AAAATCATTG ATTTCATCGA AGTTCATTCC TATACCATTA TCTTTAATAA CGATTTTATG 120

CTCCGGTTTA TCGAATAACC TAACTTCCAC TTCCGTAGCA CATGCATCGT AGGCATTCGC 180

TATCAACTCG GCAATCGCAG GAACAGTGTG CGAATACAAT CTTTACACCC AAATGTTCGA 240

TTACGGTTGG CTCGAAACTC AATTTCAATT 270

(2) INFORMATIONS POUR LA SEQ ID NO: 89A:

(i) CARACTERISTIQUES DE LA SEQUENCE:
(A) LONGUEUR: 267 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 89A:

AATTATGAAC ACACGCATCA TCGTTTCGGC TGCGTTCGTT GCGTTGGCAT TAGCAGGTTG 60

CGGCTCAATC AATAATGTAA CCGTTTCCGA CCAGAAACTT CAGGAACGTG CCGCGTTTGC 120

CTTGGGCGTC ACCAATGCCG TAAAAATCAG CAACCGCAGC AATGAAGGCA TACGCATCAA 180

CTTTACCGCA ACTGTGGGTA AGCGCGTGAC CAATGCTATG TTACCAGTGT AATCAGCACA 240

ATCGGCGTTA CCACTTCCGA TGCAATT 267

(2) INFORMATIONS POUR LA SEQ ID NO: 94A:

(i) CARACTERISTIQUES DE LA SEQUENCE:
(A) LONGUEUR: 308 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 94A:

AATTTGTTGG GCAGATGGCC GTGAATCAGC AGGTGGGCGA CTTCTTCAAA CTCGCATTTT 60

TGTGCCAAAT CCAGAATGTC GTAACCGCGA TACGTCAAAT CGTTGCCGGT ACGCAACGGT 120

ACACAAAGCG GTATTACCGG CCGCAACGCC AGAAAGCGCA ACGGATTTTT AGGTTTGAGG 180

GTCGGGGTTT GAGTAGTTTC AGTCATGGTA TTTCTCCTTT GTGTTTTTAT GGGTTTCGGG 240

TTTTCAGACG ACCGATGCGG ATTTGTTGAA AGGCAGTCTG AAAGCGGTAA ATCATTTTTG 300

AAACAATT 308

(2) INFORMATIONS POUR LA SEQ ID NO: 95A:

(i) CARACTERISTIQUES DE LA SEQUENCE:
(A) LONGUEUR: 286 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 95A :

AATTCGGAGG AGCAGTACCG CCAAGCGTTG CTCGCCTATT CCGGCGGTGA TAAAACAGAC 60

GAGGGTATCC GCCTGATGCA ACAGAGCGAT TACGGCAACT TGTCCTACCA CATCCGTAAT 120

AAAAACATGC TTTTCATTTT TTCGGCAAGC AATGACGCAC AAGCTCAGCC CAACACAACT 180

GACCCTATTG CCATTTTATG AAAAAGACGC TCAAAAAGGC ATTATCACAG TTGCAGGCGT 240

AGACCGCAGT GGAGAAAAGT TCAATGGCTC CAACCATTGC GGAATT 286

(2) INFORMATIONS POUR LA SEQ ID NO: 98A :

(i) CARACTERISTIQUES DE LA SEQUENCE:
(A) LONGUEUR: 316 paires de bases
(B) TYPE: nucléotide
(C) NOMBRE DE BRINS: simple
(D) CONFIGURATION: linéaire

(ii) TYPE DE MOLECULE: ADN (génomique)

(iii) HYPOTHETIQUE: NON

(iv) ANTI-SENS: NON

(xi) DESCRIPTION DE LA SEQUENCE: SEQ ID NO: 98A

AATTTGTCGG CAATCTTCCC GGGTCGCTTT ATTTTGTGCA GGCATTATTT TTCATTTTTG 60

GCTTGACAGT TTGGAGATAT TGTGTATCGG GGGGGGGTAT TTGCTGACGT AAAAAACTAT 120

AAACGCCGCA GCAAAATATG GCTGACTATA TTATTGACTT TGATTTTGTC CTGCGCGGTG 180

ATGGATAAAA TCGCCAGCGA TAAAGATTTG CGAGAACCTG ATGCCGGCCT GTTGTTGAAT 240

ATTTTCGACC TGTAATTACG ATTTGGCTTC CGCGCCGGCA CAATATGCCG CCAAGCGGCG 300

CCCACATTTT GGAAGC 316

LISTE DE SEQUENCES

<110> Sanofi Pasteur
      INSERM

<120> Acides nucléiques et polypeptides spécifiques des
      souches pathogènes du genre Neisseria

<130> BET 06P0039

<140>
<141>

<150> FR 98 13 693
<151> 1998-10-30

<160> 129

<170> PatentIn Ver. 2.1

<210> 1
<211> 858
<212> ADN
<213> Neisseria meningitidis

<220>
<221> CDS
<222> (1)..(855)

<400> 1
atg tct gaa gaa aaa ttg aaa atg agt ttc gag cca acc gta atc gaa    48
Met Ser Glu Glu Lys Leu Lys Met Ser Phe Glu Pro Thr Val Ile Glu
  1               5                  10                  15

cat ttg ggt gta aag atg tat tcg cac act gtt cct gcg att gcc gag    96
His Leu Gly Val Lys Met Tyr Ser His Thr Val Pro Ala Ile Ala Glu
             20                  25                  30

ttg ata gcg aat gcc tac gat gca tgt gct acg gaa gtg gaa gtt agg    144
Leu Ile Ala Asn Ala Tyr Asp Ala Cys Ala Thr Glu Val Glu Val Arg
         35                  40                  45

tta ttc gat aaa ccg gag cat aaa atc gtt atc aaa gat aat ggt ata    192
Leu Phe Asp Lys Pro Glu His Lys Ile Val Ile Lys Asp Asn Gly Ile
     50                  55                  60

gga atg agc ttc gat gaa atc aat gat ttt tat ttg aga atc ggt cgg    240
Gly Met Ser Phe Asp Glu Ile Asn Asp Phe Tyr Leu Arg Ile Gly Arg
 65                  70                  75                  80

aac aga agg gaa gaa aaa caa gct tcc ccg tgc gga aga att cca acg    288
Asn Arg Arg Glu Glu Lys Gln Ala Ser Pro Cys Gly Arg Ile Pro Thr
                 85                  90                  95

ggt aaa aaa ggc ctt ggt aaa ttg gca tta ttc ggg ctt ggc aac aaa    336
Gly Lys Lys Gly Leu Gly Lys Leu Ala Leu Phe Gly Leu Gly Asn Lys
            100                 105                 110

att gaa att tct act atc cag gga aac gaa agg gtt act ttt act ttg    384
Ile Glu Ile Ser Thr Ile Gln Gly Asn Glu Arg Val Thr Phe Thr Leu

        115                 120                 125

gat tat gca gag att cga aga agc aag ggt att tat caa ccg gag ttt 432
Asp Tyr Ala Glu Ile Arg Arg Ser Lys Gly Ile Tyr Gln Pro Glu Phe
    130                 135                 140

cga aaa gaa tct gtt gaa tcc aat atc gaa agc ggg aca acc ata act 480
Arg Lys Glu Ser Val Glu Ser Asn Ile Glu Ser Gly Thr Thr Ile Thr
145                 150                 155                 160

tta acc gaa ctg acg aaa aag caa gga tat ccg tta gat aat tat gta 528
Leu Thr Glu Leu Thr Lys Lys Gln Gly Tyr Pro Leu Asp Asn Tyr Val
                165                 170                 175

gag cat ctt tcc cgc ttg ttt gat ttt ccg gct cag gat ttt aaa atc 576
Glu His Leu Ser Arg Leu Phe Asp Phe Pro Ala Gln Asp Phe Lys Ile
            180                 185                 190

aaa gta agc ttg aac ggc tct gaa cct aaa atc att gat gga aat cta 624
Lys Val Ser Leu Asn Gly Ser Glu Pro Lys Ile Ile Asp Gly Asn Leu
        195                 200                 205

aaa tat gat ctt gtt acc cca caa ttc gaa tgg gaa tac cag gat tta 672
Lys Tyr Asp Leu Val Thr Pro Gln Phe Glu Trp Glu Tyr Gln Asp Leu
    210                 215                 220

gca acc aat att tca tcg tta tct tca aaa ttc gaa cag tat gaa tac 720
Ala Thr Asn Ile Ser Ser Leu Ser Ser Lys Phe Glu Gln Tyr Glu Tyr
225                 230                 235                 240

agc gga tta ata caa ggt aag ttc att aca acg gaa aaa cct tta aag 768
Ser Gly Leu Ile Gln Gly Lys Phe Ile Thr Thr Glu Lys Pro Leu Lys
                245                 250                 255

aat aat atg aaa ggt att acc ttg ttt gcc aac ggc aga atg gta aat 816
Asn Asn Met Lys Gly Ile Thr Leu Phe Ala Asn Gly Arg Met Val Asn
            260                 265                 270

atg ccc gag ttt ttc act gat agc gaa tcc agc cat ttc taa 858
Met Pro Glu Phe Phe Thr Asp Ser Glu Ser Ser His Phe
        275                 280                 285

<210> 2
<211> 285
<212> PRT
<213> Neisseria meningitidis

<400> 2
Met Ser Glu Glu Lys Leu Lys Met Ser Phe Glu Pro Thr Val Ile Glu
1               5                   10                  15

His Leu Gly Val Lys Met Tyr Ser His Thr Val Pro Ala Ile Ala Glu
            20                  25                  30

Leu Ile Ala Asn Ala Tyr Asp Ala Cys Ala Thr Glu Val Glu Val Arg
        35                  40                  45

Leu Phe Asp Lys Pro Glu His Lys Ile Val Ile Lys Asp Asn Gly Ile
    50                  55                  60

Gly Met Ser Phe Asp Glu Ile Asn Asp Phe Tyr Leu Arg Ile Gly Arg
65                  70                  75                  80

Asn Arg Arg Glu Glu Lys Gln Ala Ser Pro Cys Gly Arg Ile Pro Thr
                85                  90                  95

Gly Lys Lys Gly Leu Gly Lys Leu Ala Leu Phe Gly Leu Gly Asn Lys
            100                 105                 110

Ile Glu Ile Ser Thr Ile Gln Gly Asn Glu Arg Val Thr Phe Thr Leu
        115                 120                 125

Asp Tyr Ala Glu Ile Arg Arg Ser Lys Gly Ile Tyr Gln Pro Glu Phe
    130                 135                 140

Arg Lys Glu Ser Val Glu Ser Asn Ile Glu Ser Gly Thr Thr Ile Thr
145                 150                 155                 160

Leu Thr Glu Leu Thr Lys Lys Gln Gly Tyr Pro Leu Asp Asn Tyr Val
                165                 170                 175

Glu His Leu Ser Arg Leu Phe Asp Phe Pro Ala Gln Asp Phe Lys Ile
            180                 185                 190

Lys Val Ser Leu Asn Gly Ser Glu Pro Lys Ile Ile Asp Gly Asn Leu
        195                 200                 205

Lys Tyr Asp Leu Val Thr Pro Gln Phe Glu Trp Glu Tyr Gln Asp Leu
    210                 215                 220

Ala Thr Asn Ile Ser Ser Leu Ser Ser Lys Phe Glu Gln Tyr Glu Tyr
225                 230                 235                 240

Ser Gly Leu Ile Gln Gly Lys Phe Ile Thr Thr Glu Lys Pro Leu Lys
                245                 250                 255

Asn Asn Met Lys Gly Ile Thr Leu Phe Ala Asn Gly Arg Met Val Asn
            260                 265                 270

Met Pro Glu Phe Phe Thr Asp Ser Glu Ser Ser His Phe
        275                 280                 285

<210> 3
<211> 1101
<212> ADN
<213> Neisseria meningitidis

<220>
<221> CDS
<222> (1)..(1098)

<400> 3
atg aaa cac tta ctc atc gat ttt gaa aac gtc cag ccg caa aac tta     48
Met Lys His Leu Leu Ile Asp Phe Glu Asn Val Gln Pro Gln Asn Leu
1               5                   10                  15

gac aaa tta cta acc gaa aat acc cat att tgg cta ttt ata ggt gta     96

Asp Lys Leu Leu Thr Glu Asn Thr His Ile Trp Leu Phe Ile Gly Val
            20                  25                  30

tta cac aaa atg tta cct att agt ctg gtg caa tcc cta cta cgt ttc 144
Leu His Lys Met Leu Pro Ile Ser Leu Val Gln Ser Leu Leu Arg Phe
        35                  40                  45

ggc gaa cgt gtc cat ctt gtc cag tta caa aaa acg ggg aaa aac gca 192
Gly Glu Arg Val His Leu Val Gln Leu Gln Lys Thr Gly Lys Asn Ala
    50                  55                  60

ttg gat ttt tac ctg tcc tat tac ctc gga caa att acc gcc aca gac 240
Leu Asp Phe Tyr Leu Ser Tyr Tyr Leu Gly Gln Ile Thr Ala Thr Asp
65                  70                  75                  80

ccc aat gcc caa atc ggc ata ctc tcg cgt gat gga gga tac gat gtt 288
Pro Asn Ala Gln Ile Gly Ile Leu Ser Arg Asp Gly Gly Tyr Asp Val
                85                  90                  95

ctg gtc gaa cat att ttg aaa aac cac cag gcg aag ggt atc gtg cgc 336
Leu Val Glu His Ile Leu Lys Asn His Gln Ala Lys Gly Ile Val Arg
            100                 105                 110

cta gcc aat ata gat gaa gta caa cat cag aaa att gct acc gaa ccg 384
Leu Ala Asn Ile Asp Glu Val Gln His Gln Lys Ile Ala Thr Glu Pro
        115                 120                 125

ccg tca gca ttg ctg gaa aac act cct cag cct gaa acc acc ctc aaa 432
Pro Ser Ala Leu Leu Glu Asn Thr Pro Gln Pro Glu Thr Thr Leu Lys
    130                 135                 140

cca cag caa cca tta act tcc tat ttc caa gca gcc cta act gca ctg 480
Pro Gln Gln Pro Leu Thr Ser Tyr Phe Gln Ala Ala Leu Thr Ala Leu
145                 150                 155                 160

cgc cgc ccc gac gct ttc cgc ccc tgc cgc ctg cat aac ctg cga caa 528
Arg Arg Pro Asp Ala Phe Arg Pro Cys Arg Leu His Asn Leu Arg Gln
                165                 170                 175

aat ctg cgt aag cat att ttg agt gat ttg ttt aaa gaa aaa acc gat 576
Asn Leu Arg Lys His Ile Leu Ser Asp Leu Phe Lys Glu Lys Thr Asp
            180                 185                 190

gaa gaa tgc gaa ata acc act gct aac gtt atc aat aaa ctc aaa gca 624
Glu Glu Cys Glu Ile Thr Thr Ala Asn Val Ile Asn Lys Leu Lys Ala
        195                 200                 205

caa aac ttc atc agc att gat gaa cag gaa acc gtt tcc tac cat ctc 672
Gln Asn Phe Ile Ser Ile Asp Glu Gln Glu Thr Val Ser Tyr His Leu
    210                 215                 220

agt gat aat gat ttg tta caa aga atc caa cgc cat att tta agc caa 720
Ser Asp Asn Asp Leu Leu Gln Arg Ile Gln Arg His Ile Leu Ser Gln
225                 230                 235                 240

cgt ccc aaa acc tac gct gat ttt caa gcc gtc gtg caa aac cga gca 768
Arg Pro Lys Thr Tyr Ala Asp Phe Gln Ala Val Val Gln Asn Arg Ala
                245                 250                 255

gat gca ctt cac tta aca gtc ggt acc aac gac att caa tcc ttt gcg 816

Asp Ala Leu His Leu Thr Val Gly Thr Asn Asp Ile Gln Ser Phe Ala
            260                 265                 270

cga cat ttg cgc gac caa aac ctg atc cgc caa aac aat ggg aaa att     864
Arg His Leu Arg Asp Gln Asn Leu Ile Arg Gln Asn Asn Gly Lys Ile
        275                 280                 285

gaa tat gca ccg ttt act gaa cct aaa cca cag cca acg ccc aag cag     912
Glu Tyr Ala Pro Phe Thr Glu Pro Lys Pro Gln Pro Thr Pro Lys Gln
    290                 295                 300

cct aaa aaa acc gca tgg gaa cct gat gaa att att tgg aaa aaa gtg     960
Pro Lys Lys Thr Ala Trp Glu Pro Asp Glu Ile Ile Trp Lys Lys Val
305                 310                 315                 320

att gcc gcg tta tcg tta aag aac cgt cct aat aaa acc aaa act tta     1008
Ile Ala Ala Leu Ser Leu Lys Asn Arg Pro Asn Lys Thr Lys Thr Leu
                325                 330                 335

cgc aat aca atc cag gca ctc aca aaa tcc aat gca caa gaa act gac     1056
Arg Asn Thr Ile Gln Ala Leu Thr Lys Ser Asn Ala Gln Glu Thr Asp
            340                 345                 350

aaa ctg cta caa cat tta caa gat gac cca agt cct acg tat tga         1101
Lys Leu Leu Gln His Leu Gln Asp Asp Pro Ser Pro Thr Tyr
        355                 360                 365

<210> 4
<211> 366
<212> PRT
<213> Neisseria meningitidis

<400> 4
Met Lys His Leu Leu Ile Asp Phe Glu Asn Val Gln Pro Gln Asn Leu
1               5                   10                  15

Asp Lys Leu Leu Thr Glu Asn Thr His Ile Trp Leu Phe Ile Gly Val
            20                  25                  30

Leu His Lys Met Leu Pro Ile Ser Leu Val Gln Ser Leu Leu Arg Phe
        35                  40                  45

Gly Glu Arg Val His Leu Val Gln Leu Gln Lys Thr Gly Lys Asn Ala
    50                  55                  60

Leu Asp Phe Tyr Leu Ser Tyr Tyr Leu Gly Gln Ile Thr Ala Thr Asp
65                  70                  75                  80

Pro Asn Ala Gln Ile Gly Ile Leu Ser Arg Asp Gly Gly Tyr Asp Val
                85                  90                  95

Leu Val Glu His Ile Leu Lys Asn His Gln Ala Lys Gly Ile Val Arg
            100                 105                 110

Leu Ala Asn Ile Asp Glu Val Gln His Gln Lys Ile Ala Thr Glu Pro
        115                 120                 125

Pro Ser Ala Leu Leu Glu Asn Thr Pro Gln Pro Glu Thr Thr Leu Lys
    130                 135                 140

Pro Gln Gln Pro Leu Thr Ser Tyr Phe Gln Ala Ala Leu Thr Ala Leu
145                 150                 155                 160

Arg Arg Pro Asp Ala Phe Arg Pro Cys Arg Leu His Asn Leu Arg Gln
                165                 170                 175

Asn Leu Arg Lys His Ile Leu Ser Asp Leu Phe Lys Glu Lys Thr Asp
            180                 185                 190

Glu Glu Cys Glu Ile Thr Thr Ala Asn Val Ile Asn Lys Leu Lys Ala
        195                 200                 205

Gln Asn Phe Ile Ser Ile Asp Glu Gln Glu Thr Val Ser Tyr His Leu
    210                 215                 220

Ser Asp Asn Asp Leu Leu Gln Arg Ile Gln Arg His Ile Leu Ser Gln
225                 230                 235                 240

Arg Pro Lys Thr Tyr Ala Asp Phe Gln Ala Val Val Gln Asn Arg Ala
                245                 250                 255

Asp Ala Leu His Leu Thr Val Gly Thr Asn Asp Ile Gln Ser Phe Ala
            260                 265                 270

Arg His Leu Arg Asp Gln Asn Leu Ile Arg Gln Asn Asn Gly Lys Ile
        275                 280                 285

Glu Tyr Ala Pro Phe Thr Glu Pro Lys Pro Gln Pro Thr Pro Lys Gln
    290                 295                 300

Pro Lys Lys Thr Ala Trp Glu Pro Asp Glu Ile Ile Trp Lys Lys Val
305                 310                 315                 320

Ile Ala Ala Leu Ser Leu Lys Asn Arg Pro Asn Lys Thr Lys Thr Leu
                325                 330                 335

Arg Asn Thr Ile Gln Ala Leu Thr Lys Ser Asn Ala Gln Glu Thr Asp
            340                 345                 350

Lys Leu Leu Gln His Leu Gln Asp Asp Pro Ser Pro Thr Tyr
        355                 360                 365

<210> 5
<211> 1572
<212> ADN
<213> Neisseria meningitidis

<220>
<221> CDS
<222> (1)..(1569)

<400> 5
atg aaa aaa tcc ctt ttc gtt ctc ttt ctg tat tca tcc cta ctt acc     48
Met Lys Lys Ser Leu Phe Val Leu Phe Leu Tyr Ser Ser Leu Leu Thr
1               5                   10                  15

gcc agc gaa atc gcc tat cgc ttt gta ttc gga att gaa acc tta ccg     96

Ala Ser Glu Ile Ala Tyr Arg Phe Val Phe Gly Ile Glu Thr Leu Pro
            20                  25                  30

gct gca aaa atg gca gaa acg ttt gcg ctg aca ttt atg att gct gcg 144
Ala Ala Lys Met Ala Glu Thr Phe Ala Leu Thr Phe Met Ile Ala Ala
        35                  40                  45

ctg tat ctg ttt gcg cgt tat aag gct tcg cgg ctg ctg att gcg gtg 192
Leu Tyr Leu Phe Ala Arg Tyr Lys Ala Ser Arg Leu Leu Ile Ala Val
    50                  55                  60

ttt ttc gcg ttc agc att att gcc aac aat gta cat tat gcg gtt tat 240
Phe Phe Ala Phe Ser Ile Ile Ala Asn Asn Val His Tyr Ala Val Tyr
65                  70                  75                  80

caa agt tgg atg acg ggc atc aat tat tgg ctg atg ctg aaa gag att 288
Gln Ser Trp Met Thr Gly Ile Asn Tyr Trp Leu Met Leu Lys Glu Ile
                85                  90                  95

acc gaa gtc ggc agt gcg ggc gcg tcg atg ttg gat aag ttg tgg ctg 336
Thr Glu Val Gly Ser Ala Gly Ala Ser Met Leu Asp Lys Leu Trp Leu
            100                 105                 110

cct gcg ttg tgg ggc gtg ttg gaa gtc atg ttg ttt tgc agc ctt gcc 384
Pro Ala Leu Trp Gly Val Leu Glu Val Met Leu Phe Cys Ser Leu Ala
        115                 120                 125

aag ttc cac cgt aag acg cat ttt tct gcc gat ata ctg ttt gcc ttc 432
Lys Phe His Arg Lys Thr His Phe Ser Ala Asp Ile Leu Phe Ala Phe
    130                 135                 140

cta atg ctg atg att ttc gtg cgt tcg ttc gac acg aaa caa gag cac 480
Leu Met Leu Met Ile Phe Val Arg Ser Phe Asp Thr Lys Gln Glu His
145                 150                 155                 160

ggt att tcg ccc aaa ccg aca tac agc cgc atc aaa gcc aat tat ttc 528
Gly Ile Ser Pro Lys Pro Thr Tyr Ser Arg Ile Lys Ala Asn Tyr Phe
                165                 170                 175

agc ttc ggt tat ttt gtc gga cgc gtg ttg ccg tat cag ttg ttt gat 576
Ser Phe Gly Tyr Phe Val Gly Arg Val Leu Pro Tyr Gln Leu Phe Asp
            180                 185                 190

tta agc agg att ccc gcc ttt aag cag cct gct cca agc aaa atc ggg 624
Leu Ser Arg Ile Pro Ala Phe Lys Gln Pro Ala Pro Ser Lys Ile Gly
        195                 200                 205

cag ggc agt gtt caa aat atc gtc ctg att atg ggc gaa agc gaa agc 672
Gln Gly Ser Val Gln Asn Ile Val Leu Ile Met Gly Glu Ser Glu Ser
    210                 215                 220

gcg gcg cat ttg aag ctg ttt ggc tac gga cgc gaa act tcg ccg ttt 720
Ala Ala His Leu Lys Leu Phe Gly Tyr Gly Arg Glu Thr Ser Pro Phe
225                 230                 235                 240

tta acc cgg ctg tcg caa gcc gat ttt aag ccg att gtg aaa caa agt 768
Leu Thr Arg Leu Ser Gln Ala Asp Phe Lys Pro Ile Val Lys Gln Ser
                245                 250                 255

tat tcc gca ggc ttt atg act gca gtg tcc ctg ccc agt ttt ttc aat 816

Tyr Ser Ala Gly Phe Met Thr Ala Val Ser Leu Pro Ser Phe Phe Asn
            260                 265                 270

gcg ata ccg cac gcc aac ggc ttg gaa caa atc agc ggc ggc gat act 864
Ala Ile Pro His Ala Asn Gly Leu Glu Gln Ile Ser Gly Gly Asp Thr
        275                 280                 285

aat atg ttc cgc ctc gcc aaa gag cag ggc tat gaa acg tat ttt tac 912
Asn Met Phe Arg Leu Ala Lys Glu Gln Gly Tyr Glu Thr Tyr Phe Tyr
    290                 295                 300

agc gca cag gcg gaa aac gag atg gcg att ttg aac tta atc ggt aag 960
Ser Ala Gln Ala Glu Asn Glu Met Ala Ile Leu Asn Leu Ile Gly Lys
305                 310                 315                 320

aaa tgg ata gac cat ctg att cag ccg acg cag ctt ggc tac ggc aac 1008
Lys Trp Ile Asp His Leu Ile Gln Pro Thr Gln Leu Gly Tyr Gly Asn
                325                 330                 335

ggc gac aat atg ccc gat gag aag ctg ctg ccg ctg ttc gac aaa atc 1056
Gly Asp Asn Met Pro Asp Glu Lys Leu Leu Pro Leu Phe Asp Lys Ile
            340                 345                 350

aat ttg cag cag ggc agg cat ttt atc gtg ttg cac caa cgt ggt tcg 1104
Asn Leu Gln Gln Gly Arg His Phe Ile Val Leu His Gln Arg Gly Ser
        355                 360                 365

cac gcc cca tac agc gca ttg ttg cag cct caa gat aaa gta ttc ggc 1152
His Ala Pro Tyr Ser Ala Leu Leu Gln Pro Gln Asp Lys Val Phe Gly
    370                 375                 380

gaa ctt att gtg gat aag tac gac aac acc atc cac aaa acc gac caa 1200
Glu Leu Ile Val Asp Lys Tyr Asp Asn Thr Ile His Lys Thr Asp Gln
385                 390                 395                 400

atg att caa acc gta ttc gag cag ctg caa aag cag cct gac ggc aac 1248
Met Ile Gln Thr Val Phe Glu Gln Leu Gln Lys Gln Pro Asp Gly Asn
                405                 410                 415

tgg ctg ttt gcc tat acc tcc gat cat ggc cag tat gtt cgc caa gat 1296
Trp Leu Phe Ala Tyr Thr Ser Asp His Gly Gln Tyr Val Arg Gln Asp
            420                 425                 430

atc tac aat caa ggc acg gtg cag ccc gac agc tat ctc gtg ccg ctg 1344
Ile Tyr Asn Gln Gly Thr Val Gln Pro Asp Ser Tyr Leu Val Pro Leu
        435                 440                 445

gtg ttg tac agc tcg aat aag gcc gtg caa cag gct gcc aac cag gct 1392
Val Leu Tyr Ser Ser Asn Lys Ala Val Gln Gln Ala Ala Asn Gln Ala
    450                 455                 460

ttt gcg cct tgc gag att gcc ttc cat cag cag ctt tca acg ttc ctg 1440
Phe Ala Pro Cys Glu Ile Ala Phe His Gln Gln Leu Ser Thr Phe Leu
465                 470                 475                 480

att cac acg ttg ggc tac gat atg ccg gtt tca ggt tgt cgc gaa ggc 1488
Ile His Thr Leu Gly Tyr Asp Met Pro Val Ser Gly Cys Arg Glu Gly
                485                 490                 495

tcg gta acg ggc aac ctg att acg ggt gat gca ggc agc ttg aac att 1536

Ser Val Thr Gly Asn Leu Ile Thr Gly Asp Ala Gly Ser Leu Asn Ile
            500                 505                 510

cgc gac ggc aag gcg gaa tat gtt tat ccg caa tga                    1572
Arg Asp Gly Lys Ala Glu Tyr Val Tyr Pro Gln
        515                 520


<210> 6
<211> 523
<212> PRT
<213> Neisseria meningitidis

<400> 6
Met Lys Lys Ser Leu Phe Val Leu Phe Leu Tyr Ser Ser Leu Leu Thr
1               5                   10                  15

Ala Ser Glu Ile Ala Tyr Arg Phe Val Phe Gly Ile Glu Thr Leu Pro
            20                  25                  30

Ala Ala Lys Met Ala Glu Thr Phe Ala Leu Thr Phe Met Ile Ala Ala
        35                  40                  45

Leu Tyr Leu Phe Ala Arg Tyr Lys Ala Ser Arg Leu Leu Ile Ala Val
    50                  55                  60

Phe Phe Ala Phe Ser Ile Ile Ala Asn Asn Val His Tyr Ala Val Tyr
65                  70                  75                  80

Gln Ser Trp Met Thr Gly Ile Asn Tyr Trp Leu Met Leu Lys Glu Ile
                85                  90                  95

Thr Glu Val Gly Ser Ala Gly Ala Ser Met Leu Asp Lys Leu Trp Leu
            100                 105                 110

Pro Ala Leu Trp Gly Val Leu Glu Val Met Leu Phe Cys Ser Leu Ala
        115                 120                 125

Lys Phe His Arg Lys Thr His Phe Ser Ala Asp Ile Leu Phe Ala Phe
    130                 135                 140

Leu Met Leu Met Ile Phe Val Arg Ser Phe Asp Thr Lys Gln Glu His
145                 150                 155                 160

Gly Ile Ser Pro Lys Pro Thr Tyr Ser Arg Ile Lys Ala Asn Tyr Phe
                165                 170                 175

Ser Phe Gly Tyr Phe Val Gly Arg Val Leu Pro Tyr Gln Leu Phe Asp
            180                 185                 190

Leu Ser Arg Ile Pro Ala Phe Lys Gln Pro Ala Pro Ser Lys Ile Gly
        195                 200                 205

Gln Gly Ser Val Gln Asn Ile Val Leu Ile Met Gly Glu Ser Glu Ser
    210                 215                 220

Ala Ala His Leu Lys Leu Phe Gly Tyr Gly Arg Glu Thr Ser Pro Phe
225                 230                 235                 240

Leu Thr Arg Leu Ser Gln Ala Asp Phe Lys Pro Ile Val Lys Gln Ser

245                 250                 255

Tyr Ser Ala Gly Phe Met Thr Ala Val Ser Leu Pro Ser Phe Phe Asn
            260                 265                 270

Ala Ile Pro His Ala Asn Gly Leu Glu Gln Ile Ser Gly Gly Asp Thr
        275                 280                 285

Asn Met Phe Arg Leu Ala Lys Glu Gln Gly Tyr Glu Thr Tyr Phe Tyr
    290                 295                 300

Ser Ala Gln Ala Glu Asn Glu Met Ala Ile Leu Asn Leu Ile Gly Lys
305                 310                 315                 320

Lys Trp Ile Asp His Leu Ile Gln Pro Thr Gln Leu Gly Tyr Gly Asn
                325                 330                 335

Gly Asp Asn Met Pro Asp Glu Lys Leu Leu Pro Leu Phe Asp Lys Ile
            340                 345                 350

Asn Leu Gln Gln Gly Arg His Phe Ile Val Leu His Gln Arg Gly Ser
        355                 360                 365

His Ala Pro Tyr Ser Ala Leu Leu Gln Pro Gln Asp Lys Val Phe Gly
    370                 375                 380

Glu Leu Ile Val Asp Lys Tyr Asp Asn Thr Ile His Lys Thr Asp Gln
385                 390                 395                 400

Met Ile Gln Thr Val Phe Glu Gln Leu Gln Lys Gln Pro Asp Gly Asn
                405                 410                 415

Trp Leu Phe Ala Tyr Thr Ser Asp His Gly Gln Tyr Val Arg Gln Asp
            420                 425                 430

Ile Tyr Asn Gln Gly Thr Val Gln Pro Asp Ser Tyr Leu Val Pro Leu
        435                 440                 445

Val Leu Tyr Ser Ser Asn Lys Ala Val Gln Gln Ala Ala Asn Gln Ala
    450                 455                 460

Phe Ala Pro Cys Glu Ile Ala Phe His Gln Gln Leu Ser Thr Phe Leu
465                 470                 475                 480

Ile His Thr Leu Gly Tyr Asp Met Pro Val Ser Gly Cys Arg Glu Gly
                485                 490                 495

Ser Val Thr Gly Asn Leu Ile Thr Gly Asp Ala Gly Ser Leu Asn Ile
            500                 505                 510

Arg Asp Gly Lys Ala Glu Tyr Val Tyr Pro Gln
        515                 520

<210> 7
<211> 3204
<212> ADN
<213> Neisseria meningitidis

<220>
<221> CDS
<222> (1)..(3201)

<400> 7
atg cga acg acc cca acc ttc cct aca aaa act ttc aaa ccg gct gcc 48
Met Arg Thr Thr Pro Thr Phe Pro Thr Lys Thr Phe Lys Pro Ala Ala
1               5                   10                  15

atg gcg tta gct gtt gca aca aca ctt tct gcc tgc tta ggc ggc ggc 96
Met Ala Leu Ala Val Ala Thr Thr Leu Ser Ala Cys Leu Gly Gly Gly
            20                  25                  30

ggc ggc act tct gcg ccc gac ttc aat gca ggc ggc acc ggt atc ggc 144
Gly Gly Thr Ser Ala Pro Asp Phe Asn Ala Gly Gly Thr Gly Ile Gly
        35                  40                  45

agc aac agc aga gca aca aca gcg aaa tca gca gca gta tct tac gcc 192
Ser Asn Ser Arg Ala Thr Thr Ala Lys Ser Ala Ala Val Ser Tyr Ala
    50                  55                  60

ggt atc aag aac gaa atg tgc aaa gac aga agc atg ctc tgt gcc ggt 240
Gly Ile Lys Asn Glu Met Cys Lys Asp Arg Ser Met Leu Cys Ala Gly
65                  70                  75                  80

cgg gat gac gtt gcg gtt aca gac agg gat gcc aaa atc aat gcc ccc 288
Arg Asp Asp Val Ala Val Thr Asp Arg Asp Ala Lys Ile Asn Ala Pro
                85                  90                  95

ccc ccg aat ctg cat acc gga gac ttt aca aac cca aat gac gca tac 336
Pro Pro Asn Leu His Thr Gly Asp Phe Thr Asn Pro Asn Asp Ala Tyr
            100                 105                 110

aag aat ttg atc aac ctc aaa cct gca att gaa gca ggc tat aca gga 384
Lys Asn Leu Ile Asn Leu Lys Pro Ala Ile Glu Ala Gly Tyr Thr Gly
        115                 120                 125

cgc ggg gta gag gta ggt atc gtc gat aca ggc gaa tcc gtc ggc agc 432
Arg Gly Val Glu Val Gly Ile Val Asp Thr Gly Glu Ser Val Gly Ser
    130                 135                 140

ata tcc ttt ccc gaa ctg tat ggc aga aaa gaa cac ggc tat aac gaa 480
Ile Ser Phe Pro Glu Leu Tyr Gly Arg Lys Glu His Gly Tyr Asn Glu
145                 150                 155                 160

aat tac aaa aac tat acg gcg tat atg cgg aag gaa gcg cct gaa gac 528
Asn Tyr Lys Asn Tyr Thr Ala Tyr Met Arg Lys Glu Ala Pro Glu Asp
                165                 170                 175

gga ggc ggt aaa gac att aaa gct tct ttc gac gat gag gcc gtt ata 576
Gly Gly Gly Lys Asp Ile Lys Ala Ser Phe Asp Asp Glu Ala Val Ile
            180                 185                 190

gag act gaa gca aag ccg acg gat atc cgc cac gta aaa gaa atc gga 624
Glu Thr Glu Ala Lys Pro Thr Asp Ile Arg His Val Lys Glu Ile Gly
        195                 200                 205

cac atc gat gtg gtc tcc cat att att ggc ggg cgt tcc gtg gac ggc 672
His Ile Asp Val Val Ser His Ile Ile Gly Gly Arg Ser Val Asp Gly
    210                 215                 220

aga cct gca ggc ggt att gcg ccc gat gcg acg cta cac ata atg aat 720
Arg Pro Ala Gly Gly Ile Ala Pro Asp Ala Thr Leu His Ile Met Asn
225                 230                 235                 240

acg cat gat gga acc aag aac gaa ata atg tct gca gcc atc cgc aat 768
Thr His Asp Gly Thr Lys Asn Glu Ile Met Ser Ala Ala Ile Arg Asn
                245                 250                 255

gca tgg gtc aag ctg ggc gaa cgt ggc gtg cgc atc gtc aat aac agt 816
Ala Trp Val Lys Leu Gly Glu Arg Gly Val Arg Ile Val Asn Asn Ser
            260                 265                 270

ttt gga aca aca tcg agg gca ggc act gcc gac cat ttc caa ata gcc 864
Phe Gly Thr Thr Ser Arg Ala Gly Thr Ala Asp His Phe Gln Ile Ala
        275                 280                 285

aat tcg gag gag cag tac cgc caa gcg ttg ctc gcc tat tcc ggc ggt 912
Asn Ser Glu Glu Gln Tyr Arg Gln Ala Leu Leu Ala Tyr Ser Gly Gly
    290                 295                 300

gat aaa aca gac gag ggt atc cgc ctg atg caa cag agc gat tac ggc 960
Asp Lys Thr Asp Glu Gly Ile Arg Leu Met Gln Gln Ser Asp Tyr Gly
305                 310                 315                 320

aac ttg tcc tac cac atc cgt aat aaa aac atg ctt ttc att ttt tcg 1008
Asn Leu Ser Tyr His Ile Arg Asn Lys Asn Met Leu Phe Ile Phe Ser
                325                 330                 335

gca agc aat gac gca caa gct cag ccc aac aca ctg acc cta ttg cca 1056
Ala Ser Asn Asp Ala Gln Ala Gln Pro Asn Thr Leu Thr Leu Leu Pro
            340                 345                 350

ttt tat gaa aaa gat gct caa aaa ggc att atc aca gtc gca ggc gta 1104
Phe Tyr Glu Lys Asp Ala Gln Lys Gly Ile Ile Thr Val Ala Gly Val
        355                 360                 365

gac cgc agt gga gaa aag ttc aat ggc tcc aac cat tgc gga att act 1152
Asp Arg Ser Gly Glu Lys Phe Asn Gly Ser Asn His Cys Gly Ile Thr
    370                 375                 380

gcc atg tgg tgc cta tcg gca ccc tat gaa gca agc gtc cgt ttc acc 1200
Ala Met Trp Cys Leu Ser Ala Pro Tyr Glu Ala Ser Val Arg Phe Thr
385                 390                 395                 400

cgt aca aac ccg att caa att gcc gga aca tcc ttt tcc gca ccc atc 1248
Arg Thr Asn Pro Ile Gln Ile Ala Gly Thr Ser Phe Ser Ala Pro Ile
                405                 410                 415

gta acc ggc acg gcg gct ctg ctg ctg cag aaa tac ccg tgg atg agc 1296
Val Thr Gly Thr Ala Ala Leu Leu Leu Gln Lys Tyr Pro Trp Met Ser
            420                 425                 430

aac gac aac ctg cgt acc acg ctg ctg aca acg gct cag gac atc ggt 1344
Asn Asp Asn Leu Arg Thr Thr Leu Leu Thr Thr Ala Gln Asp Ile Gly
        435                 440                 445

gca gtc ggc gtg gac agc aag ttc ggc tgg gga ctg ctg gat gcg ggt 1392
Ala Val Gly Val Asp Ser Lys Phe Gly Trp Gly Leu Leu Asp Ala Gly
    450                 455                 460

aag gcc atg aac gga ccc gcg tcc ttt ccg ttc ggc gac ttt acc gcc 1440
Lys Ala Met Asn Gly Pro Ala Ser Phe Pro Phe Gly Asp Phe Thr Ala
465                 470                 475                 480

gat acg aaa ggt aca tcc gat att gcc tac tcc ttc cgt aac gac att 1488
Asp Thr Lys Gly Thr Ser Asp Ile Ala Tyr Ser Phe Arg Asn Asp Ile
                485                 490                 495

tca ggc acg ggc ggc ctg atc aaa aaa ggc ggc agc caa ctg caa ctg 1536
Ser Gly Thr Gly Gly Leu Ile Lys Lys Gly Gly Ser Gln Leu Gln Leu
            500                 505                 510

cac ggc aac aac acc tat acg ggc aaa acc att atc gaa ggc ggt tcg 1584
His Gly Asn Asn Thr Tyr Thr Gly Lys Thr Ile Ile Glu Gly Gly Ser
        515                 520                 525

ctg gtg ttg tac ggc aac aac aaa tcg gat atg cgc gtc gaa acc aaa 1632
Leu Val Leu Tyr Gly Asn Asn Lys Ser Asp Met Arg Val Glu Thr Lys
    530                 535                 540

ggt gcg ctg att tat aac ggg gcg gca tcc ggc ggt agc ctg aac agc 1680
Gly Ala Leu Ile Tyr Asn Gly Ala Ala Ser Gly Gly Ser Leu Asn Ser
545                 550                 555                 560

gac ggc att gtc tat ctg gca gat acc gac cga tcc ggc gca aac gaa 1728
Asp Gly Ile Val Tyr Leu Ala Asp Thr Asp Arg Ser Gly Ala Asn Glu
                565                 570                 575

acc gtg cac atc aaa ggc gat ctg cag ctg ggc ggc gaa ggt acg ctg 1776
Thr Val His Ile Lys Gly Asp Leu Gln Leu Gly Gly Glu Gly Thr Leu
            580                 585                 590

tac aca cgt ttg ggc aaa ctg ctg aaa gtg gac ggt acg gcg atg acc 1824
Tyr Thr Arg Leu Gly Lys Leu Leu Lys Val Asp Gly Thr Ala Met Thr
        595                 600                 605

ggc ggc aag ctg tac atg tcg gca cgc ggc aaa ggg gca ggc tat ctc 1872
Gly Gly Lys Leu Tyr Met Ser Ala Arg Gly Lys Gly Ala Gly Tyr Leu
    610                 615                 620

aac cgt acc gga caa cgt gtt ccc ttc ctg agt gcc gcc aaa atc ggg 1920
Asn Arg Thr Gly Gln Arg Val Pro Phe Leu Ser Ala Ala Lys Ile Gly
625                 630                 635                 640

cgg gat tat tct ttc ttc aca aac atc gaa acc gac ggt ggt ctg ctg 1968
Arg Asp Tyr Ser Phe Phe Thr Asn Ile Glu Thr Asp Gly Gly Leu Leu
                645                 650                 655

gct tcc ctc gac agc gtc gaa aaa aca gcg ggc agt gaa ggc gac acg 2016
Ala Ser Leu Asp Ser Val Glu Lys Thr Ala Gly Ser Glu Gly Asp Thr
            660                 665                 670

ctg tcc tat tat gtc cgt cgc ggc aat gcg gca cgg act gct tcg gca 2064
Leu Ser Tyr Tyr Val Arg Arg Gly Asn Ala Ala Arg Thr Ala Ser Ala
        675                 680                 685

gcg gca cat tcc gcg ccc gcc ggt ctg aaa cac gcc gta gaa cag ggc 2112
Ala Ala His Ser Ala Pro Ala Gly Leu Lys His Ala Val Glu Gln Gly
    690                 695                 700

ggc agc aat ctg gaa aac ctg atg gtc gaa ctg gat gcc tcc gaa tca 2160
Gly Ser Asn Leu Glu Asn Leu Met Val Glu Leu Asp Ala Ser Glu Ser
705                 710                 715                 720

tcc gca aca ccc gag acg gtt gaa act gcg gcc gcc gac cgc aca gat 2208
Ser Ala Thr Pro Glu Thr Val Glu Thr Ala Ala Ala Asp Arg Thr Asp
                725                 730                 735

atg ccg ggc atc cgc ccc tac ggc gca act ttc cgc gca gcg gca gcc 2256
Met Pro Gly Ile Arg Pro Tyr Gly Ala Thr Phe Arg Ala Ala Ala Ala
            740                 745                 750

gta cag cat gcg aat gcc gcc gac ggt gta cgc atc ttc aac agt ctc 2304
Val Gln His Ala Asn Ala Ala Asp Gly Val Arg Ile Phe Asn Ser Leu
        755                 760                 765

gcc gct acc gtc tat gcc gac agt acc gcc gcc cat gcc gat atg cag 2352
Ala Ala Thr Val Tyr Ala Asp Ser Thr Ala Ala His Ala Asp Met Gln
    770                 775                 780

gga cgc cgg ctg aaa gcc gta tcg gac ggg ttg gac cac aac gct acg 2400
Gly Arg Arg Leu Lys Ala Val Ser Asp Gly Leu Asp His Asn Ala Thr
785                 790                 795                 800

ggt ctg cgc gtc atc gcg caa acc caa cag gac ggt gga acg tgg gaa 2448
Gly Leu Arg Val Ile Ala Gln Thr Gln Gln Asp Gly Gly Thr Trp Glu
                805                 810                 815

cag ggc ggt gtt gaa ggc aaa atg cgc ggc agt acc caa acc gtc ggc 2496
Gln Gly Gly Val Glu Gly Lys Met Arg Gly Ser Thr Gln Thr Val Gly
            820                 825                 830

att gcc gcg aaa acc ggc gaa aat acg aca gca gcc gcc aca ctg ggc 2544
Ile Ala Ala Lys Thr Gly Glu Asn Thr Thr Ala Ala Ala Thr Leu Gly
        835                 840                 845

atg gga cac agc aca tgg agc gaa aac agt gca aat gca aaa acc gac 2592
Met Gly His Ser Thr Trp Ser Glu Asn Ser Ala Asn Ala Lys Thr Asp
    850                 855                 860

agc att agt ctg ttt gca ggc ata cgg cac gat gcg ggc gat atc ggc 2640
Ser Ile Ser Leu Phe Ala Gly Ile Arg His Asp Ala Gly Asp Ile Gly
865                 870                 875                 880

tat ctc aaa ggc ctg ttc tcc tac gga cgc tac aaa aac agc atc agc 2688
Tyr Leu Lys Gly Leu Phe Ser Tyr Gly Arg Tyr Lys Asn Ser Ile Ser
                885                 890                 895

cgc agc acc ggt gcg gac gaa cat gcg gaa ggc agc gtc aac ggc acg 2736
Arg Ser Thr Gly Ala Asp Glu His Ala Glu Gly Ser Val Asn Gly Thr
            900                 905                 910

ctg atg cag ctg ggc gca ctg ggc ggt gtc aac gtt ccg ttt gcc gca 2784
Leu Met Gln Leu Gly Ala Leu Gly Gly Val Asn Val Pro Phe Ala Ala
        915                 920                 925

acg gga gat ttg acg gtc gaa ggc ggt ctg cgc tac gac ctg ctc aaa 2832
Thr Gly Asp Leu Thr Val Glu Gly Gly Leu Arg Tyr Asp Leu Leu Lys
    930                 935                 940

cag gat gca ttc gcc gaa aaa ggc agt gct ttg ggc tgg agc ggc aac 2880
Gln Asp Ala Phe Ala Glu Lys Gly Ser Ala Leu Gly Trp Ser Gly Asn
945                 950                 955                 960

agc ctc act gaa ggc aca ctg gtc gga ctc gcg ggt ctg aag ctg tcg 2928
Ser Leu Thr Glu Gly Thr Leu Val Gly Leu Ala Gly Leu Lys Leu Ser
                965                 970                 975

caa ccc ttg agc gat aaa gcc gtc ctg ttt gca acg gcg ggc gtg gaa 2976
Gln Pro Leu Ser Asp Lys Ala Val Leu Phe Ala Thr Ala Gly Val Glu
            980                 985                 990

cgc gac ctg aac gga cgc gac tac acg gta acg ggc ggc ttt acc ggc 3024
Arg Asp Leu Asn Gly Arg Asp Tyr Thr Val Thr Gly Gly Phe Thr Gly
        995                 1000                1005

gcg act gca gca acc ggc aag acg ggg gca cgc aat atg ccg cac acc 3072
Ala Thr Ala Ala Thr Gly Lys Thr Gly Ala Arg Asn Met Pro His Thr
    1010                1015                1020

cgc ctg gtt gcc ggt ctg ggc gcg gat gtc gaa ttc ggc aac ggc tgg 3120
Arg Leu Val Ala Gly Leu Gly Ala Asp Val Glu Phe Gly Asn Gly Trp
1025                1030                1035                1040

aac ggc ttg gca cgt tac agc tac gcc ggt tcc aaa cag tac ggc aac 3168
Asn Gly Leu Ala Arg Tyr Ser Tyr Ala Gly Ser Lys Gln Tyr Gly Asn
                1045                1050                1055

cac agc gga cga gtc ggc gta ggc tac cgg ttc tga 3204
His Ser Gly Arg Val Gly Val Gly Tyr Arg Phe
            1060                1065

<210> 8
<211> 1067
<212> PRT
<213> Neisseria meningitidis

<400> 8
Met Arg Thr Thr Pro Thr Phe Pro Thr Lys Thr Phe Lys Pro Ala Ala
1               5                   10                  15

Met Ala Leu Ala Val Ala Thr Thr Leu Ser Ala Cys Leu Gly Gly Gly
            20                  25                  30

Gly Gly Thr Ser Ala Pro Asp Phe Asn Ala Gly Gly Thr Gly Ile Gly
        35                  40                  45

Ser Asn Ser Arg Ala Thr Thr Ala Lys Ser Ala Ala Val Ser Tyr Ala
    50                  55                  60

Gly Ile Lys Asn Glu Met Cys Lys Asp Arg Ser Met Leu Cys Ala Gly
65                  70                  75                  80

Arg Asp Asp Val Ala Val Thr Asp Arg Asp Ala Lys Ile Asn Ala Pro
                85                  90                  95

Pro Pro Asn Leu His Thr Gly Asp Phe Thr Asn Pro Asn Asp Ala Tyr
            100                 105                 110

Lys Asn Leu Ile Asn Leu Lys Pro Ala Ile Glu Ala Gly Tyr Thr Gly
        115                 120                 125

Arg Gly Val Glu Val Gly Ile Val Asp Thr Gly Glu Ser Val Gly Ser
    130                 135                 140

Ile Ser Phe Pro Glu Leu Tyr Gly Arg Lys Glu His Gly Tyr Asn Glu
145                 150                 155                 160

Asn Tyr Lys Asn Tyr Thr Ala Tyr Met Arg Lys Glu Ala Pro Glu Asp
                165                 170                 175

Gly Gly Gly Lys Asp Ile Lys Ala Ser Phe Asp Asp Glu Ala Val Ile
            180                 185                 190

Glu Thr Glu Ala Lys Pro Thr Asp Ile Arg His Val Lys Glu Ile Gly
        195                 200                 205

His Ile Asp Val Val Ser His Ile Ile Gly Gly Arg Ser Val Asp Gly
    210                 215                 220

Arg Pro Ala Gly Gly Ile Ala Pro Asp Ala Thr Leu His Ile Met Asn
225                 230                 235                 240

Thr His Asp Gly Thr Lys Asn Glu Ile Met Ser Ala Ala Ile Arg Asn
                245                 250                 255

Ala Trp Val Lys Leu Gly Glu Arg Gly Val Arg Ile Val Asn Asn Ser
            260                 265                 270

Phe Gly Thr Thr Ser Arg Ala Gly Thr Ala Asp His Phe Gln Ile Ala
        275                 280                 285

Asn Ser Glu Glu Gln Tyr Arg Gln Ala Leu Leu Ala Tyr Ser Gly Gly
    290                 295                 300

Asp Lys Thr Asp Glu Gly Ile Arg Leu Met Gln Gln Ser Asp Tyr Gly
305                 310                 315                 320

Asn Leu Ser Tyr His Ile Arg Asn Lys Asn Met Leu Phe Ile Phe Ser
                325                 330                 335

Ala Ser Asn Asp Ala Gln Ala Gln Pro Asn Thr Leu Thr Leu Leu Pro
            340                 345                 350

Phe Tyr Glu Lys Asp Ala Gln Lys Gly Ile Ile Thr Val Ala Gly Val
        355                 360                 365

Asp Arg Ser Gly Glu Lys Phe Asn Gly Ser Asn His Cys Gly Ile Thr
    370                 375                 380

Ala Met Trp Cys Leu Ser Ala Pro Tyr Glu Ala Ser Val Arg Phe Thr
385                 390                 395                 400

Arg Thr Asn Pro Ile Gln Ile Ala Gly Thr Ser Phe Ser Ala Pro Ile
                405                 410                 415

Val Thr Gly Thr Ala Ala Leu Leu Leu Gln Lys Tyr Pro Trp Met Ser
            420                 425                 430

Asn Asp Asn Leu Arg Thr Thr Leu Leu Thr Thr Ala Gln Asp Ile Gly
        435                 440                 445

Ala Val Gly Val Asp Ser Lys Phe Gly Trp Gly Leu Leu Asp Ala Gly
    450                 455                 460

Lys Ala Met Asn Gly Pro Ala Ser Phe Pro Phe Gly Asp Phe Thr Ala
465                 470                 475                 480

Asp Thr Lys Gly Thr Ser Asp Ile Ala Tyr Ser Phe Arg Asn Asp Ile
                485                 490                 495

Ser Gly Thr Gly Gly Leu Ile Lys Lys Gly Gly Ser Gln Leu Gln Leu
            500                 505                 510

His Gly Asn Asn Thr Tyr Thr Gly Lys Thr Ile Ile Glu Gly Gly Ser
        515                 520                 525

Leu Val Leu Tyr Gly Asn Asn Lys Ser Asp Met Arg Val Glu Thr Lys
    530                 535                 540

Gly Ala Leu Ile Tyr Asn Gly Ala Ala Ser Gly Gly Ser Leu Asn Ser
545                 550                 555                 560

Asp Gly Ile Val Tyr Leu Ala Asp Thr Asp Arg Ser Gly Ala Asn Glu
                565                 570                 575

Thr Val His Ile Lys Gly Asp Leu Gln Leu Gly Gly Glu Gly Thr Leu
            580                 585                 590

Tyr Thr Arg Leu Gly Lys Leu Leu Lys Val Asp Gly Thr Ala Met Thr
        595                 600                 605

Gly Gly Lys Leu Tyr Met Ser Ala Arg Gly Lys Gly Ala Gly Tyr Leu
    610                 615                 620

Asn Arg Thr Gly Gln Arg Val Pro Phe Leu Ser Ala Ala Lys Ile Gly
625                 630                 635                 640

Arg Asp Tyr Ser Phe Phe Thr Asn Ile Glu Thr Asp Gly Gly Leu Leu
                645                 650                 655

Ala Ser Leu Asp Ser Val Glu Lys Thr Ala Gly Ser Glu Gly Asp Thr
            660                 665                 670

Leu Ser Tyr Tyr Val Arg Arg Gly Asn Ala Ala Arg Thr Ala Ser Ala
        675                 680                 685

Ala Ala His Ser Ala Pro Ala Gly Leu Lys His Ala Val Glu Gln Gly
    690                 695                 700

Gly Ser Asn Leu Glu Asn Leu Met Val Glu Leu Asp Ala Ser Glu Ser
705                 710                 715                 720

Ser Ala Thr Pro Glu Thr Val Glu Thr Ala Ala Ala Asp Arg Thr Asp
                725                 730                 735

Met Pro Gly Ile Arg Pro Tyr Gly Ala Thr Phe Arg Ala Ala Ala Ala
            740                 745                 750

Val Gln His Ala Asn Ala Ala Asp Gly Val Arg Ile Phe Asn Ser Leu
        755                 760                 765

Ala Ala Thr Val Tyr Ala Asp Ser Thr Ala Ala His Ala Asp Met Gln
    770                 775                 780

Gly Arg Arg Leu Lys Ala Val Ser Asp Gly Leu Asp His Asn Ala Thr
785                 790                 795                 800

Gly Leu Arg Val Ile Ala Gln Thr Gln Gln Asp Gly Gly Thr Trp Glu
                805                 810                 815

Gln Gly Gly Val Glu Gly Lys Met Arg Gly Ser Thr Gln Thr Val Gly
            820                 825                 830

Ile Ala Ala Lys Thr Gly Glu Asn Thr Thr Ala Ala Ala Thr Leu Gly
        835                 840                 845

Met Gly His Ser Thr Trp Ser Glu Asn Ser Ala Asn Ala Lys Thr Asp
    850                 855                 860

Ser Ile Ser Leu Phe Ala Gly Ile Arg His Asp Ala Gly Asp Ile Gly
865                 870                 875                 880

Tyr Leu Lys Gly Leu Phe Ser Tyr Gly Arg Tyr Lys Asn Ser Ile Ser
                885                 890                 895

Arg Ser Thr Gly Ala Asp Glu His Ala Glu Gly Ser Val Asn Gly Thr
            900                 905                 910

Leu Met Gln Leu Gly Ala Leu Gly Gly Val Asn Val Pro Phe Ala Ala
        915                 920                 925

Thr Gly Asp Leu Thr Val Glu Gly Gly Leu Arg Tyr Asp Leu Leu Lys
    930                 935                 940

Gln Asp Ala Phe Ala Glu Lys Gly Ser Ala Leu Gly Trp Ser Gly Asn
945                 950                 955                 960

Ser Leu Thr Glu Gly Thr Leu Val Gly Leu Ala Gly Leu Lys Leu Ser
                965                 970                 975

Gln Pro Leu Ser Asp Lys Ala Val Leu Phe Ala Thr Ala Gly Val Glu
            980                 985                 990

Arg Asp Leu Asn Gly Arg Asp Tyr Thr Val Thr Gly Gly Phe Thr Gly
        995                 1000                1005

Ala Thr Ala Ala Thr Gly Lys Thr Gly Ala Arg Asn Met Pro His Thr
    1010                1015                1020

Arg Leu Val Ala Gly Leu Gly Ala Asp Val Glu Phe Gly Asn Gly Trp
025 1030 1035 1040

Asn Gly Leu Ala Arg Tyr Ser Tyr Ala Gly Ser Lys Gln Tyr Gly Asn
                1045                1050                1055

His Ser Gly Arg Val Gly Val Gly Tyr Arg Phe
            1060                1065

<210> 9
<211> 339
<212> ADN
<213> Neisseria meningitidis

<220>
<221> CDS
<222> (1)..(336)

<400> 9
atg aac aca cgc atc atc gtt tcg gct gcg ttc gtt gcg ttg gca tta 48
Met Asn Thr Arg Ile Ile Val Ser Ala Ala Phe Val Ala Leu Ala Leu
1               5                   10                  15

gca ggt tgc ggc tca atc aat aat gta acc gtt tcc gac cag aaa ctt 96
Ala Gly Cys Gly Ser Ile Asn Asn Val Thr Val Ser Asp Gln Lys Leu
            20                  25                  30

cag gaa cgt gcc gcg ttt gcc ttg ggc gtc agc caa aat gcc gta aaa 144
Gln Glu Arg Ala Ala Phe Ala Leu Gly Val Ser Gln Asn Ala Val Lys
        35                  40                  45

atc agc aac cgc agc aat gaa agc ata cgc atc aac ttt acc gca act 192
Ile Ser Asn Arg Ser Asn Glu Ser Ile Arg Ile Asn Phe Thr Ala Thr
    50                  55                  60

gtg ggt aag cgc gtg agc caa tgc tat gtt acc agt gta atc agc aca 240
Val Gly Lys Arg Val Ser Gln Cys Tyr Val Thr Ser Val Ile Ser Thr
65                  70                  75                  80

atc ggc gtt acc act tcc gat gca att tgt ttg gga ggc gga acg cac 288
Ile Gly Val Thr Thr Ser Asp Ala Ile Cys Leu Gly Gly Gly Thr His
                85                  90                  95

aaa ggc aaa agt caa tgc aat gct ttg ctt aaa gcg gca ggc cgt tgc 336
Lys Gly Lys Ser Gln Cys Asn Ala Leu Leu Lys Ala Ala Gly Arg Cys
            100                 105                 110

taa 339


<210> 10
<211> 112
<212> PRT
<213> Neisseria meningitidis

<400> 10
Met Asn Thr Arg Ile Ile Val Ser Ala Ala Phe Val Ala Leu Ala Leu
1               5                   10                  15

Ala Gly Cys Gly Ser Ile Asn Asn Val Thr Val Ser Asp Gln Lys Leu
            20                  25                  30

Gln Glu Arg Ala Ala Phe Ala Leu Gly Val Ser Gln Asn Ala Val Lys
        35                  40                  45

Ile Ser Asn Arg Ser Asn Glu Ser Ile Arg Ile Asn Phe Thr Ala Thr

50                  55                  60

Val Gly Lys Arg Val Ser Gln Cys Tyr Val Thr Ser Val Ile Ser Thr
65                  70                  75                  80

Ile Gly Val Thr Thr Ser Asp Ala Ile Cys Leu Gly Gly Gly Thr His
                85                  90                  95

Lys Gly Lys Ser Gln Cys Asn Ala Leu Leu Lys Ala Ala Gly Arg Cys
            100                 105                 110

<210> 11
<211> 1314
<212> ADN
<213> Neisseria meningitidis

<220>
<221> CDS
<222> (1)..(1311)

<400> 11
atg ctg acg ttt atc gga ctg ctg att atc ggg gtc atc gta tgg ctg      48
Met Leu Thr Phe Ile Gly Leu Leu Ile Ile Gly Val Ile Val Trp Leu
1               5                   10                  15

ctg ctg acg gaa aaa gtg tcg ccc atc atc gca tta atc ttg gtg ccg      96
Leu Leu Thr Glu Lys Val Ser Pro Ile Ile Ala Leu Ile Leu Val Pro
            20                  25                  30

ctg ttt ggg gcg ttg ctg gcg ggg ttt gat gta tcc caa tta aaa gaa      144
Leu Phe Gly Ala Leu Leu Ala Gly Phe Asp Val Ser Gln Leu Lys Glu
        35                  40                  45

ttt tat tcg ggc ggc acc aaa tcg gtg atg cag att gtg att atg ttt      192
Phe Tyr Ser Gly Gly Thr Lys Ser Val Met Gln Ile Val Ile Met Phe
    50                  55                  60

atg ttt tcc att ttg ttt ttt gga atc atg aac gat gtg ggg ctg ttc      240
Met Phe Ser Ile Leu Phe Phe Gly Ile Met Asn Asp Val Gly Leu Phe
65                  70                  75                  80

cgt ccg atg ata ggc ggt ttg att aag ctg act cgg ggt aat atc gtg      288
Arg Pro Met Ile Gly Gly Leu Ile Lys Leu Thr Arg Gly Asn Ile Val
                85                  90                  95

gca gtg agt gtg ggg acg gtc ttg gtg tcg gtg gtg gcg cag ttg gac      336
Ala Val Ser Val Gly Thr Val Leu Val Ser Val Val Ala Gln Leu Asp
            100                 105                 110

ggg gcg ggt gcg acg acg ttt tta ttg gtc gtc ccc gcc ctt ttg ccg      384
Gly Ala Gly Ala Thr Thr Phe Leu Leu Val Val Pro Ala Leu Leu Pro
        115                 120                 125

ctt tac aag cgt ctg cat atg aat cct tac ctg ctg ttt ttg ctg ctg      432
Leu Tyr Lys Arg Leu His Met Asn Pro Tyr Leu Leu Phe Leu Leu Leu
    130                 135                 140

act tcc agt gcg gga ttg att aac ctt ctg ccg tgg ggc ggg ccg acc      480

Thr Ser Ser Ala Gly Leu Ile Asn Leu Leu Pro Trp Gly Gly Pro Thr
145                 150                 155                 160

ggg cgg gtt gca agc gtg ttg ggc gca gat gtg ggc gaa ttg tat aaa     528
Gly Arg Val Ala Ser Val Leu Gly Ala Asp Val Gly Glu Leu Tyr Lys
                165                 170                 175

cct ttg ttg acg gtg caa att atc ggt gtg gtg ttt atc ctt gcg ctg     576
Pro Leu Leu Thr Val Gln Ile Ile Gly Val Val Phe Ile Leu Ala Leu
            180                 185                 190

tcc ctg ctt ttg ggt gtg cgt gaa aaa agg cgg att gtc cgg gag ttg     624
Ser Leu Leu Leu Gly Val Arg Glu Lys Arg Arg Ile Val Arg Glu Leu
        195                 200                 205

ggc gcg ttg ccc gcc gtg gcg gat ttg ata aag ccg gtg cct ttg tcg     672
Gly Ala Leu Pro Ala Val Ala Asp Leu Ile Lys Pro Val Pro Leu Ser
    210                 215                 220

gaa gaa gaa caa aaa ttg gcg cgt ccg aaa ctg ttt tgg tgg aat gtc     720
Glu Glu Glu Gln Lys Leu Ala Arg Pro Lys Leu Phe Trp Trp Asn Val
225                 230                 235                 240

ctg ctg ttt ttg gcg gcg atg agc ctg ctt ttt tcg ggc atc ttc ccg     768
Leu Leu Phe Leu Ala Ala Met Ser Leu Leu Phe Ser Gly Ile Phe Pro
                245                 250                 255

ccg ggt tat gta ttt atg ctg gct gca acg gcg gcg ttg ctt ttg aat     816
Pro Gly Tyr Val Phe Met Leu Ala Ala Thr Ala Ala Leu Leu Leu Asn
            260                 265                 270

tac cgc agc ccg cag gaa cag atg gag cgg att tat gcc cac gcc ggc     864
Tyr Arg Ser Pro Gln Glu Gln Met Glu Arg Ile Tyr Ala His Ala Gly
        275                 280                 285

ggc gcg gtg atg atg gcg tcc att att ttg gcg gca ggt acg ttt ttg     912
Gly Ala Val Met Met Ala Ser Ile Ile Leu Ala Ala Gly Thr Phe Leu
    290                 295                 300

ggg att ttg aag ggt gcg ggg atg ttg gac gcg att tcc aaa gac att     960
Gly Ile Leu Lys Gly Ala Gly Met Leu Asp Ala Ile Ser Lys Asp Ile
305                 310                 315                 320

gtg cat atc ctg ccg gac gcg ctg ctg cct tat ctg cat att gcc atc     1008
Val His Ile Leu Pro Asp Ala Leu Leu Pro Tyr Leu His Ile Ala Ile
                325                 330                 335

ggt gtg ttg ggc att ccg ctt gag ttg gtt ttg agt acg gac gct tat     1056
Gly Val Leu Gly Ile Pro Leu Glu Leu Val Leu Ser Thr Asp Ala Tyr
            340                 345                 350

tat ttc gga ctg ttt ccg att gtg gag cag att acc tcg cag gcg ggc     1104
Tyr Phe Gly Leu Phe Pro Ile Val Glu Gln Ile Thr Ser Gln Ala Gly
        355                 360                 365

gtg gcg ccc gaa gca gca ggt tat gcg atg ttg atc ggc agt atc gtc     1152
Val Ala Pro Glu Ala Ala Gly Tyr Ala Met Leu Ile Gly Ser Ile Val
    370                 375                 380

ggc act ttt gtt acg ccg ctt tcg ccg gct ttg tgg atg ggc ttg ggt     1200

Gly Thr Phe Val Thr Pro Leu Ser Pro Ala Leu Trp Met Gly Leu Gly
385                 390                 395                 400

ttg gcg aaa ttg tcg atg ggc aaa cac atc cgt tat tcg ttt ttt tgg     1248
Leu Ala Lys Leu Ser Met Gly Lys His Ile Arg Tyr Ser Phe Phe Trp
                405                 410                 415

gcg tgg ggt ttg tcg ctg gcg ata ttg gcc agt tcg ata gcg gca gga     1296
Ala Trp Gly Leu Ser Leu Ala Ile Leu Ala Ser Ser Ile Ala Ala Gly
            420                 425                 430

atc gtg cct ctg ccg taa                                             1314
Ile Val Pro Leu Pro
        435

<210> 12
<211> 437
<212> PRT
<213> Neisseria meningitidis

<400> 12
Met Leu Thr Phe Ile Gly Leu Leu Ile Ile Gly Val Ile Val Trp Leu
  1               5                  10                  15

Leu Leu Thr Glu Lys Val Ser Pro Ile Ile Ala Leu Ile Leu Val Pro
             20                  25                  30

Leu Phe Gly Ala Leu Leu Ala Gly Phe Asp Val Ser Gln Leu Lys Glu
         35                  40                  45

Phe Tyr Ser Gly Gly Thr Lys Ser Val Met Gln Ile Val Ile Met Phe
     50                  55                  60

Met Phe Ser Ile Leu Phe Phe Gly Ile Met Asn Asp Val Gly Leu Phe
 65                  70                  75                  80

Arg Pro Met Ile Gly Gly Leu Ile Lys Leu Thr Arg Gly Asn Ile Val
                 85                  90                  95

Ala Val Ser Val Gly Thr Val Leu Val Ser Val Val Ala Gln Leu Asp
            100                 105                 110

Gly Ala Gly Ala Thr Thr Phe Leu Leu Val Val Pro Ala Leu Leu Pro
        115                 120                 125

Leu Tyr Lys Arg Leu His Met Asn Pro Tyr Leu Leu Phe Leu Leu Leu
    130                 135                 140

Thr Ser Ser Ala Gly Leu Ile Asn Leu Leu Pro Trp Gly Gly Pro Thr
145                 150                 155                 160

Gly Arg Val Ala Ser Val Leu Gly Ala Asp Val Gly Glu Leu Tyr Lys
                165                 170                 175

Pro Leu Leu Thr Val Gln Ile Ile Gly Val Val Phe Ile Leu Ala Leu
            180                 185                 190

Ser Leu Leu Leu Gly Val Arg Glu Lys Arg Arg Ile Val Arg Glu Leu
        195                 200                 205

Gly Ala Leu Pro Ala Val Ala Asp Leu Ile Lys Pro Val Pro Leu Ser
    210                 215                 220

Glu Glu Glu Gln Lys Leu Ala Arg Pro Lys Leu Phe Trp Trp Asn Val
225                 230                 235                 240

Leu Leu Phe Leu Ala Ala Met Ser Leu Leu Phe Ser Gly Ile Phe Pro
                245                 250                 255

Pro Gly Tyr Val Phe Met Leu Ala Ala Thr Ala Ala Leu Leu Leu Asn
            260                 265                 270

Tyr Arg Ser Pro Gln Glu Gln Met Glu Arg Ile Tyr Ala His Ala Gly
        275                 280                 285

Gly Ala Val Met Met Ala Ser Ile Ile Leu Ala Ala Gly Thr Phe Leu
    290                 295                 300

Gly Ile Leu Lys Gly Ala Gly Met Leu Asp Ala Ile Ser Lys Asp Ile
305                 310                 315                 320

Val His Ile Leu Pro Asp Ala Leu Leu Pro Tyr Leu His Ile Ala Ile
                325                 330                 335

Gly Val Leu Gly Ile Pro Leu Glu Leu Val Leu Ser Thr Asp Ala Tyr
            340                 345                 350

Tyr Phe Gly Leu Phe Pro Ile Val Glu Gln Ile Thr Ser Gln Ala Gly
        355                 360                 365

Val Ala Pro Glu Ala Ala Gly Tyr Ala Met Leu Ile Gly Ser Ile Val
    370                 375                 380

Gly Thr Phe Val Thr Pro Leu Ser Pro Ala Leu Trp Met Gly Leu Gly
385                 390                 395                 400

Leu Ala Lys Leu Ser Met Gly Lys His Ile Arg Tyr Ser Phe Phe Trp
                405                 410                 415

Ala Trp Gly Leu Ser Leu Ala Ile Leu Ala Ser Ser Ile Ala Ala Gly
            420                 425                 430

Ile Val Pro Leu Pro
        435

<210> 13
<211> 1155
<212> ADN
<213> Neisseria meningitidis

<220>
<221> CDS
<222> (1)..(1152)

<400> 13
atg ggc atc cat ctc gac ttc ggc att agt cct aaa acg ttc cga cag      48
Met Gly Ile His Leu Asp Phe Gly Ile Ser Pro Lys Thr Phe Arg Gln

1                 5                   10                  15

act tat ctg tat caa aag ccc aag ctc ttt aaa gga gcg gtt cgg aat     96
Thr Tyr Leu Tyr Gln Lys Pro Lys Leu Phe Lys Gly Ala Val Arg Asn
                20                  25                  30

ctc gaa gcc gca tct tgt aaa tat atc aac gag ata tac caa cga gca     144
Leu Glu Ala Ala Ser Cys Lys Tyr Ile Asn Glu Ile Tyr Gln Arg Ala
            35                  40                  45

gac cca acc gca ccg ctg ttt cat ctg cgt aaa aaa ggc gca atc gtt     192
Asp Pro Thr Ala Pro Leu Phe His Leu Arg Lys Lys Gly Ala Ile Val
        50                  55                  60

cct aaa gaa gaa tac gtc gaa agt ttc gac gat ttg ggc aaa act cgc     240
Pro Lys Glu Glu Tyr Val Glu Ser Phe Asp Asp Leu Gly Lys Thr Arg
 65                  70                  75                  80

tac cgt ttt att aaa tcc gtt atc tac gaa cat atg aag aat ggt gcg     288
Tyr Arg Phe Ile Lys Ser Val Ile Tyr Glu His Met Lys Asn Gly Ala
                    85                  90                  95

tcg tta gtc tat aac cat att aac aac gag ccg ttt tca gac cat atc     336
Ser Leu Val Tyr Asn His Ile Asn Asn Glu Pro Phe Ser Asp His Ile
                100                 105                 110

gcc cgt caa gtc gcc cgc ttt gcc ggc gca cat act att gtt agt gga     384
Ala Arg Gln Val Ala Arg Phe Ala Gly Ala His Thr Ile Val Ser Gly
            115                 120                 125

tat ctt gct ttt ggc agc gac gaa tct tat aaa aac cat tgg gat acc     432
Tyr Leu Ala Phe Gly Ser Asp Glu Ser Tyr Lys Asn His Trp Asp Thr
    130                 135                 140

cgc gat gtg tat gcc atc cag ctt ttc ggc aag aaa cgt tgg caa ctt     480
Arg Asp Val Tyr Ala Ile Gln Leu Phe Gly Lys Lys Arg Trp Gln Leu
145                 150                 155                 160

act gcc cct gat ttc cct atg cca ttg tat atg caa cag act aaa gat     528
Thr Ala Pro Asp Phe Pro Met Pro Leu Tyr Met Gln Gln Thr Lys Asp
                165                 170                 175

act gat att tcc att cct gaa cat atc gat atg gat att atc ctt gaa     576
Thr Asp Ile Ser Ile Pro Glu His Ile Asp Met Asp Ile Ile Leu Glu
                180                 185                 190

gca ggt gat gtc ctc tac atc cca cgc ggt tgg tgg cac aga cct atc     624
Ala Gly Asp Val Leu Tyr Ile Pro Arg Gly Trp Trp His Arg Pro Ile
            195                 200                 205

ccg ctc ggc tgt gaa acc ttc cac ttc gct gtc ggt acc ttc ccg ccc     672
Pro Leu Gly Cys Glu Thr Phe His Phe Ala Val Gly Thr Phe Pro Pro
    210                 215                 220

aac ggc tat aat tac ctc gag tgg cta atg aag aaa ttc ccc acg ata     720
Asn Gly Tyr Asn Tyr Leu Glu Trp Leu Met Lys Lys Phe Pro Thr Ile
225                 230                 235                 240

gaa agt ctg cgc cac agt ttc tca gac tgg gag caa gat agg acg cgt     768
Glu Ser Leu Arg His Ser Phe Ser Asp Trp Glu Gln Asp Arg Thr Arg

                245                 250                 255

atc aac gat act gcc gca caa att gct gcc atg att gcc gac ccc gtc 816
Ile Asn Asp Thr Ala Ala Gln Ile Ala Ala Met Ile Ala Asp Pro Val
            260                 265                 270

aat tac gaa gcc ttc agt gaa gac ttc ctc ggc aaa gaa cgc acc gat 864
Asn Tyr Glu Ala Phe Ser Glu Asp Phe Leu Gly Lys Glu Arg Thr Asp
        275                 280                 285

acc gct ttt cat ctc gaa cag ttc gcg aat ccc aac gct act ccg ctt 912
Thr Ala Phe His Leu Glu Gln Phe Ala Asn Pro Asn Ala Thr Pro Leu
    290                 295                 300

tca gac gac gtc agg ttg aga cta aat gcc aat aat ttg gat acg ttg 960
Ser Asp Asp Val Arg Leu Arg Leu Asn Ala Asn Asn Leu Asp Thr Leu
305                 310                 315                 320

gaa aag gga tat ttg att ggg aat ggg atg aag ata agc gta gat gaa 1008
Glu Lys Gly Tyr Leu Ile Gly Asn Gly Met Lys Ile Ser Val Asp Glu
                325                 330                 335

ttg ggg aaa aaa gtg tta gaa cac atc ggt aag aat gaa ccg tta ttg 1056
Leu Gly Lys Lys Val Leu Glu His Ile Gly Lys Asn Glu Pro Leu Leu
            340                 345                 350

ttg aaa aat cta ctg gtt aac ttc aat cag gga aaa cat gaa gaa gtt 1104
Leu Lys Asn Leu Leu Val Asn Phe Asn Gln Gly Lys His Glu Glu Val
        355                 360                 365

agg aag ttg att tat cag ttg ata gag tta gat ttt ctg gaa ctt ttg 1152
Arg Lys Leu Ile Tyr Gln Leu Ile Glu Leu Asp Phe Leu Glu Leu Leu
    370                 375                 380

tga 1155

<210> 14
<211> 384
<212> PRT
<213> Neisseria meningitidis

<400> 14
Met Gly Ile His Leu Asp Phe Gly Ile Ser Pro Lys Thr Phe Arg Gln
1               5                   10                  15

Thr Tyr Leu Tyr Gln Lys Pro Lys Leu Phe Lys Gly Ala Val Arg Asn
            20                  25                  30

Leu Glu Ala Ala Ser Cys Lys Tyr Ile Asn Glu Ile Tyr Gln Arg Ala
        35                  40                  45

Asp Pro Thr Ala Pro Leu Phe His Leu Arg Lys Lys Gly Ala Ile Val
    50                  55                  60

Pro Lys Glu Glu Tyr Val Glu Ser Phe Asp Asp Leu Gly Lys Thr Arg
65                  70                  75                  80

Tyr Arg Phe Ile Lys Ser Val Ile Tyr Glu His Met Lys Asn Gly Ala
                85                  90                  95

Ser Leu Val Tyr Asn His Ile Asn Asn Glu Pro Phe Ser Asp His Ile
            100                 105                 110

Ala Arg Gln Val Ala Arg Phe Ala Gly Ala His Thr Ile Val Ser Gly
        115                 120                 125

Tyr Leu Ala Phe Gly Ser Asp Glu Ser Tyr Lys Asn His Trp Asp Thr
    130                 135                 140

Arg Asp Val Tyr Ala Ile Gln Leu Phe Gly Lys Lys Arg Trp Gln Leu
145                 150                 155                 160

Thr Ala Pro Asp Phe Pro Met Pro Leu Tyr Met Gln Gln Thr Lys Asp
                165                 170                 175

Thr Asp Ile Ser Ile Pro Glu His Ile Asp Met Asp Ile Ile Leu Glu
            180                 185                 190

Ala Gly Asp Val Leu Tyr Ile Pro Arg Gly Trp Trp His Arg Pro Ile
        195                 200                 205

Pro Leu Gly Cys Glu Thr Phe His Phe Ala Val Gly Thr Phe Pro Pro
    210                 215                 220

Asn Gly Tyr Asn Tyr Leu Glu Trp Leu Met Lys Lys Phe Pro Thr Ile
225                 230                 235                 240

Glu Ser Leu Arg His Ser Phe Ser Asp Trp Glu Gln Asp Arg Thr Arg
                245                 250                 255

Ile Asn Asp Thr Ala Ala Gln Ile Ala Ala Met Ile Ala Asp Pro Val
            260                 265                 270

Asn Tyr Glu Ala Phe Ser Glu Asp Phe Leu Gly Lys Glu Arg Thr Asp
        275                 280                 285

Thr Ala Phe His Leu Glu Gln Phe Ala Asn Pro Asn Ala Thr Pro Leu
    290                 295                 300

Ser Asp Asp Val Arg Leu Arg Leu Asn Ala Asn Asn Leu Asp Thr Leu
305                 310                 315                 320

Glu Lys Gly Tyr Leu Ile Gly Asn Gly Met Lys Ile Ser Val Asp Glu
                325                 330                 335

Leu Gly Lys Lys Val Leu Glu His Ile Gly Lys Asn Glu Pro Leu Leu
            340                 345                 350

Leu Lys Asn Leu Leu Val Asn Phe Asn Gln Gly Lys His Glu Glu Val
        355                 360                 365

Arg Lys Leu Ile Tyr Gln Leu Ile Glu Leu Asp Phe Leu Glu Leu Leu
    370                 375                 380

<210> 15
<211> 717
<212> ADN

<213> Neisseria meningitidis

<220>
<221> CDS
<222> (1)..(714)

<400> 15
atg aat aga ccc aag caa ccc ttc ttc cgt ccc gaa gtc gcc gtt gcc     48
Met Asn Arg Pro Lys Gln Pro Phe Phe Arg Pro Glu Val Ala Val Ala
1               5                   10                  15

cgc caa acc agc ctg acg ggt aaa gtg att ctg aca cga ccg ttg tca     96
Arg Gln Thr Ser Leu Thr Gly Lys Val Ile Leu Thr Arg Pro Leu Ser
            20                  25                  30

ttt tcc cta tgg acg aca ttt gca tcg ata tct gcg tta ttg att atc     144
Phe Ser Leu Trp Thr Thr Phe Ala Ser Ile Ser Ala Leu Leu Ile Ile
        35                  40                  45

ctg ttt ttg ata ttt ggt aac tat acg cga aag aca aca gtg gag gga     192
Leu Phe Leu Ile Phe Gly Asn Tyr Thr Arg Lys Thr Thr Val Glu Gly
    50                  55                  60

caa att tta cct gca tcg ggc gta atc agg gtg tat gca ccg gat acg     240
Gln Ile Leu Pro Ala Ser Gly Val Ile Arg Val Tyr Ala Pro Asp Thr
65                  70                  75                  80

ggg aca att aca gcg aaa ttc gtg gaa gat gga gaa aag gtt aag gct     288
Gly Thr Ile Thr Ala Lys Phe Val Glu Asp Gly Glu Lys Val Lys Ala
                85                  90                  95

ggc gac aag cta ttt gcg ctt tcg acc tca cgt ttc ggc gca gga gat     336
Gly Asp Lys Leu Phe Ala Leu Ser Thr Ser Arg Phe Gly Ala Gly Asp
            100                 105                 110

agc gtg cag cag cag ttg aaa acg gag gca gtt ttg aag aaa acg ttg     384
Ser Val Gln Gln Gln Leu Lys Thr Glu Ala Val Leu Lys Lys Thr Leu
        115                 120                 125

gca gaa cag gaa ctg ggt cgt ctg aag ctg ata cac ggg aat gaa acg     432
Ala Glu Gln Glu Leu Gly Arg Leu Lys Leu Ile His Gly Asn Glu Thr
    130                 135                 140

cgc agc ctt aaa gca act gtc gaa cgt ttg gaa aac cag aaa ctc cat     480
Arg Ser Leu Lys Ala Thr Val Glu Arg Leu Glu Asn Gln Lys Leu His
145                 150                 155                 160

att tcg caa cag ata gac ggt cag aaa agg cgc att aga ctt gcg gaa     528
Ile Ser Gln Gln Ile Asp Gly Gln Lys Arg Arg Ile Arg Leu Ala Glu
                165                 170                 175

gaa atg ttg cag aaa tat cgt ttc cta tcc gcc aat gat gca gtg cca     576
Glu Met Leu Gln Lys Tyr Arg Phe Leu Ser Ala Asn Asp Ala Val Pro
            180                 185                 190

aaa caa gaa atg atg aat gtc aag gca gag ctt tta gag cag aaa gcc     624
Lys Gln Glu Met Met Asn Val Lys Ala Glu Leu Leu Glu Gln Lys Ala
        195                 200                 205

aaa ctt gat gcc tac cgc cga gaa gaa gtc ggg ctg ctt cag gaa atc     672

Lys Leu Asp Ala Tyr Arg Arg Glu Glu Val Gly Leu Leu Gln Glu Ile
    210                 215                 220

cgc acg cag aat ctg aca ttg gcc agc ctc ccc caa gcg gca tga        717
Arg Thr Gln Asn Leu Thr Leu Ala Ser Leu Pro Gln Ala Ala
225                 230                 235


<210> 16
<211> 238
<212> PRT
<213> Neisseria meningitidis

<400> 16
Met Asn Arg Pro Lys Gln Pro Phe Phe Arg Pro Glu Val Ala Val Ala
  1               5                  10                  15

Arg Gln Thr Ser Leu Thr Gly Lys Val Ile Leu Thr Arg Pro Leu Ser
             20                  25                  30

Phe Ser Leu Trp Thr Thr Phe Ala Ser Ile Ser Ala Leu Leu Ile Ile
         35                  40                  45

Leu Phe Leu Ile Phe Gly Asn Tyr Thr Arg Lys Thr Thr Val Glu Gly
     50                  55                  60

Gln Ile Leu Pro Ala Ser Gly Val Ile Arg Val Tyr Ala Pro Asp Thr
 65                  70                  75                  80

Gly Thr Ile Thr Ala Lys Phe Val Glu Asp Gly Glu Lys Val Lys Ala
                 85                  90                  95

Gly Asp Lys Leu Phe Ala Leu Ser Thr Ser Arg Phe Gly Ala Gly Asp
            100                 105                 110

Ser Val Gln Gln Gln Leu Lys Thr Glu Ala Val Leu Lys Lys Thr Leu
        115                 120                 125

Ala Glu Gln Glu Leu Gly Arg Leu Lys Leu Ile His Gly Asn Glu Thr
    130                 135                 140

Arg Ser Leu Lys Ala Thr Val Glu Arg Leu Glu Asn Gln Lys Leu His
145                 150                 155                 160

Ile Ser Gln Gln Ile Asp Gly Gln Lys Arg Arg Ile Arg Leu Ala Glu
                165                 170                 175

Glu Met Leu Gln Lys Tyr Arg Phe Leu Ser Ala Asn Asp Ala Val Pro
            180                 185                 190

Lys Gln Glu Met Met Asn Val Lys Ala Glu Leu Leu Glu Gln Lys Ala
        195                 200                 205

Lys Leu Asp Ala Tyr Arg Arg Glu Glu Val Gly Leu Leu Gln Glu Ile
    210                 215                 220

Arg Thr Gln Asn Leu Thr Leu Ala Ser Leu Pro Gln Ala Ala
225                 230                 235

<210> 17
<211> 690
<212> ADN
<213> Neisseria meningitidis

<220>
<221> CDS
<222> (1)..(687)

<400> 17
atg atg aat gtc gag gca gag ctt tta gag cag aaa gcc aaa ctt gat    48
Met Met Asn Val Glu Ala Glu Leu Leu Glu Gln Lys Ala Lys Leu Asp
1               5                   10                  15

gcc tac ggc cga gaa gaa gcc ggg ctg ctt cag gaa atc cgc acg cag    96
Ala Tyr Gly Arg Glu Glu Ala Gly Leu Leu Gln Glu Ile Arg Thr Gln
            20                  25                  30

aat ctg aca ttg gcc agc ctc ccc aaa cgg cat gag aca gaa caa agc    144
Asn Leu Thr Leu Ala Ser Leu Pro Lys Arg His Glu Thr Glu Gln Ser
        35                  40                  45

cag ctt gaa cgc acc atg gcc gat att tct caa gaa gtt ttg gat ttt    192
Gln Leu Glu Arg Thr Met Ala Asp Ile Ser Gln Glu Val Leu Asp Phe
    50                  55                  60

gaa atg cgc tct gaa caa atc atc cgt gca gga cgg tcg ggt tat ata    240
Glu Met Arg Ser Glu Gln Ile Ile Arg Ala Gly Arg Ser Gly Tyr Ile
65                  70                  75                  80

gca ata ccg aac gtc gaa gtc gga cag cag gtt gat cct tcc aaa ctg    288
Ala Ile Pro Asn Val Glu Val Gly Gln Gln Val Asp Pro Ser Lys Leu
                85                  90                  95

ctc ttg agc att gtt ccc gaa cgt acc gag cta tat gcc cat cta tat    336
Leu Leu Ser Ile Val Pro Glu Arg Thr Glu Leu Tyr Ala His Leu Tyr
            100                 105                 110

atc ccc agc agt gca gca ggc ttt atc aag ccg aaa gac aag gtt gtc    384
Ile Pro Ser Ser Ala Ala Gly Phe Ile Lys Pro Lys Asp Lys Val Val
        115                 120                 125

cta cgt tat cag gca tat ccc tat caa aaa ttc ggg ctt gct tcc ggc    432
Leu Arg Tyr Gln Ala Tyr Pro Tyr Gln Lys Phe Gly Leu Ala Ser Gly
    130                 135                 140

agt gtc gta tca gta gca aaa acg gca ctg ggc aga cag gaa ttg tcg    480
Ser Val Val Ser Val Ala Lys Thr Ala Leu Gly Arg Gln Glu Leu Ser
145                 150                 155                 160

gga ttg ggc atg gta tcc tcc gat ttg gcg aag agc aac gaa cct gtt    528
Gly Leu Gly Met Val Ser Ser Asp Leu Ala Lys Ser Asn Glu Pro Val
                165                 170                 175

tat ctc gtg aaa ata aaa ccc gac aaa cca acc atc act gca tac ggt    576
Tyr Leu Val Lys Ile Lys Pro Asp Lys Pro Thr Ile Thr Ala Tyr Gly
            180                 185                 190

gag gaa aaa ccg ctg caa atc ggc atg acg ttg gaa gca gac atc ctg    624

Glu Glu Lys Pro Leu Gln Ile Gly Met Thr Leu Glu Ala Asp Ile Leu
        195                 200                 205

cac gag aaa cgg cgg ctg tac gaa tgg gta ttg gag ctg att tat agt 672
His Glu Lys Arg Arg Leu Tyr Glu Trp Val Leu Glu Leu Ile Tyr Ser
    210                 215                 220

atg tcg ggc aaa ctg taa 690
Met Ser Gly Lys Leu
225

<210> 18
<211> 229
<212> PRT
<213> Neisseria meningitidis

<400> 18
Met Met Asn Val Glu Ala Glu Leu Leu Glu Gln Lys Ala Lys Leu Asp
1               5                   10                  15

Ala Tyr Gly Arg Glu Glu Ala Gly Leu Leu Gln Glu Ile Arg Thr Gln
            20                  25                  30

Asn Leu Thr Leu Ala Ser Leu Pro Lys Arg His Glu Thr Glu Gln Ser
        35                  40                  45

Gln Leu Glu Arg Thr Met Ala Asp Ile Ser Gln Glu Val Leu Asp Phe
    50                  55                  60

Glu Met Arg Ser Glu Gln Ile Ile Arg Ala Gly Arg Ser Gly Tyr Ile
65                  70                  75                  80

Ala Ile Pro Asn Val Glu Val Gly Gln Gln Val Asp Pro Ser Lys Leu
                85                  90                  95

Leu Leu Ser Ile Val Pro Glu Arg Thr Glu Leu Tyr Ala His Leu Tyr
            100                 105                 110

Ile Pro Ser Ser Ala Ala Gly Phe Ile Lys Pro Lys Asp Lys Val Val
        115                 120                 125

Leu Arg Tyr Gln Ala Tyr Pro Tyr Gln Lys Phe Gly Leu Ala Ser Gly
    130                 135                 140

Ser Val Val Ser Val Ala Lys Thr Ala Leu Gly Arg Gln Glu Leu Ser
145                 150                 155                 160

Gly Leu Gly Met Val Ser Ser Asp Leu Ala Lys Ser Asn Glu Pro Val
                165                 170                 175

Tyr Leu Val Lys Ile Lys Pro Asp Lys Pro Thr Ile Thr Ala Tyr Gly
            180                 185                 190

Glu Glu Lys Pro Leu Gln Ile Gly Met Thr Leu Glu Ala Asp Ile Leu
        195                 200                 205

His Glu Lys Arg Arg Leu Tyr Glu Trp Val Leu Glu Leu Ile Tyr Ser
    210                 215                 220

Met Ser Gly Lys Leu
225

<210> 19
<211> 1743
<212> ADN
<213> Neisseria meningitidis

<220>
<221> CDS
<222> (1)..(1740)

<400> 19
atg aaa ttt ttt cct gct cca tgt ctg ttg gtt atc ctg gct gtc ata 48
Met Lys Phe Phe Pro Ala Pro Cys Leu Leu Val Ile Leu Ala Val Ile
1               5                   10                  15

ccc ctt aaa acc tta gct gcc gat gaa aac gat gca gaa ctt atc cgt 96
Pro Leu Lys Thr Leu Ala Ala Asp Glu Asn Asp Ala Glu Leu Ile Arg
            20                  25                  30

tcc atg cag cgt cag cag cac ata gat gct gaa ttg tta act gat gca 144
Ser Met Gln Arg Gln Gln His Ile Asp Ala Glu Leu Leu Thr Asp Ala
        35                  40                  45

aat gtc cgt ttc gag caa cca ttg gag aag aac aat tat gtc ctg agt 192
Asn Val Arg Phe Glu Gln Pro Leu Glu Lys Asn Asn Tyr Val Leu Ser
    50                  55                  60

gaa gat gaa aca ccg tgt act cgg gta aat tac att agt tta gat gat 240
Glu Asp Glu Thr Pro Cys Thr Arg Val Asn Tyr Ile Ser Leu Asp Asp
65                  70                  75                  80

aag acg gcg cgc aaa ttt tct ttt ctt cct tct gtg ctc atg aaa gaa 288
Lys Thr Ala Arg Lys Phe Ser Phe Leu Pro Ser Val Leu Met Lys Glu
                85                  90                  95

aca gct ttt aaa act ggg atg tgt tta ggt tcc aat aat ttg agc agg 336
Thr Ala Phe Lys Thr Gly Met Cys Leu Gly Ser Asn Asn Leu Ser Arg
            100                 105                 110

cta caa aaa gcc gcg caa cag ata ctg att gtg cgt ggc tac ctc act 384
Leu Gln Lys Ala Ala Gln Gln Ile Leu Ile Val Arg Gly Tyr Leu Thr
        115                 120                 125

tcc caa gct att atc caa cca cag aat atg gat tcg gga att ctg aaa 432
Ser Gln Ala Ile Ile Gln Pro Gln Asn Met Asp Ser Gly Ile Leu Lys
    130                 135                 140

tta cgg gta tca gca ggc gaa atc agg gat atc cgc tat gaa gaa aaa 480
Leu Arg Val Ser Ala Gly Glu Ile Arg Asp Ile Arg Tyr Glu Glu Lys
145                 150                 155                 160

cgg gat gcg aag tct gcc gag ggc agt att agt gca ttc aat aac aaa 528
Arg Asp Ala Lys Ser Ala Glu Gly Ser Ile Ser Ala Phe Asn Asn Lys
                165                 170                 175

ctt ccc tta tat agg aac aaa att ctc aat ctt cgc gat gta gag cag 576

Leu Pro Leu Tyr Arg Asn Lys Ile Leu Asn Leu Arg Asp Val Glu Gln
            180                 185                 190

ggc ttg gaa aac ctg cgt cgt ttg ccg agt gtt aaa aca gat att cag 624
Gly Leu Glu Asn Leu Arg Arg Leu Pro Ser Val Lys Thr Asp Ile Gln
        195                 200                 205

att ata ccg tcc gaa gaa gaa ggc aaa agc gat tta cag atc aaa tgg 672
Ile Ile Pro Ser Glu Glu Glu Gly Lys Ser Asp Leu Gln Ile Lys Trp
    210                 215                 220

cag cag aat aaa ccc ata cgg ttc agt atc ggt ata gat gat gcg ggc 720
Gln Gln Asn Lys Pro Ile Arg Phe Ser Ile Gly Ile Asp Asp Ala Gly
225                 230                 235                 240

ggc aaa acg acc ggc aaa tat caa gga aat gtc gct tta tcg tcc gat 768
Gly Lys Thr Thr Gly Lys Tyr Gln Gly Asn Val Ala Leu Ser Ser Asp
                245                 250                 255

aac cct ttg ggc tta agc gat tcg ttt tat gtt tca tat gga cgc ggt 816
Asn Pro Leu Gly Leu Ser Asp Ser Phe Tyr Val Ser Tyr Gly Arg Gly
            260                 265                 270

ttg gtg cac aaa acg gac ttg act gct gcc acc ggt acg gaa act gaa 864
Leu Val His Lys Thr Asp Leu Thr Ala Ala Thr Gly Thr Glu Thr Glu
        275                 280                 285

agc gga tcc aga agt tac agc gtg cat tat tcg gtg ccc gta aaa aaa 912
Ser Gly Ser Arg Ser Tyr Ser Val His Tyr Ser Val Pro Val Lys Lys
    290                 295                 300

tgg ctg ttt tct ttt aat cac aat gga cat cgt tac cac gaa gca acc 960
Trp Leu Phe Ser Phe Asn His Asn Gly His Arg Tyr His Glu Ala Thr
305                 310                 315                 320

gaa ggc tat tcc gtc aat tac gat tac aac ggc aaa caa tat cag agc 1008
Glu Gly Tyr Ser Val Asn Tyr Asp Tyr Asn Gly Lys Gln Tyr Gln Ser
                325                 330                 335

agc ctg gcc gcc gag cgc atg ctt tgg ccc ccc agc ttt cct caa act 1056
Ser Leu Ala Ala Glu Arg Met Leu Trp Pro Pro Ser Phe Pro Gln Thr
            340                 345                 350

tca gtc cga atg aaa tta tgg aca cgc caa acc tat aaa tac atc gac 1104
Ser Val Arg Met Lys Leu Trp Thr Arg Gln Thr Tyr Lys Tyr Ile Asp
        355                 360                 365

gat gcc gaa atc gaa gtg caa cgc cgc cgc tct gca ggc tgg gaa gcc 1152
Asp Ala Glu Ile Glu Val Gln Arg Arg Arg Ser Ala Gly Trp Glu Ala
    370                 375                 380

gaa ttg cgc cac cgt gct tac ctc cac cgt tgg cag ctt gac ggc aag 1200
Glu Leu Arg His Arg Ala Tyr Leu His Arg Trp Gln Leu Asp Gly Lys
385                 390                 395                 400

ttg tct tac aaa cgc ggg acc ggc atg cgc caa agt atg ccc gca cct 1248
Leu Ser Tyr Lys Arg Gly Thr Gly Met Arg Gln Ser Met Pro Ala Pro
                405                 410                 415

gaa gaa aac ggc ggc ggt act att cca gcc aca tcc cgt atg aaa atc 1296

Glu Glu Asn Gly Gly Gly Thr Ile Pro Ala Thr Ser Arg Met Lys Ile
            420                 425                 430

ata acc gcc gga ttg gat gca gcg gcc ccg tct atg ttg ggc aaa cag 1344
Ile Thr Ala Gly Leu Asp Ala Ala Ala Pro Ser Met Leu Gly Lys Gln
        435                 440                 445

cag ttt ttc tac gca acc gcc att caa gct caa tgg aac aaa acg cct 1392
Gln Phe Phe Tyr Ala Thr Ala Ile Gln Ala Gln Trp Asn Lys Thr Pro
    450                 455                 460

ttg gtt gcc caa gac aag ttg tct atc ggc agc cgc tac acc gtt cgc 1440
Leu Val Ala Gln Asp Lys Leu Ser Ile Gly Ser Arg Tyr Thr Val Arg
465                 470                 475                 480

gga ttt gat ggg gag cag agt ctt ttc gga gag cga ggt ttc tac tgg 1488
Gly Phe Asp Gly Glu Gln Ser Leu Phe Gly Glu Arg Gly Phe Tyr Trp
                485                 490                 495

cag aat act tta act tgg tat ttt cat ccg aac cat cag ttc tat ctc 1536
Gln Asn Thr Leu Thr Trp Tyr Phe His Pro Asn His Gln Phe Tyr Leu
            500                 505                 510

ggt gcg gac tat ggc cgc gta tct ggc gaa agt gca caa tat gta tcg 1584
Gly Ala Asp Tyr Gly Arg Val Ser Gly Glu Ser Ala Gln Tyr Val Ser
        515                 520                 525

ggc aag cag ctg atg ggt gca gtg gtc ggc ttc aga gga ggg cat aaa 1632
Gly Lys Gln Leu Met Gly Ala Val Val Gly Phe Arg Gly Gly His Lys
    530                 535                 540

gta ggc ggt atg ttt gct tat gat ctg ttt gcc ggc aag ccg ctt cat 1680
Val Gly Gly Met Phe Ala Tyr Asp Leu Phe Ala Gly Lys Pro Leu His
545                 550                 555                 560

aaa ccc aaa ggc ttt cag acg acc aac acc gtt tac ggc ttc aac ttg 1728
Lys Pro Lys Gly Phe Gln Thr Thr Asn Thr Val Tyr Gly Phe Asn Leu
                565                 570                 575

aat tac agt ttc taa 1743
Asn Tyr Ser Phe
            580

<210> 20
<211> 580
<212> PRT
<213> Neisseria meningitidis

<400> 20
Met Lys Phe Phe Pro Ala Pro Cys Leu Leu Val Ile Leu Ala Val Ile
1               5                   10                  15

Pro Leu Lys Thr Leu Ala Ala Asp Glu Asn Asp Ala Glu Leu Ile Arg
            20                  25                  30

Ser Met Gln Arg Gln Gln His Ile Asp Ala Glu Leu Leu Thr Asp Ala
        35                  40                  45

Asn Val Arg Phe Glu Gln Pro Leu Glu Lys Asn Asn Tyr Val Leu Ser

50                  55                  60

Glu Asp Glu Thr Pro Cys Thr Arg Val Asn Tyr Ile Ser Leu Asp Asp
65                  70                  75                  80

Lys Thr Ala Arg Lys Phe Ser Phe Leu Pro Ser Val Leu Met Lys Glu
                85                  90                  95

Thr Ala Phe Lys Thr Gly Met Cys Leu Gly Ser Asn Asn Leu Ser Arg
            100                 105                 110

Leu Gln Lys Ala Ala Gln Gln Ile Leu Ile Val Arg Gly Tyr Leu Thr
        115                 120                 125

Ser Gln Ala Ile Ile Gln Pro Gln Asn Met Asp Ser Gly Ile Leu Lys
    130                 135                 140

Leu Arg Val Ser Ala Gly Glu Ile Arg Asp Ile Arg Tyr Glu Glu Lys
145                 150                 155                 160

Arg Asp Ala Lys Ser Ala Glu Gly Ser Ile Ser Ala Phe Asn Asn Lys
                165                 170                 175

Leu Pro Leu Tyr Arg Asn Lys Ile Leu Asn Leu Arg Asp Val Glu Gln
            180                 185                 190

Gly Leu Glu Asn Leu Arg Arg Leu Pro Ser Val Lys Thr Asp Ile Gln
        195                 200                 205

Ile Ile Pro Ser Glu Glu Glu Gly Lys Ser Asp Leu Gln Ile Lys Trp
    210                 215                 220

Gln Gln Asn Lys Pro Ile Arg Phe Ser Ile Gly Ile Asp Asp Ala Gly
225                 230                 235                 240

Gly Lys Thr Thr Gly Lys Tyr Gln Gly Asn Val Ala Leu Ser Ser Asp
                245                 250                 255

Asn Pro Leu Gly Leu Ser Asp Ser Phe Tyr Val Ser Tyr Gly Arg Gly
            260                 265                 270

Leu Val His Lys Thr Asp Leu Thr Ala Ala Thr Gly Thr Glu Thr Glu
        275                 280                 285

Ser Gly Ser Arg Ser Tyr Ser Val His Tyr Ser Val Pro Val Lys Lys
    290                 295                 300

Trp Leu Phe Ser Phe Asn His Asn Gly His Arg Tyr His Glu Ala Thr
305                 310                 315                 320

Glu Gly Tyr Ser Val Asn Tyr Asp Tyr Asn Gly Lys Gln Tyr Gln Ser
                325                 330                 335

Ser Leu Ala Ala Glu Arg Met Leu Trp Pro Pro Ser Phe Pro Gln Thr
            340                 345                 350

Ser Val Arg Met Lys Leu Trp Thr Arg Gln Thr Tyr Lys Tyr Ile Asp
        355                 360                 365

Asp Ala Glu Ile Glu Val Gln Arg Arg Arg Ser Ala Gly Trp Glu Ala

370                 375                 380

Glu Leu Arg His Arg Ala Tyr Leu His Arg Trp Gln Leu Asp Gly Lys
385                 390                 395                 400

Leu Ser Tyr Lys Arg Gly Thr Gly Met Arg Gln Ser Met Pro Ala Pro
                405                 410                 415

Glu Glu Asn Gly Gly Gly Thr Ile Pro Ala Thr Ser Arg Met Lys Ile
            420                 425                 430

Ile Thr Ala Gly Leu Asp Ala Ala Ala Pro Ser Met Leu Gly Lys Gln
        435                 440                 445

Gln Phe Phe Tyr Ala Thr Ala Ile Gln Ala Gln Trp Asn Lys Thr Pro
    450                 455                 460

Leu Val Ala Gln Asp Lys Leu Ser Ile Gly Ser Arg Tyr Thr Val Arg
465                 470                 475                 480

Gly Phe Asp Gly Glu Gln Ser Leu Phe Gly Glu Arg Gly Phe Tyr Trp
                485                 490                 495

Gln Asn Thr Leu Thr Trp Tyr Phe His Pro Asn His Gln Phe Tyr Leu
            500                 505                 510

Gly Ala Asp Tyr Gly Arg Val Ser Gly Glu Ser Ala Gln Tyr Val Ser
        515                 520                 525

Gly Lys Gln Leu Met Gly Ala Val Val Gly Phe Arg Gly Gly His Lys
    530                 535                 540

Val Gly Gly Met Phe Ala Tyr Asp Leu Phe Ala Gly Lys Pro Leu His
545                 550                 555                 560

Lys Pro Lys Gly Phe Gln Thr Thr Asn Thr Val Tyr Gly Phe Asn Leu
                565                 570                 575

Asn Tyr Ser Phe
            580

<210> 21
<211> 411
<212> ADN
<213> Neisseria meningitidis

<220>
<221> CDS
<222> (1)..(408)

<400> 21
atg att gaa ttt gtc cga gcc aaa aaa cgg ctg ctt tgg gca ttt gtg      48
Met Ile Glu Phe Val Arg Ala Lys Lys Arg Leu Leu Trp Ala Phe Val
1               5                   10                  15

ctt ttg ctt gtg tgg acg tgc ggt tac cga tac gcc gcc gac aag gcc      96
Leu Leu Leu Val Trp Thr Cys Gly Tyr Arg Tyr Ala Ala Asp Lys Ala
            20                  25                  30

gaa gcg aaa caa acc gcc ctg att gcc acc tat cgg cat tct tct atg     144
Glu Ala Lys Gln Thr Ala Leu Ile Ala Thr Tyr Arg His Ser Ser Met
        35                  40                  45

gtt gcg gcg gaa caa tac gcc ttg cag ctt aaa aaa gcg cag gac gaa     192
Val Ala Ala Glu Gln Tyr Ala Leu Gln Leu Lys Lys Ala Gln Asp Glu
    50                  55                  60

agg cag cgg tgg tac gac ttt tcc caa aaa caa gga aga aag ccc gtg     240
Arg Gln Arg Trp Tyr Asp Phe Ser Gln Lys Gln Gly Arg Lys Pro Val
65                  70                  75                  80

aaa aaa cag tat ccg ccg caa acg aaa aaa gcc ggc tat ctg aaa acc     288
Lys Lys Gln Tyr Pro Pro Gln Thr Lys Lys Ala Gly Tyr Leu Lys Thr
                85                  90                  95

aag gaa gaa ctg ctt gcg gaa ttg gct tgc ctt aaa gcg gaa atg gct     336
Lys Glu Glu Leu Leu Ala Glu Leu Ala Cys Leu Lys Ala Glu Met Ala
            100                 105                 110

gcc cta aaa aag ctc gat gcc tta atc tat ggg aaa gaa gtg cgg cag     384
Ala Leu Lys Lys Leu Asp Ala Leu Ile Tyr Gly Lys Glu Val Arg Gln
        115                 120                 125

aaa gaa cgc aac tcg tcg cag ggt taa                                 411
Lys Glu Arg Asn Ser Ser Gln Gly
    130                 135


<210> 22
<211> 136
<212> PRT
<213> Neisseria meningitidis

<400> 22
Met Ile Glu Phe Val Arg Ala Lys Lys Arg Leu Leu Trp Ala Phe Val
1               5                   10                  15

Leu Leu Leu Val Trp Thr Cys Gly Tyr Arg Tyr Ala Ala Asp Lys Ala
            20                  25                  30

Glu Ala Lys Gln Thr Ala Leu Ile Ala Thr Tyr Arg His Ser Ser Met
        35                  40                  45

Val Ala Ala Glu Gln Tyr Ala Leu Gln Leu Lys Lys Ala Gln Asp Glu
    50                  55                  60

Arg Gln Arg Trp Tyr Asp Phe Ser Gln Lys Gln Gly Arg Lys Pro Val
65                  70                  75                  80

Lys Lys Gln Tyr Pro Pro Gln Thr Lys Lys Ala Gly Tyr Leu Lys Thr
                85                  90                  95

Lys Glu Glu Leu Leu Ala Glu Leu Ala Cys Leu Lys Ala Glu Met Ala
            100                 105                 110

Ala Leu Lys Lys Leu Asp Ala Leu Ile Tyr Gly Lys Glu Val Arg Gln
        115                 120                 125

Lys Glu Arg Asn Ser Ser Gln Gly
    130                 135

<210> 23
<211> 924
<212> ADN
<213> Neisseria meningitidis

<220>
<221> CDS
<222> (1)..(921)

<400> 23
atg caa tac agc aca ctg gca gga caa acc gac aac tcc ctc gtt tcc     48
Met Gln Tyr Ser Thr Leu Ala Gly Gln Thr Asp Asn Ser Leu Val Ser
  1               5                  10                  15

aat aat ttc ggg ttt ttg cgc ctg ccg ctt aat ttt atg ccg tat gaa     96
Asn Asn Phe Gly Phe Leu Arg Leu Pro Leu Asn Phe Met Pro Tyr Glu
             20                  25                  30

agt cat gcc gat tgg gtt att acc ggc gtg cct tat gat atg gcg gtt    144
Ser His Ala Asp Trp Val Ile Thr Gly Val Pro Tyr Asp Met Ala Val
         35                  40                  45

tca ggg cgt tcc ggc gcg cgt ttc ggt cct gaa gcc atc cgg cgc gcc    192
Ser Gly Arg Ser Gly Ala Arg Phe Gly Pro Glu Ala Ile Arg Arg Ala
     50                  55                  60

tcc gtc aac ctc gct tgg gag cac cgc agg ttt cca tgg aca ttt gat    240
Ser Val Asn Leu Ala Trp Glu His Arg Arg Phe Pro Trp Thr Phe Asp
 65                  70                  75                  80

gtg cgc gaa cgc ctg aac att att gat tgc ggc gac ttg gtt ttt tct    288
Val Arg Glu Arg Leu Asn Ile Ile Asp Cys Gly Asp Leu Val Phe Ser
                 85                  90                  95

ttt ggc gac agc agg gat ttt gtc gaa aaa atg gaa gcg cac gcc ggc    336
Phe Gly Asp Ser Arg Asp Phe Val Glu Lys Met Glu Ala His Ala Gly
            100                 105                 110

aaa tta ctt tct tcc ggc aaa cgc tgt ttg agt ttg ggc ggc gac cat    384
Lys Leu Leu Ser Ser Gly Lys Arg Cys Leu Ser Leu Gly Gly Asp His
        115                 120                 125

ttc att acc ctc ccg ttg ttg cgc gcc cac gcc cgc tat ttc ggc aaa    432
Phe Ile Thr Leu Pro Leu Leu Arg Ala His Ala Arg Tyr Phe Gly Lys
    130                 135                 140

ctc gca ctg att cat ttt gac gcg cac acc gac acc tac gac aac ggc    480
Leu Ala Leu Ile His Phe Asp Ala His Thr Asp Thr Tyr Asp Asn Gly
145                 150                 155                 160

agc gaa tac gac cac ggt acg atg ttc tat acc gcc ccc aag gaa ggc    528
Ser Glu Tyr Asp His Gly Thr Met Phe Tyr Thr Ala Pro Lys Glu Gly
                165                 170                 175

ctc atc gac ccg tcc cgt tcc gta caa atc ggc ata cgt acc gaa cac    576

Leu Ile Asp Pro Ser Arg Ser Val Gln Ile Gly Ile Arg Thr Glu His
            180                 185                 190

agt aaa aaa ttg cct ttt act gtg ttg acc gcc ccc caa gtt aat gaa      624
Ser Lys Lys Leu Pro Phe Thr Val Leu Thr Ala Pro Gln Val Asn Glu
        195                 200                 205

gac agt gtt gaa gag acc gtc cgt aaa atc aaa gaa acc gtc ggc aat      672
Asp Ser Val Glu Glu Thr Val Arg Lys Ile Lys Glu Thr Val Gly Asn
    210                 215                 220

atg ccc gtt tac ctg act ttc gac ata gac tgc ctc gac ccg tcg ttc      720
Met Pro Val Tyr Leu Thr Phe Asp Ile Asp Cys Leu Asp Pro Ser Phe
225                 230                 235                 240

gcc ccc ggg acc ggt acg ccc gta tgc ggc ggc ttg agc agc gac agg      768
Ala Pro Gly Thr Gly Thr Pro Val Cys Gly Gly Leu Ser Ser Asp Arg
                245                 250                 255

gca tta aaa atc cta cgt ggg ctg acg gat ctc gac atc gtc ggt atg      816
Ala Leu Lys Ile Leu Arg Gly Leu Thr Asp Leu Asp Ile Val Gly Met
            260                 265                 270

gat gtt gta gaa gtt gcc ccc tct tac gac caa tcc gac att acc gct      864
Asp Val Val Glu Val Ala Pro Ser Tyr Asp Gln Ser Asp Ile Thr Ala
        275                 280                 285

ttg gcc ggc gcc aca att gcc ttg gaa atg ctt tac ctt caa ggt gcg      912
Leu Ala Gly Ala Thr Ile Ala Leu Glu Met Leu Tyr Leu Gln Gly Ala
    290                 295                 300

aaa aag gac tga                                                      924
Lys Lys Asp
305


<210> 24
<211> 307
<212> PRT
<213> Neisseria meningitidis

<400> 24
Met Gln Tyr Ser Thr Leu Ala Gly Gln Thr Asp Asn Ser Leu Val Ser
1               5                   10                  15

Asn Asn Phe Gly Phe Leu Arg Leu Pro Leu Asn Phe Met Pro Tyr Glu
            20                  25                  30

Ser His Ala Asp Trp Val Ile Thr Gly Val Pro Tyr Asp Met Ala Val
        35                  40                  45

Ser Gly Arg Ser Gly Ala Arg Phe Gly Pro Glu Ala Ile Arg Arg Ala
    50                  55                  60

Ser Val Asn Leu Ala Trp Glu His Arg Arg Phe Pro Trp Thr Phe Asp
65                  70                  75                  80

Val Arg Glu Arg Leu Asn Ile Ile Asp Cys Gly Asp Leu Val Phe Ser
                85                  90                  95

Phe Gly Asp Ser Arg Asp Phe Val Glu Lys Met Glu Ala His Ala Gly
                100                 105                 110

Lys Leu Leu Ser Ser Gly Lys Arg Cys Leu Ser Leu Gly Gly Asp His
            115                 120                 125

Phe Ile Thr Leu Pro Leu Leu Arg Ala His Ala Arg Tyr Phe Gly Lys
    130                 135                 140

Leu Ala Leu Ile His Phe Asp Ala His Thr Asp Thr Tyr Asp Asn Gly
145                 150                 155                 160

Ser Glu Tyr Asp His Gly Thr Met Phe Tyr Thr Ala Pro Lys Glu Gly
                165                 170                 175

Leu Ile Asp Pro Ser Arg Ser Val Gln Ile Gly Ile Arg Thr Glu His
                180                 185                 190

Ser Lys Lys Leu Pro Phe Thr Val Leu Thr Ala Pro Gln Val Asn Glu
            195                 200                 205

Asp Ser Val Glu Glu Thr Val Arg Lys Ile Lys Glu Thr Val Gly Asn
    210                 215                 220

Met Pro Val Tyr Leu Thr Phe Asp Ile Asp Cys Leu Asp Pro Ser Phe
225                 230                 235                 240

Ala Pro Gly Thr Gly Thr Pro Val Cys Gly Gly Leu Ser Ser Asp Arg
                245                 250                 255

Ala Leu Lys Ile Leu Arg Gly Leu Thr Asp Leu Asp Ile Val Gly Met
                260                 265                 270

Asp Val Val Glu Val Ala Pro Ser Tyr Asp Gln Ser Asp Ile Thr Ala
            275                 280                 285

Leu Ala Gly Ala Thr Ile Ala Leu Glu Met Leu Tyr Leu Gln Gly Ala
    290                 295                 300

Lys Lys Asp
305

<210> 25
<211> 426
<212> ADN
<213> Neisseria meningitidis

<220>
<221> CDS
<222> (1)..(423)

<400> 25
atg gag cag tcg ggc aaa ttc agt tgg tct gcg gca gct ttt tgg gac      48
Met Glu Gln Ser Gly Lys Phe Ser Trp Ser Ala Ala Ala Phe Trp Asp
  1               5                  10                  15

att ccc tac ccc gtc acc agg cgg att gcc tca agt ttg tat tcg acc      96
Ile Pro Tyr Pro Val Thr Arg Arg Ile Ala Ser Ser Leu Tyr Ser Thr

20                  25                  30

gaa tat ttt gtc gta tgc ttt ctg cgt ttg atg cca ctc tct ccg tgt     144
Glu Tyr Phe Val Val Cys Phe Leu Arg Leu Met Pro Leu Ser Pro Cys
        35                  40                  45

aat ctg tat ttt gtc acc cat ctg cgt acc aat gaa tcg gaa ata gaa     192
Asn Leu Tyr Phe Val Thr His Leu Arg Thr Asn Glu Ser Glu Ile Glu
    50                  55                  60

aga tgg tct gct gtt ccc tgc caa ata gta ttg aac gac ggc aag tcg     240
Arg Trp Ser Ala Val Pro Cys Gln Ile Val Leu Asn Asp Gly Lys Ser
65                  70                  75                  80

gaa ttc ggc gga ttc gca ttt gaa gtg caa ctt tcc cta aca gaa aaa     288
Glu Phe Gly Gly Phe Ala Phe Glu Val Gln Leu Ser Leu Thr Glu Lys
                85                  90                  95

ggc cag tat gcg gta gca tac gac ctt tcc tgc aag aaa gat tgc cat     336
Gly Gln Tyr Ala Val Ala Tyr Asp Leu Ser Cys Lys Lys Asp Cys His
            100                 105                 110

gag cta cac gca act gac cca agg cga acg ata cca cat cca ata cct     384
Glu Leu His Ala Thr Asp Pro Arg Arg Thr Ile Pro His Pro Ile Pro
        115                 120                 125

gtc ccg cca ctg cac cgt cac cga aat cgc caa aca gct taa             426
Val Pro Pro Leu His Arg His Arg Asn Arg Gln Thr Ala
    130                 135                 140

<210> 26
<211> 141
<212> PRT
<213> Neisseria meningitidis

<400> 26
Met Glu Gln Ser Gly Lys Phe Ser Trp Ser Ala Ala Ala Phe Trp Asp
1               5                   10                  15

Ile Pro Tyr Pro Val Thr Arg Arg Ile Ala Ser Ser Leu Tyr Ser Thr
            20                  25                  30

Glu Tyr Phe Val Val Cys Phe Leu Arg Leu Met Pro Leu Ser Pro Cys
        35                  40                  45

Asn Leu Tyr Phe Val Thr His Leu Arg Thr Asn Glu Ser Glu Ile Glu
    50                  55                  60

Arg Trp Ser Ala Val Pro Cys Gln Ile Val Leu Asn Asp Gly Lys Ser
65                  70                  75                  80

Glu Phe Gly Gly Phe Ala Phe Glu Val Gln Leu Ser Leu Thr Glu Lys
                85                  90                  95

Gly Gln Tyr Ala Val Ala Tyr Asp Leu Ser Cys Lys Lys Asp Cys His
            100                 105                 110

Glu Leu His Ala Thr Asp Pro Arg Arg Thr Ile Pro His Pro Ile Pro
        115                 120                 125

Val Pro Pro Leu His Arg His Arg Asn Arg Gln Thr Ala
    130                 135                 140

<210> 27
<211> 351
<212> ADN
<213> Neisseria meningitidis

<220>
<221> CDS
<222> (1)..(348)

<400> 27
atg caa aac ggc ggg gga aag att tac cag acg gcg gac aat gtg gaa      48
Met Gln Asn Gly Gly Gly Lys Ile Tyr Gln Thr Ala Asp Asn Val Glu
  1               5                  10                  15

ggg att atg ctg ttg aag gta gta cct gag cgt acc gtt tcg gca gat      96
Gly Ile Met Leu Leu Lys Val Val Pro Glu Arg Thr Val Ser Ala Asp
             20                  25                  30

gca aaa acc aga gac ccg atg tgg gac aat gcg gct tta cag acc agc     144
Ala Lys Thr Arg Asp Pro Met Trp Asp Asn Ala Ala Leu Gln Thr Ser
         35                  40                  45

gaa ggc gta aat ttt att gct cgt ttc cta gga ttt ttt agc gat ggg     192
Glu Gly Val Asn Phe Ile Ala Arg Phe Leu Gly Phe Phe Ser Asp Gly
     50                  55                  60

gaa tac cgc tat gtg gat gtc ctg caa ccc aac cat tcc gat att att     240
Glu Tyr Arg Tyr Val Asp Val Leu Gln Pro Asn His Ser Asp Ile Ile
 65                  70                  75                  80

cgg tat tca ggt aaa gat ttt ccg cta aat caa ata ctt aac cat ata     288
Arg Tyr Ser Gly Lys Asp Phe Pro Leu Asn Gln Ile Leu Asn His Ile
                 85                  90                  95

cac ccc gcc cgt tat gcg gta acg ttc gaa aac aat gtc gat tcc aag     336
His Pro Ala Arg Tyr Ala Val Thr Phe Glu Asn Asn Val Asp Ser Lys
            100                 105                 110

ctg cgc agg cac tga                                                 351
Leu Arg Arg His
        115

<210> 28
<211> 116
<212> PRT
<213> Neisseria meningitidis

<400> 28
Met Gln Asn Gly Gly Gly Lys Ile Tyr Gln Thr Ala Asp Asn Val Glu
  1               5                  10                  15

Gly Ile Met Leu Leu Lys Val Val Pro Glu Arg Thr Val Ser Ala Asp
             20                  25                  30

Ala Lys Thr Arg Asp Pro Met Trp Asp Asn Ala Ala Leu Gln Thr Ser
        35                  40                  45

Glu Gly Val Asn Phe Ile Ala Arg Phe Leu Gly Phe Phe Ser Asp Gly
    50                  55                  60

Glu Tyr Arg Tyr Val Asp Val Leu Gln Pro Asn His Ser Asp Ile Ile
65                  70                  75                  80

Arg Tyr Ser Gly Lys Asp Phe Pro Leu Asn Gln Ile Leu Asn His Ile
                85                  90                  95

His Pro Ala Arg Tyr Ala Val Thr Phe Glu Asn Asn Val Asp Ser Lys
            100                 105                 110

Leu Arg Arg His
        115

<210> 29
<211> 1404
<212> ADN
<213> Neisseria meningitidis

<220>
<221> CDS
<222> (1)..(1401)

<400> 29
atg aca ttg ctc aat cta atg ata atg caa gat tac ggt att tcc gtt 48
Met Thr Leu Leu Asn Leu Met Ile Met Gln Asp Tyr Gly Ile Ser Val
1               5                   10                  15

tgc ctg aca ctg acg ccc tat ttg caa cat gaa cta ttt tcg gct atg 96
Cys Leu Thr Leu Thr Pro Tyr Leu Gln His Glu Leu Phe Ser Ala Met
            20                  25                  30

aaa tcc tat ttt tcc aaa tat atc cta ccc gtt tca ctt ttt acc ttg 144
Lys Ser Tyr Phe Ser Lys Tyr Ile Leu Pro Val Ser Leu Phe Thr Leu
        35                  40                  45

cca cta tcc ctt tcc cca tcc gtt tcg gct ttt acg ctg cct gaa gca 192
Pro Leu Ser Leu Ser Pro Ser Val Ser Ala Phe Thr Leu Pro Glu Ala
    50                  55                  60

tgg cgg gcg gcg cag caa cat tcg gct gat ttt caa gcg tcc cat tac 240
Trp Arg Ala Ala Gln Gln His Ser Ala Asp Phe Gln Ala Ser His Tyr
65                  70                  75                  80

cag cgt gat gca gtg cgc gca cgg caa caa caa gcc aag gcc gca ttc 288
Gln Arg Asp Ala Val Arg Ala Arg Gln Gln Gln Ala Lys Ala Ala Phe
                85                  90                  95

ctt ccc cat gta tcc gcc aat gcc agc tac cag cgc cag ccg cca tcg 336
Leu Pro His Val Ser Ala Asn Ala Ser Tyr Gln Arg Gln Pro Pro Ser
            100                 105                 110

att tct tcc acc cgc gaa aca cag gga tgg agc gtg cag gtg gga caa 384

Ile Ser Ser Thr Arg Glu Thr Gln Gly Trp Ser Val Gln Val Gly Gln
        115                 120                 125

acc tta ttt gac gct gcc aaa ttt gca caa tac cgc caa agc agg ttc 432
Thr Leu Phe Asp Ala Ala Lys Phe Ala Gln Tyr Arg Gln Ser Arg Phe
    130                 135                 140

gat acg cag gct gca gaa cag cgt ttc gat gcg gca cgc gaa gaa ttg 480
Asp Thr Gln Ala Ala Glu Gln Arg Phe Asp Ala Ala Arg Glu Glu Leu
145                 150                 155                 160

ctg ttg aaa gtt gcc gaa agt tat ttc aac gtt tta ctc agc cga gac 528
Leu Leu Lys Val Ala Glu Ser Tyr Phe Asn Val Leu Leu Ser Arg Asp
                165                 170                 175

acc gtt gcc gcc cat gcg gcg gaa aaa gag gct tat gcc cag cag gta 576
Thr Val Ala Ala His Ala Ala Glu Lys Glu Ala Tyr Ala Gln Gln Val
            180                 185                 190

agg cag gcg cag gct tta ttc aat aaa ggt gct gcc acc gcg ctg gat 624
Arg Gln Ala Gln Ala Leu Phe Asn Lys Gly Ala Ala Thr Ala Leu Asp
        195                 200                 205

att cac gaa gcc aaa gcc ggt tac gac aat gcc ctg gcc caa gaa atc 672
Ile His Glu Ala Lys Ala Gly Tyr Asp Asn Ala Leu Ala Gln Glu Ile
    210                 215                 220

gcc gta ttg gct gag aaa caa acc tat gaa aac cag ttg aac gac tac 720
Ala Val Leu Ala Glu Lys Gln Thr Tyr Glu Asn Gln Leu Asn Asp Tyr
225                 230                 235                 240

acc gac ctg gat agc aaa caa atc gag gcc ata gat acc gcc aac ctg 768
Thr Asp Leu Asp Ser Lys Gln Ile Glu Ala Ile Asp Thr Ala Asn Leu
                245                 250                 255

ttg gca cgc tat ctg ccc aag ctg gaa cgt tac agt ctg gat gaa tgg 816
Leu Ala Arg Tyr Leu Pro Lys Leu Glu Arg Tyr Ser Leu Asp Glu Trp
            260                 265                 270

cag cgc att gcc tta tcc aac aat cat gaa tac cgg atg cag cag ctt 864
Gln Arg Ile Ala Leu Ser Asn Asn His Glu Tyr Arg Met Gln Gln Leu
        275                 280                 285

gcc ctg caa agc agc gga cag gcg ctt cgg gca gca cag aac agc cgc 912
Ala Leu Gln Ser Ser Gly Gln Ala Leu Arg Ala Ala Gln Asn Ser Arg
    290                 295                 300

tat ccc acc gtt tct gcc cat gtc ggc tat cag aat aac ctc tac act 960
Tyr Pro Thr Val Ser Ala His Val Gly Tyr Gln Asn Asn Leu Tyr Thr
305                 310                 315                 320

tca tct gcg cag aat aat gac tac cac tat cgg ggc aaa ggg atg agc 1008
Ser Ser Ala Gln Asn Asn Asp Tyr His Tyr Arg Gly Lys Gly Met Ser
                325                 330                 335

gtc ggc gta cag ttg aat ttg ccg ctt tat acc ggc gga gaa ttg tcg 1056
Val Gly Val Gln Leu Asn Leu Pro Leu Tyr Thr Gly Gly Glu Leu Ser
            340                 345                 350

ggc aaa atc cat gaa gcc gaa gcg caa tac ggg gcc gcc gaa gca cag 1104

Gly Lys Ile His Glu Ala Glu Ala Gln Tyr Gly Ala Ala Glu Ala Gln
        355                 360                 365

ctg acc gca acc gag cgg cac atc aaa ctc gcc gta cgc cag gct tat    1152
Leu Thr Ala Thr Glu Arg His Ile Lys Leu Ala Val Arg Gln Ala Tyr
    370                 375                 380

acc gaa agc ggt gcg gcg cgt tac caa atc atg gcg caa gaa cgg gtt    1200
Thr Glu Ser Gly Ala Ala Arg Tyr Gln Ile Met Ala Gln Glu Arg Val
385                 390                 395                 400

ttg gaa agc agc cgt ttg aaa ctg aaa tcg acc gaa acc ggc caa caa    1248
Leu Glu Ser Ser Arg Leu Lys Leu Lys Ser Thr Glu Thr Gly Gln Gln
                405                 410                 415

tac ggc atc cgc aac cgg ctg gaa gta ata cgg gcg cgg cag gaa gtc    1296
Tyr Gly Ile Arg Asn Arg Leu Glu Val Ile Arg Ala Arg Gln Glu Val
            420                 425                 430

gcc caa gca gaa cag aaa ctg gct caa gca cgg tat aaa ttc atg ctg    1344
Ala Gln Ala Glu Gln Lys Leu Ala Gln Ala Arg Tyr Lys Phe Met Leu
        435                 440                 445

gct tat ttg cgc ttg gtg aaa gag agc ggg tta ggg ttg gaa acg gta    1392
Ala Tyr Leu Arg Leu Val Lys Glu Ser Gly Leu Gly Leu Glu Thr Val
    450                 455                 460

ttt gcg gaa taa                                                    1404
Phe Ala Glu
465

<210> 30
<211> 467
<212> PRT
<213> Neisseria meningitidis

<400> 30
Met Thr Leu Leu Asn Leu Met Ile Met Gln Asp Tyr Gly Ile Ser Val
1               5                   10                  15

Cys Leu Thr Leu Thr Pro Tyr Leu Gln His Glu Leu Phe Ser Ala Met
            20                  25                  30

Lys Ser Tyr Phe Ser Lys Tyr Ile Leu Pro Val Ser Leu Phe Thr Leu
        35                  40                  45

Pro Leu Ser Leu Ser Pro Ser Val Ser Ala Phe Thr Leu Pro Glu Ala
    50                  55                  60

Trp Arg Ala Ala Gln Gln His Ser Ala Asp Phe Gln Ala Ser His Tyr
65                  70                  75                  80

Gln Arg Asp Ala Val Arg Ala Arg Gln Gln Gln Ala Lys Ala Ala Phe
                85                  90                  95

Leu Pro His Val Ser Ala Asn Ala Ser Tyr Gln Arg Gln Pro Pro Ser
            100                 105                 110

Ile Ser Ser Thr Arg Glu Thr Gln Gly Trp Ser Val Gln Val Gly Gln

115                 120                 125

Thr Leu Phe Asp Ala Ala Lys Phe Ala Gln Tyr Arg Gln Ser Arg Phe
    130                 135                 140

Asp Thr Gln Ala Ala Glu Gln Arg Phe Asp Ala Ala Arg Glu Glu Leu
145                 150                 155                 160

Leu Leu Lys Val Ala Glu Ser Tyr Phe Asn Val Leu Leu Ser Arg Asp
                165                 170                 175

Thr Val Ala Ala His Ala Ala Glu Lys Glu Ala Tyr Ala Gln Gln Val
            180                 185                 190

Arg Gln Ala Gln Ala Leu Phe Asn Lys Gly Ala Ala Thr Ala Leu Asp
        195                 200                 205

Ile His Glu Ala Lys Ala Gly Tyr Asp Asn Ala Leu Ala Gln Glu Ile
    210                 215                 220

Ala Val Leu Ala Glu Lys Gln Thr Tyr Glu Asn Gln Leu Asn Asp Tyr
225                 230                 235                 240

Thr Asp Leu Asp Ser Lys Gln Ile Glu Ala Ile Asp Thr Ala Asn Leu
                245                 250                 255

Leu Ala Arg Tyr Leu Pro Lys Leu Glu Arg Tyr Ser Leu Asp Glu Trp
            260                 265                 270

Gln Arg Ile Ala Leu Ser Asn Asn His Glu Tyr Arg Met Gln Gln Leu
        275                 280                 285

Ala Leu Gln Ser Ser Gly Gln Ala Leu Arg Ala Ala Gln Asn Ser Arg
    290                 295                 300

Tyr Pro Thr Val Ser Ala His Val Gly Tyr Gln Asn Asn Leu Tyr Thr
305                 310                 315                 320

Ser Ser Ala Gln Asn Asn Asp Tyr His Tyr Arg Gly Lys Gly Met Ser
                325                 330                 335

Val Gly Val Gln Leu Asn Leu Pro Leu Tyr Thr Gly Gly Glu Leu Ser
            340                 345                 350

Gly Lys Ile His Glu Ala Glu Ala Gln Tyr Gly Ala Ala Glu Ala Gln
        355                 360                 365

Leu Thr Ala Thr Glu Arg His Ile Lys Leu Ala Val Arg Gln Ala Tyr
    370                 375                 380

Thr Glu Ser Gly Ala Ala Arg Tyr Gln Ile Met Ala Gln Glu Arg Val
385                 390                 395                 400

Leu Glu Ser Ser Arg Leu Lys Leu Lys Ser Thr Glu Thr Gly Gln Gln
                405                 410                 415

Tyr Gly Ile Arg Asn Arg Leu Glu Val Ile Arg Ala Arg Gln Glu Val
            420                 425                 430

Ala Gln Ala Glu Gln Lys Leu Ala Gln Ala Arg Tyr Lys Phe Met Leu

435                 440                 445

Ala Tyr Leu Arg Leu Val Lys Glu Ser Gly Leu Gly Leu Glu Thr Val
    450                 455                 460

Phe Ala Glu
465

<210> 31
<211> 696
<212> ADN
<213> Neisseria meningitidis

<220>
<221> CDS
<222> (1)..(693)

<400> 31
atg aaa caa tcc gcc cga ata aaa aat atg gat cag aca tta aaa aat      48
Met Lys Gln Ser Ala Arg Ile Lys Asn Met Asp Gln Thr Leu Lys Asn
1               5                   10                  15

aca ttg ggc att tgc gcg ctt tta gcc ttt tgt ttt ggc gcg gcc atc      96
Thr Leu Gly Ile Cys Ala Leu Leu Ala Phe Cys Phe Gly Ala Ala Ile
            20                  25                  30

gca tca ggt tat cac ttg gaa tat gaa tac ggc tac cgt tat tct gcc      144
Ala Ser Gly Tyr His Leu Glu Tyr Glu Tyr Gly Tyr Arg Tyr Ser Ala
        35                  40                  45

gtg ggt gct ttg gct tcg gtt gta ttt tta tta tta ttg gca cgc ggt      192
Val Gly Ala Leu Ala Ser Val Val Phe Leu Leu Leu Leu Ala Arg Gly
    50                  55                  60

ttc ccg cgc gtt tct tca gtt gtt tta ctg att tac gtc ggc aca acc      240
Phe Pro Arg Val Ser Ser Val Val Leu Leu Ile Tyr Val Gly Thr Thr
65                  70                  75                  80

gcc cta tat ttg ccg gtc ggc tgg ctg tat ggt gcg ccg tct tat cag      288
Ala Leu Tyr Leu Pro Val Gly Trp Leu Tyr Gly Ala Pro Ser Tyr Gln
                85                  90                  95

ata gtc ggt tcg ata ttg gaa agc aat cct gcc gag gcg cgt gaa ttt      336
Ile Val Gly Ser Ile Leu Glu Ser Asn Pro Ala Glu Ala Arg Glu Phe
            100                 105                 110

gtc ggc aat ctt ccc ggg tcg ctt tat ttt gtg cag gca tta ttt ttc      384
Val Gly Asn Leu Pro Gly Ser Leu Tyr Phe Val Gln Ala Leu Phe Phe
        115                 120                 125

att ttt ggc ttg aca gtt tgg aga tat tgt gta tcg ggg ggg gta ttt      432
Ile Phe Gly Leu Thr Val Trp Arg Tyr Cys Val Ser Gly Gly Val Phe
    130                 135                 140

gct gac gta aaa aac tat aaa cgc cgc agc aaa ata tgg ctg act ata      480
Ala Asp Val Lys Asn Tyr Lys Arg Arg Ser Lys Ile Trp Leu Thr Ile
145                 150                 155                 160

tta ttg act ttg att ttg tcc tgc gcg gtg atg gat aaa atc gcc agc     528
Leu Leu Thr Leu Ile Leu Ser Cys Ala Val Met Asp Lys Ile Ala Ser
                165                 170                 175

gat aaa gat ttg cga gaa cct gat gcc ggc ctg ttg ttg aat att ttc     576
Asp Lys Asp Leu Arg Glu Pro Asp Ala Gly Leu Leu Leu Asn Ile Phe
            180                 185                 190

gac ctg tat tac gat ttg gct tcc gcg ccg gca cca ata tgt cgc caa     624
Asp Leu Tyr Tyr Asp Leu Ala Ser Ala Pro Ala Pro Ile Cys Arg Gln
        195                 200                 205

gcg cgc cca cat ttt gga agc agc aaa aaa agc gtc aac atg gca tat     672
Ala Arg Pro His Phe Gly Ser Ser Lys Lys Ser Val Asn Met Ala Tyr
    210                 215                 220

ccg tca tgt tgc gcc caa gta taa                                     696
Pro Ser Cys Cys Ala Gln Val
225                 230


<210> 32
<211> 231
<212> PRT
<213> Neisseria meningitidis

<400> 32
Met Lys Gln Ser Ala Arg Ile Lys Asn Met Asp Gln Thr Leu Lys Asn
1               5                   10                  15

Thr Leu Gly Ile Cys Ala Leu Leu Ala Phe Cys Phe Gly Ala Ala Ile
            20                  25                  30

Ala Ser Gly Tyr His Leu Glu Tyr Glu Tyr Gly Tyr Arg Tyr Ser Ala
        35                  40                  45

Val Gly Ala Leu Ala Ser Val Val Phe Leu Leu Leu Leu Ala Arg Gly
    50                  55                  60

Phe Pro Arg Val Ser Ser Val Val Leu Leu Ile Tyr Val Gly Thr Thr
65                  70                  75                  80

Ala Leu Tyr Leu Pro Val Gly Trp Leu Tyr Gly Ala Pro Ser Tyr Gln
                85                  90                  95

Ile Val Gly Ser Ile Leu Glu Ser Asn Pro Ala Glu Ala Arg Glu Phe
            100                 105                 110

Val Gly Asn Leu Pro Gly Ser Leu Tyr Phe Val Gln Ala Leu Phe Phe
        115                 120                 125

Ile Phe Gly Leu Thr Val Trp Arg Tyr Cys Val Ser Gly Gly Val Phe
    130                 135                 140

Ala Asp Val Lys Asn Tyr Lys Arg Arg Ser Lys Ile Trp Leu Thr Ile
145                 150                 155                 160

Leu Leu Thr Leu Ile Leu Ser Cys Ala Val Met Asp Lys Ile Ala Ser
                165                 170                 175

Asp Lys Asp Leu Arg Glu Pro Asp Ala Gly Leu Leu Leu Asn Ile Phe
            180                 185                 190

Asp Leu Tyr Tyr Asp Leu Ala Ser Ala Pro Ala Pro Ile Cys Arg Gln
        195                 200                 205

Ala Arg Pro His Phe Gly Ser Ser Lys Lys Ser Val Asn Met Ala Tyr
    210                 215                 220

Pro Ser Cys Cys Ala Gln Val
225                 230

<210> 33
<211> 909
<212> ADN
<213> Neisseria meningitidis

<220>
<221> CDS
<222> (1)..(906)

<400> 33
atg aat gtt tac ggt ttc cca ttg ccc gat acg cct ttt ttg agt cgg 48
Met Asn Val Tyr Gly Phe Pro Leu Pro Asp Thr Pro Phe Leu Ser Arg
1               5                   10                  15

acc aaa ggg ctg ttg ata aac ggt tac cat ttc acc gcc cac gcg acg 96
Thr Lys Gly Leu Leu Ile Asn Gly Tyr His Phe Thr Ala His Ala Thr
            20                  25                  30

aat ctt tcg ctg ccg cag act ttg ggg ctg ccg gga gag ccg aac aat 144
Asn Leu Ser Leu Pro Gln Thr Leu Gly Leu Pro Gly Glu Pro Asn Asn
        35                  40                  45

aac att gtc agc ttg gcg aag cag gcg ggt ttt cgg acg gcg tgg ctg 192
Asn Ile Val Ser Leu Ala Lys Gln Ala Gly Phe Arg Thr Ala Trp Leu
    50                  55                  60

tct aat caa gga atg ttg ggg cat ttt gcc aac gaa att tcc acc tat 240
Ser Asn Gln Gly Met Leu Gly His Phe Ala Asn Glu Ile Ser Thr Tyr
65                  70                  75                  80

gcc cta cgc agc gat tat ccg tgg ttt acc caa agg ggt gat tat ggc 288
Ala Leu Arg Ser Asp Tyr Pro Trp Phe Thr Gln Arg Gly Asp Tyr Gly
                85                  90                  95

aaa agc gcg ggg ttg agc gac cgc ctt ttg ttg ccg gcg ttc aaa cgg 336
Lys Ser Ala Gly Leu Ser Asp Arg Leu Leu Leu Pro Ala Phe Lys Arg
            100                 105                 110

gtt ttg ata gga aat gca ggc acg aag cct cgg ctg att gtg atg cac 384
Val Leu Ile Gly Asn Ala Gly Thr Lys Pro Arg Leu Ile Val Met His
        115                 120                 125

ctg atg ggt tcg cac agt gat ttt tgc aca cgt ttg gat aag gat gcg 432
Leu Met Gly Ser His Ser Asp Phe Cys Thr Arg Leu Asp Lys Asp Ala
    130                 135                 140

cgg cgg ttt cag tat caa act gaa aaa ata tcc tgc tat gtt tcc acc     480
Arg Arg Phe Gln Tyr Gln Thr Glu Lys Ile Ser Cys Tyr Val Ser Thr
145                 150                 155                 160

atc gcg caa acc gat aaa ttt tta gaa gat aca gtt aag ata ttg aat     528
Ile Ala Gln Thr Asp Lys Phe Leu Glu Asp Thr Val Lys Ile Leu Asn
                165                 170                 175

gaa aat aaa gaa agc tgg tct ttg gtt tac ttt tcc gac cac ggt ttg     576
Glu Asn Lys Glu Ser Trp Ser Leu Val Tyr Phe Ser Asp His Gly Leu
            180                 185                 190

atg cat gtc ggt aaa ggc ggc gag cga acg ttg aca cat ggt gcg tgg     624
Met His Val Gly Lys Gly Gly Glu Arg Thr Leu Thr His Gly Ala Trp
        195                 200                 205

aag cgt caa agc tac ggc gtg ccg ctg gtt aaa att tcg tcc gat gac     672
Lys Arg Gln Ser Tyr Gly Val Pro Leu Val Lys Ile Ser Ser Asp Asp
    210                 215                 220

acg cgg cgc gaa atg att aaa gtg agg cgc agc gcg ttt aat ttt tta     720
Thr Arg Arg Glu Met Ile Lys Val Arg Arg Ser Ala Phe Asn Phe Leu
225                 230                 235                 240

cgc gga ttc ggc agt tgg acg ggt atc gaa acc gac gag ttg ccc gat     768
Arg Gly Phe Gly Ser Trp Thr Gly Ile Glu Thr Asp Glu Leu Pro Asp
                245                 250                 255

gac ggc tat gat ttt tgg ggg aat gtt ccc gat gtg cag ggc gaa ggc     816
Asp Gly Tyr Asp Phe Trp Gly Asn Val Pro Asp Val Gln Gly Glu Gly
            260                 265                 270

aat aac ctt gcc ttt atc gac gga ctg ccc gac gac ccc gcg ccg tgg     864
Asn Asn Leu Ala Phe Ile Asp Gly Leu Pro Asp Asp Pro Ala Pro Trp
        275                 280                 285

tat gcg gga aaa ggc aaa tcg act aaa aat acg tct aaa aaa tga         909
Tyr Ala Gly Lys Gly Lys Ser Thr Lys Asn Thr Ser Lys Lys
    290                 295                 300

<210> 34
<211> 302
<212> PRT
<213> Neisseria meningitidis

<400> 34
Met Asn Val Tyr Gly Phe Pro Leu Pro Asp Thr Pro Phe Leu Ser Arg
  1               5                  10                  15

Thr Lys Gly Leu Leu Ile Asn Gly Tyr His Phe Thr Ala His Ala Thr
             20                  25                  30

Asn Leu Ser Leu Pro Gln Thr Leu Gly Leu Pro Gly Glu Pro Asn Asn
         35                  40                  45

Asn Ile Val Ser Leu Ala Lys Gln Ala Gly Phe Arg Thr Ala Trp Leu
     50                  55                  60

Ser Asn Gln Gly Met Leu Gly His Phe Ala Asn Glu Ile Ser Thr Tyr

65                  70                  75                  80

Ala Leu Arg Ser Asp Tyr Pro Trp Phe Thr Gln Arg Gly Asp Tyr Gly
                85                  90                  95

Lys Ser Ala Gly Leu Ser Asp Arg Leu Leu Leu Pro Ala Phe Lys Arg
            100                 105                 110

Val Leu Ile Gly Asn Ala Gly Thr Lys Pro Arg Leu Ile Val Met His
        115                 120                 125

Leu Met Gly Ser His Ser Asp Phe Cys Thr Arg Leu Asp Lys Asp Ala
    130                 135                 140

Arg Arg Phe Gln Tyr Gln Thr Glu Lys Ile Ser Cys Tyr Val Ser Thr
145                 150                 155                 160

Ile Ala Gln Thr Asp Lys Phe Leu Glu Asp Thr Val Lys Ile Leu Asn
                165                 170                 175

Glu Asn Lys Glu Ser Trp Ser Leu Val Tyr Phe Ser Asp His Gly Leu
            180                 185                 190

Met His Val Gly Lys Gly Gly Glu Arg Thr Leu Thr His Gly Ala Trp
        195                 200                 205

Lys Arg Gln Ser Tyr Gly Val Pro Leu Val Lys Ile Ser Ser Asp Asp
    210                 215                 220

Thr Arg Arg Glu Met Ile Lys Val Arg Arg Ser Ala Phe Asn Phe Leu
225                 230                 235                 240

Arg Gly Phe Gly Ser Trp Thr Gly Ile Glu Thr Asp Glu Leu Pro Asp
                245                 250                 255

Asp Gly Tyr Asp Phe Trp Gly Asn Val Pro Asp Val Gln Gly Glu Gly
            260                 265                 270

Asn Asn Leu Ala Phe Ile Asp Gly Leu Pro Asp Asp Pro Ala Pro Trp
        275                 280                 285

Tyr Ala Gly Lys Gly Lys Ser Thr Lys Asn Thr Ser Lys Lys
    290                 295                 300

<210> 35
<211> 864
<212> ADN
<213> Neisseria meningitidis

<220>
<221> CDS
<222> (1)..(861)

<400> 35
atg atg agt caa cac tct gcc gga gca cgt ttc cgc caa gcc gtg aaa     48
Met Met Ser Gln His Ser Ala Gly Ala Arg Phe Arg Gln Ala Val Lys
1               5                   10                  15

gaa tcg aat ccg ctt gcc gtc gcc ggt tgc gtc aat gct tat ttt gca 96
Glu Ser Asn Pro Leu Ala Val Ala Gly Cys Val Asn Ala Tyr Phe Ala
            20                  25                  30

cga ttg gcc acc caa agc ggt ttc aaa gcc atc tat ctg tcc ggc ggc 144
Arg Leu Ala Thr Gln Ser Gly Phe Lys Ala Ile Tyr Leu Ser Gly Gly
        35                  40                  45

ggc gtg gca gcc tgt tct tgc ggt atc cct gat ttg ggc att acc aca 192
Gly Val Ala Ala Cys Ser Cys Gly Ile Pro Asp Leu Gly Ile Thr Thr
    50                  55                  60

atg gaa gat gtg ctg atc gac gca cga cgc att acg gac aac gtg gat 240
Met Glu Asp Val Leu Ile Asp Ala Arg Arg Ile Thr Asp Asn Val Asp
65                  70                  75                  80

acg cct ctg ctg gtg gac atc gat gtg ggt tgg ggc ggt gca ttc aat 288
Thr Pro Leu Leu Val Asp Ile Asp Val Gly Trp Gly Gly Ala Phe Asn
                85                  90                  95

att gcc cgt acc att cgc aac ttt gaa cgc gcc ggt gtt gca gcg gtt 336
Ile Ala Arg Thr Ile Arg Asn Phe Glu Arg Ala Gly Val Ala Ala Val
            100                 105                 110

cac atc gaa gat cag gta gcg caa aaa cgc tgc ggc cac cgt ccg aac 384
His Ile Glu Asp Gln Val Ala Gln Lys Arg Cys Gly His Arg Pro Asn
        115                 120                 125

aaa gcc att gta tct aaa gat gaa atg gtc gac cgt atc aaa gct gcc 432
Lys Ala Ile Val Ser Lys Asp Glu Met Val Asp Arg Ile Lys Ala Ala
    130                 135                 140

gta gat gcg cgc gtt gat gag aac ttc gtg att atg gcg cgt acc gat 480
Val Asp Ala Arg Val Asp Glu Asn Phe Val Ile Met Ala Arg Thr Asp
145                 150                 155                 160

gcg ctg gcg gta gaa ggt ttg gat gcc gct atc gaa cgc gcc caa gct 528
Ala Leu Ala Val Glu Gly Leu Asp Ala Ala Ile Glu Arg Ala Gln Ala
                165                 170                 175

tgt gtc gaa gcc ggt gcg gac atg att ttc cct gaa gcc atg acc gat 576
Cys Val Glu Ala Gly Ala Asp Met Ile Phe Pro Glu Ala Met Thr Asp
            180                 185                 190

ttg aac atg tac cgc caa ttt gca gat gcg gtg aaa gtg ccc gtg ttg 624
Leu Asn Met Tyr Arg Gln Phe Ala Asp Ala Val Lys Val Pro Val Leu
        195                 200                 205

gcg aac att acc gag ttt ggt tcc act ccg ctt tat acc caa agc gag 672
Ala Asn Ile Thr Glu Phe Gly Ser Thr Pro Leu Tyr Thr Gln Ser Glu
    210                 215                 220

ctg gct gaa aac ggc gtg tcg ctg gtg ctg tat ccg ctg tca tcg ttc 720
Leu Ala Glu Asn Gly Val Ser Leu Val Leu Tyr Pro Leu Ser Ser Phe
225                 230                 235                 240

cgt gca gca agc aaa gcc gct ctg aat gtt tac gaa gcg att atg cgc 768
Arg Ala Ala Ser Lys Ala Ala Leu Asn Val Tyr Glu Ala Ile Met Arg
                245                 250                 255

gat ggc act tca ggc ggc ggt ggt gga cag tat gca aac ccg tgc cga    816
Asp Gly Thr Ser Gly Gly Gly Gly Gly Gln Tyr Ala Asn Pro Cys Arg
            260                 265                 270

gct gta cga gca tct gaa cta tca tgc ctt cga gca aaa act gga taa    864
Ala Val Arg Ala Ser Glu Leu Ser Cys Leu Arg Ala Lys Thr Gly
        275                 280                 285

<210> 36
<211> 287
<212> PRT
<213> Neisseria meningitidis

<400> 36
Met Met Ser Gln His Ser Ala Gly Ala Arg Phe Arg Gln Ala Val Lys
1               5                   10                  15

Glu Ser Asn Pro Leu Ala Val Ala Gly Cys Val Asn Ala Tyr Phe Ala
            20                  25                  30

Arg Leu Ala Thr Gln Ser Gly Phe Lys Ala Ile Tyr Leu Ser Gly Gly
        35                  40                  45

Gly Val Ala Ala Cys Ser Cys Gly Ile Pro Asp Leu Gly Ile Thr Thr
    50                  55                  60

Met Glu Asp Val Leu Ile Asp Ala Arg Arg Ile Thr Asp Asn Val Asp
65                  70                  75                  80

Thr Pro Leu Leu Val Asp Ile Asp Val Gly Trp Gly Gly Ala Phe Asn
                85                  90                  95

Ile Ala Arg Thr Ile Arg Asn Phe Glu Arg Ala Gly Val Ala Ala Val
            100                 105                 110

His Ile Glu Asp Gln Val Ala Gln Lys Arg Cys Gly His Arg Pro Asn
        115                 120                 125

Lys Ala Ile Val Ser Lys Asp Glu Met Val Asp Arg Ile Lys Ala Ala
    130                 135                 140

Val Asp Ala Arg Val Asp Glu Asn Phe Val Ile Met Ala Arg Thr Asp
145                 150                 155                 160

Ala Leu Ala Val Glu Gly Leu Asp Ala Ala Ile Glu Arg Ala Gln Ala
                165                 170                 175

Cys Val Glu Ala Gly Ala Asp Met Ile Phe Pro Glu Ala Met Thr Asp
            180                 185                 190

Leu Asn Met Tyr Arg Gln Phe Ala Asp Ala Val Lys Val Pro Val Leu
        195                 200                 205

Ala Asn Ile Thr Glu Phe Gly Ser Thr Pro Leu Tyr Thr Gln Ser Glu
    210                 215                 220

Leu Ala Glu Asn Gly Val Ser Leu Val Leu Tyr Pro Leu Ser Ser Phe
225                 230                 235                 240

Arg Ala Ala Ser Lys Ala Ala Leu Asn Val Tyr Glu Ala Ile Met Arg
                245                 250                 255

Asp Gly Thr Ser Gly Gly Gly Gly Gly Gln Tyr Ala Asn Pro Cys Arg
            260                 265                 270

Ala Val Arg Ala Ser Glu Leu Ser Cys Leu Arg Ala Lys Thr Gly
        275                 280                 285

<210> 37
<211> 921
<212> ADN
<213> Neisseria meningitidis

<220>
<221> CDS
<222> (1)..(918)

<400> 37
atg cct tcg agc aaa aac tgg ata aat tgt ttc aaa aat gat tta ccg 48
Met Pro Ser Ser Lys Asn Trp Ile Asn Cys Phe Lys Asn Asp Leu Pro
1               5                   10                  15

ctt tca gac tgc ctt tca aca aat ccg cat cgg tcg tct gaa aac ccg 96
Leu Ser Asp Cys Leu Ser Thr Asn Pro His Arg Ser Ser Glu Asn Pro
            20                  25                  30

aaa ccc ata aaa aca caa agg aga aat acc atg act gaa act act caa 144
Lys Pro Ile Lys Thr Gln Arg Arg Asn Thr Met Thr Glu Thr Thr Gln
        35                  40                  45

acc ccg acc ctc aaa cct aaa aaa tcc gtt gcg ctt tct ggc gtt gcg 192
Thr Pro Thr Leu Lys Pro Lys Lys Ser Val Ala Leu Ser Gly Val Ala
    50                  55                  60

gcc ggt aat acc gct ttg tgt acc gtt ggc cgt acc ggc aac gat ttg 240
Ala Gly Asn Thr Ala Leu Cys Thr Val Gly Arg Thr Gly Asn Asp Leu
65                  70                  75                  80

agc tat cgc ggt tac gac att ctg gat ttg gca caa aaa tgt gag ttt 288
Ser Tyr Arg Gly Tyr Asp Ile Leu Asp Leu Ala Gln Lys Cys Glu Phe
                85                  90                  95

gaa gaa gtt gcc cac ctg ctg att cac ggc cat tta ccc aac aaa ttc 336
Glu Glu Val Ala His Leu Leu Ile His Gly His Leu Pro Asn Lys Phe
            100                 105                 110

gag ctg gcc gct tat aaa gcc aag ctc aaa tcc atg cgc ggc ctg cct 384
Glu Leu Ala Ala Tyr Lys Ala Lys Leu Lys Ser Met Arg Gly Leu Pro
        115                 120                 125

atc cgt gtg att aaa gtt ttg gaa agc ctg cct gca cat acc cat ccg 432
Ile Arg Val Ile Lys Val Leu Glu Ser Leu Pro Ala His Thr His Pro
    130                 135                 140

atg gac gtg atg cgt acc ggc gta tcc atg ctg ggc tgt gtt cat cct 480
Met Asp Val Met Arg Thr Gly Val Ser Met Leu Gly Cys Val His Pro
145                 150                 155                 160

gaa cgt gaa ggc cat ccg gaa agc gaa gcg cgc gac att gcc gac aaa     528
Glu Arg Glu Gly His Pro Glu Ser Glu Ala Arg Asp Ile Ala Asp Lys
                165                 170                 175

ctg atc gcc agc ctc ggc agt atc ctc ttg tac tgg tat caa tat tcg     576
Leu Ile Ala Ser Leu Gly Ser Ile Leu Leu Tyr Trp Tyr Gln Tyr Ser
            180                 185                 190

cac aac ggc aaa cgc att gaa gtt gaa agc gaa gaa gag acc atc ggc     624
His Asn Gly Lys Arg Ile Glu Val Glu Ser Glu Glu Glu Thr Ile Gly
        195                 200                 205

ggt cat ttc ctg cac ctg ttg cac ggc aaa cgc cca agc gaa tca cac     672
Gly His Phe Leu His Leu Leu His Gly Lys Arg Pro Ser Glu Ser His
    210                 215                 220

atc aaa gcc atg cac gtt tca ctg att ctg tat gcc gaa cac gag ttc     720
Ile Lys Ala Met His Val Ser Leu Ile Leu Tyr Ala Glu His Glu Phe
225                 230                 235                 240

aac gct tct acc ttt acc gcc cgc gtg atc gcc ggt aca ggc tct gat     768
Asn Ala Ser Thr Phe Thr Ala Arg Val Ile Ala Gly Thr Gly Ser Asp
                245                 250                 255

atg tac tcc agc att acc gga gca atc ggc gcg ttg aaa ggt ccg aaa     816
Met Tyr Ser Ser Ile Thr Gly Ala Ile Gly Ala Leu Lys Gly Pro Lys
            260                 265                 270

cac ggc ggc gcg aac gaa ggg ctt acg ata ttc aaa aac gct acc gca     864
His Gly Gly Ala Asn Glu Gly Leu Thr Ile Phe Lys Asn Ala Thr Ala
        275                 280                 285

atg ccg acg aag ccg aag ccg aca tcc gcg aac gca tcg gcc gca aag     912
Met Pro Thr Lys Pro Lys Pro Thr Ser Ala Asn Ala Ser Ala Ala Lys
    290                 295                 300

aaa tcg tga                                                         921
Lys Ser
305

<210> 38
<211> 306
<212> PRT
<213> Neisseria meningitidis

<400> 38
Met Pro Ser Ser Lys Asn Trp Ile Asn Cys Phe Lys Asn Asp Leu Pro
1               5                   10                  15

Leu Ser Asp Cys Leu Ser Thr Asn Pro His Arg Ser Ser Glu Asn Pro
            20                  25                  30

Lys Pro Ile Lys Thr Gln Arg Arg Asn Thr Met Thr Glu Thr Thr Gln
        35                  40                  45

Thr Pro Thr Leu Lys Pro Lys Lys Ser Val Ala Leu Ser Gly Val Ala
    50                  55                  60

Ala Gly Asn Thr Ala Leu Cys Thr Val Gly Arg Thr Gly Asn Asp Leu
65                  70                  75                  80

Ser Tyr Arg Gly Tyr Asp Ile Leu Asp Leu Ala Gln Lys Cys Glu Phe
                85                  90                  95

Glu Glu Val Ala His Leu Leu Ile His Gly His Leu Pro Asn Lys Phe
            100                 105                 110

Glu Leu Ala Ala Tyr Lys Ala Lys Leu Lys Ser Met Arg Gly Leu Pro
        115                 120                 125

Ile Arg Val Ile Lys Val Leu Glu Ser Leu Pro Ala His Thr His Pro
    130                 135                 140

Met Asp Val Met Arg Thr Gly Val Ser Met Leu Gly Cys Val His Pro
145                 150                 155                 160

Glu Arg Glu Gly His Pro Glu Ser Glu Ala Arg Asp Ile Ala Asp Lys
                165                 170                 175

Leu Ile Ala Ser Leu Gly Ser Ile Leu Leu Tyr Trp Tyr Gln Tyr Ser
            180                 185                 190

His Asn Gly Lys Arg Ile Glu Val Glu Ser Glu Glu Glu Thr Ile Gly
        195                 200                 205

Gly His Phe Leu His Leu Leu His Gly Lys Arg Pro Ser Glu Ser His
    210                 215                 220

Ile Lys Ala Met His Val Ser Leu Ile Leu Tyr Ala Glu His Glu Phe
225                 230                 235                 240

Asn Ala Ser Thr Phe Thr Ala Arg Val Ile Ala Gly Thr Gly Ser Asp
                245                 250                 255

Met Tyr Ser Ser Ile Thr Gly Ala Ile Gly Ala Leu Lys Gly Pro Lys
            260                 265                 270

His Gly Gly Ala Asn Glu Gly Leu Thr Ile Phe Lys Asn Ala Thr Ala
        275                 280                 285

Met Pro Thr Lys Pro Lys Pro Thr Ser Ala Asn Ala Ser Ala Ala Lys
    290                 295                 300

Lys Ser
305

<210> 39
<211> 945
<212> ADN
<213> Neisseria meningitidis

<220>
<221> CDS
<222> (1)..(942)

<400> 39

atg cac cta tgt gga aag tat tat gga gta aat atg aag ctg cgt gat 48
Met His Leu Cys Gly Lys Tyr Tyr Gly Val Asn Met Lys Leu Arg Asp
1               5                   10                  15

tta ctg atg gga ata ttc ttg gca gtt tct gcg gcc ctt ctg aat gca 96
Leu Leu Met Gly Ile Phe Leu Ala Val Ser Ala Ala Leu Leu Asn Ala
            20                  25                  30

acc atc ggc ata ttc agc aag ata ttg atg gag cag ggc ttg tct gtt 144
Thr Ile Gly Ile Phe Ser Lys Ile Leu Met Glu Gln Gly Leu Ser Val
        35                  40                  45

cag cat att gca ttt ttg aaa act ttg aca ggt ttc gtg ttt atc agc 192
Gln His Ile Ala Phe Leu Lys Thr Leu Thr Gly Phe Val Phe Ile Ser
    50                  55                  60

att ttg ctt tgc cgt acc ggt ttt acc aga cag att gcg gat att tca 240
Ile Leu Leu Cys Arg Thr Gly Phe Thr Arg Gln Ile Ala Asp Ile Ser
65                  70                  75                  80

aga aag aaa gag gca att ttg ccg ttg ctg tta aaa gta gca att tgt 288
Arg Lys Lys Glu Ala Ile Leu Pro Leu Leu Leu Lys Val Ala Ile Cys
                85                  90                  95

gct ttt ttc gga att tat acg ttg ttt ttc ttt gaa acc aca gct tat 336
Ala Phe Phe Gly Ile Tyr Thr Leu Phe Phe Phe Glu Thr Thr Ala Tyr
            100                 105                 110

caa tat ggc aat gct gcg aat gta gta gtt gta tta atg gca tcg gct 384
Gln Tyr Gly Asn Ala Ala Asn Val Val Val Val Leu Met Ala Ser Ala
        115                 120                 125

gcc gta tct gcc ttg ata ttg gac agc ata ctg tta gat gaa cgt att 432
Ala Val Ser Ala Leu Ile Leu Asp Ser Ile Leu Leu Asp Glu Arg Ile
    130                 135                 140

tgc att tct tca gtc gtc ggt gtg ggt ttg gca gta ttg ggg atc gca 480
Cys Ile Ser Ser Val Val Gly Val Gly Leu Ala Val Leu Gly Ile Ala
145                 150                 155                 160

atg att tct tgg act gga gaa gga agt tta ggg ttg att ctg aat gcc 528
Met Ile Ser Trp Thr Gly Glu Gly Ser Leu Gly Leu Ile Leu Asn Ala
                165                 170                 175

gca ctg gcg ggc tcg ggc tac ggt tgt ttt tcc gtt ttg att aag aaa 576
Ala Leu Ala Gly Ser Gly Tyr Gly Cys Phe Ser Val Leu Ile Lys Lys
            180                 185                 190

ttc ggc cta aac ggc ggt att tat ttg aca cgg ata ttg atg ttt ttt 624
Phe Gly Leu Asn Gly Gly Ile Tyr Leu Thr Arg Ile Leu Met Phe Phe
        195                 200                 205

gga agt att ttt ttg ttt atc cct tca ttg gaa ggt att gag gat ata 672
Gly Ser Ile Phe Leu Phe Ile Pro Ser Leu Glu Gly Ile Glu Asp Ile
    210                 215                 220

cat tgg caa tgg tct ttt att ccg cca ctc ttg gca ttg tct tta ttg 720
His Trp Gln Trp Ser Phe Ile Pro Pro Leu Leu Ala Leu Ser Leu Leu
225                 230                 235                 240

ccg acg att tta gga ttt tat tgt aca act aaa gca ttg gat tat ttg     768
Pro Thr Ile Leu Gly Phe Tyr Cys Thr Thr Lys Ala Leu Asp Tyr Leu
                245                 250                 255

agt gct gcg aag gta cag gta act gaa ttg gcc gag cca ttg ttt gct     816
Ser Ala Ala Lys Val Gln Val Thr Glu Leu Ala Glu Pro Leu Phe Ala
            260                 265                 270

gcc gta ctg gct tgg ttg ttt ttg aat gaa ata ccg gaa gga cgc ttc     864
Ala Val Leu Ala Trp Leu Phe Leu Asn Glu Ile Pro Glu Gly Arg Phe
        275                 280                 285

ttt gtc ggc gcc att ctg att att gcc ggt att gtg tct atc aat ggg     912
Phe Val Gly Ala Ile Leu Ile Ile Ala Gly Ile Val Ser Ile Asn Gly
    290                 295                 300

ctg tat cga cca ttg ttg aag cga att gaa taa                         945
Leu Tyr Arg Pro Leu Leu Lys Arg Ile Glu
305                 310


<210> 40
<211> 314
<212> PRT
<213> Neisseria meningitidis

<400> 40
Met His Leu Cys Gly Lys Tyr Tyr Gly Val Asn Met Lys Leu Arg Asp
1               5                   10                  15

Leu Leu Met Gly Ile Phe Leu Ala Val Ser Ala Ala Leu Leu Asn Ala
            20                  25                  30

Thr Ile Gly Ile Phe Ser Lys Ile Leu Met Glu Gln Gly Leu Ser Val
        35                  40                  45

Gln His Ile Ala Phe Leu Lys Thr Leu Thr Gly Phe Val Phe Ile Ser
    50                  55                  60

Ile Leu Leu Cys Arg Thr Gly Phe Thr Arg Gln Ile Ala Asp Ile Ser
65                  70                  75                  80

Arg Lys Lys Glu Ala Ile Leu Pro Leu Leu Leu Lys Val Ala Ile Cys
                85                  90                  95

Ala Phe Phe Gly Ile Tyr Thr Leu Phe Phe Phe Glu Thr Thr Ala Tyr
            100                 105                 110

Gln Tyr Gly Asn Ala Ala Asn Val Val Val Val Leu Met Ala Ser Ala
        115                 120                 125

Ala Val Ser Ala Leu Ile Leu Asp Ser Ile Leu Leu Asp Glu Arg Ile
    130                 135                 140

Cys Ile Ser Ser Val Val Gly Val Gly Leu Ala Val Leu Gly Ile Ala
145                 150                 155                 160

Met Ile Ser Trp Thr Gly Glu Gly Ser Leu Gly Leu Ile Leu Asn Ala
                165                 170                 175

Ala Leu Ala Gly Ser Gly Tyr Gly Cys Phe Ser Val Leu Ile Lys Lys
            180                 185                 190

Phe Gly Leu Asn Gly Gly Ile Tyr Leu Thr Arg Ile Leu Met Phe Phe
        195                 200                 205

Gly Ser Ile Phe Leu Phe Ile Pro Ser Leu Glu Gly Ile Glu Asp Ile
    210                 215                 220

His Trp Gln Trp Ser Phe Ile Pro Pro Leu Leu Ala Leu Ser Leu Leu
225                 230                 235                 240

Pro Thr Ile Leu Gly Phe Tyr Cys Thr Thr Lys Ala Leu Asp Tyr Leu
                245                 250                 255

Ser Ala Ala Lys Val Gln Val Thr Glu Leu Ala Glu Pro Leu Phe Ala
            260                 265                 270

Ala Val Leu Ala Trp Leu Phe Leu Asn Glu Ile Pro Glu Gly Arg Phe
        275                 280                 285

Phe Val Gly Ala Ile Leu Ile Ile Ala Gly Ile Val Ser Ile Asn Gly
    290                 295                 300

Leu Tyr Arg Pro Leu Leu Lys Arg Ile Glu
305                 310

<210> 41
<211> 2610
<212> ADN
<213> Neisseria meningitidis

<220>
<221> CDS
<222> (1)..(2607)

<400> 41
atg gct gcc aac caa cgt tac cgc aaa ccg ctg ccc ggt acg gat ttg      48
Met Ala Ala Asn Gln Arg Tyr Arg Lys Pro Leu Pro Gly Thr Asp Leu
1               5                   10                  15

gaa tac tac gac gcg cgt gcg gcg tgt gag gac atc aag ccc ggc tct      96
Glu Tyr Tyr Asp Ala Arg Ala Ala Cys Glu Asp Ile Lys Pro Gly Ser
            20                  25                  30

tac gac aag ctg cct tac acg agc cgc att ttg gcg gag aat ttg gtc     144
Tyr Asp Lys Leu Pro Tyr Thr Ser Arg Ile Leu Ala Glu Asn Leu Val
        35                  40                  45

aac cgc gcg gac aaa gtc gat ttg ccg acg ctg caa agc tgg ctg ggt     192
Asn Arg Ala Asp Lys Val Asp Leu Pro Thr Leu Gln Ser Trp Leu Gly
    50                  55                  60

cag ctg att gag gga aaa cag gaa atc gac ttt cct tgg tat ccg gcg     240
Gln Leu Ile Glu Gly Lys Gln Glu Ile Asp Phe Pro Trp Tyr Pro Ala
65                  70                  75                  80

cgg gtg gtg tgc cac gat att ctg ggg cag acc gcg ttg gtg gat ttg     288

Arg Val Val Cys His Asp Ile Leu Gly Gln Thr Ala Leu Val Asp Leu
                85                  90                  95

gca ggt ctg cgc gat gcg att gcc gaa aaa ggc ggc gat cct gcc aaa     336
Ala Gly Leu Arg Asp Ala Ile Ala Glu Lys Gly Gly Asp Pro Ala Lys
            100                 105                 110

gtg aat ccg gtg gtt gca aaa ccc agc ttc atc gtc gac cac tct ctg     384
Val Asn Pro Val Val Ala Lys Pro Ser Phe Ile Val Asp His Ser Leu
        115                 120                 125

gcc gtt gaa tgc ggc ggc tac gac ccc gat gcc ttc cgc aaa aac cgc     432
Ala Val Glu Cys Gly Gly Tyr Asp Pro Asp Ala Phe Arg Lys Asn Arg
    130                 135                 140

caa atc gaa gac aga cgt aac gaa gac cgt ttc cac ttc atc aac tgg     480
Gln Ile Glu Asp Arg Arg Asn Glu Asp Arg Phe His Phe Ile Asn Trp
145                 150                 155                 160

aca aaa acc gca ttt gaa aat gtg gac gtg att ccg gcg ggc aac ggc     528
Thr Lys Thr Ala Phe Glu Asn Val Asp Val Ile Pro Ala Gly Asn Gly
                165                 170                 175

atc atg cac caa atc aat cta gaa aaa atg tcg ccc gtc gtc caa gtc     576
Ile Met His Gln Ile Asn Leu Glu Lys Met Ser Pro Val Val Gln Val
            180                 185                 190

aaa aac ggc gtg gcg ttc ccc gat acc tgc gtc ggc acg gat tcg cac     624
Lys Asn Gly Val Ala Phe Pro Asp Thr Cys Val Gly Thr Asp Ser His
        195                 200                 205

acg ccg cac gtc gat gcg ctg ggc gtg att tcc gtg ggc gtg ggc gga     672
Thr Pro His Val Asp Ala Leu Gly Val Ile Ser Val Gly Val Gly Gly
    210                 215                 220

ttg gaa gcg gaa acc gtg atg ctg ggt cgc gcg tcc atg atg cgc ctg     720
Leu Glu Ala Glu Thr Val Met Leu Gly Arg Ala Ser Met Met Arg Leu
225                 230                 235                 240

ccc gat att gtc ggc gtt gag ctg aac ggc aaa cgg cag gcg ggc att     768
Pro Asp Ile Val Gly Val Glu Leu Asn Gly Lys Arg Gln Ala Gly Ile
                245                 250                 255

acg gcg acg gat att gtg ttg gca ctg acc gag ttt ctg cgc aaa gaa     816
Thr Ala Thr Asp Ile Val Leu Ala Leu Thr Glu Phe Leu Arg Lys Glu
            260                 265                 270

cgc gtg gtc ggg gcg ttt gtc gaa ttc ttc ggc gag ggc gcg aga agc     864
Arg Val Val Gly Ala Phe Val Glu Phe Phe Gly Glu Gly Ala Arg Ser
        275                 280                 285

ctg tct atc ggc gac cgc gcg acc att tcc aac atg acg ccg gag ttc     912
Leu Ser Ile Gly Asp Arg Ala Thr Ile Ser Asn Met Thr Pro Glu Phe
    290                 295                 300

ggc gcg act gcc gcg atg ttc gct att gat gag caa acc att gat tat     960
Gly Ala Thr Ala Ala Met Phe Ala Ile Asp Glu Gln Thr Ile Asp Tyr
305                 310                 315                 320

ttg aaa ctg acc gga cgc gac gac gcg cag gtg aaa ttg gtg gaa acc     1008

Leu Lys Leu Thr Gly Arg Asp Asp Ala Gln Val Lys Leu Val Glu Thr
                325                 330                 335

tac gcc aaa acc gca ggc tta tgg gca gat gcc ttg aaa acc gcc gtt 1056
Tyr Ala Lys Thr Ala Gly Leu Trp Ala Asp Ala Leu Lys Thr Ala Val
            340                 345                 350

tat ccg cgc gtt ttg aaa ttt gat ttg agc agc gta acg cgc aat atg 1104
Tyr Pro Arg Val Leu Lys Phe Asp Leu Ser Ser Val Thr Arg Asn Met
        355                 360                 365

gca ggc ccg agc aac ccg cac gcg cgt ttt gcg acc gcc gat ttg gcc 1152
Ala Gly Pro Ser Asn Pro His Ala Arg Phe Ala Thr Ala Asp Leu Ala
    370                 375                 380

agc aaa ggc ttg gct aaa cct tac gaa gag cct tca gac ggc caa atg 1200
Ser Lys Gly Leu Ala Lys Pro Tyr Glu Glu Pro Ser Asp Gly Gln Met
385                 390                 395                 400

ccc gac ggc gcg gtc atc atc gcc gcg att acc agt tgc acc aac act 1248
Pro Asp Gly Ala Val Ile Ile Ala Ala Ile Thr Ser Cys Thr Asn Thr
                405                 410                 415

tcc aac ccg cgc aac gtt gtt gcc gcc gcg ctc ttg gcg cgc aac gcc 1296
Ser Asn Pro Arg Asn Val Val Ala Ala Ala Leu Leu Ala Arg Asn Ala
            420                 425                 430

aac tgc ttc ggg ctg aaa cgc aaa ccg tgg gtc aaa acc tcg ttt gcc 1344
Asn Cys Phe Gly Leu Lys Arg Lys Pro Trp Val Lys Thr Ser Phe Ala
        435                 440                 445

ccc ggt tcg aaa gtg gcg gaa att tat ttg aaa gaa gca ggc ctg ctg 1392
Pro Gly Ser Lys Val Ala Glu Ile Tyr Leu Lys Glu Ala Gly Leu Leu
    450                 455                 460

ccc gaa atg gaa aaa ctc ggc ttc ggt atc gtc gcc ttc gcc tgc acc 1440
Pro Glu Met Glu Lys Leu Gly Phe Gly Ile Val Ala Phe Ala Cys Thr
465                 470                 475                 480

acc tgc aac ggc atg agt ggc gcg ctg gat ccg aaa atc cag aaa gaa 1488
Thr Cys Asn Gly Met Ser Gly Ala Leu Asp Pro Lys Ile Gln Lys Glu
                485                 490                 495

atc atc gac cgc gat ttg tac gcc acc gcc gta tta tca ggc aac cgc 1536
Ile Ile Asp Arg Asp Leu Tyr Ala Thr Ala Val Leu Ser Gly Asn Arg
            500                 505                 510

aac ttc gac ggc cgt gtc cat ccg tat gcg aaa cag gct ttc ctc gct 1584
Asn Phe Asp Gly Arg Val His Pro Tyr Ala Lys Gln Ala Phe Leu Ala
        515                 520                 525

tcg cct ccg ttg gtc gtt gcc tac gcg ctg gca ggc agt atc cgt ttc 1632
Ser Pro Pro Leu Val Val Ala Tyr Ala Leu Ala Gly Ser Ile Arg Phe
    530                 535                 540

gat att gaa aac gac gta ctc ggc gtt gca gac ggc aag gaa atc cgc 1680
Asp Ile Glu Asn Asp Val Leu Gly Val Ala Asp Gly Lys Glu Ile Arg
545                 550                 555                 560

ctg aaa gac att tgg cct gcc gat gaa gaa atc gat gcc gtc gtt gcc 1728

Leu Lys Asp Ile Trp Pro Ala Asp Glu Glu Ile Asp Ala Val Val Ala
                565                 570                 575

gaa tat gtg aaa ccg cag cag ttc cgc gat gtg tat gta ccg atg ttc 1776
Glu Tyr Val Lys Pro Gln Gln Phe Arg Asp Val Tyr Val Pro Met Phe
            580                 585                 590

gac acc ggc aca gcg caa aaa gca cct agt ccg ctg tac gat tgg cgt 1824
Asp Thr Gly Thr Ala Gln Lys Ala Pro Ser Pro Leu Tyr Asp Trp Arg
        595                 600                 605

ccg atg tcc acc tac atc cgc cgt ccg cct tac tgg gaa ggc gcg ctg 1872
Pro Met Ser Thr Tyr Ile Arg Arg Pro Pro Tyr Trp Glu Gly Ala Leu
    610                 615                 620

gca ggg gaa cgc aca tta aga ggt atg cgt ccg ctg gcg att ttg ccc 1920
Ala Gly Glu Arg Thr Leu Arg Gly Met Arg Pro Leu Ala Ile Leu Pro
625                 630                 635                 640

gac aac atc acc acc gac cac ctc tcg ccg tcc aat gcg att ttg gcc 1968
Asp Asn Ile Thr Thr Asp His Leu Ser Pro Ser Asn Ala Ile Leu Ala
                645                 650                 655

gtc agt gcc gca ggc gag tat ttg gcg aaa atg ggt ttg cct gaa gaa 2016
Val Ser Ala Ala Gly Glu Tyr Leu Ala Lys Met Gly Leu Pro Glu Glu
            660                 665                 670

gac ttc aac tct tac gca acc cac cgc ggc gac cac ttg acc gcc caa 2064
Asp Phe Asn Ser Tyr Ala Thr His Arg Gly Asp His Leu Thr Ala Gln
        675                 680                 685

cgc gct acc ttc gcc aat ccg aaa ctg ttt aac gaa atg gtg aaa aac 2112
Arg Ala Thr Phe Ala Asn Pro Lys Leu Phe Asn Glu Met Val Lys Asn
    690                 695                 700

gaa gac ggc agc gtg cgc caa ggc tcg ttc gcc cgc gtc gaa ccc gaa 2160
Glu Asp Gly Ser Val Arg Gln Gly Ser Phe Ala Arg Val Glu Pro Glu
705                 710                 715                 720

ggc gaa acc atg cgc atg tgg gaa gcc atc gaa acc tat atg aac cgc 2208
Gly Glu Thr Met Arg Met Trp Glu Ala Ile Glu Thr Tyr Met Asn Arg
                725                 730                 735

aaa cag ccg ctc atc atc att gcc ggt gcg gac tat ggt caa ggc tca 2256
Lys Gln Pro Leu Ile Ile Ile Ala Gly Ala Asp Tyr Gly Gln Gly Ser
            740                 745                 750

agc cgc gac tgg gct gca aaa ggc gta cgc ctc gcc ggc gta gaa gcg 2304
Ser Arg Asp Trp Ala Ala Lys Gly Val Arg Leu Ala Gly Val Glu Ala
        755                 760                 765

att gtt gcc gaa ggc ttc gag cgt atc cac cgc acc aac ctt atc ggc 2352
Ile Val Ala Glu Gly Phe Glu Arg Ile His Arg Thr Asn Leu Ile Gly
    770                 775                 780

atg ggc gtg ttg ccg ctg cag ttc aaa ccc gac acc aac cgc cat acc 2400
Met Gly Val Leu Pro Leu Gln Phe Lys Pro Asp Thr Asn Arg His Thr
785                 790                 795                 800

ctg caa ctg gac ggt acg gaa acc tac gac gtg gtc ggc gaa cgc aca 2448

Leu Gln Leu Asp Gly Thr Glu Thr Tyr Asp Val Val Gly Glu Arg Thr
                805                 810                 815

ccg cgc tgc gac ctg acc ctc gtg att cac cgt aaa aac ggc gaa acc 2496
Pro Arg Cys Asp Leu Thr Leu Val Ile His Arg Lys Asn Gly Glu Thr
            820                 825                 830

gtc gaa gtt ccc gtt acc tgc cgc ctc gat act gca gaa gaa gta ttg 2544
Val Glu Val Pro Val Thr Cys Arg Leu Asp Thr Ala Glu Glu Val Leu
        835                 840                 845

gta tat gaa gcc ggc ggc gtg ttg caa cgg ttt gca cag gat ttt ttg 2592
Val Tyr Glu Ala Gly Gly Val Leu Gln Arg Phe Ala Gln Asp Phe Leu
    850                 855                 860

gaa ggg aac gcg gct tag 2610
Glu Gly Asn Ala Ala
865

<210> 42
<211> 869
<212> PRT
<213> Neisseria meningitidis

<400> 42
Met Ala Ala Asn Gln Arg Tyr Arg Lys Pro Leu Pro Gly Thr Asp Leu
1               5                   10                  15

Glu Tyr Tyr Asp Ala Arg Ala Ala Cys Glu Asp Ile Lys Pro Gly Ser
            20                  25                  30

Tyr Asp Lys Leu Pro Tyr Thr Ser Arg Ile Leu Ala Glu Asn Leu Val
        35                  40                  45

Asn Arg Ala Asp Lys Val Asp Leu Pro Thr Leu Gln Ser Trp Leu Gly
    50                  55                  60

Gln Leu Ile Glu Gly Lys Gln Glu Ile Asp Phe Pro Trp Tyr Pro Ala
65                  70                  75                  80

Arg Val Val Cys His Asp Ile Leu Gly Gln Thr Ala Leu Val Asp Leu
                85                  90                  95

Ala Gly Leu Arg Asp Ala Ile Ala Glu Lys Gly Gly Asp Pro Ala Lys
            100                 105                 110

Val Asn Pro Val Val Ala Lys Pro Ser Phe Ile Val Asp His Ser Leu
        115                 120                 125

Ala Val Glu Cys Gly Gly Tyr Asp Pro Asp Ala Phe Arg Lys Asn Arg
    130                 135                 140

Gln Ile Glu Asp Arg Arg Asn Glu Asp Arg Phe His Phe Ile Asn Trp
145                 150                 155                 160

Thr Lys Thr Ala Phe Glu Asn Val Asp Val Ile Pro Ala Gly Asn Gly
                165                 170                 175

Ile Met His Gln Ile Asn Leu Glu Lys Met Ser Pro Val Val Gln Val

            180                 185                 190

Lys Asn Gly Val Ala Phe Pro Asp Thr Cys Val Gly Thr Asp Ser His
        195                 200                 205

Thr Pro His Val Asp Ala Leu Gly Val Ile Ser Val Gly Val Gly Gly
    210                 215                 220

Leu Glu Ala Glu Thr Val Met Leu Gly Arg Ala Ser Met Met Arg Leu
225                 230                 235                 240

Pro Asp Ile Val Gly Val Glu Leu Asn Gly Lys Arg Gln Ala Gly Ile
                245                 250                 255

Thr Ala Thr Asp Ile Val Leu Ala Leu Thr Glu Phe Leu Arg Lys Glu
            260                 265                 270

Arg Val Val Gly Ala Phe Val Glu Phe Phe Gly Glu Gly Ala Arg Ser
        275                 280                 285

Leu Ser Ile Gly Asp Arg Ala Thr Ile Ser Asn Met Thr Pro Glu Phe
    290                 295                 300

Gly Ala Thr Ala Ala Met Phe Ala Ile Asp Glu Gln Thr Ile Asp Tyr
305                 310                 315                 320

Leu Lys Leu Thr Gly Arg Asp Asp Ala Gln Val Lys Leu Val Glu Thr
                325                 330                 335

Tyr Ala Lys Thr Ala Gly Leu Trp Ala Asp Ala Leu Lys Thr Ala Val
            340                 345                 350

Tyr Pro Arg Val Leu Lys Phe Asp Leu Ser Ser Val Thr Arg Asn Met
        355                 360                 365

Ala Gly Pro Ser Asn Pro His Ala Arg Phe Ala Thr Ala Asp Leu Ala
    370                 375                 380

Ser Lys Gly Leu Ala Lys Pro Tyr Glu Glu Pro Ser Asp Gly Gln Met
385                 390                 395                 400

Pro Asp Gly Ala Val Ile Ile Ala Ala Ile Thr Ser Cys Thr Asn Thr
                405                 410                 415

Ser Asn Pro Arg Asn Val Val Ala Ala Ala Leu Leu Ala Arg Asn Ala
            420                 425                 430

Asn Cys Phe Gly Leu Lys Arg Lys Pro Trp Val Lys Thr Ser Phe Ala
        435                 440                 445

Pro Gly Ser Lys Val Ala Glu Ile Tyr Leu Lys Glu Ala Gly Leu Leu
    450                 455                 460

Pro Glu Met Glu Lys Leu Gly Phe Gly Ile Val Ala Phe Ala Cys Thr
465                 470                 475                 480

Thr Cys Asn Gly Met Ser Gly Ala Leu Asp Pro Lys Ile Gln Lys Glu
                485                 490                 495

Ile Ile Asp Arg Asp Leu Tyr Ala Thr Ala Val Leu Ser Gly Asn Arg

            500                 505                 510

Asn Phe Asp Gly Arg Val His Pro Tyr Ala Lys Gln Ala Phe Leu Ala
        515                 520                 525

Ser Pro Pro Leu Val Val Ala Tyr Ala Leu Ala Gly Ser Ile Arg Phe
    530                 535                 540

Asp Ile Glu Asn Asp Val Leu Gly Val Ala Asp Gly Lys Glu Ile Arg
545                 550                 555                 560

Leu Lys Asp Ile Trp Pro Ala Asp Glu Glu Ile Asp Ala Val Val Ala
                565                 570                 575

Glu Tyr Val Lys Pro Gln Gln Phe Arg Asp Val Tyr Val Pro Met Phe
            580                 585                 590

Asp Thr Gly Thr Ala Gln Lys Ala Pro Ser Pro Leu Tyr Asp Trp Arg
        595                 600                 605

Pro Met Ser Thr Tyr Ile Arg Arg Pro Pro Tyr Trp Glu Gly Ala Leu
    610                 615                 620

Ala Gly Glu Arg Thr Leu Arg Gly Met Arg Pro Leu Ala Ile Leu Pro
625                 630                 635                 640

Asp Asn Ile Thr Thr Asp His Leu Ser Pro Ser Asn Ala Ile Leu Ala
                645                 650                 655

Val Ser Ala Ala Gly Glu Tyr Leu Ala Lys Met Gly Leu Pro Glu Glu
            660                 665                 670

Asp Phe Asn Ser Tyr Ala Thr His Arg Gly Asp His Leu Thr Ala Gln
        675                 680                 685

Arg Ala Thr Phe Ala Asn Pro Lys Leu Phe Asn Glu Met Val Lys Asn
    690                 695                 700

Glu Asp Gly Ser Val Arg Gln Gly Ser Phe Ala Arg Val Glu Pro Glu
705                 710                 715                 720

Gly Glu Thr Met Arg Met Trp Glu Ala Ile Glu Thr Tyr Met Asn Arg
                725                 730                 735

Lys Gln Pro Leu Ile Ile Ile Ala Gly Ala Asp Tyr Gly Gln Gly Ser
            740                 745                 750

Ser Arg Asp Trp Ala Ala Lys Gly Val Arg Leu Ala Gly Val Glu Ala
        755                 760                 765

Ile Val Ala Glu Gly Phe Glu Arg Ile His Arg Thr Asn Leu Ile Gly
    770                 775                 780

Met Gly Val Leu Pro Leu Gln Phe Lys Pro Asp Thr Asn Arg His Thr
785                 790                 795                 800

Leu Gln Leu Asp Gly Thr Glu Thr Tyr Asp Val Val Gly Glu Arg Thr
                805                 810                 815

Pro Arg Cys Asp Leu Thr Leu Val Ile His Arg Lys Asn Gly Glu Thr

            820                 825                 830

Val Glu Val Pro Val Thr Cys Arg Leu Asp Thr Ala Glu Glu Val Leu
        835                 840                 845

Val Tyr Glu Ala Gly Gly Val Leu Gln Arg Phe Ala Gln Asp Phe Leu
    850                 855                 860

Glu Gly Asn Ala Ala
865

<210> 43
<211> 1170
<212> ADN
<213> Neisseria meningitidis

<220>
<221> CDS
<222> (1)..(1167)

<400> 43
atg ccg caa att aaa att ccc gcc gtt tac tac cgt ggc ggt aca tca 48
Met Pro Gln Ile Lys Ile Pro Ala Val Tyr Tyr Arg Gly Gly Thr Ser
1               5                   10                  15

aaa ggc gtg ttt ttc aaa cgt tcc gac ctg ccc gag gcg gcg cgg gaa 96
Lys Gly Val Phe Phe Lys Arg Ser Asp Leu Pro Glu Ala Ala Arg Glu
            20                  25                  30

gcg gga agc gca cgc gac aaa atc ctc ttg cgc gta ctc ggc agc ccg 144
Ala Gly Ser Ala Arg Asp Lys Ile Leu Leu Arg Val Leu Gly Ser Pro
        35                  40                  45

gac ccc tac ggc aag cag ata gac ggt ttg ggc aac gcc agt tcg tcc 192
Asp Pro Tyr Gly Lys Gln Ile Asp Gly Leu Gly Asn Ala Ser Ser Ser
    50                  55                  60

acc agc aaa gcc gtg att ttg gac aag tcc gaa cgc acc gat cac gat 240
Thr Ser Lys Ala Val Ile Leu Asp Lys Ser Glu Arg Thr Asp His Asp
65                  70                  75                  80

gtc gat tac ctt ttc ggg caa gtt tcc atc gac aaa cct ttt gtc gat 288
Val Asp Tyr Leu Phe Gly Gln Val Ser Ile Asp Lys Pro Phe Val Asp
                85                  90                  95

tgg agt ggc aac tgc ggc aac ctc acc gcc gcc gtg ggc gca ttt gcc 336
Trp Ser Gly Asn Cys Gly Asn Leu Thr Ala Ala Val Gly Ala Phe Ala
            100                 105                 110

atc gag caa ggc ttg gtc gat aaa tcc aaa atc cct tca gac ggc ccg 384
Ile Glu Gln Gly Leu Val Asp Lys Ser Lys Ile Pro Ser Asp Gly Pro
        115                 120                 125

tgt acc gtc aaa atc tgg cag aaa aac atc ggc aaa acc att att gcc 432
Cys Thr Val Lys Ile Trp Gln Lys Asn Ile Gly Lys Thr Ile Ile Ala
    130                 135                 140

cat gta ccg atg caa aac ggc gca gtt ttg gaa aca ggc gat ttt gag 480

His Val Pro Met Gln Asn Gly Ala Val Leu Glu Thr Gly Asp Phe Glu
145                 150                 155                 160

ctc gac ggc gta acg ttc ccg gca gcc gaa gta caa atc gaa ttt ctt     528
Leu Asp Gly Val Thr Phe Pro Ala Ala Glu Val Gln Ile Glu Phe Leu
                165                 170                 175

gat cca gcc gac ggc gaa ggc agt atg ttc cca acc ggc aat ttg gtc     576
Asp Pro Ala Asp Gly Glu Gly Ser Met Phe Pro Thr Gly Asn Leu Val
            180                 185                 190

gat gaa att gat gtg ccg aat ata ggc cgt ttg aaa gcc acg ctc atc     624
Asp Glu Ile Asp Val Pro Asn Ile Gly Arg Leu Lys Ala Thr Leu Ile
        195                 200                 205

aac gcg ggc att ccg acc gtt ttc ctg aat gcc gcc gac ttg ggc tac     672
Asn Ala Gly Ile Pro Thr Val Phe Leu Asn Ala Ala Asp Leu Gly Tyr
    210                 215                 220

acg ggc aaa gag ttg caa gac gac atc aac aac gat gcc gca gct ttg     720
Thr Gly Lys Glu Leu Gln Asp Asp Ile Asn Asn Asp Ala Ala Ala Leu
225                 230                 235                 240

gaa aaa ttc gag aaa atc cgc gct tac ggt gcg ctg aaa atg ggt cta     768
Glu Lys Phe Glu Lys Ile Arg Ala Tyr Gly Ala Leu Lys Met Gly Leu
                245                 250                 255

atc agc gac gta tcc gaa gct gcc gcc cgc gcg cac acg ccg aaa gtc     816
Ile Ser Asp Val Ser Glu Ala Ala Ala Arg Ala His Thr Pro Lys Val
            260                 265                 270

gcc ttc gtc gcg ccc gcc gcc gat tac acc gcc tcc agt ggc aaa acc     864
Ala Phe Val Ala Pro Ala Ala Asp Tyr Thr Ala Ser Ser Gly Lys Thr
        275                 280                 285

gtg aat gcc gcc gac atc gat ttg ctg gta cgc gcc ctg agc atg ggc     912
Val Asn Ala Ala Asp Ile Asp Leu Leu Val Arg Ala Leu Ser Met Gly
    290                 295                 300

aaa ttg cac cac gcg atg atg ggt acc gcc tct gtt gcc att gcg acc     960
Lys Leu His His Ala Met Met Gly Thr Ala Ser Val Ala Ile Ala Thr
305                 310                 315                 320

gcc gcc gcc gtg ccc ggt acg ctg gtc aac ctt gcc gca ggg gcg gga     1008
Ala Ala Ala Val Pro Gly Thr Leu Val Asn Leu Ala Ala Gly Ala Gly
                325                 330                 335

acg cgt aaa gaa gtg cgc ttc ggg cat cct tcc ggc aca ttg cgc gtc     1056
Thr Arg Lys Glu Val Arg Phe Gly His Pro Ser Gly Thr Leu Arg Val
            340                 345                 350

ggt gca gcc gcc gaa tgt cag gac gga caa tgg acg gcc acc aaa gcg     1104
Gly Ala Ala Ala Glu Cys Gln Asp Gly Gln Trp Thr Ala Thr Lys Ala
        355                 360                 365

gtt atg agc cgc agc gca cgc gtg atg atg gaa ggt tgg gtc agg gtg     1152
Val Met Ser Arg Ser Ala Arg Val Met Met Glu Gly Trp Val Arg Val
    370                 375                 380

ccg gaa gat tgt ttt taa                                             1170

Pro Glu Asp Cys Phe
385

<210> 44
<211> 389
<212> PRT
<213> Neisseria meningitidis

<400> 44
Met Pro Gln Ile Lys Ile Pro Ala Val Tyr Tyr Arg Gly Gly Thr Ser
1               5                   10                  15

Lys Gly Val Phe Phe Lys Arg Ser Asp Leu Pro Glu Ala Ala Arg Glu
            20                  25                  30

Ala Gly Ser Ala Arg Asp Lys Ile Leu Leu Arg Val Leu Gly Ser Pro
        35                  40                  45

Asp Pro Tyr Gly Lys Gln Ile Asp Gly Leu Gly Asn Ala Ser Ser Ser
    50                  55                  60

Thr Ser Lys Ala Val Ile Leu Asp Lys Ser Glu Arg Thr Asp His Asp
65                  70                  75                  80

Val Asp Tyr Leu Phe Gly Gln Val Ser Ile Asp Lys Pro Phe Val Asp
                85                  90                  95

Trp Ser Gly Asn Cys Gly Asn Leu Thr Ala Ala Val Gly Ala Phe Ala
            100                 105                 110

Ile Glu Gln Gly Leu Val Asp Lys Ser Lys Ile Pro Ser Asp Gly Pro
        115                 120                 125

Cys Thr Val Lys Ile Trp Gln Lys Asn Ile Gly Lys Thr Ile Ile Ala
    130                 135                 140

His Val Pro Met Gln Asn Gly Ala Val Leu Glu Thr Gly Asp Phe Glu
145                 150                 155                 160

Leu Asp Gly Val Thr Phe Pro Ala Ala Glu Val Gln Ile Glu Phe Leu
                165                 170                 175

Asp Pro Ala Asp Gly Glu Gly Ser Met Phe Pro Thr Gly Asn Leu Val
            180                 185                 190

Asp Glu Ile Asp Val Pro Asn Ile Gly Arg Leu Lys Ala Thr Leu Ile
        195                 200                 205

Asn Ala Gly Ile Pro Thr Val Phe Leu Asn Ala Ala Asp Leu Gly Tyr
    210                 215                 220

Thr Gly Lys Glu Leu Gln Asp Asp Ile Asn Asn Asp Ala Ala Ala Leu
225                 230                 235                 240

Glu Lys Phe Glu Lys Ile Arg Ala Tyr Gly Ala Leu Lys Met Gly Leu
                245                 250                 255

Ile Ser Asp Val Ser Glu Ala Ala Ala Arg Ala His Thr Pro Lys Val
            260                 265                 270

Ala Phe Val Ala Pro Ala Ala Asp Tyr Thr Ala Ser Ser Gly Lys Thr
        275                 280                 285

Val Asn Ala Ala Asp Ile Asp Leu Leu Val Arg Ala Leu Ser Met Gly
    290                 295                 300

Lys Leu His His Ala Met Met Gly Thr Ala Ser Val Ala Ile Ala Thr
305                 310                 315                 320

Ala Ala Ala Val Pro Gly Thr Leu Val Asn Leu Ala Ala Gly Ala Gly
                325                 330                 335

Thr Arg Lys Glu Val Arg Phe Gly His Pro Ser Gly Thr Leu Arg Val
            340                 345                 350

Gly Ala Ala Ala Glu Cys Gln Asp Gly Gln Trp Thr Ala Thr Lys Ala
        355                 360                 365

Val Met Ser Arg Ser Ala Arg Val Met Met Glu Gly Trp Val Arg Val
    370                 375                 380

Pro Glu Asp Cys Phe
385

<210> 45
<211> 954
<212> ADN
<213> Neisseria meningitidis

<220>
<221> CDS
<222> (1)..(951)

<400> 45
atg cgc acg ccg ttt tgt tgg gca tac gcc aat gcc gcc cga ata tcg 48
Met Arg Thr Pro Phe Cys Trp Ala Tyr Ala Asn Ala Ala Arg Ile Ser
1               5                   10                  15

gca atg ctg ccg gcg tgt tgg gcg cag gcg atg ttg gcc gaa gta atc 96
Ala Met Leu Pro Ala Cys Trp Ala Gln Ala Met Leu Ala Glu Val Ile
            20                  25                  30

agc tgc aac aag gct tcg tcg ctg ccg cag cct tcg gcg aga tcg gcg 144
Ser Cys Asn Lys Ala Ser Ser Leu Pro Gln Pro Ser Ala Arg Ser Ala
        35                  40                  45

ttt aaa tca acc tgc ttc atg ggt gat tct ccg tat ttg gtt cag ata 192
Phe Lys Ser Thr Cys Phe Met Gly Asp Ser Pro Tyr Leu Val Gln Ile
    50                  55                  60

gac ttg gtt ttt gcg ccg cag ggc ggt ggc ttc ttt caa gcc gat tat 240
Asp Leu Val Phe Ala Pro Gln Gly Gly Gly Phe Phe Gln Ala Asp Tyr
65                  70                  75                  80

ttt gaa ttt gac ttt gct gcc gaa gcg cac ctg tgc cag cct gcc caa 288
Phe Glu Phe Asp Phe Ala Ala Glu Ala His Leu Cys Gln Pro Ala Gln
                85                  90                  95

atc ggc ggc ggc aac ggt agc gat ttt cgg ata acc gcc ggt ggt ttg     336
Ile Gly Gly Gly Asn Gly Ser Asp Phe Arg Ile Thr Ala Gly Gly Leu
            100                 105                 110

cgc atc ggc cag cag gat aat cgg ttt gcc gcc ggg cgg cac ctg cac     384
Arg Ile Gly Gln Gln Asp Asn Arg Phe Ala Ala Gly Arg His Leu His
        115                 120                 125

ggt tcc tgc ctg aac agc gtg gga cag cat ttc caa agg ttg cga cag     432
Gly Ser Cys Leu Asn Ser Val Gly Gln His Phe Gln Arg Leu Arg Gln
    130                 135                 140

ggt cag cgg ctg tcc gtc gaa gcg gta gcc cat gcg gtt gct atc gct     480
Gly Gln Arg Leu Ser Val Glu Ala Val Ala His Ala Val Ala Ile Ala
145                 150                 155                 160

ttg cag cgt cca cgt ttc ccg ttc cag att cag acg ccc ttt ttc act     528
Leu Gln Arg Pro Arg Phe Pro Phe Gln Ile Gln Thr Pro Phe Phe Thr
                165                 170                 175

gaa agc ggc ata ttc cga cga agg aac aag gtg gat ggt atc ggt aaa     576
Glu Ser Gly Ile Phe Arg Arg Arg Asn Lys Val Asp Gly Ile Gly Lys
            180                 185                 190

cgg tat cgg ggc aat gcc gac ttt gga caa ttc ctg cgc acc ttt gcc     624
Arg Tyr Arg Gly Asn Ala Asp Phe Gly Gln Phe Leu Arg Thr Phe Ala
        195                 200                 205

gat ggg gag ata atc gcc ttt ttg cag cat tct gcc ctg atg gcc gcc     672
Asp Gly Glu Ile Ile Ala Phe Leu Gln His Ser Ala Leu Met Ala Ala
    210                 215                 220

gaa acc ggc ttt cag gtc ggt gct tct cga acc cat cac ttc cgg cac     720
Glu Thr Gly Phe Gln Val Gly Ala Ser Arg Thr His His Phe Arg His
225                 230                 235                 240

atc aaa tcc gcc cgc cac gca cac ata gcc gta cat gcc ctg cac ggc     768
Ile Lys Ser Ala Arg His Ala His Ile Ala Val His Ala Leu His Gly
                245                 250                 255

acg cac cat ttt caa ggt ctg ccc ttt gcg ggc ggt ata acg cca ata     816
Thr His His Phe Gln Gly Leu Pro Phe Ala Gly Gly Ile Thr Pro Ile
            260                 265                 270

cga ata gac cgg ttc gcc gtc caa ttc cgc ctg ata cac ggc acc ggt     864
Arg Ile Asp Arg Phe Ala Val Gln Phe Arg Leu Ile His Gly Thr Gly
        275                 280                 285

gag aca aaa cgg cgt atc ccg ttc aaa cac cag cat tat ccc gcc caa     912
Glu Thr Lys Arg Arg Ile Pro Phe Lys His Gln His Tyr Pro Ala Gln
    290                 295                 300

agc gat ttc gat tgc ggc cgt gcc ttc gtc gtt gcc caa taa             954
Ser Asp Phe Asp Cys Gly Arg Ala Phe Val Val Ala Gln
305                 310                 315

<210> 46
<211> 317

<212> PRT
<213> Neisseria meningitidis

<400> 46
Met Arg Thr Pro Phe Cys Trp Ala Tyr Ala Asn Ala Ala Arg Ile Ser
1               5                   10                  15

Ala Met Leu Pro Ala Cys Trp Ala Gln Ala Met Leu Ala Glu Val Ile
            20                  25                  30

Ser Cys Asn Lys Ala Ser Ser Leu Pro Gln Pro Ser Ala Arg Ser Ala
        35                  40                  45

Phe Lys Ser Thr Cys Phe Met Gly Asp Ser Pro Tyr Leu Val Gln Ile
    50                  55                  60

Asp Leu Val Phe Ala Pro Gln Gly Gly Gly Phe Phe Gln Ala Asp Tyr
65                  70                  75                  80

Phe Glu Phe Asp Phe Ala Ala Glu Ala His Leu Cys Gln Pro Ala Gln
                85                  90                  95

Ile Gly Gly Gly Asn Gly Ser Asp Phe Arg Ile Thr Ala Gly Gly Leu
            100                 105                 110

Arg Ile Gly Gln Gln Asp Asn Arg Phe Ala Ala Gly Arg His Leu His
        115                 120                 125

Gly Ser Cys Leu Asn Ser Val Gly Gln His Phe Gln Arg Leu Arg Gln
    130                 135                 140

Gly Gln Arg Leu Ser Val Glu Ala Val Ala His Ala Val Ala Ile Ala
145                 150                 155                 160

Leu Gln Arg Pro Arg Phe Pro Phe Gln Ile Gln Thr Pro Phe Phe Thr
                165                 170                 175

Glu Ser Gly Ile Phe Arg Arg Arg Asn Lys Val Asp Gly Ile Gly Lys
            180                 185                 190

Arg Tyr Arg Gly Asn Ala Asp Phe Gly Gln Phe Leu Arg Thr Phe Ala
        195                 200                 205

Asp Gly Glu Ile Ile Ala Phe Leu Gln His Ser Ala Leu Met Ala Ala
    210                 215                 220

Glu Thr Gly Phe Gln Val Gly Ala Ser Arg Thr His His Phe Arg His
225                 230                 235                 240

Ile Lys Ser Ala Arg His Ala His Ile Ala Val His Ala Leu His Gly
                245                 250                 255

Thr His His Phe Gln Gly Leu Pro Phe Ala Gly Gly Ile Thr Pro Ile
            260                 265                 270

Arg Ile Asp Arg Phe Ala Val Gln Phe Arg Leu Ile His Gly Thr Gly
        275                 280                 285

Glu Thr Lys Arg Arg Ile Pro Phe Lys His Gln His Tyr Pro Ala Gln
    290                 295                 300

Ser Asp Phe Asp Cys Gly Arg Ala Phe Val Val Ala Gln
305                 310                 315

<210> 47
<211> 648
<212> ADN
<213> Neisseria meningitidis

<220>
<221> CDS
<222> (1)..(645)

<400> 47
atg aga ata gag atc aca cca atc agc gaa tcc gct ttg gtc tgc cga      48
Met Arg Ile Glu Ile Thr Pro Ile Ser Glu Ser Ala Leu Val Cys Arg
1               5                   10                  15

ctg aat gcg cct tcc gaa ctg ggc aaa cag caa aag ttg tgg gcg ttt      96
Leu Asn Ala Pro Ser Glu Leu Gly Lys Gln Gln Lys Leu Trp Ala Phe
            20                  25                  30

gcc gct gcg ctc ggg cag cac gac agg att gag gaa gtg gtg gtc ggc      144
Ala Ala Ala Leu Gly Gln His Asp Arg Ile Glu Glu Val Val Val Gly
        35                  40                  45

atg aac aat ctg acc gtg ttc acc cgt ttc gat acc gat ttg gcg acg      192
Met Asn Asn Leu Thr Val Phe Thr Arg Phe Asp Thr Asp Leu Ala Thr
    50                  55                  60

ctt gcc gat gaa ttg caa tat gtg tgg gaa cac acc gcc gtt aca gac      240
Leu Ala Asp Glu Leu Gln Tyr Val Trp Glu His Thr Ala Val Thr Asp
65                  70                  75                  80

cat cag ggc aaa ctg gtg gaa att ccc gtc tgc tac ggc ggc gaa tac      288
His Gln Gly Lys Leu Val Glu Ile Pro Val Cys Tyr Gly Gly Glu Tyr
                85                  90                  95

ggc ccg gat ttg gcg gaa gtc gct gct ttc cat cag acg gtt att tcc      336
Gly Pro Asp Leu Ala Glu Val Ala Ala Phe His Gln Thr Val Ile Ser
            100                 105                 110

gaa atc gtc cgc cgc cat acg gcg caa act tat acc gta ttt atg atg      384
Glu Ile Val Arg Arg His Thr Ala Gln Thr Tyr Thr Val Phe Met Met
        115                 120                 125

ggc ttc cag cct ggt ttc cct tat ctg ggc ggc ttg ccc gaa gca ttg      432
Gly Phe Gln Pro Gly Phe Pro Tyr Leu Gly Gly Leu Pro Glu Ala Leu
    130                 135                 140

cac acg ccc cgc cgt gcc gtg ccg aga acg tcc gtt cct gcc ggt tcg      480
His Thr Pro Arg Arg Ala Val Pro Arg Thr Ser Val Pro Ala Gly Ser
145                 150                 155                 160

gtc ggt atc ggc ggc agt cag acc ggt gtg tat ccg ttc gct tcg ccc      528
Val Gly Ile Gly Gly Ser Gln Thr Gly Val Tyr Pro Phe Ala Ser Pro
                165                 170                 175

ggc ggc tgg cag att atc ggc aga acc gaa tta ccc ttg ttc cga gcc     576
Gly Gly Trp Gln Ile Ile Gly Arg Thr Glu Leu Pro Leu Phe Arg Ala
            180                 185                 190

gat ttg aat ccg ccg acc ctg ctg gcg gcg ggt gac caa gtc cgc ttt     624
Asp Leu Asn Pro Pro Thr Leu Leu Ala Ala Gly Asp Gln Val Arg Phe
        195                 200                 205

gtt gca gaa agg att gag cca tga                                     648
Val Ala Glu Arg Ile Glu Pro
    210                 215


<210> 48
<211> 215
<212> PRT
<213> Neisseria meningitidis

<400> 48
Met Arg Ile Glu Ile Thr Pro Ile Ser Glu Ser Ala Leu Val Cys Arg
1               5                   10                  15

Leu Asn Ala Pro Ser Glu Leu Gly Lys Gln Gln Lys Leu Trp Ala Phe
            20                  25                  30

Ala Ala Ala Leu Gly Gln His Asp Arg Ile Glu Glu Val Val Val Gly
        35                  40                  45

Met Asn Asn Leu Thr Val Phe Thr Arg Phe Asp Thr Asp Leu Ala Thr
    50                  55                  60

Leu Ala Asp Glu Leu Gln Tyr Val Trp Glu His Thr Ala Val Thr Asp
65                  70                  75                  80

His Gln Gly Lys Leu Val Glu Ile Pro Val Cys Tyr Gly Gly Glu Tyr
                85                  90                  95

Gly Pro Asp Leu Ala Glu Val Ala Ala Phe His Gln Thr Val Ile Ser
            100                 105                 110

Glu Ile Val Arg Arg His Thr Ala Gln Thr Tyr Thr Val Phe Met Met
        115                 120                 125

Gly Phe Gln Pro Gly Phe Pro Tyr Leu Gly Gly Leu Pro Glu Ala Leu
    130                 135                 140

His Thr Pro Arg Arg Ala Val Pro Arg Thr Ser Val Pro Ala Gly Ser
145                 150                 155                 160

Val Gly Ile Gly Gly Ser Gln Thr Gly Val Tyr Pro Phe Ala Ser Pro
                165                 170                 175

Gly Gly Trp Gln Ile Ile Gly Arg Thr Glu Leu Pro Leu Phe Arg Ala
            180                 185                 190

Asp Leu Asn Pro Pro Thr Leu Leu Ala Ala Gly Asp Gln Val Arg Phe
        195                 200                 205

Val Ala Glu Arg Ile Glu Pro
    210                 215

<210> 49
<211> 930
<212> ADN
<213> Neisseria meningitidis

<220>
<221> CDS
<222> (1)..(927)

<400> 49
atg att cac gtt tcg gca gtg cag gca ccg gcg cat att cag gat acc     48
Met Ile His Val Ser Ala Val Gln Ala Pro Ala His Ile Gln Asp Thr
1               5                   10                  15

gga cgc tac gga cac cgg cgt tac ggc atc ggt cat gcc ggt gcg atg     96
Gly Arg Tyr Gly His Arg Arg Tyr Gly Ile Gly His Ala Gly Ala Met
            20                  25                  30

gac acg gtt gct ttg gcg gcg ggt aat att tta ttg ggc aac gac gaa     144
Asp Thr Val Ala Leu Ala Ala Gly Asn Ile Leu Leu Gly Asn Asp Glu
        35                  40                  45

ggc acg gcc gca atc gaa atc gct ttg ggc ggg ata atg ctg gtg ttt     192
Gly Thr Ala Ala Ile Glu Ile Ala Leu Gly Gly Ile Met Leu Val Phe
    50                  55                  60

gaa cgg gat acg ccg ttt tgt ctc acc ggt gcc gtg tat cag gcg gaa     240
Glu Arg Asp Thr Pro Phe Cys Leu Thr Gly Ala Val Tyr Gln Ala Glu
65                  70                  75                  80

ttg gac ggc gaa ccg gtc tat tcg tat tgg cgt tat acc gcc cgc aaa     288
Leu Asp Gly Glu Pro Val Tyr Ser Tyr Trp Arg Tyr Thr Ala Arg Lys
                85                  90                  95

ggg cag acc ttg aaa atg gtg cgt gcc gtg cag ggc atg tac ggc tat     336
Gly Gln Thr Leu Lys Met Val Arg Ala Val Gln Gly Met Tyr Gly Tyr
            100                 105                 110

gtg tgc gtg gcg ggc gga ttt gat gtg ccg gaa gtg atg ggt tcg aga     384
Val Cys Val Ala Gly Gly Phe Asp Val Pro Glu Val Met Gly Ser Arg
        115                 120                 125

agc acc gac ctg aaa gcc ggt ttc ggc ggc cat cag ggc aga atg ctg     432
Ser Thr Asp Leu Lys Ala Gly Phe Gly Gly His Gln Gly Arg Met Leu
    130                 135                 140

caa aaa ggc gat tat ctc ccc atc ggc aaa ggt gcg cag gaa ttg tcc     480
Gln Lys Gly Asp Tyr Leu Pro Ile Gly Lys Gly Ala Gln Glu Leu Ser
145                 150                 155                 160

aaa gtc ggc att gcc ccg ata ccg ttt acc gat acc atc cac ctt gtt     528
Lys Val Gly Ile Ala Pro Ile Pro Phe Thr Asp Thr Ile His Leu Val
                165                 170                 175

cct tcg tcg gaa tat gcc gct ttc agt gaa aaa ggg cgt ctg aat ctg     576
Pro Ser Ser Glu Tyr Ala Ala Phe Ser Glu Lys Gly Arg Leu Asn Leu
            180                 185                 190

gaa cgg gaa acg tgg acg ctg caa agc gat agc aac cgc atg ggc tac     624
Glu Arg Glu Thr Trp Thr Leu Gln Ser Asp Ser Asn Arg Met Gly Tyr
        195                 200                 205

cgc ttc gac gga cag ccg ctg acc ctg tcg caa cct ttg gaa atg ctg     672
Arg Phe Asp Gly Gln Pro Leu Thr Leu Ser Gln Pro Leu Glu Met Leu
    210                 215                 220

tcc cac gct gtt cag gca gga acc gtg cag gtg ccg ccc ggc ggc aaa     720
Ser His Ala Val Gln Ala Gly Thr Val Gln Val Pro Pro Gly Gly Lys
225                 230                 235                 240

ccg att atc ctg ctg gcc gat gcg caa acc acc ggc ggt tat ccg aaa     768
Pro Ile Ile Leu Leu Ala Asp Ala Gln Thr Thr Gly Gly Tyr Pro Lys
                245                 250                 255

atc gct acc gtt gcc gcc gcc gat ttg ggc agg ctg gca cag gtg cgc     816
Ile Ala Thr Val Ala Ala Ala Asp Leu Gly Arg Leu Ala Gln Val Arg
            260                 265                 270

ttc ggc agc aaa gtc aaa ttc aaa ata atc ggc ttg aaa gaa gcc acc     864
Phe Gly Ser Lys Val Lys Phe Lys Ile Ile Gly Leu Lys Glu Ala Thr
        275                 280                 285

gcc ctg cgg cgc aaa aac caa gtc tat ctg aac caa ata cgg aga atc     912
Ala Leu Arg Arg Lys Asn Gln Val Tyr Leu Asn Gln Ile Arg Arg Ile
    290                 295                 300

acc cat gaa gca ggt tga                                             930
Thr His Glu Ala Gly
305

<210> 50
<211> 309
<212> PRT
<213> Neisseria meningitidis

<400> 50
Met Ile His Val Ser Ala Val Gln Ala Pro Ala His Ile Gln Asp Thr
1               5                   10                  15

Gly Arg Tyr Gly His Arg Arg Tyr Gly Ile Gly His Ala Gly Ala Met
            20                  25                  30

Asp Thr Val Ala Leu Ala Ala Gly Asn Ile Leu Leu Gly Asn Asp Glu
        35                  40                  45

Gly Thr Ala Ala Ile Glu Ile Ala Leu Gly Gly Ile Met Leu Val Phe
    50                  55                  60

Glu Arg Asp Thr Pro Phe Cys Leu Thr Gly Ala Val Tyr Gln Ala Glu
65                  70                  75                  80

Leu Asp Gly Glu Pro Val Tyr Ser Tyr Trp Arg Tyr Thr Ala Arg Lys
                85                  90                  95

Gly Gln Thr Leu Lys Met Val Arg Ala Val Gln Gly Met Tyr Gly Tyr
            100                 105                 110

Val Cys Val Ala Gly Gly Phe Asp Val Pro Glu Val Met Gly Ser Arg
        115                 120                 125

Ser Thr Asp Leu Lys Ala Gly Phe Gly Gly His Gln Gly Arg Met Leu
    130                 135                 140

Gln Lys Gly Asp Tyr Leu Pro Ile Gly Lys Gly Ala Gln Glu Leu Ser
145                 150                 155                 160

Lys Val Gly Ile Ala Pro Ile Pro Phe Thr Asp Thr Ile His Leu Val
                165                 170                 175

Pro Ser Ser Glu Tyr Ala Ala Phe Ser Glu Lys Gly Arg Leu Asn Leu
            180                 185                 190

Glu Arg Glu Thr Trp Thr Leu Gln Ser Asp Ser Asn Arg Met Gly Tyr
        195                 200                 205

Arg Phe Asp Gly Gln Pro Leu Thr Leu Ser Gln Pro Leu Glu Met Leu
    210                 215                 220

Ser His Ala Val Gln Ala Gly Thr Val Gln Val Pro Pro Gly Gly Lys
225                 230                 235                 240

Pro Ile Ile Leu Leu Ala Asp Ala Gln Thr Thr Gly Gly Tyr Pro Lys
                245                 250                 255

Ile Ala Thr Val Ala Ala Ala Asp Leu Gly Arg Leu Ala Gln Val Arg
            260                 265                 270

Phe Gly Ser Lys Val Lys Phe Lys Ile Ile Gly Leu Lys Glu Ala Thr
        275                 280                 285

Ala Leu Arg Arg Lys Asn Gln Val Tyr Leu Asn Gln Ile Arg Arg Ile
    290                 295                 300

Thr His Glu Ala Gly
305

<210> 51
<211> 2094
<212> ADN
<213> Neisseria meningitidis

<220>
<221> CDS
<222> (1)..(2091)

<400> 51
atg aat tcg acc gca agt aaa acc ctg aaa gga ttg tcg ctg gtg ttt      48
Met Asn Ser Thr Ala Ser Lys Thr Leu Lys Gly Leu Ser Leu Val Phe
1               5                   10                  15

ttc gcc tct gga ttc tgc gcc ctg att tac cag gtc agc tgg cag agg      96
Phe Ala Ser Gly Phe Cys Ala Leu Ile Tyr Gln Val Ser Trp Gln Arg
            20                  25                  30

ctt cta ttc agt cac ata ggt atc gat ttg agt tcg att act gtc att 144
Leu Leu Phe Ser His Ile Gly Ile Asp Leu Ser Ser Ile Thr Val Ile
        35                  40                  45

att tct gta ttt atg gtc ggc ttg ggt gta ggt gcg tat ttc ggt gga 192
Ile Ser Val Phe Met Val Gly Leu Gly Val Gly Ala Tyr Phe Gly Gly
    50                  55                  60

cgc att gct gac cgt ttt cct tca agt atc atc ccc ctg ttt tgc atc 240
Arg Ile Ala Asp Arg Phe Pro Ser Ser Ile Ile Pro Leu Phe Cys Ile
65                  70                  75                  80

gct gaa gta tcc atc ggt ctg ttc ggt ttg gta agc agg ggt ctg att 288
Ala Glu Val Ser Ile Gly Leu Phe Gly Leu Val Ser Arg Gly Leu Ile
                85                  90                  95

tcc ggc ttg ggg cat ctt tta gtt gag gct gat ttg ccc atc atc gct 336
Ser Gly Leu Gly His Leu Leu Val Glu Ala Asp Leu Pro Ile Ile Ala
            100                 105                 110

gct gcc aat ttc ctc tta ttg ctg ctt cct acc ttt atg atg ggc gcg 384
Ala Ala Asn Phe Leu Leu Leu Leu Leu Pro Thr Phe Met Met Gly Ala
        115                 120                 125

acc ttg ccc ttg ctg acc tgt ttt ttt aac cgg aaa ata cat aat gtt 432
Thr Leu Pro Leu Leu Thr Cys Phe Phe Asn Arg Lys Ile His Asn Val
    130                 135                 140

ggc gag tct atc ggt acc tta tat ttt ttc aac act ttg ggt gcg gca 480
Gly Glu Ser Ile Gly Thr Leu Tyr Phe Phe Asn Thr Leu Gly Ala Ala
145                 150                 155                 160

ctc gga tcg ctt gcc gcc gcc gaa ttt ttc tac gtc ttt ttt acc ctc 528
Leu Gly Ser Leu Ala Ala Ala Glu Phe Phe Tyr Val Phe Phe Thr Leu
                165                 170                 175

tcc caa acc att gcg ctg aca gcc tgc ttt aac ctt ctg att gct gct 576
Ser Gln Thr Ile Ala Leu Thr Ala Cys Phe Asn Leu Leu Ile Ala Ala
            180                 185                 190

tca gta tgc tgc gtt aca gaa agg atg gat ata gtg aac act aaa ccg 624
Ser Val Cys Cys Val Thr Glu Arg Met Asp Ile Val Asn Thr Lys Pro
        195                 200                 205

aat act agt ttg att tat atg ctt tct ttc ctt agc ggc tta ttg agc 672
Asn Thr Ser Leu Ile Tyr Met Leu Ser Phe Leu Ser Gly Leu Leu Ser
    210                 215                 220

ttg ggt ata gaa gtc ttg tgg gta agg atg ttt tcg ttc gca gca cag 720
Leu Gly Ile Glu Val Leu Trp Val Arg Met Phe Ser Phe Ala Ala Gln
225                 230                 235                 240

tcc gtg cct cag gca ttt tca ttt act ctt gcc tat ttt ctg acc ggt 768
Ser Val Pro Gln Ala Phe Ser Phe Thr Leu Ala Tyr Phe Leu Thr Gly
                245                 250                 255

atc gcc gtc ggc gcg tat ttt ggc aaa cgg att tgc cgc agc cgc ttt 816
Ile Ala Val Gly Ala Tyr Phe Gly Lys Arg Ile Cys Arg Ser Arg Phe
            260                 265                 270

gtt gat att ccc ttt atc ggg cag tgc ttc ttg tgg gcg ggt att gcc     864
Val Asp Ile Pro Phe Ile Gly Gln Cys Phe Leu Trp Ala Gly Ile Ala
        275                 280                 285

gac ttt ttg att ttg ggt gct gcg tgg ttg ttg acg ggt ttt tcc ggc     912
Asp Phe Leu Ile Leu Gly Ala Ala Trp Leu Leu Thr Gly Phe Ser Gly
    290                 295                 300

ttc gtc cac cac gcc ggt atc ttc att acc ctg tct gcc gtc gtc aga     960
Phe Val His His Ala Gly Ile Phe Ile Thr Leu Ser Ala Val Val Arg
305                 310                 315                 320

ggg ttg att ttc ccg ctc gta cac cat gtg ggt acg gat ggc aac aaa     1008
Gly Leu Ile Phe Pro Leu Val His His Val Gly Thr Asp Gly Asn Lys
                325                 330                 335

tcc gga cga cag gtt tcc aat gtt tat ttc gcc aac gtt gcc ggc agt     1056
Ser Gly Arg Gln Val Ser Asn Val Tyr Phe Ala Asn Val Ala Gly Ser
            340                 345                 350

gca ttg ggt ccg gtc ctt atc ggc ttt gtg ata ctt gat ttc ttg tcc     1104
Ala Leu Gly Pro Val Leu Ile Gly Phe Val Ile Leu Asp Phe Leu Ser
        355                 360                 365

acc caa cag att tac ctg ctc atc tgt ttg att tct gct gct gtc cct     1152
Thr Gln Gln Ile Tyr Leu Leu Ile Cys Leu Ile Ser Ala Ala Val Pro
    370                 375                 380

ttg ttt tgt aca ctg ttc caa aaa agt ctc cga ctg aat gca gtg tcg     1200
Leu Phe Cys Thr Leu Phe Gln Lys Ser Leu Arg Leu Asn Ala Val Ser
385                 390                 395                 400

gta gca gtt tcc cta atg ttc ggc atc ctc atg ttc cta ctg ccg gat     1248
Val Ala Val Ser Leu Met Phe Gly Ile Leu Met Phe Leu Leu Pro Asp
                405                 410                 415

tct gtc ttt caa aat att gct gac cgt ccg gat cgg ctg att gaa aac     1296
Ser Val Phe Gln Asn Ile Ala Asp Arg Pro Asp Arg Leu Ile Glu Asn
            420                 425                 430

aaa cac ggc att gtt gcg gtt tac cat aga gat ggt gat aag gtt gtt     1344
Lys His Gly Ile Val Ala Val Tyr His Arg Asp Gly Asp Lys Val Val
        435                 440                 445

tat ggg gcg aat gta tac gac ggc gca tac aat acc gat gta ttc aat     1392
Tyr Gly Ala Asn Val Tyr Asp Gly Ala Tyr Asn Thr Asp Val Phe Asn
    450                 455                 460

agt gtc aac ggc atc gaa cgt gcc tat ctg cta ccc tcc ctg aag tct     1440
Ser Val Asn Gly Ile Glu Arg Ala Tyr Leu Leu Pro Ser Leu Lys Ser
465                 470                 475                 480

ggc ata cgc cgc att ttc gtc gtt gga ttg agt aca ggt tcg tgg gcg     1488
Gly Ile Arg Arg Ile Phe Val Val Gly Leu Ser Thr Gly Ser Trp Ala
                485                 490                 495

cgc gtc ttg tct gcc att ccg gaa atg cag tcg atg atc gtt gcg gaa     1536
Arg Val Leu Ser Ala Ile Pro Glu Met Gln Ser Met Ile Val Ala Glu
            500                 505                 510

atc aat ccg gca tac cgt agc ctt atc gcg gac gag ccg caa atc gcc 1584
Ile Asn Pro Ala Tyr Arg Ser Leu Ile Ala Asp Glu Pro Gln Ile Ala
        515                 520                 525

ccg ctt ttg cag gac aaa cgt gtt gaa att gta ttg gat gac ggt agg 1632
Pro Leu Leu Gln Asp Lys Arg Val Glu Ile Val Leu Asp Asp Gly Arg
    530                 535                 540

aaa tgg ctg cgt cgc cat cct gat gaa aaa ttc gac ctg att ttg atg 1680
Lys Trp Leu Arg Arg His Pro Asp Glu Lys Phe Asp Leu Ile Leu Met
545                 550                 555                 560

aat acg act tgg tac tgg cgt gcc tat tcc acc aac ctg ttg agt gcg 1728
Asn Thr Thr Trp Tyr Trp Arg Ala Tyr Ser Thr Asn Leu Leu Ser Ala
                565                 570                 575

gaa ttt tta aaa cag gtg caa agc cac ctt acc ccg gat ggt att gta 1776
Glu Phe Leu Lys Gln Val Gln Ser His Leu Thr Pro Asp Gly Ile Val
            580                 585                 590

atg ttt aat acc acg cac agc ccg cat gct ttt gct acc gcc gta cac 1824
Met Phe Asn Thr Thr His Ser Pro His Ala Phe Ala Thr Ala Val His
        595                 600                 605

agt att ccc tat gca tac cgc tat ggg cat atg gta gtc ggc tcg gca 1872
Ser Ile Pro Tyr Ala Tyr Arg Tyr Gly His Met Val Val Gly Ser Ala
    610                 615                 620

acc ccg gta gtt ttc cct aat aaa gaa ctg ctc aag caa cgt ctc tcc 1920
Thr Pro Val Val Phe Pro Asn Lys Glu Leu Leu Lys Gln Arg Leu Ser
625                 630                 635                 640

cgg ttg att tgg ccg gaa agc ggc agg cac gta ttt gac agc agc acc 1968
Arg Leu Ile Trp Pro Glu Ser Gly Arg His Val Phe Asp Ser Ser Thr
                645                 650                 655

gtg gat gct gca gca caa aag gtt gtc tct cgt atg ctg att cag atg 2016
Val Asp Ala Ala Ala Gln Lys Val Val Ser Arg Met Leu Ile Gln Met
            660                 665                 670

acg gaa cct tcg gct ggg gcg gaa gtc att acc gac gat aat atg att 2064
Thr Glu Pro Ser Ala Gly Ala Glu Val Ile Thr Asp Asp Asn Met Ile
        675                 680                 685

gta gaa tac aaa tac ggc aga ggg att taa 2094
Val Glu Tyr Lys Tyr Gly Arg Gly Ile
    690                 695

<210> 52
<211> 697
<212> PRT
<213> Neisseria meningitidis

<400> 52
Met Asn Ser Thr Ala Ser Lys Thr Leu Lys Gly Leu Ser Leu Val Phe
1               5                   10                  15

Phe Ala Ser Gly Phe Cys Ala Leu Ile Tyr Gln Val Ser Trp Gln Arg
            20                  25                  30

Leu Leu Phe Ser His Ile Gly Ile Asp Leu Ser Ser Ile Thr Val Ile
        35                  40                  45

Ile Ser Val Phe Met Val Gly Leu Gly Val Gly Ala Tyr Phe Gly Gly
    50                  55                  60

Arg Ile Ala Asp Arg Phe Pro Ser Ser Ile Ile Pro Leu Phe Cys Ile
65                  70                  75                  80

Ala Glu Val Ser Ile Gly Leu Phe Gly Leu Val Ser Arg Gly Leu Ile
                85                  90                  95

Ser Gly Leu Gly His Leu Leu Val Glu Ala Asp Leu Pro Ile Ile Ala
            100                 105                 110

Ala Ala Asn Phe Leu Leu Leu Leu Leu Pro Thr Phe Met Met Gly Ala
        115                 120                 125

Thr Leu Pro Leu Leu Thr Cys Phe Phe Asn Arg Lys Ile His Asn Val
    130                 135                 140

Gly Glu Ser Ile Gly Thr Leu Tyr Phe Phe Asn Thr Leu Gly Ala Ala
145                 150                 155                 160

Leu Gly Ser Leu Ala Ala Ala Glu Phe Phe Tyr Val Phe Phe Thr Leu
                165                 170                 175

Ser Gln Thr Ile Ala Leu Thr Ala Cys Phe Asn Leu Leu Ile Ala Ala
            180                 185                 190

Ser Val Cys Cys Val Thr Glu Arg Met Asp Ile Val Asn Thr Lys Pro
        195                 200                 205

Asn Thr Ser Leu Ile Tyr Met Leu Ser Phe Leu Ser Gly Leu Leu Ser
    210                 215                 220

Leu Gly Ile Glu Val Leu Trp Val Arg Met Phe Ser Phe Ala Ala Gln
225                 230                 235                 240

Ser Val Pro Gln Ala Phe Ser Phe Thr Leu Ala Tyr Phe Leu Thr Gly
                245                 250                 255

Ile Ala Val Gly Ala Tyr Phe Gly Lys Arg Ile Cys Arg Ser Arg Phe
            260                 265                 270

Val Asp Ile Pro Phe Ile Gly Gln Cys Phe Leu Trp Ala Gly Ile Ala
        275                 280                 285

Asp Phe Leu Ile Leu Gly Ala Ala Trp Leu Leu Thr Gly Phe Ser Gly
    290                 295                 300

Phe Val His His Ala Gly Ile Phe Ile Thr Leu Ser Ala Val Val Arg
305                 310                 315                 320

Gly Leu Ile Phe Pro Leu Val His His Val Gly Thr Asp Gly Asn Lys
                325                 330                 335

Ser Gly Arg Gln Val Ser Asn Val Tyr Phe Ala Asn Val Ala Gly Ser
            340                 345                 350

Ala Leu Gly Pro Val Leu Ile Gly Phe Val Ile Leu Asp Phe Leu Ser
        355                 360                 365

Thr Gln Gln Ile Tyr Leu Leu Ile Cys Leu Ile Ser Ala Ala Val Pro
    370                 375                 380

Leu Phe Cys Thr Leu Phe Gln Lys Ser Leu Arg Leu Asn Ala Val Ser
385                 390                 395                 400

Val Ala Val Ser Leu Met Phe Gly Ile Leu Met Phe Leu Leu Pro Asp
                405                 410                 415

Ser Val Phe Gln Asn Ile Ala Asp Arg Pro Asp Arg Leu Ile Glu Asn
            420                 425                 430

Lys His Gly Ile Val Ala Val Tyr His Arg Asp Gly Asp Lys Val Val
        435                 440                 445

Tyr Gly Ala Asn Val Tyr Asp Gly Ala Tyr Asn Thr Asp Val Phe Asn
    450                 455                 460

Ser Val Asn Gly Ile Glu Arg Ala Tyr Leu Leu Pro Ser Leu Lys Ser
465                 470                 475                 480

Gly Ile Arg Arg Ile Phe Val Val Gly Leu Ser Thr Gly Ser Trp Ala
                485                 490                 495

Arg Val Leu Ser Ala Ile Pro Glu Met Gln Ser Met Ile Val Ala Glu
            500                 505                 510

Ile Asn Pro Ala Tyr Arg Ser Leu Ile Ala Asp Glu Pro Gln Ile Ala
        515                 520                 525

Pro Leu Leu Gln Asp Lys Arg Val Glu Ile Val Leu Asp Asp Gly Arg
    530                 535                 540

Lys Trp Leu Arg Arg His Pro Asp Glu Lys Phe Asp Leu Ile Leu Met
545                 550                 555                 560

Asn Thr Thr Trp Tyr Trp Arg Ala Tyr Ser Thr Asn Leu Leu Ser Ala
                565                 570                 575

Glu Phe Leu Lys Gln Val Gln Ser His Leu Thr Pro Asp Gly Ile Val
            580                 585                 590

Met Phe Asn Thr Thr His Ser Pro His Ala Phe Ala Thr Ala Val His
        595                 600                 605

Ser Ile Pro Tyr Ala Tyr Arg Tyr Gly His Met Val Val Gly Ser Ala
    610                 615                 620

Thr Pro Val Val Phe Pro Asn Lys Glu Leu Leu Lys Gln Arg Leu Ser
625                 630                 635                 640

Arg Leu Ile Trp Pro Glu Ser Gly Arg His Val Phe Asp Ser Ser Thr
                645                 650                 655

Val Asp Ala Ala Ala Gln Lys Val Val Ser Arg Met Leu Ile Gln Met
            660                 665                 670

Thr Glu Pro Ser Ala Gly Ala Glu Val Ile Thr Asp Asp Asn Met Ile
        675                 680                 685

Val Glu Tyr Lys Tyr Gly Arg Gly Ile
    690                 695

<210> 53
<211> 1040
<212> PRT
<213> Neisseria meningitidis

<400> 53
Cys Leu Gly Gly Gly Gly Gly Thr Ser Ala Pro Asp Phe Asn Ala Gly
  1               5                  10                  15

Gly Thr Gly Ile Gly Ser Asn Ser Arg Ala Thr Thr Ala Lys Ser Ala
             20                  25                  30

Ala Val Ser Tyr Ala Gly Ile Lys Asn Glu Met Cys Lys Asp Arg Ser
         35                  40                  45

Met Leu Cys Ala Gly Arg Asp Asp Val Ala Val Thr Asp Arg Asp Ala
     50                  55                  60

Lys Ile Asn Ala Pro Pro Pro Asn Leu His Thr Gly Asp Phe Thr Asn
 65                  70                  75                  80

Pro Asn Asp Ala Tyr Lys Asn Leu Ile Asn Leu Lys Pro Ala Ile Glu
                 85                  90                  95

Ala Gly Tyr Thr Gly Arg Gly Val Glu Val Gly Ile Val Asp Thr Gly
            100                 105                 110

Glu Ser Val Gly Ser Ile Ser Phe Pro Glu Leu Tyr Gly Arg Lys Glu
        115                 120                 125

His Gly Tyr Asn Glu Asn Tyr Lys Asn Tyr Thr Ala Tyr Met Arg Lys
    130                 135                 140

Glu Ala Pro Glu Asp Gly Gly Gly Lys Asp Ile Lys Ala Ser Phe Asp
145                 150                 155                 160

Asp Glu Ala Val Ile Glu Thr Glu Ala Lys Pro Thr Asp Ile Arg His
                165                 170                 175

Val Lys Glu Ile Gly His Ile Asp Val Val Ser His Ile Ile Gly Gly
            180                 185                 190

Arg Ser Val Asp Gly Arg Pro Ala Gly Gly Ile Ala Pro Asp Ala Thr
        195                 200                 205

Leu His Ile Met Asn Thr His Asp Gly Thr Lys Asn Glu Ile Met Ser
    210                 215                 220

Ala Ala Ile Arg Asn Ala Trp Val Lys Leu Gly Glu Arg Gly Val Arg
225                 230                 235                 240

Ile Val Asn Asn Ser Phe Gly Thr Thr Ser Arg Ala Gly Thr Ala Asp
                245                 250                 255

His Phe Gln Ile Ala Asn Ser Glu Glu Gln Tyr Arg Gln Ala Leu Leu
            260                 265                 270

Ala Tyr Ser Gly Gly Asp Lys Thr Asp Glu Gly Ile Arg Leu Met Gln
        275                 280                 285

Gln Ser Asp Tyr Gly Asn Leu Ser Tyr His Ile Arg Asn Lys Asn Met
    290                 295                 300

Leu Phe Ile Phe Ser Ala Ser Asn Asp Ala Gln Ala Gln Pro Asn Thr
305                 310                 315                 320

Leu Thr Leu Leu Pro Phe Tyr Glu Lys Asp Ala Gln Lys Gly Ile Ile
                325                 330                 335

Thr Val Ala Gly Val Asp Arg Ser Gly Glu Lys Phe Asn Gly Ser Asn
            340                 345                 350

His Cys Gly Ile Thr Ala Met Trp Cys Leu Ser Ala Pro Tyr Glu Ala
        355                 360                 365

Ser Val Arg Phe Thr Arg Thr Asn Pro Ile Gln Ile Ala Gly Thr Ser
    370                 375                 380

Phe Ser Ala Pro Ile Val Thr Gly Thr Ala Ala Leu Leu Leu Gln Lys
385                 390                 395                 400

Tyr Pro Trp Met Ser Asn Asp Asn Leu Arg Thr Thr Leu Leu Thr Thr
                405                 410                 415

Ala Gln Asp Ile Gly Ala Val Gly Val Asp Ser Lys Phe Gly Trp Gly
            420                 425                 430

Leu Leu Asp Ala Gly Lys Ala Met Asn Gly Pro Ala Ser Phe Pro Phe
        435                 440                 445

Gly Asp Phe Thr Ala Asp Thr Lys Gly Thr Ser Asp Ile Ala Tyr Ser
    450                 455                 460

Phe Arg Asn Asp Ile Ser Gly Thr Gly Gly Leu Ile Lys Lys Gly Gly
465                 470                 475                 480

Ser Gln Leu Gln Leu His Gly Asn Asn Thr Tyr Thr Gly Lys Thr Ile
                485                 490                 495

Ile Glu Gly Gly Ser Leu Val Leu Tyr Gly Asn Asn Lys Ser Asp Met
            500                 505                 510

Arg Val Glu Thr Lys Gly Ala Leu Ile Tyr Asn Gly Ala Ala Ser Gly
        515                 520                 525

Gly Ser Leu Asn Ser Asp Gly Ile Val Tyr Leu Ala Asp Thr Asp Arg
    530                 535                 540

Ser Gly Ala Asn Glu Thr Val His Ile Lys Gly Asp Leu Gln Leu Gly
545                 550                 555                 560

Gly Glu Gly Thr Leu Tyr Thr Arg Leu Gly Lys Leu Leu Lys Val Asp
                565                 570                 575

Gly Thr Ala Met Thr Gly Gly Lys Leu Tyr Met Ser Ala Arg Gly Lys
            580                 585                 590

Gly Ala Gly Tyr Leu Asn Arg Thr Gly Gln Arg Val Pro Phe Leu Ser
        595                 600                 605

Ala Ala Lys Ile Gly Arg Asp Tyr Ser Phe Phe Thr Asn Ile Glu Thr
    610                 615                 620

Asp Gly Gly Leu Leu Ala Ser Leu Asp Ser Val Glu Lys Thr Ala Gly
625                 630                 635                 640

Ser Glu Gly Asp Thr Leu Ser Tyr Tyr Val Arg Arg Gly Asn Ala Ala
                645                 650                 655

Arg Thr Ala Ser Ala Ala Ala His Ser Ala Pro Ala Gly Leu Lys His
            660                 665                 670

Ala Val Glu Gln Gly Gly Ser Asn Leu Glu Asn Leu Met Val Glu Leu
        675                 680                 685

Asp Ala Ser Glu Ser Ser Ala Thr Pro Glu Thr Val Glu Thr Ala Ala
    690                 695                 700

Ala Asp Arg Thr Asp Met Pro Gly Ile Arg Pro Tyr Gly Ala Thr Phe
705                 710                 715                 720

Arg Ala Ala Ala Ala Val Gln His Ala Asn Ala Ala Asp Gly Val Arg
                725                 730                 735

Ile Phe Asn Ser Leu Ala Ala Thr Val Tyr Ala Asp Ser Thr Ala Ala
            740                 745                 750

His Ala Asp Met Gln Gly Arg Arg Leu Lys Ala Val Ser Asp Gly Leu
        755                 760                 765

Asp His Asn Ala Thr Gly Leu Arg Val Ile Ala Gln Thr Gln Gln Asp
    770                 775                 780

Gly Gly Thr Trp Glu Gln Gly Gly Val Glu Gly Lys Met Arg Gly Ser
785                 790                 795                 800

Thr Gln Thr Val Gly Ile Ala Ala Lys Thr Gly Glu Asn Thr Thr Ala
                805                 810                 815

Ala Ala Thr Leu Gly Met Gly His Ser Thr Trp Ser Glu Asn Ser Ala
            820                 825                 830

Asn Ala Lys Thr Asp Ser Ile Ser Leu Phe Ala Gly Ile Arg His Asp
        835                 840                 845

Ala Gly Asp Ile Gly Tyr Leu Lys Gly Leu Phe Ser Tyr Gly Arg Tyr
    850                 855                 860

Lys Asn Ser Ile Ser Arg Ser Thr Gly Ala Asp Glu His Ala Glu Gly
865                 870                 875                 880

Ser Val Asn Gly Thr Leu Met Gln Leu Gly Ala Leu Gly Gly Val Asn
                885                 890                 895

Val Pro Phe Ala Ala Thr Gly Asp Leu Thr Val Glu Gly Gly Leu Arg
            900                 905                 910

Tyr Asp Leu Leu Lys Gln Asp Ala Phe Ala Glu Lys Gly Ser Ala Leu
        915                 920                 925

Gly Trp Ser Gly Asn Ser Leu Thr Glu Gly Thr Leu Val Gly Leu Ala
    930                 935                 940

Gly Leu Lys Leu Ser Gln Pro Leu Ser Asp Lys Ala Val Leu Phe Ala
945                 950                 955                 960

Thr Ala Gly Val Glu Arg Asp Leu Asn Gly Arg Asp Tyr Thr Val Thr
                965                 970                 975

Gly Gly Phe Thr Gly Ala Thr Ala Ala Thr Gly Lys Thr Gly Ala Arg
            980                 985                 990

Asn Met Pro His Thr Arg Leu Val Ala Gly Leu Gly Ala Asp Val Glu
        995                 1000                1005

Phe Gly Asn Gly Trp Asn Gly Leu Ala Arg Tyr Ser Tyr Ala Gly Ser
   1010                 1015                1020

Lys Gln Tyr Gly Asn His Ser Gly Arg Val Gly Val Gly Tyr Arg Phe
1025                1030                1035                1040

<210> 54
<211> 858
<212> ADN
<213> Neisseria gonorrhoeae

<220>
<221> CDS
<222> (1)..(855)

<400> 54
atg tct gaa gaa aaa ttg aaa atg agt ttc gag cca acc gta atc gaa     48
Met Ser Glu Glu Lys Leu Lys Met Ser Phe Glu Pro Thr Val Ile Glu
  1               5                  10                  15

cat ttg ggt gta aag atg tat tcg cac act gtt cct gcg att gcc gag     96
His Leu Gly Val Lys Met Tyr Ser His Thr Val Pro Ala Ile Ala Glu
             20                  25                  30

ttg ata gcg aat gcc tac gat gca tgt gct acg gaa gtg gaa gtt agg    144
Leu Ile Ala Asn Ala Tyr Asp Ala Cys Ala Thr Glu Val Glu Val Arg
         35                  40                  45

tta ttc gat aaa ccg gag cat aaa atc gtt att aaa gat aat ggc ata    192
Leu Phe Asp Lys Pro Glu His Lys Ile Val Ile Lys Asp Asn Gly Ile
     50                  55                  60

gga atg agc ttc gat gaa atc aat gat ttt tat ttg aga atc ggt cgg     240
Gly Met Ser Phe Asp Glu Ile Asn Asp Phe Tyr Leu Arg Ile Gly Arg
65                  70                  75                  80

aac aga agg gaa gaa aaa caa gcc tcc ccg tgc gga aga att cca acg     288
Asn Arg Arg Glu Glu Lys Gln Ala Ser Pro Cys Gly Arg Ile Pro Thr
                85                  90                  95

ggt aaa aaa ggt ctt ggt aaa ttg gca tta ttc agg ctt ggc aac aaa     336
Gly Lys Lys Gly Leu Gly Lys Leu Ala Leu Phe Arg Leu Gly Asn Lys
            100                 105                 110

atc gaa atc tct act atc caa gga aac gaa cgg gtt act ttt act ttg     384
Ile Glu Ile Ser Thr Ile Gln Gly Asn Glu Arg Val Thr Phe Thr Leu
        115                 120                 125

gat tat gca gag att aaa aaa agt gag cgt att tat caa ccg gag ttt     432
Asp Tyr Ala Glu Ile Lys Lys Ser Glu Arg Ile Tyr Gln Pro Glu Phe
    130                 135                 140

cag aaa gag tct gtt aaa ccc aat acc gaa aac gga aca act ata act     480
Gln Lys Glu Ser Val Lys Pro Asn Thr Glu Asn Gly Thr Thr Ile Thr
145                 150                 155                 160

tta acc gag ctg acg aaa aaa caa gga tac ccg tta gat aat tat gtg     528
Leu Thr Glu Leu Thr Lys Lys Gln Gly Tyr Pro Leu Asp Asn Tyr Val
                165                 170                 175

ggg cat ctt tcc cgt tta ttt gat ttt ccg gct cag gat ttt aaa atc     576
Gly His Leu Ser Arg Leu Phe Asp Phe Pro Ala Gln Asp Phe Lys Ile
            180                 185                 190

aaa gta agc ttg aac ggc tcg gaa cca aga atc att gac gga aac cta     624
Lys Val Ser Leu Asn Gly Ser Glu Pro Arg Ile Ile Asp Gly Asn Leu
        195                 200                 205

aaa tat aat ctt gtt acc cca caa ttc gaa tgg gaa tac cag gat cta     672
Lys Tyr Asn Leu Val Thr Pro Gln Phe Glu Trp Glu Tyr Gln Asp Leu
    210                 215                 220

gca acc aat att tca tcg tta tct tca aaa ttc gaa cag tat gaa tac     720
Ala Thr Asn Ile Ser Ser Leu Ser Ser Lys Phe Glu Gln Tyr Glu Tyr
225                 230                 235                 240

agc gga tta ata caa ggt aag ttc att aca acg gaa aaa cct tta aag     768
Ser Gly Leu Ile Gln Gly Lys Phe Ile Thr Thr Glu Lys Pro Leu Lys
                245                 250                 255

aat aat atg aaa ggt att acc ttg ttt gcc aac ggc aga atg gta aat     816
Asn Asn Met Lys Gly Ile Thr Leu Phe Ala Asn Gly Arg Met Val Asn
            260                 265                 270

atg ccc gag ttt ttc act gat agc gaa tcc agc cat ttc taa             858
Met Pro Glu Phe Phe Thr Asp Ser Glu Ser Ser His Phe
        275                 280                 285

<210> 55
<211> 285

<212> PRT
<213> Neisseria gonorrhoeae

<400> 55
Met Ser Glu Glu Lys Leu Lys Met Ser Phe Glu Pro Thr Val Ile Glu
1               5                   10                  15

His Leu Gly Val Lys Met Tyr Ser His Thr Val Pro Ala Ile Ala Glu
            20                  25                  30

Leu Ile Ala Asn Ala Tyr Asp Ala Cys Ala Thr Glu Val Glu Val Arg
        35                  40                  45

Leu Phe Asp Lys Pro Glu His Lys Ile Val Ile Lys Asp Asn Gly Ile
    50                  55                  60

Gly Met Ser Phe Asp Glu Ile Asn Asp Phe Tyr Leu Arg Ile Gly Arg
65                  70                  75                  80

Asn Arg Arg Glu Glu Lys Gln Ala Ser Pro Cys Gly Arg Ile Pro Thr
                85                  90                  95

Gly Lys Lys Gly Leu Gly Lys Leu Ala Leu Phe Arg Leu Gly Asn Lys
            100                 105                 110

Ile Glu Ile Ser Thr Ile Gln Gly Asn Glu Arg Val Thr Phe Thr Leu
        115                 120                 125

Asp Tyr Ala Glu Ile Lys Lys Ser Glu Arg Ile Tyr Gln Pro Glu Phe
    130                 135                 140

Gln Lys Glu Ser Val Lys Pro Asn Thr Glu Asn Gly Thr Thr Ile Thr
145                 150                 155                 160

Leu Thr Glu Leu Thr Lys Lys Gln Gly Tyr Pro Leu Asp Asn Tyr Val
                165                 170                 175

Gly His Leu Ser Arg Leu Phe Asp Phe Pro Ala Gln Asp Phe Lys Ile
            180                 185                 190

Lys Val Ser Leu Asn Gly Ser Glu Pro Arg Ile Ile Asp Gly Asn Leu
        195                 200                 205

Lys Tyr Asn Leu Val Thr Pro Gln Phe Glu Trp Glu Tyr Gln Asp Leu
    210                 215                 220

Ala Thr Asn Ile Ser Ser Leu Ser Ser Lys Phe Glu Gln Tyr Glu Tyr
225                 230                 235                 240

Ser Gly Leu Ile Gln Gly Lys Phe Ile Thr Thr Glu Lys Pro Leu Lys
                245                 250                 255

Asn Asn Met Lys Gly Ile Thr Leu Phe Ala Asn Gly Arg Met Val Asn
            260                 265                 270

Met Pro Glu Phe Phe Thr Asp Ser Glu Ser Ser His Phe
        275                 280                 285

<210> 56
<211> 1575
<212> ADN
<213> Neisseria gonorrhoeae

<220>
<221> CDS
<222> (1)..(1572)

<400> 56
atg aaa aaa tcc ctt ttc gtt ctc ttt ctg tat tca tcc cta ctt acc 48
Met Lys Lys Ser Leu Phe Val Leu Phe Leu Tyr Ser Ser Leu Leu Thr
1               5                   10                  15

gcc agc gaa atc gcc tat cgc ttt gta ttc gga att gaa acc tta ccg 96
Ala Ser Glu Ile Ala Tyr Arg Phe Val Phe Gly Ile Glu Thr Leu Pro
            20                  25                  30

gct gca aaa atg gcg gaa acg ttt gcg ctg aca ttt atg att gct gcg 144
Ala Ala Lys Met Ala Glu Thr Phe Ala Leu Thr Phe Met Ile Ala Ala
        35                  40                  45

ctg tat ctg ttt gcg cgt tat aag gct tcg cgg ctg ctg att gcg gtg 192
Leu Tyr Leu Phe Ala Arg Tyr Lys Ala Ser Arg Leu Leu Ile Ala Val
    50                  55                  60

ttt ttc gcg ttc agc atg att gcc aac aat gtg cat tac gcg gtt tat 240
Phe Phe Ala Phe Ser Met Ile Ala Asn Asn Val His Tyr Ala Val Tyr
65                  70                  75                  80

caa agc tgg atg acg ggt att aac tat tgg ctg atg ctg aaa gag gtt 288
Gln Ser Trp Met Thr Gly Ile Asn Tyr Trp Leu Met Leu Lys Glu Val
                85                  90                  95

acc gaa gtc ggc agc gcg ggc gcg tcg atg ttg gat aag ttg tgg ctg 336
Thr Glu Val Gly Ser Ala Gly Ala Ser Met Leu Asp Lys Leu Trp Leu
            100                 105                 110

cct gct ttg tgg ggc gtg gcg gaa gtc atg ttg ttt tgc agc ctt gcc 384
Pro Ala Leu Trp Gly Val Ala Glu Val Met Leu Phe Cys Ser Leu Ala
        115                 120                 125

aag ttc cgc cgt aag acg cat ttt tct gcc gat ata ctg ttt gcc ttc 432
Lys Phe Arg Arg Lys Thr His Phe Ser Ala Asp Ile Leu Phe Ala Phe
    130                 135                 140

cta atg ctg atg att ttc gtg cgt tcg ttc gac acg aaa caa gag cac 480
Leu Met Leu Met Ile Phe Val Arg Ser Phe Asp Thr Lys Gln Glu His
145                 150                 155                 160

ggt att tcg ccc aaa ccg aca tac agc cgc atc aaa gcc aat tat ttc 528
Gly Ile Ser Pro Lys Pro Thr Tyr Ser Arg Ile Lys Ala Asn Tyr Phe
                165                 170                 175

agc ttc ggt tat ttt gtc ggg cgc gtg ttg ccg tat cag ttg ttt gat 576
Ser Phe Gly Tyr Phe Val Gly Arg Val Leu Pro Tyr Gln Leu Phe Asp
            180                 185                 190

tta agc aag atc cct gtg ttc aaa cag cct gct cca agc aaa atc ggg 624
Leu Ser Lys Ile Pro Val Phe Lys Gln Pro Ala Pro Ser Lys Ile Gly

195                 200                 205

caa ggc agt att caa aat atc gtc ctg att atg ggc gaa agc gaa agc     672
Gln Gly Ser Ile Gln Asn Ile Val Leu Ile Met Gly Glu Ser Glu Ser
    210                 215                 220

gcg gcg cat ttg aaa ttg ttt ggt tac ggg cgc gaa act tcg ccg ttt     720
Ala Ala His Leu Lys Leu Phe Gly Tyr Gly Arg Glu Thr Ser Pro Phe
225                 230                 235                 240

tta acc cgg ctg tcg caa gcc gat ttt aag ccg att gtg aaa caa agt     768
Leu Thr Arg Leu Ser Gln Ala Asp Phe Lys Pro Ile Val Lys Gln Ser
                245                 250                 255

tat tcc gca ggc ttt atg acg gca gta tcc ctg ccc agt ttc ttt aac     816
Tyr Ser Ala Gly Phe Met Thr Ala Val Ser Leu Pro Ser Phe Phe Asn
            260                 265                 270

gtc ata ccg cac gcc aac ggc ttg gaa caa atc agc ggc ggc gat acc     864
Val Ile Pro His Ala Asn Gly Leu Glu Gln Ile Ser Gly Gly Asp Thr
        275                 280                 285

aat atg ttc cgc ctc gcc aaa gag cag ggc tat gaa acg tat ttt tac     912
Asn Met Phe Arg Leu Ala Lys Glu Gln Gly Tyr Glu Thr Tyr Phe Tyr
    290                 295                 300

agt gcc cag gct gaa aac caa atg gca att ttg aac tta atc ggt aag     960
Ser Ala Gln Ala Glu Asn Gln Met Ala Ile Leu Asn Leu Ile Gly Lys
305                 310                 315                 320

aaa tgg ata gac cat ctg att cag ccg acg caa ctt ggc tac ggc aac     1008
Lys Trp Ile Asp His Leu Ile Gln Pro Thr Gln Leu Gly Tyr Gly Asn
                325                 330                 335

ggc gac aat atg ccc gat gag aag ctg ctg ccg ttg ttc gac aaa atc     1056
Gly Asp Asn Met Pro Asp Glu Lys Leu Leu Pro Leu Phe Asp Lys Ile
            340                 345                 350

aat ttg cag cag ggc agg cat ttt atc gtg ttg cac caa cgc ggt tcg     1104
Asn Leu Gln Gln Gly Arg His Phe Ile Val Leu His Gln Arg Gly Ser
        355                 360                 365

cac gcc cca tac ggc gca ttg ttg cag cct caa gat aaa gta ttc ggc     1152
His Ala Pro Tyr Gly Ala Leu Leu Gln Pro Gln Asp Lys Val Phe Gly
    370                 375                 380

gaa gcc gat att gtg gat aag tac gac aac acc atc cac aaa acc gac     1200
Glu Ala Asp Ile Val Asp Lys Tyr Asp Asn Thr Ile His Lys Thr Asp
385                 390                 395                 400

caa atg att caa acc gta ttc gag cag ctg caa aag cag cct gac ggc     1248
Gln Met Ile Gln Thr Val Phe Glu Gln Leu Gln Lys Gln Pro Asp Gly
                405                 410                 415

aac tgg ctg ttt gcc tat acc tcc gat cat ggc cag tat gtg cgc caa     1296
Asn Trp Leu Phe Ala Tyr Thr Ser Asp His Gly Gln Tyr Val Arg Gln
            420                 425                 430

gat atc tac aat caa ggc acg gtg cag ccc gac agc tat att gtg cct     1344
Asp Ile Tyr Asn Gln Gly Thr Val Gln Pro Asp Ser Tyr Ile Val Pro

435                 440                 445

ctg gtt ttg tac agc ccg gat aag gcc gtg caa cag gct gcc aac cag      1392
Leu Val Leu Tyr Ser Pro Asp Lys Ala Val Gln Gln Ala Ala Asn Gln
    450                 455                 460

gct ttt gcg cct tgc gag att gcc ttc cat cag cag ctt tca acg ttc      1440
Ala Phe Ala Pro Cys Glu Ile Ala Phe His Gln Gln Leu Ser Thr Phe
465                 470                 475                 480

ctg att cac acg ttg ggc tac gat atg ccg gtt tca ggt tgt cgc gaa      1488
Leu Ile His Thr Leu Gly Tyr Asp Met Pro Val Ser Gly Cys Arg Glu
                485                 490                 495

ggc tcg gta aca ggc aac ctg att acg ggc gat gca ggc agc ttg aac      1536
Gly Ser Val Thr Gly Asn Leu Ile Thr Gly Asp Ala Gly Ser Leu Asn
            500                 505                 510

att cgc aac ggc aag gcg gaa tat gtt tat ccg caa taa                  1575
Ile Arg Asn Gly Lys Ala Glu Tyr Val Tyr Pro Gln
        515                 520

<210> 57
<211> 524
<212> PRT
<213> Neisseria gonorrhoeae

<400> 57
Met Lys Lys Ser Leu Phe Val Leu Phe Leu Tyr Ser Ser Leu Leu Thr
1               5                   10                  15

Ala Ser Glu Ile Ala Tyr Arg Phe Val Phe Gly Ile Glu Thr Leu Pro
            20                  25                  30

Ala Ala Lys Met Ala Glu Thr Phe Ala Leu Thr Phe Met Ile Ala Ala
        35                  40                  45

Leu Tyr Leu Phe Ala Arg Tyr Lys Ala Ser Arg Leu Leu Ile Ala Val
    50                  55                  60

Phe Phe Ala Phe Ser Met Ile Ala Asn Asn Val His Tyr Ala Val Tyr
65                  70                  75                  80

Gln Ser Trp Met Thr Gly Ile Asn Tyr Trp Leu Met Leu Lys Glu Val
                85                  90                  95

Thr Glu Val Gly Ser Ala Gly Ala Ser Met Leu Asp Lys Leu Trp Leu
            100                 105                 110

Pro Ala Leu Trp Gly Val Ala Glu Val Met Leu Phe Cys Ser Leu Ala
        115                 120                 125

Lys Phe Arg Arg Lys Thr His Phe Ser Ala Asp Ile Leu Phe Ala Phe
    130                 135                 140

Leu Met Leu Met Ile Phe Val Arg Ser Phe Asp Thr Lys Gln Glu His
145                 150                 155                 160

Gly Ile Ser Pro Lys Pro Thr Tyr Ser Arg Ile Lys Ala Asn Tyr Phe

                165                 170                 175

Ser Phe Gly Tyr Phe Val Gly Arg Val Leu Pro Tyr Gln Leu Phe Asp
            180                 185                 190

Leu Ser Lys Ile Pro Val Phe Lys Gln Pro Ala Pro Ser Lys Ile Gly
        195                 200                 205

Gln Gly Ser Ile Gln Asn Ile Val Leu Ile Met Gly Glu Ser Glu Ser
    210                 215                 220

Ala Ala His Leu Lys Leu Phe Gly Tyr Gly Arg Glu Thr Ser Pro Phe
225                 230                 235                 240

Leu Thr Arg Leu Ser Gln Ala Asp Phe Lys Pro Ile Val Lys Gln Ser
                245                 250                 255

Tyr Ser Ala Gly Phe Met Thr Ala Val Ser Leu Pro Ser Phe Phe Asn
            260                 265                 270

Val Ile Pro His Ala Asn Gly Leu Glu Gln Ile Ser Gly Gly Asp Thr
        275                 280                 285

Asn Met Phe Arg Leu Ala Lys Glu Gln Gly Tyr Glu Thr Tyr Phe Tyr
    290                 295                 300

Ser Ala Gln Ala Glu Asn Gln Met Ala Ile Leu Asn Leu Ile Gly Lys
305                 310                 315                 320

Lys Trp Ile Asp His Leu Ile Gln Pro Thr Gln Leu Gly Tyr Gly Asn
                325                 330                 335

Gly Asp Asn Met Pro Asp Glu Lys Leu Leu Pro Leu Phe Asp Lys Ile
            340                 345                 350

Asn Leu Gln Gln Gly Arg His Phe Ile Val Leu His Gln Arg Gly Ser
        355                 360                 365

His Ala Pro Tyr Gly Ala Leu Leu Gln Pro Gln Asp Lys Val Phe Gly
    370                 375                 380

Glu Ala Asp Ile Val Asp Lys Tyr Asp Asn Thr Ile His Lys Thr Asp
385                 390                 395                 400

Gln Met Ile Gln Thr Val Phe Glu Gln Leu Gln Lys Gln Pro Asp Gly
                405                 410                 415

Asn Trp Leu Phe Ala Tyr Thr Ser Asp His Gly Gln Tyr Val Arg Gln
            420                 425                 430

Asp Ile Tyr Asn Gln Gly Thr Val Gln Pro Asp Ser Tyr Ile Val Pro
        435                 440                 445

Leu Val Leu Tyr Ser Pro Asp Lys Ala Val Gln Gln Ala Ala Asn Gln
    450                 455                 460

Ala Phe Ala Pro Cys Glu Ile Ala Phe His Gln Gln Leu Ser Thr Phe
465                 470                 475                 480

Leu Ile His Thr Leu Gly Tyr Asp Met Pro Val Ser Gly Cys Arg Glu

                485                 490                 495

Gly Ser Val Thr Gly Asn Leu Ile Thr Gly Asp Ala Gly Ser Leu Asn
            500                 505                 510

Ile Arg Asn Gly Lys Ala Glu Tyr Val Tyr Pro Gln
        515                 520

<210> 58
<211> 1314
<212> ADN
<213> Neisseria gonorrhoeae

<220>
<221> CDS
<222> (1)..(1311)

<400> 58
atg ctg acg ttt atc gga ttg ctg att atc ggg gtc atc gta tgg ctg 48
Met Leu Thr Phe Ile Gly Leu Leu Ile Ile Gly Val Ile Val Trp Leu
1               5                   10                  15

ttg ctg acg gaa aaa gtg tcg ccc atc atc gca tta atc ttg gtg ccg 96
Leu Leu Thr Glu Lys Val Ser Pro Ile Ile Ala Leu Ile Leu Val Pro
            20                  25                  30

ctg att ggg gcg ttg ctg gcg ggg ttt gat gta tcc caa tta aaa gaa 144
Leu Ile Gly Ala Leu Leu Ala Gly Phe Asp Val Ser Gln Leu Lys Glu
        35                  40                  45

ttt tat tcg ggc ggc acg aaa tcg gtg acg cag att gtg att atg ttt 192
Phe Tyr Ser Gly Gly Thr Lys Ser Val Thr Gln Ile Val Ile Met Phe
    50                  55                  60

atg ttt tcc att ttg ttt ttt gga atc atg aac gat gtg ggg ctg ttc 240
Met Phe Ser Ile Leu Phe Phe Gly Ile Met Asn Asp Val Gly Leu Phe
65                  70                  75                  80

cgt ccg atg ata ggc ggt ttg att aag ctg act cgg ggt aat atc gtg 288
Arg Pro Met Ile Gly Gly Leu Ile Lys Leu Thr Arg Gly Asn Ile Val
                85                  90                  95

gca gtg agt gtg ggg acg gtc ttg gtg tcg gtg gtg gca cag ttg gac 336
Ala Val Ser Val Gly Thr Val Leu Val Ser Val Val Ala Gln Leu Asp
            100                 105                 110

ggg gcg ggc gcg acg acg ttt tta tcg gtc gtc ccc gcc ctt ttg ccg 384
Gly Ala Gly Ala Thr Thr Phe Leu Ser Val Val Pro Ala Leu Leu Pro
        115                 120                 125

ctt tac aag cgt ctg cat atg aat cct tac ctg ctg ttt ttg ctg ctg 432
Leu Tyr Lys Arg Leu His Met Asn Pro Tyr Leu Leu Phe Leu Leu Leu
    130                 135                 140

act tcc agc gcg ggg cta atc aac ctt ttg ccg cgg ggc ggg ccg atc 480
Thr Ser Ser Ala Gly Leu Ile Asn Leu Leu Pro Arg Gly Gly Pro Ile
145                 150                 155                 160

ggg cgg gtt gca agc gtg ttg ggc gca gat gtg ggc gaa ttg tat aaa 528
Gly Arg Val Ala Ser Val Leu Gly Ala Asp Val Gly Glu Leu Tyr Lys
                165                 170                 175

cct ttg ttg acg gtg caa att atc ggt gtg gtg ttt atc ctt gtg ctg 576
Pro Leu Leu Thr Val Gln Ile Ile Gly Val Val Phe Ile Leu Val Leu
            180                 185                 190

tcc ctg ttt ttg ggt gtg cgt gaa aaa agg cgg att gtc cgg gag ttg 624
Ser Leu Phe Leu Gly Val Arg Glu Lys Arg Arg Ile Val Arg Glu Leu
        195                 200                 205

ggc gcg ttg ccc gcc gtg gcg gat ttg ata aag ccg gcg cct ttg tcg 672
Gly Ala Leu Pro Ala Val Ala Asp Leu Ile Lys Pro Ala Pro Leu Ser
    210                 215                 220

gaa gaa gaa caa aaa ttg gcg cgt ccg aaa ctg ttt tgg tgg aat gtc 720
Glu Glu Glu Gln Lys Leu Ala Arg Pro Lys Leu Phe Trp Trp Asn Val
225                 230                 235                 240

ctg ctg ttt ttg gcg gcg atg agc ctg ctt ttt tcg ggc atc ttc ccg 768
Leu Leu Phe Leu Ala Ala Met Ser Leu Leu Phe Ser Gly Ile Phe Pro
                245                 250                 255

ccg ggt tat gta ttt atg ctg gct gca acg gcg gcg ttg ctt ttg aat 816
Pro Gly Tyr Val Phe Met Leu Ala Ala Thr Ala Ala Leu Leu Leu Asn
            260                 265                 270

tac cgc agc ccg cag gaa cag atg gag cgg att tat gcc cac gcc ggc 864
Tyr Arg Ser Pro Gln Glu Gln Met Glu Arg Ile Tyr Ala His Ala Gly
        275                 280                 285

ggc gcg gtg atg atg gcg tcc att att ttg gcg gca ggt acg ttt ttg 912
Gly Ala Val Met Met Ala Ser Ile Ile Leu Ala Ala Gly Thr Phe Leu
    290                 295                 300

ggg att ttg aag ggc gcg ggg atg ttg gac gcg att tcc aaa gac ctt 960
Gly Ile Leu Lys Gly Ala Gly Met Leu Asp Ala Ile Ser Lys Asp Leu
305                 310                 315                 320

gtg cat atc ctg ccg gac gcg ttg ctg cct tat ctg cat att gcc atc 1008
Val His Ile Leu Pro Asp Ala Leu Leu Pro Tyr Leu His Ile Ala Ile
                325                 330                 335

ggt gtg ttg ggt att ccg ctt gag ttg gtt ttg agt acg gac gct tat 1056
Gly Val Leu Gly Ile Pro Leu Glu Leu Val Leu Ser Thr Asp Ala Tyr
            340                 345                 350

tat ttc gga ctg ttt ccg att gtg gaa cag att acc tcg cag gcg ggc 1104
Tyr Phe Gly Leu Phe Pro Ile Val Glu Gln Ile Thr Ser Gln Ala Gly
        355                 360                 365

gtt gca ccc gaa gcg gca ggc tat gcg atg ttg atc ggc agt atc gtc 1152
Val Ala Pro Glu Ala Ala Gly Tyr Ala Met Leu Ile Gly Ser Ile Val
    370                 375                 380

ggt act ttt gtt acg ccg ctt tcg ccg gct ttg tgg atg ggt ttg ggt 1200
Gly Thr Phe Val Thr Pro Leu Ser Pro Ala Leu Trp Met Gly Leu Gly
385                 390                 395                 400

ttg gcg aaa ttg tcg atg ggc aaa cac atc cgt tat tcg ttt ttc tgg     1248
Leu Ala Lys Leu Ser Met Gly Lys His Ile Arg Tyr Ser Phe Phe Trp
                405                 410                 415

gcg tgg ggt ttg tcg ctg gcg ata ttg gtc agt tcg ata gcg gca gga     1296
Ala Trp Gly Leu Ser Leu Ala Ile Leu Val Ser Ser Ile Ala Ala Gly
            420                 425                 430

atc gtg cct ctg ccg taa                                             1314
Ile Val Pro Leu Pro
        435


<210> 59
<211> 437
<212> PRT
<213> Neisseria gonorrhoeae

<400> 59
Met Leu Thr Phe Ile Gly Leu Leu Ile Ile Gly Val Ile Val Trp Leu
 1               5                  10                  15

Leu Leu Thr Glu Lys Val Ser Pro Ile Ile Ala Leu Ile Leu Val Pro
            20                  25                  30

Leu Ile Gly Ala Leu Leu Ala Gly Phe Asp Val Ser Gln Leu Lys Glu
        35                  40                  45

Phe Tyr Ser Gly Gly Thr Lys Ser Val Thr Gln Ile Val Ile Met Phe
    50                  55                  60

Met Phe Ser Ile Leu Phe Phe Gly Ile Met Asn Asp Val Gly Leu Phe
65                  70                  75                  80

Arg Pro Met Ile Gly Gly Leu Ile Lys Leu Thr Arg Gly Asn Ile Val
                85                  90                  95

Ala Val Ser Val Gly Thr Val Leu Val Ser Val Val Ala Gln Leu Asp
            100                 105                 110

Gly Ala Gly Ala Thr Thr Phe Leu Ser Val Val Pro Ala Leu Leu Pro
        115                 120                 125

Leu Tyr Lys Arg Leu His Met Asn Pro Tyr Leu Leu Phe Leu Leu Leu
    130                 135                 140

Thr Ser Ser Ala Gly Leu Ile Asn Leu Leu Pro Arg Gly Gly Pro Ile
145                 150                 155                 160

Gly Arg Val Ala Ser Val Leu Gly Ala Asp Val Gly Glu Leu Tyr Lys
                165                 170                 175

Pro Leu Leu Thr Val Gln Ile Ile Gly Val Val Phe Ile Leu Val Leu
            180                 185                 190

Ser Leu Phe Leu Gly Val Arg Glu Lys Arg Arg Ile Val Arg Glu Leu
        195                 200                 205

Gly Ala Leu Pro Ala Val Ala Asp Leu Ile Lys Pro Ala Pro Leu Ser
    210                 215                 220

Glu Glu Glu Gln Lys Leu Ala Arg Pro Lys Leu Phe Trp Trp Asn Val
225                 230                 235                 240

Leu Leu Phe Leu Ala Ala Met Ser Leu Leu Phe Ser Gly Ile Phe Pro
                245                 250                 255

Pro Gly Tyr Val Phe Met Leu Ala Ala Thr Ala Ala Leu Leu Leu Asn
            260                 265                 270

Tyr Arg Ser Pro Gln Glu Gln Met Glu Arg Ile Tyr Ala His Ala Gly
        275                 280                 285

Gly Ala Val Met Met Ala Ser Ile Ile Leu Ala Ala Gly Thr Phe Leu
    290                 295                 300

Gly Ile Leu Lys Gly Ala Gly Met Leu Asp Ala Ile Ser Lys Asp Leu
305                 310                 315                 320

Val His Ile Leu Pro Asp Ala Leu Leu Pro Tyr Leu His Ile Ala Ile
                325                 330                 335

Gly Val Leu Gly Ile Pro Leu Glu Leu Val Leu Ser Thr Asp Ala Tyr
            340                 345                 350

Tyr Phe Gly Leu Phe Pro Ile Val Glu Gln Ile Thr Ser Gln Ala Gly
        355                 360                 365

Val Ala Pro Glu Ala Ala Gly Tyr Ala Met Leu Ile Gly Ser Ile Val
    370                 375                 380

Gly Thr Phe Val Thr Pro Leu Ser Pro Ala Leu Trp Met Gly Leu Gly
385                 390                 395                 400

Leu Ala Lys Leu Ser Met Gly Lys His Ile Arg Tyr Ser Phe Phe Trp
                405                 410                 415

Ala Trp Gly Leu Ser Leu Ala Ile Leu Val Ser Ser Ile Ala Ala Gly
            420                 425                 430

Ile Val Pro Leu Pro
        435

<210> 60
<211> 1155
<212> ADN
<213> Neisseria gonorrhoeae

<220>
<221> CDS
<222> (1)..(1152)

<400> 60
atg ggc atc cat ctc gac ttc ggc att agt cct aaa acg ttc cga cag      48
Met Gly Ile His Leu Asp Phe Gly Ile Ser Pro Lys Thr Phe Arg Gln
1               5                   10                  15

act tat ctg tat caa aag ccc aag ctc ttt aaa gga gcg gtt cgg aat      96

Thr Tyr Leu Tyr Gln Lys Pro Lys Leu Phe Lys Gly Ala Val Arg Asn
            20                  25                  30

ctc gaa gcc gca tct tgt aaa tat atc aac gag ata tac caa cga gca     144
Leu Glu Ala Ala Ser Cys Lys Tyr Ile Asn Glu Ile Tyr Gln Arg Ala
        35                  40                  45

gac cca acc gca ccg ctg ttt cat ctg cgt aaa aaa ggc gca atc gtt     192
Asp Pro Thr Ala Pro Leu Phe His Leu Arg Lys Lys Gly Ala Ile Val
    50                  55                  60

cct aaa gaa gaa tac gtc gaa agt ttc gac gat ttg ggc aaa act cgc     240
Pro Lys Glu Glu Tyr Val Glu Ser Phe Asp Asp Leu Gly Lys Thr Arg
65                  70                  75                  80

tac cgt ttt att aaa tcc gtt atc tac gaa cat atg aag aat ggt gcg     288
Tyr Arg Phe Ile Lys Ser Val Ile Tyr Glu His Met Lys Asn Gly Ala
                85                  90                  95

tcg tta gtc tat aac cat att aac aac gag ccg ttt tca gac cat atc     336
Ser Leu Val Tyr Asn His Ile Asn Asn Glu Pro Phe Ser Asp His Ile
            100                 105                 110

gcc cgt caa gtc gcc cgc ttt gcc ggc gca cat act att gtt agt gga     384
Ala Arg Gln Val Ala Arg Phe Ala Gly Ala His Thr Ile Val Ser Gly
        115                 120                 125

tat ctt gct ttt ggc agc gac gaa tct tat aaa aac cat tgg gat acc     432
Tyr Leu Ala Phe Gly Ser Asp Glu Ser Tyr Lys Asn His Trp Asp Thr
    130                 135                 140

cgc gat gtg tat gcc atc cag ctt ttc ggc aag aaa cgt tgg caa ctt     480
Arg Asp Val Tyr Ala Ile Gln Leu Phe Gly Lys Lys Arg Trp Gln Leu
145                 150                 155                 160

act gcc cct gat ttc cct atg cca ttg tat atg caa cag act aaa gat     528
Thr Ala Pro Asp Phe Pro Met Pro Leu Tyr Met Gln Gln Thr Lys Asp
                165                 170                 175

act gat att tcc att cct gaa cat atc gat atg gat att atc ctt gaa     576
Thr Asp Ile Ser Ile Pro Glu His Ile Asp Met Asp Ile Ile Leu Glu
            180                 185                 190

gca ggt gat gtc ctc tac atc cca cgc ggt tgg tgg cac aga cct atc     624
Ala Gly Asp Val Leu Tyr Ile Pro Arg Gly Trp Trp His Arg Pro Ile
        195                 200                 205

ccg ctc ggc tgt gaa acc ttc cac ttc gct gtc ggt acc ttc cca cca     672
Pro Leu Gly Cys Glu Thr Phe His Phe Ala Val Gly Thr Phe Pro Pro
    210                 215                 220

aac ggc tat aat tac ctc gag tgg cta atg aag aaa ttt ccc acc ata     720
Asn Gly Tyr Asn Tyr Leu Glu Trp Leu Met Lys Lys Phe Pro Thr Ile
225                 230                 235                 240

gaa agt ctg cgc cac agt ttc tca gac tgg gag caa gat agg acg cgt     768
Glu Ser Leu Arg His Ser Phe Ser Asp Trp Glu Gln Asp Arg Thr Arg
                245                 250                 255

atc aac gat act gcc gca caa att gct gcc atg att gcc gac ccc gtc     816

Ile Asn Asp Thr Ala Ala Gln Ile Ala Ala Met Ile Ala Asp Pro Val
            260                 265                 270

aat tat gaa gcc ttc agt gaa gac ttt ctc ggc aaa gaa cgt acc gat      864
Asn Tyr Glu Ala Phe Ser Glu Asp Phe Leu Gly Lys Glu Arg Thr Asp
        275                 280                 285

acc gct ttt cat ctc gaa cag ttc gcg aat ccc aac gct act ccg ctt      912
Thr Ala Phe His Leu Glu Gln Phe Ala Asn Pro Asn Ala Thr Pro Leu
    290                 295                 300

tca gac gac gtc agg ttg aga tta aat gcc aat aat ttg gat acg ttg      960
Ser Asp Asp Val Arg Leu Arg Leu Asn Ala Asn Asn Leu Asp Thr Leu
305                 310                 315                 320

gaa aag gga tat ttg att ggg aat ggg atg aag ata agc gta gat gag      1008
Glu Lys Gly Tyr Leu Ile Gly Asn Gly Met Lys Ile Ser Val Asp Glu
                325                 330                 335

ttg ggg aaa aaa gtg tta gaa cac atc ggt aag aat gaa ccg tta ttg      1056
Leu Gly Lys Lys Val Leu Glu His Ile Gly Lys Asn Glu Pro Leu Leu
            340                 345                 350

ttg aaa aat cta ctg gtt aac ttc aat cag gca aaa cat gaa gaa gtt      1104
Leu Lys Asn Leu Leu Val Asn Phe Asn Gln Ala Lys His Glu Glu Val
        355                 360                 365

agg aag ttg atc tat cag ttg ata gag tta gat ttt ctg gaa att ttg      1152
Arg Lys Leu Ile Tyr Gln Leu Ile Glu Leu Asp Phe Leu Glu Ile Leu
    370                 375                 380

tga                                                                  1155


<210> 61
<211> 384
<212> PRT
<213> Neisseria gonorrhoeae

<400> 61
Met Gly Ile His Leu Asp Phe Gly Ile Ser Pro Lys Thr Phe Arg Gln
1               5                   10                  15

Thr Tyr Leu Tyr Gln Lys Pro Lys Leu Phe Lys Gly Ala Val Arg Asn
            20                  25                  30

Leu Glu Ala Ala Ser Cys Lys Tyr Ile Asn Glu Ile Tyr Gln Arg Ala
        35                  40                  45

Asp Pro Thr Ala Pro Leu Phe His Leu Arg Lys Lys Gly Ala Ile Val
    50                  55                  60

Pro Lys Glu Glu Tyr Val Glu Ser Phe Asp Asp Leu Gly Lys Thr Arg
65                  70                  75                  80

Tyr Arg Phe Ile Lys Ser Val Ile Tyr Glu His Met Lys Asn Gly Ala
                85                  90                  95

Ser Leu Val Tyr Asn His Ile Asn Asn Glu Pro Phe Ser Asp His Ile
            100                 105                 110

Ala Arg Gln Val Ala Arg Phe Ala Gly Ala His Thr Ile Val Ser Gly
        115                 120                 125

Tyr Leu Ala Phe Gly Ser Asp Glu Ser Tyr Lys Asn His Trp Asp Thr
    130                 135                 140

Arg Asp Val Tyr Ala Ile Gln Leu Phe Gly Lys Lys Arg Trp Gln Leu
145                 150                 155                 160

Thr Ala Pro Asp Phe Pro Met Pro Leu Tyr Met Gln Gln Thr Lys Asp
                165                 170                 175

Thr Asp Ile Ser Ile Pro Glu His Ile Asp Met Asp Ile Ile Leu Glu
            180                 185                 190

Ala Gly Asp Val Leu Tyr Ile Pro Arg Gly Trp Trp His Arg Pro Ile
        195                 200                 205

Pro Leu Gly Cys Glu Thr Phe His Phe Ala Val Gly Thr Phe Pro Pro
    210                 215                 220

Asn Gly Tyr Asn Tyr Leu Glu Trp Leu Met Lys Lys Phe Pro Thr Ile
225                 230                 235                 240

Glu Ser Leu Arg His Ser Phe Ser Asp Trp Glu Gln Asp Arg Thr Arg
                245                 250                 255

Ile Asn Asp Thr Ala Ala Gln Ile Ala Ala Met Ile Ala Asp Pro Val
            260                 265                 270

Asn Tyr Glu Ala Phe Ser Glu Asp Phe Leu Gly Lys Glu Arg Thr Asp
        275                 280                 285

Thr Ala Phe His Leu Glu Gln Phe Ala Asn Pro Asn Ala Thr Pro Leu
    290                 295                 300

Ser Asp Asp Val Arg Leu Arg Leu Asn Ala Asn Asn Leu Asp Thr Leu
305                 310                 315                 320

Glu Lys Gly Tyr Leu Ile Gly Asn Gly Met Lys Ile Ser Val Asp Glu
                325                 330                 335

Leu Gly Lys Lys Val Leu Glu His Ile Gly Lys Asn Glu Pro Leu Leu
            340                 345                 350

Leu Lys Asn Leu Leu Val Asn Phe Asn Gln Ala Lys His Glu Glu Val
        355                 360                 365

Arg Lys Leu Ile Tyr Gln Leu Ile Glu Leu Asp Phe Leu Glu Ile Leu
    370                 375                 380

<210> 62
<211> 717
<212> ADN
<213> Neisseria meningitidis

<220>

<221> CDS
<222> (1)..(714)

<400> 62
atg aat aga ccc aag caa ccc ttc ttc cgt ccc gaa gtc gcc gtt gcc     48
Met Asn Arg Pro Lys Gln Pro Phe Phe Arg Pro Glu Val Ala Val Ala
1               5                   10                  15

cgc caa acc agc ctg acg ggt aaa gtg att ctg aca cga ccg ttg tca     96
Arg Gln Thr Ser Leu Thr Gly Lys Val Ile Leu Thr Arg Pro Leu Ser
            20                  25                  30

ttt tcc cta tgg acg aca ttt gca tcg ata tct gcg tta ttg att atc     144
Phe Ser Leu Trp Thr Thr Phe Ala Ser Ile Ser Ala Leu Leu Ile Ile
        35                  40                  45

ctg ttt ttg ata ttt ggt aac tat acg cga aag aca aca gtg gag gga     192
Leu Phe Leu Ile Phe Gly Asn Tyr Thr Arg Lys Thr Thr Val Glu Gly
    50                  55                  60

caa att tta cct gca tcg ggc gta atc agg gtg tat gca ccg gat acg     240
Gln Ile Leu Pro Ala Ser Gly Val Ile Arg Val Tyr Ala Pro Asp Thr
65                  70                  75                  80

ggg aca att aca gcg aaa ttc gtg gaa gat gga gaa aag gtt aag gct     288
Gly Thr Ile Thr Ala Lys Phe Val Glu Asp Gly Glu Lys Val Lys Ala
                85                  90                  95

ggc gac aag cta ttt gcg ctt tcg acc tca cgt ttc ggc gca gga gat     336
Gly Asp Lys Leu Phe Ala Leu Ser Thr Ser Arg Phe Gly Ala Gly Asp
            100                 105                 110

agc gtg cag cag cag ttg aaa acg gag gca gtt ttg aag aaa acg ttg     384
Ser Val Gln Gln Gln Leu Lys Thr Glu Ala Val Leu Lys Lys Thr Leu
        115                 120                 125

gca gaa cag gaa ctg ggt cgt ctg aag ctg ata cac ggg aat gaa acg     432
Ala Glu Gln Glu Leu Gly Arg Leu Lys Leu Ile His Gly Asn Glu Thr
    130                 135                 140

cgc agc ctt aaa gca act gtc gaa cgt ttg gaa aac cag gaa ctc cat     480
Arg Ser Leu Lys Ala Thr Val Glu Arg Leu Glu Asn Gln Glu Leu His
145                 150                 155                 160

att tcg caa cag ata gac ggt cag aaa agg cgc att aga ctt gcg gaa     528
Ile Ser Gln Gln Ile Asp Gly Gln Lys Arg Arg Ile Arg Leu Ala Glu
                165                 170                 175

gaa atg ttg cag aaa tat cgt ttc cta tcc gcc aat gat gca gtg cca     576
Glu Met Leu Gln Lys Tyr Arg Phe Leu Ser Ala Asn Asp Ala Val Pro
            180                 185                 190

aaa caa gaa atg atg aat gtc aag gca gag ctt tta gag cag aaa gcc     624
Lys Gln Glu Met Met Asn Val Lys Ala Glu Leu Leu Glu Gln Lys Ala
        195                 200                 205

aaa ctt gat gcc tac cgc cga gaa gaa gtc ggg ctg ctt cag gaa atc     672
Lys Leu Asp Ala Tyr Arg Arg Glu Glu Val Gly Leu Leu Gln Glu Ile
    210                 215                 220

cgc acg cag aat ctg aca ttg gcc agc ctc ccc caa gcg gca tga 717
Arg Thr Gln Asn Leu Thr Leu Ala Ser Leu Pro Gln Ala Ala
225                 230                 235

<210> 63
<211> 238
<212> PRT
<213> Neisseria meningitidis

<400> 63
Met Asn Arg Pro Lys Gln Pro Phe Phe Arg Pro Glu Val Ala Val Ala
1               5                   10                  15

Arg Gln Thr Ser Leu Thr Gly Lys Val Ile Leu Thr Arg Pro Leu Ser
            20                  25                  30

Phe Ser Leu Trp Thr Thr Phe Ala Ser Ile Ser Ala Leu Leu Ile Ile
        35                  40                  45

Leu Phe Leu Ile Phe Gly Asn Tyr Thr Arg Lys Thr Thr Val Glu Gly
    50                  55                  60

Gln Ile Leu Pro Ala Ser Gly Val Ile Arg Val Tyr Ala Pro Asp Thr
65                  70                  75                  80

Gly Thr Ile Thr Ala Lys Phe Val Glu Asp Gly Glu Lys Val Lys Ala
                85                  90                  95

Gly Asp Lys Leu Phe Ala Leu Ser Thr Ser Arg Phe Gly Ala Gly Asp
            100                 105                 110

Ser Val Gln Gln Gln Leu Lys Thr Glu Ala Val Leu Lys Lys Thr Leu
        115                 120                 125

Ala Glu Gln Glu Leu Gly Arg Leu Lys Leu Ile His Gly Asn Glu Thr
    130                 135                 140

Arg Ser Leu Lys Ala Thr Val Glu Arg Leu Glu Asn Gln Glu Leu His
145                 150                 155                 160

Ile Ser Gln Gln Ile Asp Gly Gln Lys Arg Arg Ile Arg Leu Ala Glu
                165                 170                 175

Glu Met Leu Gln Lys Tyr Arg Phe Leu Ser Ala Asn Asp Ala Val Pro
            180                 185                 190

Lys Gln Glu Met Met Asn Val Lys Ala Glu Leu Leu Glu Gln Lys Ala
        195                 200                 205

Lys Leu Asp Ala Tyr Arg Arg Glu Glu Val Gly Leu Leu Gln Glu Ile
    210                 215                 220

Arg Thr Gln Asn Leu Thr Leu Ala Ser Leu Pro Gln Ala Ala
225                 230                 235

<210> 64
<211> 690

<212> ADN
<213> Neisseria gonorrhoeae

<220>
<221> CDS
<222> (1)..(687)

<400> 64
atg atg aat gtc gag gca gag ctt tta gag cag aaa gcc aaa ctt gat 48
Met Met Asn Val Glu Ala Glu Leu Leu Glu Gln Lys Ala Lys Leu Asp
1               5                   10                  15

gcc tac ggc cga gaa gaa gcc ggg ctg ctt cag gaa atc cgc acg cag 96
Ala Tyr Gly Arg Glu Glu Ala Gly Leu Leu Gln Glu Ile Arg Thr Gln
            20                  25                  30

aat ctg aca ttg gcc agc ctc ccc aaa cgg cat gag aca gaa caa agc 144
Asn Leu Thr Leu Ala Ser Leu Pro Lys Arg His Glu Thr Glu Gln Ser
        35                  40                  45

cag ctt gaa cgc acc atg gcc gat att tct caa gaa gtt ttg gat ttt 192
Gln Leu Glu Arg Thr Met Ala Asp Ile Ser Gln Glu Val Leu Asp Phe
    50                  55                  60

gaa atg cgc tct gaa caa atc atc cgt gca gga cgg tcg ggt tat ata 240
Glu Met Arg Ser Glu Gln Ile Ile Arg Ala Gly Arg Ser Gly Tyr Ile
65                  70                  75                  80

gca ata ccg aac gtc gaa gtc gga cgg cag gtt gat cct tcc aaa ctg 288
Ala Ile Pro Asn Val Glu Val Gly Arg Gln Val Asp Pro Ser Lys Leu
                85                  90                  95

ctc ttg agc att gtt ccc gaa cgt acc gag tta tat gcc cat cta tat 336
Leu Leu Ser Ile Val Pro Glu Arg Thr Glu Leu Tyr Ala His Leu Tyr
            100                 105                 110

atc ccc agc agt gca gca ggc ttt atc aag ccg aaa gac aag gtt gtc 384
Ile Pro Ser Ser Ala Ala Gly Phe Ile Lys Pro Lys Asp Lys Val Val
        115                 120                 125

cta cgt tat cag gca tat ccc tat cag aaa ttc ggg ctt gct tcc ggc 432
Leu Arg Tyr Gln Ala Tyr Pro Tyr Gln Lys Phe Gly Leu Ala Ser Gly
    130                 135                 140

agt gtc gta tca gtg gca aaa acg gca ctg ggc aga cag gaa ttg tcg 480
Ser Val Val Ser Val Ala Lys Thr Ala Leu Gly Arg Gln Glu Leu Ser
145                 150                 155                 160

gga ttg ggc atg gta tcc tcc gat ttg gcg aag agc aac gaa cct gtt 528
Gly Leu Gly Met Val Ser Ser Asp Leu Ala Lys Ser Asn Glu Pro Val
                165                 170                 175

tat ctc gtg aaa ata aaa ccc gac aaa cca acc atc act gca tac ggt 576
Tyr Leu Val Lys Ile Lys Pro Asp Lys Pro Thr Ile Thr Ala Tyr Gly
            180                 185                 190

gag gaa aaa ccg ctg caa atc ggc atg acg ctg gaa gca gac atc cta 624
Glu Glu Lys Pro Leu Gln Ile Gly Met Thr Leu Glu Ala Asp Ile Leu
        195                 200                 205

cac gag aaa cgg cgg ctg tac gaa tgg gta ttg gag ccg att tac agt     672
His Glu Lys Arg Arg Leu Tyr Glu Trp Val Leu Glu Pro Ile Tyr Ser
    210                 215                 220

atg tcg ggc agg ttg taa                                             690
Met Ser Gly Arg Leu
225

<210> 65
<211> 229
<212> PRT
<213> Neisseria gonorrhoeae

<400> 65
Met Met Asn Val Glu Ala Glu Leu Leu Glu Gln Lys Ala Lys Leu Asp
  1               5                  10                  15

Ala Tyr Gly Arg Glu Glu Ala Gly Leu Leu Gln Glu Ile Arg Thr Gln
             20                  25                  30

Asn Leu Thr Leu Ala Ser Leu Pro Lys Arg His Glu Thr Glu Gln Ser
         35                  40                  45

Gln Leu Glu Arg Thr Met Ala Asp Ile Ser Gln Glu Val Leu Asp Phe
     50                  55                  60

Glu Met Arg Ser Glu Gln Ile Ile Arg Ala Gly Arg Ser Gly Tyr Ile
 65                  70                  75                  80

Ala Ile Pro Asn Val Glu Val Gly Arg Gln Val Asp Pro Ser Lys Leu
                 85                  90                  95

Leu Leu Ser Ile Val Pro Glu Arg Thr Glu Leu Tyr Ala His Leu Tyr
            100                 105                 110

Ile Pro Ser Ser Ala Ala Gly Phe Ile Lys Pro Lys Asp Lys Val Val
        115                 120                 125

Leu Arg Tyr Gln Ala Tyr Pro Tyr Gln Lys Phe Gly Leu Ala Ser Gly
    130                 135                 140

Ser Val Val Ser Val Ala Lys Thr Ala Leu Gly Arg Gln Glu Leu Ser
145                 150                 155                 160

Gly Leu Gly Met Val Ser Ser Asp Leu Ala Lys Ser Asn Glu Pro Val
                165                 170                 175

Tyr Leu Val Lys Ile Lys Pro Asp Lys Pro Thr Ile Thr Ala Tyr Gly
            180                 185                 190

Glu Glu Lys Pro Leu Gln Ile Gly Met Thr Leu Glu Ala Asp Ile Leu
        195                 200                 205

His Glu Lys Arg Arg Leu Tyr Glu Trp Val Leu Glu Pro Ile Tyr Ser
    210                 215                 220

Met Ser Gly Arg Leu
225

<210> 66
<211> 924
<212> ADN
<213> Neisseria gonorrhoeae

<220>
<221> CDS
<222> (1)..(921)

<400> 66
atg caa tac agc aca ctg gca gga caa acc gac aac tcc ctc gtt tcc     48
Met Gln Tyr Ser Thr Leu Ala Gly Gln Thr Asp Asn Ser Leu Val Ser
1               5                   10                  15

aat aat ttc ggg ttt ttg cgc ctg ccg ctt aat ttt atg ccg tat gaa     96
Asn Asn Phe Gly Phe Leu Arg Leu Pro Leu Asn Phe Met Pro Tyr Glu
            20                  25                  30

agc cat gcc gat tgg gtt att acc ggc gtg cct tat gat atg gcg gtt     144
Ser His Ala Asp Trp Val Ile Thr Gly Val Pro Tyr Asp Met Ala Val
        35                  40                  45

tca ggg cgt tcc ggc gcg cgt ttc ggt cct gaa gcc atc cgg cgc gcc     192
Ser Gly Arg Ser Gly Ala Arg Phe Gly Pro Glu Ala Ile Arg Arg Ala
    50                  55                  60

tcc gtc aac ctc gct tgg gag cac cgc agg ttt ccg tgg aca ttt gat     240
Ser Val Asn Leu Ala Trp Glu His Arg Arg Phe Pro Trp Thr Phe Asp
65                  70                  75                  80

gtg cgc gaa cgc ctg aac att att gat tgc ggc gac ttg gtt ttt tct     288
Val Arg Glu Arg Leu Asn Ile Ile Asp Cys Gly Asp Leu Val Phe Ser
                85                  90                  95

ttt ggc gac agc agg gat ttt gtc gaa aaa atg gaa gcg cac gcc ggc     336
Phe Gly Asp Ser Arg Asp Phe Val Glu Lys Met Glu Ala His Ala Gly
            100                 105                 110

aaa tta ctt tct ttc ggc aaa cgc tgt ttg agt ttg ggc ggc gac cat     384
Lys Leu Leu Ser Phe Gly Lys Arg Cys Leu Ser Leu Gly Gly Asp His
        115                 120                 125

ttc att acc ctc ccg ttg ttg cgc gcc cac gcc cgc tat ttc ggc aaa     432
Phe Ile Thr Leu Pro Leu Leu Arg Ala His Ala Arg Tyr Phe Gly Lys
    130                 135                 140

ctc gca ctg att cat ttt gac gcg cac acc gac acc tac gac aac ggc     480
Leu Ala Leu Ile His Phe Asp Ala His Thr Asp Thr Tyr Asp Asn Gly
145                 150                 155                 160

agc gaa tac gac cac ggc acg atg ttt tat acc gcc ccc aag gaa ggc     528
Ser Glu Tyr Asp His Gly Thr Met Phe Tyr Thr Ala Pro Lys Glu Gly
                165                 170                 175

ctc atc gac ccg tcc cgt tcc gta caa atc ggc ata cgc acc gaa cac     576
Leu Ile Asp Pro Ser Arg Ser Val Gln Ile Gly Ile Arg Thr Glu His
            180                 185                 190

agt aaa aaa ttg cct ttt act gtg ttg tcc gcc ccc aaa gtc aat gaa     624
Ser Lys Lys Leu Pro Phe Thr Val Leu Ser Ala Pro Lys Val Asn Glu
        195                 200                 205

gac agt gtt gaa gag acc gtc cgt aaa atc aaa gaa acc gtc ggc aat     672
Asp Ser Val Glu Glu Thr Val Arg Lys Ile Lys Glu Thr Val Gly Asn
    210                 215                 220

atg ccc gtt tac ctg act ttc gac ata gac tgt ctc gac ccg tcg ttc     720
Met Pro Val Tyr Leu Thr Phe Asp Ile Asp Cys Leu Asp Pro Ser Phe
225                 230                 235                 240

gcc ccc ggg acc ggt acg ccc gta tgc ggc ggc ttg agc agc gac agg     768
Ala Pro Gly Thr Gly Thr Pro Val Cys Gly Gly Leu Ser Ser Asp Arg
                245                 250                 255

gca tta aaa atc cta cgt ggg ctg acg gat ctc gac atc gtc ggt atg     816
Ala Leu Lys Ile Leu Arg Gly Leu Thr Asp Leu Asp Ile Val Gly Met
            260                 265                 270

gat gtt gta gaa gtt gcc ccc tct tac gac caa tcc gac att acc gct     864
Asp Val Val Glu Val Ala Pro Ser Tyr Asp Gln Ser Asp Ile Thr Ala
        275                 280                 285

ttg gcc ggc gcc aca att gcc ttg gaa atg ctt tac ctt caa ggt gcg     912
Leu Ala Gly Ala Thr Ile Ala Leu Glu Met Leu Tyr Leu Gln Gly Ala
    290                 295                 300

aaa aag gac tga                                                     924
Lys Lys Asp
305

<210> 67
<211> 307
<212> PRT
<213> Neisseria gonorrhoeae

<400> 67
Met Gln Tyr Ser Thr Leu Ala Gly Gln Thr Asp Asn Ser Leu Val Ser
1               5                   10                  15

Asn Asn Phe Gly Phe Leu Arg Leu Pro Leu Asn Phe Met Pro Tyr Glu
            20                  25                  30

Ser His Ala Asp Trp Val Ile Thr Gly Val Pro Tyr Asp Met Ala Val
        35                  40                  45

Ser Gly Arg Ser Gly Ala Arg Phe Gly Pro Glu Ala Ile Arg Arg Ala
    50                  55                  60

Ser Val Asn Leu Ala Trp Glu His Arg Arg Phe Pro Trp Thr Phe Asp
65                  70                  75                  80

Val Arg Glu Arg Leu Asn Ile Ile Asp Cys Gly Asp Leu Val Phe Ser
                85                  90                  95

Phe Gly Asp Ser Arg Asp Phe Val Glu Lys Met Glu Ala His Ala Gly
            100                 105                 110

Lys Leu Leu Ser Phe Gly Lys Arg Cys Leu Ser Leu Gly Gly Asp His
        115                 120                 125

Phe Ile Thr Leu Pro Leu Leu Arg Ala His Ala Arg Tyr Phe Gly Lys
    130                 135                 140

Leu Ala Leu Ile His Phe Asp Ala His Thr Asp Thr Tyr Asp Asn Gly
145                 150                 155                 160

Ser Glu Tyr Asp His Gly Thr Met Phe Tyr Thr Ala Pro Lys Glu Gly
                165                 170                 175

Leu Ile Asp Pro Ser Arg Ser Val Gln Ile Gly Ile Arg Thr Glu His
            180                 185                 190

Ser Lys Lys Leu Pro Phe Thr Val Leu Ser Ala Pro Lys Val Asn Glu
        195                 200                 205

Asp Ser Val Glu Glu Thr Val Arg Lys Ile Lys Glu Thr Val Gly Asn
    210                 215                 220

Met Pro Val Tyr Leu Thr Phe Asp Ile Asp Cys Leu Asp Pro Ser Phe
225                 230                 235                 240

Ala Pro Gly Thr Gly Thr Pro Val Cys Gly Gly Leu Ser Ser Asp Arg
                245                 250                 255

Ala Leu Lys Ile Leu Arg Gly Leu Thr Asp Leu Asp Ile Val Gly Met
            260                 265                 270

Asp Val Val Glu Val Ala Pro Ser Tyr Asp Gln Ser Asp Ile Thr Ala
        275                 280                 285

Leu Ala Gly Ala Thr Ile Ala Leu Glu Met Leu Tyr Leu Gln Gly Ala
    290                 295                 300

Lys Lys Asp
305

<210> 68
<211> 1404
<212> ADN
<213> Neisseria meningitidis

<220>
<221> CDS
<222> (1)..(1401)

<400> 68
atg aca ttg ctc aat cta atg ata atg caa gat tac ggt att tcc gtt 48
Met Thr Leu Leu Asn Leu Met Ile Met Gln Asp Tyr Gly Ile Ser Val
1               5                   10                  15

tgc ctg aca ctg acg ccc tat ttg caa cat gaa cta ttt tcg gct atg 96
Cys Leu Thr Leu Thr Pro Tyr Leu Gln His Glu Leu Phe Ser Ala Met
            20                  25                  30

aaa tcc tat ttt tcc aaa tat atc cta ccc gtt tca ctt ttt acc ttg 144

Lys Ser Tyr Phe Ser Lys Tyr Ile Leu Pro Val Ser Leu Phe Thr Leu
        35                  40                  45

cca cta tcc ctt tcc cca tcc gtt tcg gct ttt acg ctg cct gaa gca 192
Pro Leu Ser Leu Ser Pro Ser Val Ser Ala Phe Thr Leu Pro Glu Ala
    50                  55                  60

tgg cgg gcg gcg cag caa cat tcg gct gat ttt caa gcg tcc cat tac 240
Trp Arg Ala Ala Gln Gln His Ser Ala Asp Phe Gln Ala Ser His Tyr
65                  70                  75                  80

cag cgt gat gca gtg cgc gca cgg caa caa caa gcc aag gcc gca ttc 288
Gln Arg Asp Ala Val Arg Ala Arg Gln Gln Gln Ala Lys Ala Ala Phe
                85                  90                  95

ctt ccc cat gta tcc gcc aat gcc agc tac cag cgc cag ccg cca tcg 336
Leu Pro His Val Ser Ala Asn Ala Ser Tyr Gln Arg Gln Pro Pro Ser
            100                 105                 110

att tct tcc acc cgc gaa aca cag gga tgg agc gtg cag gtg gga caa 384
Ile Ser Ser Thr Arg Glu Thr Gln Gly Trp Ser Val Gln Val Gly Gln
        115                 120                 125

acc tta ttt gac gct gcc aaa ttt gca caa tac cgc caa agc agg ttc 432
Thr Leu Phe Asp Ala Ala Lys Phe Ala Gln Tyr Arg Gln Ser Arg Phe
    130                 135                 140

gat acg cag gct gca gaa cag cgt ttc gat gcg gca cgc gaa gaa ttg 480
Asp Thr Gln Ala Ala Glu Gln Arg Phe Asp Ala Ala Arg Glu Glu Leu
145                 150                 155                 160

ctg ttg aaa gtt gcc gaa agt tat ttc aac gtt tta ctc agc cga gac 528
Leu Leu Lys Val Ala Glu Ser Tyr Phe Asn Val Leu Leu Ser Arg Asp
                165                 170                 175

acc gtt gcc gcc cat gcg gcg gaa aaa gag gct tat gcc cag cag gta 576
Thr Val Ala Ala His Ala Ala Glu Lys Glu Ala Tyr Ala Gln Gln Val
            180                 185                 190

agg cag gcg cag gct tta ttc aat aaa ggt gct gcc acc gcg ctg gat 624
Arg Gln Ala Gln Ala Leu Phe Asn Lys Gly Ala Ala Thr Ala Leu Asp
        195                 200                 205

att cac gaa gcc aaa gcc ggt tac gac aat gcc ctg gcc caa gaa atc 672
Ile His Glu Ala Lys Ala Gly Tyr Asp Asn Ala Leu Ala Gln Glu Ile
    210                 215                 220

gcc gta ttg gct gag aaa caa acc tat gaa aac cag ttg aac gac tac 720
Ala Val Leu Ala Glu Lys Gln Thr Tyr Glu Asn Gln Leu Asn Asp Tyr
225                 230                 235                 240

acc gac ctg gat agc aaa caa atc gag gcc ata gat acc gcc aac ctg 768
Thr Asp Leu Asp Ser Lys Gln Ile Glu Ala Ile Asp Thr Ala Asn Leu
                245                 250                 255

ttg gca cgc tat ctg ccc aag ctg gaa cgt tac agt ctg gat gaa tgg 816
Leu Ala Arg Tyr Leu Pro Lys Leu Glu Arg Tyr Ser Leu Asp Glu Trp
            260                 265                 270

cag cgc att gcc tta tcc aac aat cat gaa tac cgg atg cag cag ctt 864

Gln Arg Ile Ala Leu Ser Asn Asn His Glu Tyr Arg Met Gln Gln Leu
        275                 280                 285

gcc ctg caa agc agc gga cag gcg ctt cgg gca gca cag aac agc cgc     912
Ala Leu Gln Ser Ser Gly Gln Ala Leu Arg Ala Ala Gln Asn Ser Arg
    290                 295                 300

tat ccc acc gtt tct gcc cat gtc ggc tat cag aat aac ctc tac act     960
Tyr Pro Thr Val Ser Ala His Val Gly Tyr Gln Asn Asn Leu Tyr Thr
305                 310                 315                 320

tca tct gcg cag aat aat gac tac cac tat cgg ggc aaa ggg atg agc     1008
Ser Ser Ala Gln Asn Asn Asp Tyr His Tyr Arg Gly Lys Gly Met Ser
                325                 330                 335

gtc ggc gta cag ttg aat ttg ccg ctt tat acc ggc gga gaa ttg tcg     1056
Val Gly Val Gln Leu Asn Leu Pro Leu Tyr Thr Gly Gly Glu Leu Ser
            340                 345                 350

ggc aaa atc cat gaa gcc gaa gcg caa tac ggg gcc gcc gaa gca cag     1104
Gly Lys Ile His Glu Ala Glu Ala Gln Tyr Gly Ala Ala Glu Ala Gln
        355                 360                 365

ctg acc gca acc gag cgg cac atc aaa ctc gcc gta cgc cag gct tat     1152
Leu Thr Ala Thr Glu Arg His Ile Lys Leu Ala Val Arg Gln Ala Tyr
    370                 375                 380

acc gaa agc ggt gcg gcg cgt tac caa atc atg gcg caa gaa cgg gtt     1200
Thr Glu Ser Gly Ala Ala Arg Tyr Gln Ile Met Ala Gln Glu Arg Val
385                 390                 395                 400

ttg gaa agc agc cgt ttg aaa ctg aaa tcg acc gaa acc ggc caa caa     1248
Leu Glu Ser Ser Arg Leu Lys Leu Lys Ser Thr Glu Thr Gly Gln Gln
                405                 410                 415

tac ggc atc cgc aac cgg ctg gaa gta ata cgg gcg cgg cag gaa gtc     1296
Tyr Gly Ile Arg Asn Arg Leu Glu Val Ile Arg Ala Arg Gln Glu Val
            420                 425                 430

gcc caa gca gaa cag aaa ctg gct caa gca cgg tat aaa ttc atg ctg     1344
Ala Gln Ala Glu Gln Lys Leu Ala Gln Ala Arg Tyr Lys Phe Met Leu
        435                 440                 445

gct tat ttg cgc ttg gtg aaa gag agc ggg tta ggg ttg gaa acg gta     1392
Ala Tyr Leu Arg Leu Val Lys Glu Ser Gly Leu Gly Leu Glu Thr Val
    450                 455                 460

ttt gcg gaa taa                                                     1404
Phe Ala Glu
465

<210> 69
<211> 467
<212> PRT
<213> Neisseria meningitidis

<400> 69
Met Thr Leu Leu Asn Leu Met Ile Met Gln Asp Tyr Gly Ile Ser Val
1               5                   10                  15

Cys Leu Thr Leu Thr Pro Tyr Leu Gln His Glu Leu Phe Ser Ala Met
            20                  25                  30

Lys Ser Tyr Phe Ser Lys Tyr Ile Leu Pro Val Ser Leu Phe Thr Leu
        35                  40                  45

Pro Leu Ser Leu Ser Pro Ser Val Ser Ala Phe Thr Leu Pro Glu Ala
    50                  55                  60

Trp Arg Ala Ala Gln Gln His Ser Ala Asp Phe Gln Ala Ser His Tyr
65                  70                  75                  80

Gln Arg Asp Ala Val Arg Ala Arg Gln Gln Gln Ala Lys Ala Ala Phe
                85                  90                  95

Leu Pro His Val Ser Ala Asn Ala Ser Tyr Gln Arg Gln Pro Pro Ser
            100                 105                 110

Ile Ser Ser Thr Arg Glu Thr Gln Gly Trp Ser Val Gln Val Gly Gln
        115                 120                 125

Thr Leu Phe Asp Ala Ala Lys Phe Ala Gln Tyr Arg Gln Ser Arg Phe
    130                 135                 140

Asp Thr Gln Ala Ala Glu Gln Arg Phe Asp Ala Ala Arg Glu Glu Leu
145                 150                 155                 160

Leu Leu Lys Val Ala Glu Ser Tyr Phe Asn Val Leu Leu Ser Arg Asp
                165                 170                 175

Thr Val Ala Ala His Ala Ala Glu Lys Glu Ala Tyr Ala Gln Gln Val
            180                 185                 190

Arg Gln Ala Gln Ala Leu Phe Asn Lys Gly Ala Ala Thr Ala Leu Asp
        195                 200                 205

Ile His Glu Ala Lys Ala Gly Tyr Asp Asn Ala Leu Ala Gln Glu Ile
    210                 215                 220

Ala Val Leu Ala Glu Lys Gln Thr Tyr Glu Asn Gln Leu Asn Asp Tyr
225                 230                 235                 240

Thr Asp Leu Asp Ser Lys Gln Ile Glu Ala Ile Asp Thr Ala Asn Leu
                245                 250                 255

Leu Ala Arg Tyr Leu Pro Lys Leu Glu Arg Tyr Ser Leu Asp Glu Trp
            260                 265                 270

Gln Arg Ile Ala Leu Ser Asn Asn His Glu Tyr Arg Met Gln Gln Leu
        275                 280                 285

Ala Leu Gln Ser Ser Gly Gln Ala Leu Arg Ala Ala Gln Asn Ser Arg
    290                 295                 300

Tyr Pro Thr Val Ser Ala His Val Gly Tyr Gln Asn Asn Leu Tyr Thr
305                 310                 315                 320

Ser Ser Ala Gln Asn Asn Asp Tyr His Tyr Arg Gly Lys Gly Met Ser
                325                 330                 335

Val Gly Val Gln Leu Asn Leu Pro Leu Tyr Thr Gly Gly Glu Leu Ser
            340                 345                 350

Gly Lys Ile His Glu Ala Glu Ala Gln Tyr Gly Ala Ala Glu Ala Gln
        355                 360                 365

Leu Thr Ala Thr Glu Arg His Ile Lys Leu Ala Val Arg Gln Ala Tyr
    370                 375                 380

Thr Glu Ser Gly Ala Ala Arg Tyr Gln Ile Met Ala Gln Glu Arg Val
385                 390                 395                 400

Leu Glu Ser Ser Arg Leu Lys Leu Lys Ser Thr Glu Thr Gly Gln Gln
                405                 410                 415

Tyr Gly Ile Arg Asn Arg Leu Glu Val Ile Arg Ala Arg Gln Glu Val
            420                 425                 430

Ala Gln Ala Glu Gln Lys Leu Ala Gln Ala Arg Tyr Lys Phe Met Leu
        435                 440                 445

Ala Tyr Leu Arg Leu Val Lys Glu Ser Gly Leu Gly Leu Glu Thr Val
    450                 455                 460

Phe Ala Glu
465

<210> 70
<211> 696
<212> ADN
<213> Neisseria gonorrhoeae

<220>
<221> CDS
<222> (1)..(693)

<400> 70
atg aaa caa tcc gcc cga ata aaa aat atg gat cag aca tta aaa aat 48
Met Lys Gln Ser Ala Arg Ile Lys Asn Met Asp Gln Thr Leu Lys Asn
1               5                   10                  15

aca ttg ggc att tgc gcg ctt tta gcc ttt tgt ttt ggc gcg gcc atc 96
Thr Leu Gly Ile Cys Ala Leu Leu Ala Phe Cys Phe Gly Ala Ala Ile
            20                  25                  30

gca tca ggt tat cac ttg gaa tat gaa tac ggc tac cgt tat tct gcc 144
Ala Ser Gly Tyr His Leu Glu Tyr Glu Tyr Gly Tyr Arg Tyr Ser Ala
        35                  40                  45

gtg ggc gct ttg gct tcg gtt gta ttt tta tta tta ttg gca cgc ggc 192
Val Gly Ala Leu Ala Ser Val Val Phe Leu Leu Leu Leu Ala Arg Gly
    50                  55                  60

ttc ccg cgc gtt tct tca gtt gtt tta ctg att tac gtc ggc aca acc 240
Phe Pro Arg Val Ser Ser Val Val Leu Leu Ile Tyr Val Gly Thr Thr
65                  70                  75                  80

gcc cta tat ttg ccg gtc ggc tgg ctg tat ggt gcg cct tct tat cag     288
Ala Leu Tyr Leu Pro Val Gly Trp Leu Tyr Gly Ala Pro Ser Tyr Gln
                85                  90                  95

ata gtc ggt tcg ata ttg gaa agc aat cct gcc gag gcg cgt gaa ttt     336
Ile Val Gly Ser Ile Leu Glu Ser Asn Pro Ala Glu Ala Arg Glu Phe
            100                 105                 110

gtc ggc aat ctt ccc ggg tcg ctt tat ttt gtg cag gca tta ttt ttc     384
Val Gly Asn Leu Pro Gly Ser Leu Tyr Phe Val Gln Ala Leu Phe Phe
        115                 120                 125

att ttt ggc ttg aca gtt tgg aaa tat tgt gta tct gtg ggg gta ttt     432
Ile Phe Gly Leu Thr Val Trp Lys Tyr Cys Val Ser Val Gly Val Phe
    130                 135                 140

gct gac gta aaa aac tat aaa cgt cgc agc aaa ata tgg ctg acc ata     480
Ala Asp Val Lys Asn Tyr Lys Arg Arg Ser Lys Ile Trp Leu Thr Ile
145                 150                 155                 160

tta ttg act ttg att ttg tcc tgc gcg gtg atg gag aaa atc gcc ggc     528
Leu Leu Thr Leu Ile Leu Ser Cys Ala Val Met Glu Lys Ile Ala Gly
                165                 170                 175

gat aaa gat tgg cga gaa cct gat gcc ggc ctg ttg ttg aat att ttc     576
Asp Lys Asp Trp Arg Glu Pro Asp Ala Gly Leu Leu Leu Asn Ile Phe
            180                 185                 190

gac ctg tat tac gac ttg gct ttc cgc gcc ggc aca ata tgc cgc caa     624
Asp Leu Tyr Tyr Asp Leu Ala Phe Arg Ala Gly Thr Ile Cys Arg Gln
        195                 200                 205

gcg cgc cca cat ttt gga agc agc aaa aaa agc gtc aac atg gca tat     672
Ala Arg Pro His Phe Gly Ser Ser Lys Lys Ser Val Asn Met Ala Tyr
    210                 215                 220

ccg cca act tgc gcc caa gta taa                                     696
Pro Pro Thr Cys Ala Gln Val
225                 230


<210> 71
<211> 231
<212> PRT
<213> Neisseria gonorrhoeae

<400> 71
Met Lys Gln Ser Ala Arg Ile Lys Asn Met Asp Gln Thr Leu Lys Asn
1               5                   10                  15

Thr Leu Gly Ile Cys Ala Leu Leu Ala Phe Cys Phe Gly Ala Ala Ile
            20                  25                  30

Ala Ser Gly Tyr His Leu Glu Tyr Glu Tyr Gly Tyr Arg Tyr Ser Ala
        35                  40                  45

Val Gly Ala Leu Ala Ser Val Val Phe Leu Leu Leu Leu Ala Arg Gly
    50                  55                  60

Phe Pro Arg Val Ser Ser Val Val Leu Leu Ile Tyr Val Gly Thr Thr

65                  70                  75                  80

Ala Leu Tyr Leu Pro Val Gly Trp Leu Tyr Gly Ala Pro Ser Tyr Gln
                85                  90                  95

Ile Val Gly Ser Ile Leu Glu Ser Asn Pro Ala Glu Ala Arg Glu Phe
            100                 105                 110

Val Gly Asn Leu Pro Gly Ser Leu Tyr Phe Val Gln Ala Leu Phe Phe
        115                 120                 125

Ile Phe Gly Leu Thr Val Trp Lys Tyr Cys Val Ser Val Gly Val Phe
    130                 135                 140

Ala Asp Val Lys Asn Tyr Lys Arg Arg Ser Lys Ile Trp Leu Thr Ile
145                 150                 155                 160

Leu Leu Thr Leu Ile Leu Ser Cys Ala Val Met Glu Lys Ile Ala Gly
                165                 170                 175

Asp Lys Asp Trp Arg Glu Pro Asp Ala Gly Leu Leu Leu Asn Ile Phe
            180                 185                 190

Asp Leu Tyr Tyr Asp Leu Ala Phe Arg Ala Gly Thr Ile Cys Arg Gln
        195                 200                 205

Ala Arg Pro His Phe Gly Ser Ser Lys Lys Ser Val Asn Met Ala Tyr
    210                 215                 220

Pro Pro Thr Cys Ala Gln Val
225                 230

<210> 72
<211> 2607
<212> ADN
<213> Neisseria meningitidis

<220>
<221> CDS
<222> (1)..(2604)

<400> 72
atg gct gcc aac caa cgt tac cgc aaa ccg ctg ccc ggt acg gat ttg      48
Met Ala Ala Asn Gln Arg Tyr Arg Lys Pro Leu Pro Gly Thr Asp Leu
  1               5                  10                  15

gaa tac tac gac gcg cgt gcg gcg tgt gag ggc atc aaa ccc ggc tct      96
Glu Tyr Tyr Asp Ala Arg Ala Ala Cys Glu Gly Ile Lys Pro Gly Ser
             20                  25                  30

tac gac aag ctg cct tac acg agc cgc att ttg gcg gag aat ttg gtc     144
Tyr Asp Lys Leu Pro Tyr Thr Ser Arg Ile Leu Ala Glu Asn Leu Val
         35                  40                  45

aac cgc gcg gac aaa gtc gat ttg ccg acg ctg caa agc tgg ctg ggt     192
Asn Arg Ala Asp Lys Val Asp Leu Pro Thr Leu Gln Ser Trp Leu Gly
     50                  55                  60

cag ctg att gag gga aaa cag gaa atc gac ttt cct tgg tat ccg gcg 240
Gln Leu Ile Glu Gly Lys Gln Glu Ile Asp Phe Pro Trp Tyr Pro Ala
65                  70                  75                  80

cgg gtg gtg tgc cac gat att ctg ggg cag acc gcg ttg gtg gat ttg 288
Arg Val Val Cys His Asp Ile Leu Gly Gln Thr Ala Leu Val Asp Leu
                85                  90                  95

gca ggt ctg cgc gat gcg att gcc gaa aaa ggc ggc gat cct gcc aaa 336
Ala Gly Leu Arg Asp Ala Ile Ala Glu Lys Gly Gly Asp Pro Ala Lys
            100                 105                 110

gtg aat ccg gtg gtg caa acc cag ctc atc gtc gac cac tcg ctg gcg 384
Val Asn Pro Val Val Gln Thr Gln Leu Ile Val Asp His Ser Leu Ala
        115                 120                 125

gtg gaa tgc ggc ggc tac gac ccc gat gcg ttc cgc aaa aac cgc gaa 432
Val Glu Cys Gly Gly Tyr Asp Pro Asp Ala Phe Arg Lys Asn Arg Glu
    130                 135                 140

atc gaa gac aga cgt aac gaa gac cgt ttc cac ttc atc aac tgg aca 480
Ile Glu Asp Arg Arg Asn Glu Asp Arg Phe His Phe Ile Asn Trp Thr
145                 150                 155                 160

aaa acc gct ttt gaa aat gtg gac gtg att ccg gcg ggc aac ggc atc 528
Lys Thr Ala Phe Glu Asn Val Asp Val Ile Pro Ala Gly Asn Gly Ile
                165                 170                 175

atg cac caa atc aat cta gaa aaa atg tcg ccc gtc gtc caa gtc aaa 576
Met His Gln Ile Asn Leu Glu Lys Met Ser Pro Val Val Gln Val Lys
            180                 185                 190

aac ggc gtg gct ttc ccc gat acc tgc gtc ggc acg gat tcg cac acg 624
Asn Gly Val Ala Phe Pro Asp Thr Cys Val Gly Thr Asp Ser His Thr
        195                 200                 205

cca cac gtc gat gcg ctg ggc gtg att tcc gtg ggc gtg ggc gga ttg 672
Pro His Val Asp Ala Leu Gly Val Ile Ser Val Gly Val Gly Gly Leu
    210                 215                 220

gaa gcg gaa acc gta atg ctg gga cgc gcg tcc atg atg cgc ctg ccc 720
Glu Ala Glu Thr Val Met Leu Gly Arg Ala Ser Met Met Arg Leu Pro
225                 230                 235                 240

gat att gtc ggc gtt gag ctg aac ggc aaa cgg aag gcg ggc att acg 768
Asp Ile Val Gly Val Glu Leu Asn Gly Lys Arg Lys Ala Gly Ile Thr
                245                 250                 255

gcg acg gat att gtg ttg gca ctg acc gag ttt ctg cgc aaa gaa cgc 816
Ala Thr Asp Ile Val Leu Ala Leu Thr Glu Phe Leu Arg Lys Glu Arg
            260                 265                 270

gtg gtc ggg gcg ttt gtc gaa ttc ttc ggc gag ggc gcg aga agc ctg 864
Val Val Gly Ala Phe Val Glu Phe Phe Gly Glu Gly Ala Arg Ser Leu
        275                 280                 285

tct atc ggc gac cgc gcg acc att tcc aac atg acg ccg gag ttc ggc 912
Ser Ile Gly Asp Arg Ala Thr Ile Ser Asn Met Thr Pro Glu Phe Gly
    290                 295                 300

gcg act gcc gcg atg ttc gct att gat gag caa acc att gat tat ttg    960
Ala Thr Ala Ala Met Phe Ala Ile Asp Glu Gln Thr Ile Asp Tyr Leu
305                 310                 315                 320

aaa ctg acc gga cgc gac gac gcg cag gtg aaa ttg gtg gaa acc tac    1008
Lys Leu Thr Gly Arg Asp Asp Ala Gln Val Lys Leu Val Glu Thr Tyr
                325                 330                 335

gcc aaa acc gca ggc ttg tgg gca gat gcc ttg aaa acc gcc gtt tat    1056
Ala Lys Thr Ala Gly Leu Trp Ala Asp Ala Leu Lys Thr Ala Val Tyr
            340                 345                 350

ccg cgc gtt ttg aaa ttt gat ttg agc agc gta acg cgc aat atg gca    1104
Pro Arg Val Leu Lys Phe Asp Leu Ser Ser Val Thr Arg Asn Met Ala
        355                 360                 365

ggc ccg agc aac ccg cac gcg cgt ttt gcg acc gcc gat ttg gcc ggc    1152
Gly Pro Ser Asn Pro His Ala Arg Phe Ala Thr Ala Asp Leu Ala Gly
    370                 375                 380

aaa ggc ttg gct aaa cct tac gaa gag cct tca gac ggc caa atg cct    1200
Lys Gly Leu Ala Lys Pro Tyr Glu Glu Pro Ser Asp Gly Gln Met Pro
385                 390                 395                 400

gac ggt gca gtg att att gcc gcg att act tcc tgt acc aat act tcc    1248
Asp Gly Ala Val Ile Ile Ala Ala Ile Thr Ser Cys Thr Asn Thr Ser
                405                 410                 415

aat ccg cgc aac gtt gtc gcc gcc gcg ctg ttg gca cgc aat gcc aac    1296
Asn Pro Arg Asn Val Val Ala Ala Ala Leu Leu Ala Arg Asn Ala Asn
            420                 425                 430

cgc ctc ggc ttg caa cgc aaa cct tgg gtg aaa tct tcg ttt gcc ccg    1344
Arg Leu Gly Leu Gln Arg Lys Pro Trp Val Lys Ser Ser Phe Ala Pro
        435                 440                 445

ggt tca aaa gta gcc gaa atc tat ttg aaa gaa gca gat ctg ctg ccc    1392
Gly Ser Lys Val Ala Glu Ile Tyr Leu Lys Glu Ala Asp Leu Leu Pro
    450                 455                 460

gaa atg gaa aaa ctc ggc ttc ggt atc gtt gcc ttc gca tgt acc acc    1440
Glu Met Glu Lys Leu Gly Phe Gly Ile Val Ala Phe Ala Cys Thr Thr
465                 470                 475                 480

tgt aac ggc atg agc ggc gcg ctg gat ccg aaa atc cag aaa gaa atc    1488
Cys Asn Gly Met Ser Gly Ala Leu Asp Pro Lys Ile Gln Lys Glu Ile
                485                 490                 495

atc gac cgc gat ttg tac gcc acc gcc gta ttg tca ggc aac cgc aac    1536
Ile Asp Arg Asp Leu Tyr Ala Thr Ala Val Leu Ser Gly Asn Arg Asn
            500                 505                 510

ttt gac ggc cgt atc cat ccg tat gcg aaa cag gct ttc ctc gct tcg    1584
Phe Asp Gly Arg Ile His Pro Tyr Ala Lys Gln Ala Phe Leu Ala Ser
        515                 520                 525

cct ccg ttg gtc gtt gcc tac gcg ctg gca ggc agc atc cgt ttc gat    1632
Pro Pro Leu Val Val Ala Tyr Ala Leu Ala Gly Ser Ile Arg Phe Asp
    530                 535                 540

att gaa aac gac gta ctc ggc gtt gca gac ggc aaa gaa atc cgc ctg 1680
Ile Glu Asn Asp Val Leu Gly Val Ala Asp Gly Lys Glu Ile Arg Leu
545                 550                 555                 560

aaa gac att tgg cct acc gat gaa gaa atc gat gcc atc gtt gcc gaa 1728
Lys Asp Ile Trp Pro Thr Asp Glu Glu Ile Asp Ala Ile Val Ala Glu
                565                 570                 575

tat gtg aaa ccg cag caa ttt cgc gac gtt tat atc ccg atg ttc gac 1776
Tyr Val Lys Pro Gln Gln Phe Arg Asp Val Tyr Ile Pro Met Phe Asp
            580                 585                 590

acc ggc aca gcg caa aaa gca cca agc ccg ctg tac gac tgg cgt cca 1824
Thr Gly Thr Ala Gln Lys Ala Pro Ser Pro Leu Tyr Asp Trp Arg Pro
        595                 600                 605

atg tct acc tat atc cgc cgc cca cct tac tgg gaa ggc gca ctg gca 1872
Met Ser Thr Tyr Ile Arg Arg Pro Pro Tyr Trp Glu Gly Ala Leu Ala
    610                 615                 620

ggg gaa cgc aca tta agc ggt atg cgt ccg ctg gcg att ttg ccc gac 1920
Gly Glu Arg Thr Leu Ser Gly Met Arg Pro Leu Ala Ile Leu Pro Asp
625                 630                 635                 640

aac atc acc acc gac cat ctc tcg cca tcc aat gcg att ttg gca agc 1968
Asn Ile Thr Thr Asp His Leu Ser Pro Ser Asn Ala Ile Leu Ala Ser
                645                 650                 655

agt gcc gca ggc gaa tat ttg gca aaa atg ggt ttg cct gaa gaa gac 2016
Ser Ala Ala Gly Glu Tyr Leu Ala Lys Met Gly Leu Pro Glu Glu Asp
            660                 665                 670

ttc aac tct tac gca acc cac cgt ggc gac cac ttg acc gcc caa cgc 2064
Phe Asn Ser Tyr Ala Thr His Arg Gly Asp His Leu Thr Ala Gln Arg
        675                 680                 685

gca acc ttc gcc aat ccg aaa ctg ttt aac gaa atg gtg aga aac gaa 2112
Ala Thr Phe Ala Asn Pro Lys Leu Phe Asn Glu Met Val Arg Asn Glu
    690                 695                 700

gac ggc agc gta cgc caa ggt tcg ctg gca cgc gtt gaa ccc gaa ggc 2160
Asp Gly Ser Val Arg Gln Gly Ser Leu Ala Arg Val Glu Pro Glu Gly
705                 710                 715                 720

caa acc atg cgc atg tgg gaa gcc atc gaa acc tat atg aac cgc aaa 2208
Gln Thr Met Arg Met Trp Glu Ala Ile Glu Thr Tyr Met Asn Arg Lys
                725                 730                 735

cag ccg ctc atc atc att gcc ggc gcg gac tac ggt caa ggc tca agc 2256
Gln Pro Leu Ile Ile Ile Ala Gly Ala Asp Tyr Gly Gln Gly Ser Ser
            740                 745                 750

cgc gac tgg gct gca aaa ggc gta cgc ctc gcc ggc gtg gaa gcg att 2304
Arg Asp Trp Ala Ala Lys Gly Val Arg Leu Ala Gly Val Glu Ala Ile
        755                 760                 765

gtt gcc gaa ggc ttc gag cgt atc cac cgc acc aac ttg atc ggt atg 2352
Val Ala Glu Gly Phe Glu Arg Ile His Arg Thr Asn Leu Ile Gly Met
    770                 775                 780

ggc gtg ttg ccg ctg cag ttc aaa ccg ggt acc aac cgc cac acc ctg    2400
Gly Val Leu Pro Leu Gln Phe Lys Pro Gly Thr Asn Arg His Thr Leu
785                 790                 795                 800

caa ctg gac ggt acg gaa acc tac gac gtt gtc ggc gaa cgc aca ccg    2448
Gln Leu Asp Gly Thr Glu Thr Tyr Asp Val Val Gly Glu Arg Thr Pro
                805                 810                 815

cgc tgc gac ctg acc ctt gtg att cac cgt aaa aac ggc gag acc gtc    2496
Arg Cys Asp Leu Thr Leu Val Ile His Arg Lys Asn Gly Glu Thr Val
            820                 825                 830

gaa gtc ccc att acc tgc cgc ctc gat acc gca gaa gaa gtg ttg gta    2544
Glu Val Pro Ile Thr Cys Arg Leu Asp Thr Ala Glu Glu Val Leu Val
        835                 840                 845

tat gaa gcc ggt ggc gta ttg caa cgg ttt gca cag gat ttt ttg gaa    2592
Tyr Glu Ala Gly Gly Val Leu Gln Arg Phe Ala Gln Asp Phe Leu Glu
    850                 855                 860

ggg aac gcg gct tag                                                2607
Gly Asn Ala Ala
865


<210> 73
<211> 868
<212> PRT
<213> Neisseria meningitidis

<400> 73
Met Ala Ala Asn Gln Arg Tyr Arg Lys Pro Leu Pro Gly Thr Asp Leu
  1               5                  10                  15

Glu Tyr Tyr Asp Ala Arg Ala Ala Cys Glu Gly Ile Lys Pro Gly Ser
             20                  25                  30

Tyr Asp Lys Leu Pro Tyr Thr Ser Arg Ile Leu Ala Glu Asn Leu Val
         35                  40                  45

Asn Arg Ala Asp Lys Val Asp Leu Pro Thr Leu Gln Ser Trp Leu Gly
     50                  55                  60

Gln Leu Ile Glu Gly Lys Gln Glu Ile Asp Phe Pro Trp Tyr Pro Ala
 65                  70                  75                  80

Arg Val Val Cys His Asp Ile Leu Gly Gln Thr Ala Leu Val Asp Leu
                 85                  90                  95

Ala Gly Leu Arg Asp Ala Ile Ala Glu Lys Gly Gly Asp Pro Ala Lys
            100                 105                 110

Val Asn Pro Val Val Gln Thr Gln Leu Ile Val Asp His Ser Leu Ala
        115                 120                 125

Val Glu Cys Gly Gly Tyr Asp Pro Asp Ala Phe Arg Lys Asn Arg Glu
    130                 135                 140

Ile Glu Asp Arg Arg Asn Glu Asp Arg Phe His Phe Ile Asn Trp Thr
145                 150                 155                 160

Lys Thr Ala Phe Glu Asn Val Asp Val Ile Pro Ala Gly Asn Gly Ile
                165                 170                 175

Met His Gln Ile Asn Leu Glu Lys Met Ser Pro Val Val Gln Val Lys
            180                 185                 190

Asn Gly Val Ala Phe Pro Asp Thr Cys Val Gly Thr Asp Ser His Thr
        195                 200                 205

Pro His Val Asp Ala Leu Gly Val Ile Ser Val Gly Val Gly Gly Leu
    210                 215                 220

Glu Ala Glu Thr Val Met Leu Gly Arg Ala Ser Met Met Arg Leu Pro
225                 230                 235                 240

Asp Ile Val Gly Val Glu Leu Asn Gly Lys Arg Lys Ala Gly Ile Thr
                245                 250                 255

Ala Thr Asp Ile Val Leu Ala Leu Thr Glu Phe Leu Arg Lys Glu Arg
            260                 265                 270

Val Val Gly Ala Phe Val Glu Phe Phe Gly Glu Gly Ala Arg Ser Leu
        275                 280                 285

Ser Ile Gly Asp Arg Ala Thr Ile Ser Asn Met Thr Pro Glu Phe Gly
    290                 295                 300

Ala Thr Ala Ala Met Phe Ala Ile Asp Glu Gln Thr Ile Asp Tyr Leu
305                 310                 315                 320

Lys Leu Thr Gly Arg Asp Asp Ala Gln Val Lys Leu Val Glu Thr Tyr
                325                 330                 335

Ala Lys Thr Ala Gly Leu Trp Ala Asp Ala Leu Lys Thr Ala Val Tyr
            340                 345                 350

Pro Arg Val Leu Lys Phe Asp Leu Ser Ser Val Thr Arg Asn Met Ala
        355                 360                 365

Gly Pro Ser Asn Pro His Ala Arg Phe Ala Thr Ala Asp Leu Ala Gly
    370                 375                 380

Lys Gly Leu Ala Lys Pro Tyr Glu Glu Pro Ser Asp Gly Gln Met Pro
385                 390                 395                 400

Asp Gly Ala Val Ile Ile Ala Ala Ile Thr Ser Cys Thr Asn Thr Ser
                405                 410                 415

Asn Pro Arg Asn Val Val Ala Ala Ala Leu Leu Ala Arg Asn Ala Asn
            420                 425                 430

Arg Leu Gly Leu Gln Arg Lys Pro Trp Val Lys Ser Ser Phe Ala Pro
        435                 440                 445

Gly Ser Lys Val Ala Glu Ile Tyr Leu Lys Glu Ala Asp Leu Leu Pro
    450                 455                 460

Glu Met Glu Lys Leu Gly Phe Gly Ile Val Ala Phe Ala Cys Thr Thr
465                 470                 475                 480

Cys Asn Gly Met Ser Gly Ala Leu Asp Pro Lys Ile Gln Lys Glu Ile
                485                 490                 495

Ile Asp Arg Asp Leu Tyr Ala Thr Ala Val Leu Ser Gly Asn Arg Asn
            500                 505                 510

Phe Asp Gly Arg Ile His Pro Tyr Ala Lys Gln Ala Phe Leu Ala Ser
        515                 520                 525

Pro Pro Leu Val Val Ala Tyr Ala Leu Ala Gly Ser Ile Arg Phe Asp
    530                 535                 540

Ile Glu Asn Asp Val Leu Gly Val Ala Asp Gly Lys Glu Ile Arg Leu
545                 550                 555                 560

Lys Asp Ile Trp Pro Thr Asp Glu Glu Ile Asp Ala Ile Val Ala Glu
                565                 570                 575

Tyr Val Lys Pro Gln Gln Phe Arg Asp Val Tyr Ile Pro Met Phe Asp
            580                 585                 590

Thr Gly Thr Ala Gln Lys Ala Pro Ser Pro Leu Tyr Asp Trp Arg Pro
        595                 600                 605

Met Ser Thr Tyr Ile Arg Arg Pro Pro Tyr Trp Glu Gly Ala Leu Ala
    610                 615                 620

Gly Glu Arg Thr Leu Ser Gly Met Arg Pro Leu Ala Ile Leu Pro Asp
625                 630                 635                 640

Asn Ile Thr Thr Asp His Leu Ser Pro Ser Asn Ala Ile Leu Ala Ser
                645                 650                 655

Ser Ala Ala Gly Glu Tyr Leu Ala Lys Met Gly Leu Pro Glu Glu Asp
            660                 665                 670

Phe Asn Ser Tyr Ala Thr His Arg Gly Asp His Leu Thr Ala Gln Arg
        675                 680                 685

Ala Thr Phe Ala Asn Pro Lys Leu Phe Asn Glu Met Val Arg Asn Glu
    690                 695                 700

Asp Gly Ser Val Arg Gln Gly Ser Leu Ala Arg Val Glu Pro Glu Gly
705                 710                 715                 720

Gln Thr Met Arg Met Trp Glu Ala Ile Glu Thr Tyr Met Asn Arg Lys
                725                 730                 735

Gln Pro Leu Ile Ile Ile Ala Gly Ala Asp Tyr Gly Gln Gly Ser Ser
            740                 745                 750

Arg Asp Trp Ala Ala Lys Gly Val Arg Leu Ala Gly Val Glu Ala Ile
        755                 760                 765

Val Ala Glu Gly Phe Glu Arg Ile His Arg Thr Asn Leu Ile Gly Met
    770                 775                 780

Gly Val Leu Pro Leu Gln Phe Lys Pro Gly Thr Asn Arg His Thr Leu
785                 790                 795                 800

Gln Leu Asp Gly Thr Glu Thr Tyr Asp Val Val Gly Glu Arg Thr Pro
                805                 810                 815

Arg Cys Asp Leu Thr Leu Val Ile His Arg Lys Asn Gly Glu Thr Val
            820                 825                 830

Glu Val Pro Ile Thr Cys Arg Leu Asp Thr Ala Glu Glu Val Leu Val
        835                 840                 845

Tyr Glu Ala Gly Gly Val Leu Gln Arg Phe Ala Gln Asp Phe Leu Glu
    850                 855                 860

Gly Asn Ala Ala
865

<210> 74
<211> 1170
<212> ADN
<213> Neisseria meningitidis

<220>
<221> CDS
<222> (1)..(1167)

<400> 74
atg ccg caa att aaa att ccc gcc gtt tac tac cgt ggc ggt aca tca    48
Met Pro Gln Ile Lys Ile Pro Ala Val Tyr Tyr Arg Gly Gly Thr Ser
  1               5                  10                  15

aaa ggc gtg ttt ttc aaa cgt tcc gac ctg ccc gag gcg gcg cgg gaa    96
Lys Gly Val Phe Phe Lys Arg Ser Asp Leu Pro Glu Ala Ala Arg Glu
             20                  25                  30

gcg gga agc gca cgc gac aaa atc ctc ttg cgc gta ctc ggc agc ccg    144
Ala Gly Ser Ala Arg Asp Lys Ile Leu Leu Arg Val Leu Gly Ser Pro
         35                  40                  45

gat ccc tac ggc aag cag ata gac ggt ttg ggc aac gcc agc tcg tcc    192
Asp Pro Tyr Gly Lys Gln Ile Asp Gly Leu Gly Asn Ala Ser Ser Ser
     50                  55                  60

acc agc aag gcg gtg att ttg gac aag tcc gaa cgc gcc gat cac gat    240
Thr Ser Lys Ala Val Ile Leu Asp Lys Ser Glu Arg Ala Asp His Asp
 65                  70                  75                  80

gtc gat tac ctt ttc ggg caa gtt tcc atc gac aaa cct ttt gtc gat    288
Val Asp Tyr Leu Phe Gly Gln Val Ser Ile Asp Lys Pro Phe Val Asp
                 85                  90                  95

tgg agt ggc aac tgc ggc aac ctc acc gcc gcc gtg ggc gca ttt gcc    336
Trp Ser Gly Asn Cys Gly Asn Leu Thr Ala Ala Val Gly Ala Phe Ala
            100                 105                 110

atc gag caa ggc ttg gtc gat aaa ggc aag att cct tca gac ggc atc    384
Ile Glu Gln Gly Leu Val Asp Lys Gly Lys Ile Pro Ser Asp Gly Ile
        115                 120                 125

tgc aca gtc aaa atc tgg cag aaa aac atc ggc aaa acc att att gcc     432
Cys Thr Val Lys Ile Trp Gln Lys Asn Ile Gly Lys Thr Ile Ile Ala
    130                 135                 140

cat gta ccg atg caa aac ggc gca gtt ttg gaa aca ggc gat ttt gag     480
His Val Pro Met Gln Asn Gly Ala Val Leu Glu Thr Gly Asp Phe Glu
145                 150                 155                 160

ctc gac ggc gta acg ttc ccg gca gcc gaa gta caa atc gaa ttt ctt     528
Leu Asp Gly Val Thr Phe Pro Ala Ala Glu Val Gln Ile Glu Phe Leu
                165                 170                 175

gat cca gcc gac ggc gaa ggc agt atg ttc cca acc ggc aat ttg gtc     576
Asp Pro Ala Asp Gly Glu Gly Ser Met Phe Pro Thr Gly Asn Leu Val
            180                 185                 190

gat gaa att gat gtg ccg aat ata ggc cgt ttg aaa gcc acg ctc atc     624
Asp Glu Ile Asp Val Pro Asn Ile Gly Arg Leu Lys Ala Thr Leu Ile
        195                 200                 205

aac gcg ggc att ccg acc gtt ttc ctg aat gcc gcc gac ttg ggc tac     672
Asn Ala Gly Ile Pro Thr Val Phe Leu Asn Ala Ala Asp Leu Gly Tyr
    210                 215                 220

acg ggc aaa gag ttg caa gac gac atc aac aac gat gcc gca gct ttg     720
Thr Gly Lys Glu Leu Gln Asp Asp Ile Asn Asn Asp Ala Ala Ala Leu
225                 230                 235                 240

gaa aaa ttc gag aaa atc cgc gct tac ggt gcg ctg aaa atg ggt ctg     768
Glu Lys Phe Glu Lys Ile Arg Ala Tyr Gly Ala Leu Lys Met Gly Leu
                245                 250                 255

atc agc gac gta tcc gaa gct gcc gcc cgc gcg cac acg ccg aaa gtc     816
Ile Ser Asp Val Ser Glu Ala Ala Ala Arg Ala His Thr Pro Lys Val
            260                 265                 270

gcc ttc gtc gcg ccc gcc gcc gat tac acc gcc tcc agt ggc aaa acc     864
Ala Phe Val Ala Pro Ala Ala Asp Tyr Thr Ala Ser Ser Gly Lys Thr
        275                 280                 285

gtg aat gcc gcc gac atc gat ttg ctg gta cgc gcc ctg agc atg ggc     912
Val Asn Ala Ala Asp Ile Asp Leu Leu Val Arg Ala Leu Ser Met Gly
    290                 295                 300

aaa ttg cac cac gcg atg atg ggt acc gcc tct gtt gcc att gcg acc     960
Lys Leu His His Ala Met Met Gly Thr Ala Ser Val Ala Ile Ala Thr
305                 310                 315                 320

gcc gcc gcc gtg ccc ggt acg ctg gtc aac ctt gcc gca ggc ggc gga     1008
Ala Ala Ala Val Pro Gly Thr Leu Val Asn Leu Ala Ala Gly Gly Gly
                325                 330                 335

acg cgt aaa gaa gtg cgc ttc ggg cat cct tcc ggc aca ttg cgc gtc     1056
Thr Arg Lys Glu Val Arg Phe Gly His Pro Ser Gly Thr Leu Arg Val
            340                 345                 350

ggt gca gcc gcc gaa tgt cag gac gga caa tgg acg gcc acc aaa gcg     1104
Gly Ala Ala Ala Glu Cys Gln Asp Gly Gln Trp Thr Ala Thr Lys Ala
        355                 360                 365

gtt atg agc cgc agc gca cgc gtg atg atg gaa ggt tgg gtc agg gtg     1152
Val Met Ser Arg Ser Ala Arg Val Met Met Glu Gly Trp Val Arg Val
    370                 375                 380

ccg gaa gat tgt ttt taa                                             1170
Pro Glu Asp Cys Phe
385

<210> 75
<211> 389
<212> PRT
<213> Neisseria meningitidis

<400> 75
Met Pro Gln Ile Lys Ile Pro Ala Val Tyr Tyr Arg Gly Gly Thr Ser
 1               5                  10                  15

Lys Gly Val Phe Phe Lys Arg Ser Asp Leu Pro Glu Ala Ala Arg Glu
            20                  25                  30

Ala Gly Ser Ala Arg Asp Lys Ile Leu Leu Arg Val Leu Gly Ser Pro
        35                  40                  45

Asp Pro Tyr Gly Lys Gln Ile Asp Gly Leu Gly Asn Ala Ser Ser Ser
    50                  55                  60

Thr Ser Lys Ala Val Ile Leu Asp Lys Ser Glu Arg Ala Asp His Asp
65                  70                  75                  80

Val Asp Tyr Leu Phe Gly Gln Val Ser Ile Asp Lys Pro Phe Val Asp
                85                  90                  95

Trp Ser Gly Asn Cys Gly Asn Leu Thr Ala Ala Val Gly Ala Phe Ala
            100                 105                 110

Ile Glu Gln Gly Leu Val Asp Lys Gly Lys Ile Pro Ser Asp Gly Ile
        115                 120                 125

Cys Thr Val Lys Ile Trp Gln Lys Asn Ile Gly Lys Thr Ile Ile Ala
    130                 135                 140

His Val Pro Met Gln Asn Gly Ala Val Leu Glu Thr Gly Asp Phe Glu
145                 150                 155                 160

Leu Asp Gly Val Thr Phe Pro Ala Ala Glu Val Gln Ile Glu Phe Leu
                165                 170                 175

Asp Pro Ala Asp Gly Glu Gly Ser Met Phe Pro Thr Gly Asn Leu Val
            180                 185                 190

Asp Glu Ile Asp Val Pro Asn Ile Gly Arg Leu Lys Ala Thr Leu Ile
        195                 200                 205

Asn Ala Gly Ile Pro Thr Val Phe Leu Asn Ala Ala Asp Leu Gly Tyr
    210                 215                 220

Thr Gly Lys Glu Leu Gln Asp Asp Ile Asn Asn Asp Ala Ala Ala Leu
225                 230                 235                 240

Glu Lys Phe Glu Lys Ile Arg Ala Tyr Gly Ala Leu Lys Met Gly Leu
                245                 250                 255

Ile Ser Asp Val Ser Glu Ala Ala Ala Arg Ala His Thr Pro Lys Val
            260                 265                 270

Ala Phe Val Ala Pro Ala Ala Asp Tyr Thr Ala Ser Ser Gly Lys Thr
        275                 280                 285

Val Asn Ala Ala Asp Ile Asp Leu Leu Val Arg Ala Leu Ser Met Gly
    290                 295                 300

Lys Leu His His Ala Met Met Gly Thr Ala Ser Val Ala Ile Ala Thr
305                 310                 315                 320

Ala Ala Ala Val Pro Gly Thr Leu Val Asn Leu Ala Ala Gly Gly Gly
                325                 330                 335

Thr Arg Lys Glu Val Arg Phe Gly His Pro Ser Gly Thr Leu Arg Val
            340                 345                 350

Gly Ala Ala Ala Glu Cys Gln Asp Gly Gln Trp Thr Ala Thr Lys Ala
        355                 360                 365

Val Met Ser Arg Ser Ala Arg Val Met Met Glu Gly Trp Val Arg Val
    370                 375                 380

Pro Glu Asp Cys Phe
385

<210> 76
<211> 2094
<212> ADN
<213> Neisseria gonorrhoeae

<220>
<221> CDS
<222> (1)..(2091)

<400> 76
atg aat tcg acc gca agt aaa acc ctg aaa gga ttg tcg ctg gtg ttt      48
Met Asn Ser Thr Ala Ser Lys Thr Leu Lys Gly Leu Ser Leu Val Phe
  1               5                  10                  15

ttc gcc tct ggc ttc tgc gcc ctg att tac cag gtc agc tgg cag agg      96
Phe Ala Ser Gly Phe Cys Ala Leu Ile Tyr Gln Val Ser Trp Gln Arg
             20                  25                  30

ctt cta ttc agc cac ata ggt atc gat ttg agt tcg att act gtc att     144
Leu Leu Phe Ser His Ile Gly Ile Asp Leu Ser Ser Ile Thr Val Ile
         35                  40                  45

att tct gta ttt atg gtc ggc ttg ggt gta ggt gcg tat ttc ggc gga     192
Ile Ser Val Phe Met Val Gly Leu Gly Val Gly Ala Tyr Phe Gly Gly
     50                  55                  60

cgc att gct gac cgt ttt cct tca agt atc atc ccc ctg ttt tgc atc     240
Arg Ile Ala Asp Arg Phe Pro Ser Ser Ile Ile Pro Leu Phe Cys Ile

65                  70                  75                  80

gct gaa gta tcc atc ggt ctg ttc ggt ttg gta agc aag ggt ctg att     288
Ala Glu Val Ser Ile Gly Leu Phe Gly Leu Val Ser Lys Gly Leu Ile
                85                  90                  95

tcc ggc ttg ggg cat ctt tta gtt gag gct gat ttg ccc atc atc gct     336
Ser Gly Leu Gly His Leu Leu Val Glu Ala Asp Leu Pro Ile Ile Ala
            100                 105                 110

gct gcc aat ttc ctc tta ttg ctg ctt cct acc ttt atg atg ggc gcg     384
Ala Ala Asn Phe Leu Leu Leu Leu Leu Pro Thr Phe Met Met Gly Ala
        115                 120                 125

acc ttg ccc ttg ctg acc tgt ttt ttt aac cgg aaa ata cat aat gtt     432
Thr Leu Pro Leu Leu Thr Cys Phe Phe Asn Arg Lys Ile His Asn Val
    130                 135                 140

ggc gag tct atc ggt acc tta tat ttt ttc aac act ttg ggt gcg gca     480
Gly Glu Ser Ile Gly Thr Leu Tyr Phe Phe Asn Thr Leu Gly Ala Ala
145                 150                 155                 160

ctc gga tcg ctt gcc gcc gcc gaa ttt ttc tac gtc ttt ttt acc ctc     528
Leu Gly Ser Leu Ala Ala Ala Glu Phe Phe Tyr Val Phe Phe Thr Leu
                165                 170                 175

tcc caa acc att gcg ctg aca gcc tgc ctt aac ctt ctg att gct gct     576
Ser Gln Thr Ile Ala Leu Thr Ala Cys Leu Asn Leu Leu Ile Ala Ala
            180                 185                 190

tca gta tgc tgc gtt aca gaa agg atg gat atg gtg aac act aaa ccg     624
Ser Val Cys Cys Val Thr Glu Arg Met Asp Met Val Asn Thr Lys Pro
        195                 200                 205

aat act agt gtg att aat atg ctt tct ttc ctt acc gga tta ttg agc     672
Asn Thr Ser Val Ile Asn Met Leu Ser Phe Leu Thr Gly Leu Leu Ser
    210                 215                 220

ttg ggt ata gaa gtc ttg tgg gta agg atg ttt tcg ttc gca gca cag     720
Leu Gly Ile Glu Val Leu Trp Val Arg Met Phe Ser Phe Ala Ala Gln
225                 230                 235                 240

tcc gtg cct cag gca ttt tca ttt att ctt gcc tgt ttt ctg acc ggt     768
Ser Val Pro Gln Ala Phe Ser Phe Ile Leu Ala Cys Phe Leu Thr Gly
                245                 250                 255

atc gcc gtc ggc gcg tat ttt ggc aaa cgg att tgc cgc agc cgc ttt     816
Ile Ala Val Gly Ala Tyr Phe Gly Lys Arg Ile Cys Arg Ser Arg Phe
            260                 265                 270

gtt gat att ccc ttt atc ggg cag tgc ttc ttg tgg gcg ggt att gcc     864
Val Asp Ile Pro Phe Ile Gly Gln Cys Phe Leu Trp Ala Gly Ile Ala
        275                 280                 285

gat ttt ttg att ttg ggt gct gcg tgg ttg ttg acg ggt ttt tcc ggt     912
Asp Phe Leu Ile Leu Gly Ala Ala Trp Leu Leu Thr Gly Phe Ser Gly
    290                 295                 300

ttc gtc cac cac gcc ggt att ttc att acc ctg tct gcc gtc gtc agg     960
Phe Val His His Ala Gly Ile Phe Ile Thr Leu Ser Ala Val Val Arg

305                 310                 315                 320

ggg ttg att ttc cca ctt gta cac cat gtg ggt acg gat ggc aac aaa      1008
Gly Leu Ile Phe Pro Leu Val His His Val Gly Thr Asp Gly Asn Lys
                325                 330                 335

tcc gga cga cag gtt tcc aat gtt tat ttc gcc aac gtt gcc ggc agt      1056
Ser Gly Arg Gln Val Ser Asn Val Tyr Phe Ala Asn Val Ala Gly Ser
            340                 345                 350

gca ttg ggt ccg gtc ctt atc ggc ttt gtg ata ctt gat ttg ttg tcc      1104
Ala Leu Gly Pro Val Leu Ile Gly Phe Val Ile Leu Asp Leu Leu Ser
        355                 360                 365

acc caa cag att tac ctg ctc atc tgt ttg att tct gct gct gtc cct      1152
Thr Gln Gln Ile Tyr Leu Leu Ile Cys Leu Ile Ser Ala Ala Val Pro
    370                 375                 380

ttg ttt tgt aca ctg ttc caa aaa agt ctc cga ctc aat gca gtg tcg      1200
Leu Phe Cys Thr Leu Phe Gln Lys Ser Leu Arg Leu Asn Ala Val Ser
385                 390                 395                 400

gta gca gtt tcc cta atg ttc ggc atc ctc atg ttc cta ctg ccg gat      1248
Val Ala Val Ser Leu Met Phe Gly Ile Leu Met Phe Leu Leu Pro Asp
                405                 410                 415

tct gtc ttt caa aat att gct ggc cgt ccg gat agg ttg att gaa aac      1296
Ser Val Phe Gln Asn Ile Ala Gly Arg Pro Asp Arg Leu Ile Glu Asn
            420                 425                 430

aaa cac ggc att gtt gcg gtt tac cat aga gat ggt gat aag gtt gtt      1344
Lys His Gly Ile Val Ala Val Tyr His Arg Asp Gly Asp Lys Val Val
        435                 440                 445

tat ggg gcg aat gta tac gac ggc gca tac aat acc gat ata ttc aat      1392
Tyr Gly Ala Asn Val Tyr Asp Gly Ala Tyr Asn Thr Asp Ile Phe Asn
    450                 455                 460

agt gtc aac ggc atc gaa cgt gcc tat ctg cta ccc tcc ctg aag tcc      1440
Ser Val Asn Gly Ile Glu Arg Ala Tyr Leu Leu Pro Ser Leu Lys Ser
465                 470                 475                 480

ggc ata cgc cgc att ttc gtc gtt gga ttg agt aca ggt tcg tgg gcg      1488
Gly Ile Arg Arg Ile Phe Val Val Gly Leu Ser Thr Gly Ser Trp Ala
                485                 490                 495

cgc gtc ttg tct gcc att ccg gaa atg cag tcg atg atc gtt gcg gaa      1536
Arg Val Leu Ser Ala Ile Pro Glu Met Gln Ser Met Ile Val Ala Glu
            500                 505                 510

atc aat ccg gca tac cgt agc ctt atc gcg gac gag ccg caa atc gca      1584
Ile Asn Pro Ala Tyr Arg Ser Leu Ile Ala Asp Glu Pro Gln Ile Ala
        515                 520                 525

ccg ctt ttg cag gac aaa cgt gtt gaa att gta ttg gat gac ggt agg      1632
Pro Leu Leu Gln Asp Lys Arg Val Glu Ile Val Leu Asp Asp Gly Arg
    530                 535                 540

aaa tgg ctg cgt cgc cat cct gat gaa aaa ttc gac ctg att ttg atg      1680
Lys Trp Leu Arg Arg His Pro Asp Glu Lys Phe Asp Leu Ile Leu Met

545                 550                 555                 560

aat tcg act tgg tac tgg cgt gcc tat tcc act aac ctg ttg agt gcg     1728
Asn Ser Thr Trp Tyr Trp Arg Ala Tyr Ser Thr Asn Leu Leu Ser Ala
                565                 570                 575

gaa ttt tta aaa cag gtg caa agc cac ctt acc ccg gat ggt att gta     1776
Glu Phe Leu Lys Gln Val Gln Ser His Leu Thr Pro Asp Gly Ile Val
            580                 585                 590

atg ttt aat acc acg cac agc ccg cat gct ttt gct acc gcc gta cac     1824
Met Phe Asn Thr Thr His Ser Pro His Ala Phe Ala Thr Ala Val His
        595                 600                 605

agt att ccc tat gca tac cgc tac ggg cat atg gta gtc ggc tcg gca     1872
Ser Ile Pro Tyr Ala Tyr Arg Tyr Gly His Met Val Val Gly Ser Ala
    610                 615                 620

acc ccg gta gtt ttc cct aat aaa gaa ctg ctc aag caa cgc ctt tcc     1920
Thr Pro Val Val Phe Pro Asn Lys Glu Leu Leu Lys Gln Arg Leu Ser
625                 630                 635                 640

cgg ttg att tgg ccg gaa agc ggc agg cac gta ttt gac agc agc acc     1968
Arg Leu Ile Trp Pro Glu Ser Gly Arg His Val Phe Asp Ser Ser Thr
                645                 650                 655

gtg gat gct gca gca caa aag gtt gtc tct cgt atg ctg att cgg atg     2016
Val Asp Ala Ala Ala Gln Lys Val Val Ser Arg Met Leu Ile Arg Met
            660                 665                 670

acg gaa cct tcg gct ggg gcg gaa gtc att act gac gat aat atg att     2064
Thr Glu Pro Ser Ala Gly Ala Glu Val Ile Thr Asp Asp Asn Met Ile
        675                 680                 685

gta gaa tac aaa tac ggc aga ggg att taa                             2094
Val Glu Tyr Lys Tyr Gly Arg Gly Ile
    690                 695

<210> 77
<211> 697
<212> PRT
<213> Neisseria gonorrhoeae

<400> 77
Met Asn Ser Thr Ala Ser Lys Thr Leu Lys Gly Leu Ser Leu Val Phe
1               5                   10                  15

Phe Ala Ser Gly Phe Cys Ala Leu Ile Tyr Gln Val Ser Trp Gln Arg
            20                  25                  30

Leu Leu Phe Ser His Ile Gly Ile Asp Leu Ser Ser Ile Thr Val Ile
        35                  40                  45

Ile Ser Val Phe Met Val Gly Leu Gly Val Gly Ala Tyr Phe Gly Gly
    50                  55                  60

Arg Ile Ala Asp Arg Phe Pro Ser Ser Ile Ile Pro Leu Phe Cys Ile
65                  70                  75                  80

Ala Glu Val Ser Ile Gly Leu Phe Gly Leu Val Ser Lys Gly Leu Ile
                85                  90                  95

Ser Gly Leu Gly His Leu Leu Val Glu Ala Asp Leu Pro Ile Ile Ala
            100                 105                 110

Ala Ala Asn Phe Leu Leu Leu Leu Leu Pro Thr Phe Met Met Gly Ala
        115                 120                 125

Thr Leu Pro Leu Leu Thr Cys Phe Phe Asn Arg Lys Ile His Asn Val
    130                 135                 140

Gly Glu Ser Ile Gly Thr Leu Tyr Phe Phe Asn Thr Leu Gly Ala Ala
145                 150                 155                 160

Leu Gly Ser Leu Ala Ala Ala Glu Phe Phe Tyr Val Phe Phe Thr Leu
                165                 170                 175

Ser Gln Thr Ile Ala Leu Thr Ala Cys Leu Asn Leu Leu Ile Ala Ala
            180                 185                 190

Ser Val Cys Cys Val Thr Glu Arg Met Asp Met Val Asn Thr Lys Pro
        195                 200                 205

Asn Thr Ser Val Ile Asn Met Leu Ser Phe Leu Thr Gly Leu Leu Ser
    210                 215                 220

Leu Gly Ile Glu Val Leu Trp Val Arg Met Phe Ser Phe Ala Ala Gln
225                 230                 235                 240

Ser Val Pro Gln Ala Phe Ser Phe Ile Leu Ala Cys Phe Leu Thr Gly
                245                 250                 255

Ile Ala Val Gly Ala Tyr Phe Gly Lys Arg Ile Cys Arg Ser Arg Phe
            260                 265                 270

Val Asp Ile Pro Phe Ile Gly Gln Cys Phe Leu Trp Ala Gly Ile Ala
        275                 280                 285

Asp Phe Leu Ile Leu Gly Ala Ala Trp Leu Leu Thr Gly Phe Ser Gly
    290                 295                 300

Phe Val His His Ala Gly Ile Phe Ile Thr Leu Ser Ala Val Val Arg
305                 310                 315                 320

Gly Leu Ile Phe Pro Leu Val His His Val Gly Thr Asp Gly Asn Lys
                325                 330                 335

Ser Gly Arg Gln Val Ser Asn Val Tyr Phe Ala Asn Val Ala Gly Ser
            340                 345                 350

Ala Leu Gly Pro Val Leu Ile Gly Phe Val Ile Leu Asp Leu Leu Ser
        355                 360                 365

Thr Gln Gln Ile Tyr Leu Leu Ile Cys Leu Ile Ser Ala Ala Val Pro
    370                 375                 380

Leu Phe Cys Thr Leu Phe Gln Lys Ser Leu Arg Leu Asn Ala Val Ser
385                 390                 395                 400

Val Ala Val Ser Leu Met Phe Gly Ile Leu Met Phe Leu Leu Pro Asp
                405                 410                 415

Ser Val Phe Gln Asn Ile Ala Gly Arg Pro Asp Arg Leu Ile Glu Asn
            420                 425                 430

Lys His Gly Ile Val Ala Val Tyr His Arg Asp Gly Asp Lys Val Val
        435                 440                 445

Tyr Gly Ala Asn Val Tyr Asp Gly Ala Tyr Asn Thr Asp Ile Phe Asn
    450                 455                 460

Ser Val Asn Gly Ile Glu Arg Ala Tyr Leu Leu Pro Ser Leu Lys Ser
465                 470                 475                 480

Gly Ile Arg Arg Ile Phe Val Val Gly Leu Ser Thr Gly Ser Trp Ala
                485                 490                 495

Arg Val Leu Ser Ala Ile Pro Glu Met Gln Ser Met Ile Val Ala Glu
            500                 505                 510

Ile Asn Pro Ala Tyr Arg Ser Leu Ile Ala Asp Glu Pro Gln Ile Ala
        515                 520                 525

Pro Leu Leu Gln Asp Lys Arg Val Glu Ile Val Leu Asp Asp Gly Arg
    530                 535                 540

Lys Trp Leu Arg Arg His Pro Asp Glu Lys Phe Asp Leu Ile Leu Met
545                 550                 555                 560

Asn Ser Thr Trp Tyr Trp Arg Ala Tyr Ser Thr Asn Leu Leu Ser Ala
                565                 570                 575

Glu Phe Leu Lys Gln Val Gln Ser His Leu Thr Pro Asp Gly Ile Val
            580                 585                 590

Met Phe Asn Thr Thr His Ser Pro His Ala Phe Ala Thr Ala Val His
        595                 600                 605

Ser Ile Pro Tyr Ala Tyr Arg Tyr Gly His Met Val Val Gly Ser Ala
    610                 615                 620

Thr Pro Val Val Phe Pro Asn Lys Glu Leu Leu Lys Gln Arg Leu Ser
625                 630                 635                 640

Arg Leu Ile Trp Pro Glu Ser Gly Arg His Val Phe Asp Ser Ser Thr
                645                 650                 655

Val Asp Ala Ala Ala Gln Lys Val Val Ser Arg Met Leu Ile Arg Met
            660                 665                 670

Thr Glu Pro Ser Ala Gly Ala Glu Val Ile Thr Asp Asp Asn Met Ile
        675                 680                 685

Val Glu Tyr Lys Tyr Gly Arg Gly Ile
    690                 695

<210> 78

<211> 39
<212> ADN
<213> Séquence artificielle

<220>
<223> Description de la séquence artificielle : amorce de PCR

<400> 78
gctctagacc accatgtctg aagaaaaatt gaaaatgag 39

<210> 79
<211> 32
<212> ADN
<213> Séquence artificielle

<220>
<223> Description de la séquence artificielle: amorce de
PCR

<400> 79
cgggatccag aaatggctgg attcgctatc ag 32

<210> 80
<211> 32
<212> ADN
<213> Séquence artificielle

<220>
<223> Description de la séquence artificielle: amorce de
PCR

<400> 80
gctctagacc accatgaaac acttactcat cg 32

<210> 81
<211> 27
<212> ADN
<213> Séquence artificielle

<220>
<223> Description de la séquence artificielle: amorce de
PCR

<400> 81
cgggatccaa tacgtaggac ttgggtc 27

<210> 82
<211> 35
<212> ADN
<213> Séquence artificielle

<220>
<223> Description de la séquence artificielle: amorce de
PCR

<400> 82

gctctagacc accatgaaaa aatccctttt cgttc 35

<210> 83
<211> 31
<212> ADN
<213> Séquence artificielle

<220>
<223> Description de la séquence artificielle: amorce de
      PCR

<400> 83
cgggatccat tgcggataaa catattccgc c 31

<210> 84
<211> 34
<212> ADN
<213> Séquence artificielle

<220>
<223> Description de la séquence artificielle: amorce de
      PCR

<400> 84
gctctagacc accatgcgaa cgaccccaac cttc 34

<210> 85
<211> 30
<212> ADN
<213> Séquence artificielle

<220>
<223> Description de la séquence artificielle: amorce de
      PCR

<400> 85
cgggatccag aaccggtagc ctacgccgac 30

<210> 86
<211> 36
<212> ADN
<213> Séquence artificielle

<220>
<223> Description de la séquence artificielle: amorce de
      PCR

<400> 86
gctctagacc accatgaaca cacgcatcat cgtttc 36

<210> 87
<211> 30
<212> ADN
<213> Séquence artificielle

<220>
<223> Description de la séquence artificielle: amorce de
      PCR

<400> 87
cgggatccag caacggcctg ccgctttaag                                        30


<210> 88
<211> 34
<212> ADN
<213> Séquence artificielle

<220>
<223> Description de la séquence artificielle: amorce de
      PCR

<400> 88
gctctagacc accatgctga cgtttatcgg actg                                   34


<210> 89
<211> 27
<212> ADN
<213> Séquence artificielle

<220>
<223> Description de la séquence artificielle: amorce de
      PCR

<400> 89
cgggatccac ggcagaggca cgattcc                                           27


<210> 90
<211> 34
<212> ADN
<213> Séquence artificielle

<220>
<223> Description de la séquence artificielle: amorce de
      PCR

<400> 90
gctctagacc accatgggca tccatctcga cttc                                   34


<210> 91
<211> 33
<212> ADN
<213> Séquence artificielle

<220>
<223> Description de la séquence artificielle: amorce de
      PCR

<400> 91
cgggatccac aaaagttcca gaaaatctaa ctc                                    33

<210> 92
<211> 33
<212> ADN
<213> Séquence artificielle

<220>
<223> Description de la séquence artificielle: amorce de
      PCR

<400> 92
gctctagacc accatgaata gacccaagca acc                                33


<210> 93
<211> 26
<212> ADN
<213> Séquence artificielle

<220>
<223> Description de la séquence artificielle: amorce de
      PCR

<400> 93
cgggatccat gccgcttggg ggaggc                                        26


<210> 94
<211> 34
<212> ADN
<213> Séquence artificielle

<220>
<223> Description de la séquence artificielle: amorce de
      PCR

<400> 94
gctctagacc accatgatga atgtcgaggc agag                               34


<210> 95
<211> 26
<212> ADN
<213> Séquence artificielle

<220>
<223> Description de la séquence artificielle: amorce de
      PCR

<400> 95
cgggatccac agtttgcccg acatac                                        26


<210> 96
<211> 33
<212> ADN
<213> Séquence artificielle

<220>
<223> Description de la séquence artificielle: amorce de
      PCR

<400> 96
gctctagacc accatgaaat tttttcctgc tcc                                33

<210> 97
<211> 62
<212> ADN
<213> Séquence artificielle

<220>
<223> Description de la séquence artificielle: amorce de
      PCR

<400> 97
gaagatctag aaactgtaat tcaagttgaa ggaagatcta gaaactgtaa ttcaagttga 60
ag                                                                  62

<210> 98
<211> 33
<212> ADN
<213> Séquence artificielle

<220>
<223> Description de la séquence artificielle: amorce de
      PCR

<400> 98
gctctagacc accatgattg aatttgtccg agc                                33

<210> 99
<211> 54
<212> ADN
<213> Séquence artificielle

<220>
<223> Description de la séquence artificielle: amorce de
      PCR

<400> 99
cgggatccaa ccctgcgacg agttgcgcgg gatccaaccc tgcgacgagt tgcg         54

<210> 100
<211> 33
<212> ADN
<213> Séquence artificielle

<220>
<223> Description de la séquence artificielle: amorce de
      PCR

<400> 100
gctctagacc accatgcaat acagcacact ggc                                33

<210> 101
<211> 30

<212> ADN
<213> Séquence artificielle

<220>
<223> Description de la séquence artificielle: amorce de
      PCR

<400> 101
cgggatccag tcctttttcg caccttgaag                                        30


<210> 102
<211> 34
<212> ADN
<213> Séquence artificielle

<220>
<223> Description de la séquence artificielle: amorce de
      PCR

<400> 102
gctctagacc accatggagc agtcgggcaa attc                                   34


<210> 103
<211> 30
<212> ADN
<213> Séquence artificielle

<220>
<223> Description de la séquence artificielle: amorce de
      PCR

<400> 103
cgggatccaa gctgtttggc gatttcggtg                                        30


<210> 104
<211> 34
<212> ADN
<213> Séquence artificielle

<220>
<223> Description de la séquence artificielle: amorce de
      PCR

<400> 104
gctctagacc accatgcaaa acggcggggg aaag                                   34


<210> 105
<211> 30
<212> ADN
<213> Séquence artificielle

<220>
<223> Description de la séquence artificielle: amorce de
      PCR

<400> 105

cgggatccag tgcctgcgca gcttggaatc 30

<210> 106
<211> 40
<212> ADN
<213> Séquence artificielle

<220>
<223> Description de la séquence artificielle: amorce de
      PCR

<400> 106
gctctagacc accatgacat tgctcaatct aatgataatg 40

<210> 107
<211> 33
<212> ADN
<213> Séquence artificielle

<220>
<223> Description de la séquence artificielle: amorce de
      PCR

<400> 107
cgggatccat tccgcaaata cctgtttcca acc 33

<210> 108
<211> 30
<212> ADN
<213> Séquence artificielle

<220>
<223> Description de la séquence artificielle: amorce de
      PCR

<400> 108
gctctagacc accatgaaac aatccgcccg 30

<210> 109
<211> 28
<212> ADN
<213> Séquence artificielle

<220>
<223> Description de la séquence artificielle: amorce de
      PCR

<400> 109
cgggatccat acttgggcgc aacatgac 28

<210> 110
<211> 33
<212> ADN
<213> Séquence artificielle

<220>
<223> Description de la séquence artificielle: amorce de
      PCR

<400> 110
gctctagacc accatgaatg tttacggttt ccc                                    33


<210> 111
<211> 32
<212> ADN
<213> Séquence artificielle

<220>
<223> Description de la séquence artificielle: amorce de
      PCR

<400> 111
cgggatccat tttttagacg tatttttagt cg                                     32


<210> 112
<211> 34
<212> ADN
<213> Séquence artificielle

<220>
<223> Description de la séquence artificielle: amorce de
      PCR

<400> 112
gctctagacc accatgatga gtcaacactc tgcc                                   34


<210> 113
<211> 29
<212> ADN
<213> Séquence artificielle

<220>
<223> Description de la séquence artificielle: amorce de
      PCR

<400> 113
cgggatccat ccagtttttg ctcgaaggc                                         29


<210> 114
<211> 34
<212> ADN
<213> Séquence artificielle

<220>
<223> Description de la séquence artificielle: amorce de
      PCR

<400> 114
gctctagacc accatgcctt cgagcaaaaa ctgg                                   34

<210> 115
<211> 32
<212> ADN
<213> Séquence artificielle

<220>
<223> Description de la séquence artificielle: amorce de
      PCR

<400> 115
cgggatccat cgttcttcaa tctccacaaa cg                                32


<210> 116
<211> 31
<212> ADN
<213> Séquence artificielle

<220>
<223> Description de la séquence artificielle: amorce de
      PCR

<400> 116
gctctagacc accatgcacc tatgtggaaa g                                 31


<210> 117
<211> 29
<212> ADN
<213> Séquence artificielle

<220>
<223> Description de la séquence artificielle: amorce de
      PCR

<400> 117
cgggatccat tcaattcgct tcaacaatg                                    29


<210> 118
<211> 36
<212> ADN
<213> Séquence artificielle

<220>
<223> Description de la séquence artificielle: amorce de
      PCR

<400> 118
ggactagtcc accatggctg ccaaccaacg ttaccg                            36


<210> 119
<211> 33
<212> ADN
<213> Séquence artificielle

<220>
<223> Description de la séquence artificielle: amorce de
      PCR

<400> 119
gaagatctaa gccgcgttcc cttccaaaaa atc                                33

<210> 120
<211> 34
<212> ADN
<213> Séquence artificielle

<220>
<223> Description de la séquence artificielle: amorce de
      PCR

<400> 120
gctctagacc accatgccgc aaattaaaat tccc                               34

<210> 121
<211> 29
<212> ADN
<213> Séquence artificielle

<220>
<223> Description de la séquence artificielle: amorce de
      PCR

<400> 121
cgggatccaa aaacaatctt ccggcaccc                                     29

<210> 122
<211> 33
<212> ADN
<213> Séquence artificielle

<220>
<223> Description de la séquence artificielle: amorce de
      PCR

<400> 122
gctctagacc accatgcgca cgccgttttg ttg                                33

<210> 123
<211> 29
<212> ADN
<213> Séquence artificielle

<220>
<223> Description de la séquence artificielle: amorce de
      PCR

<400> 123
cgggatccat tgggcaacga cgaaggcac                                     29

<210> 124
<211> 33
<212> ADN

<213> Séquence artificielle

<220>
<223> Description de la séquence artificielle: amorce de
      PCR

<400> 124
gctctagacc accatgagaa tagagatcac acc                                33


<210> 125
<211> 27
<212> ADN
<213> Séquence artificielle

<220>
<223> Description de la séquence artificielle: amorce de
      PCR

<400> 125
cgggatccat ggctcaatcc tttctgc                                       27


<210> 126
<211> 34
<212> ADN
<213> Séquence artificielle

<220>
<223> Description de la séquence artificielle: amorce de
      PCR

<400> 126
gctctagacc accatgattc acgtttcggc agtg                               34


<210> 127
<211> 29
<212> ADN
<213> Séquence artificielle

<220>
<223> Description de la séquence artificielle: amorce de
      PCR

<400> 127
cgggatccaa cctgcttcat gggtgattc                                     29


<210> 128
<211> 36
<212> ADN
<213> Séquence artificielle

<220>
<223> Description de la séquence artificielle: amorce de
      PCR

<400> 128
gctctagacc accatgaatt cgaccgcaag taaaac                             36

<210> 129
<211> 28
<212> ADN
<213> Séquence artificielle

<220>
<223> Description de la séquence artificielle: amorce de
      PCR

<400> 129
cgggatccaa atccctctgc cgtatttg                                    28

## Revendications

1. Acide nucléique sous forme isolée codant pour un polypeptide spécifique des souches pathogènes du genre *Neisseria,* ou fragment antigénique de celui-ci à l'exclusion des séquences SEQ ID n°70A, 73A, 74A, 77A, 80A, 81A, 87A, 88A, 89A, 94A, 95A et 98A, la séquence d'acides aminés dudit polypeptide spécifique étant identique ou homologue d'une séquence choisie parmi les séquences du groupe II, le groupe II étant constitué par les séquences SEQ ID NO :24, SEQ ID NO :50, SEQ ID NO :4, SEQ ID NO :6, SEQ ID NO :10 à SEQ ID NO :22 (numéros pairs), SEQ ID NO :26 à SEQ ID n°48 (numéros pairs), SEQ ID NO :52 et la séquence SEQ ID n°53.

2. Acide nucléique selon la revendication 1 dont la séquence nucléotidique est identique ou homologue d'une séquence choisie parmi les séquences du groupe I, le groupe I étant constitué par les séquences SEQ ID NO :23, SEQ ID NO :49, SEQ ID NO :3, SEQ ID NO :5 et SEQ ID NO :9 à SEQ ID NO :21 (numéros impairs), SEQ ID NO :25 à SEQ ID n°47 (numéros impairs), et SEQ ID NO :51.

3. Acide nucléique selon la revendication 1 codant pour un polypeptide spécifique des souches pathogènes du genre *Neisseria,* ou fragment antigénique de celui-ci, la séquence d'acides aminés dudit polypeptide spécifique étant choisie parmi les séquences SEQ ID n°57 à 77 (numéros impairs).

4. Acide nucléique selon la revendication 3, ayant une séquence nucléotidique choisie parmi les séquences SEQ ID n° 56 à 76 (numéros pairs).

5. Polypeptide spécifique des souches pathogènes du genre Neisseria, et fragments antigéniques de celui-ci, la séquence d'acides aminés dudit polypeptide spécifique étant identique ou homologue d'une séquence choisie parmi les séquences du groupe II, constitué par les séquences SEQ ID NO :24, SEQ ID NO :50, SEQ ID NO :4, SEQ ID NO :6, SEQ ID NO :10 à SEQ ID NO :22 (numéros pairs), SEQ ID NO :26 à SEQ ID n°48 (numéros pairs), SEQ ID NO :52 et la séquence SEQ ID n°53.

6. Polypeptide selon la revendication 5 spécifique des souches pathogènes du genre *Neisseria,* et fragments antigéniques de celui-ci, la séquence d'acides aminés dudit polypeptide spécifique étant choisie parmi les séquences SEQ ID n° 57 à 77 (numéros pairs).

7. Vecteur d'expression comprenant une cassette d'expression dans laquelle une séquence nucléotidique telle que définie dans l'une des revendications 1 à 4 est placée dans des conditions permettant son expression dans une cellule hôte.

8. Cellule hôte transformée par le vecteur d'expression selon la revendication 7.

9. Composition pharmaceutique comprenant :
a) un acide nucléique selon l'une des revendications 1 à 4, sous forme nue ou en association avec au moins un agent facilitant la transfection ;
b) ou un vecteur vaccinal comprenant une séquence nucléotidique telle que définie dans l'une des revendications 1 à 4, tel que notamment un virus ou une bactérie ;
c) ou un polypeptide selon l'une des revendications 5 ou 6 ;
éventuellement en association avec un véhicule pharmaceutiquement acceptable.

10. Anticorps monospécifique dirigé contre un polypeptide selon l'une des revendications 5 ou 6.

11. Utilisation d'un acide nucléique selon l'une des revendications 1 à 4 ou d'un polypeptide spécifique des souches pathogènes de *Neisseria* ou de fragments antigéniques de celui-ci selon l'une des revendications 5 ou 6 pour la fabrication d'une composition pharmaceutique destinée à la vaccination contre *Neisseria.*
